(19) Europäisches Patentamt
European Patent Office
Office européen des brevets

(11) Publication number: **0 191 989 B1**

(12) # EUROPEAN PATENT SPECIFICATION

(45) Date of publication of patent specification: **02.09.92**

(51) Int. Cl.[5]: **C07D 413/04**, C07D 413/14, C07D 417/14, A61K 31/42

(21) Application number: **85309197.3**

(22) Date of filing: **17.12.85**

The file contains technical information submitted after the application was filed and not included in this specification

(54) **Antibiotic isoxazole derivatives, their production and use.**

(30) Priority: **18.12.84 PCT/JP84/00602**
**25.06.85 PCT/JP85/00358**
**04.09.85 JP 195075/85**
**11.11.85 JP 253188/85**

(43) Date of publication of application:
**27.08.86 Bulletin 86/35**

(45) Publication of the grant of the patent:
**02.09.92 Bulletin 92/36**

(84) Designated Contracting States:
**AT BE CH DE FR GB IT LI LU NL SE**

(56) References cited:

**CHEMICAL ABSTRACTS, vol. 87, no. 19, November 7, 1977, Columbus, Ohio, USA VOITENKO, A.D. "Some N-acyl derivatives of 3-isoxazolidinone" page 581, column 2, abstract-no. 152 060d & Tezisy Dokl.-Konf. Molodykh Uch. Sint. Issled. Biol. Akt. Soedin. 1974, 27**

**CHEMICAL ABSTRACTS, vol. 86, no. 15, April 11, 1977, Columbus, Ohio, USA VOITENKO, A.D.; KASTRONS, J.; LIEPINS, E.; GERMANE, S. "Acyl derivatives of 3-isoxazolidinone and their properties" page 498, column 1, abstract-no. 106 447s & Khim.-Farm.Zh. 1976, 10(9), 27-31**

(73) Proprietor: **TAKEDA CHEMICAL INDUSTRIES, LTD.**
**1-1, Doshomachi 4-chome**
**Chuo-ku, OSAKA(JP)**

(72) Inventor: **Yoshioka, Koichi**
**5-7 Oharano-kamisatotorimicho**
**Nishikyo-ku Kyoto 610-11(JP)**
Inventor: **Harada, Setsuo**
**3-31 Seiwadainishi 2-chome**
**Kawanishi Hyogo 666-01(JP)**
Inventor: **Ochiai, Michihiko**
**23-13 Senriyamahigashi, 1-chome**
**Suita Osaka 565(JP)**

(74) Representative: **Laredo, Jack Joseph et al**
**Elkington and Fife Prospect House 8 Pembroke Road**
**Sevenoaks, Kent TN13 1XR(GB)**

EP 0 191 989 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel 2-(4-substituted-amino-3-oxo-2-isoxazolidinyl)-5-oxo-2-tetrahydrofurancarboxylic acid derivatives exhibiting excellent antimicrobial and β-lactamase inhibitory activities, to processes for production thereof and to uses thereof.

Recently, a novel antibiotic TAN-588 (hereinafter referred to in some instances briefly as "TAN-588") exhibiting antimicrobial activity against gram-positive and gram-negative bacteria has been harvested from new species of microorganisms belonging to the genera Empedobacter and Lysobacter as isolated from soil. The present inventors conducted research to elucidate the chemical structure of the said antibiotic TAN-588, and as a result, it has been found that the antibiotic TAN-588 has a peculiar skeleton consisting of a 3-oxoisoxazolidine ring having 5-oxo-2-tetrahydrofurancarboxylic acid bonded at its nitrogen atom [cf. Nature Vol. 325 No. 7000, pp. 179-180 (8 January, 1987)].

Heretofore, there has been reported a synthesis of a compound consisting of a 3-oxoisoxazolidine ring having a 1-methylacetic acid group introduced at its nitrogen atom [Tsuji and Yamana; Heterocycles, 8, 153 (1977)1. However, it has been reported that the compound having the said 1-methylacetic acid group was not observed to exhibit antimicrobial activity.

The present inventors synthesized derivatives of the antibiotic TAN-588 exhibiting antimicrobial activity, and as a result, it was found that the said derivatives possess excellent antimicrobial activity. The finding was followed by further research leading to this invention.

The invention is concerned with:

(a) a 2-((4S)-amino-3-oxo-2-isoxazolidinyl)-5-oxo-2-tetrahydrofurancarboxylic acid derivative of the formula (I') or a pharmaceutically acceptable salt thereof, selected from:

(1) sodium 2-{(4S)-4-[2-(2-amino-4-thiazolyl)-(Z)-2-(methoxyimino)acetamido]-3-oxo-2-isoxasolidinyl}-5-oxo-2-tetrahydrofurancarboxylate;

(2) sodium 2-{(4S)-4-[D-2-(4-ethyl-2,3-dioxo-1-piperazinocarboxamido)-2-phenylacetamido]-3-oxo-2-isoxazolidinyl}-5-oxo-2-tetrahydrofurancarboxylate;

(3) sodium 2-{(4S)-4-[D-2-(3-methylsulfonyl-2-oxoimidazolidine-1-carboxamido)-2-phenylacetamido]-3-oxo-2-isoxazolidinyl}-5-oxo-2-tetrahydrofurancarboxylate;

(4) sodium 2-{(4S)-4-[D-2-(3-furfurylidene-amino-2-oxoimidazolidine-1-carboxamido-2-phenylacetamido]-3-oxo-2-isoxazolidinyl}-5-oxo-2-tetrahydrofurancarboxylate;

(5) sodium 2-[(4S)-4-(2-thienylacetamido)-3-oxo-2-isoxazolidinyl]-5-oxo-2-tetrahydrofurancarboxylate;

(6) sodium 2-[(4S)-4-(2-cyanoacetamido)-3-oxo-2-isoxazolidinyl]-5-oxo-2-tetrahydrofurancarboxylate;

(7) sodium 2-{(4S)-4-[(1H)-tetrazolylacetamido]-3-oxo-2-isoxazolidinyl}-5-oxo-2-tetrahydrofurancarboxylate;

(8) sodium 2-[(4S)-4-(2-6-dimethoxybenzamido)-3-oxo-2-isoxazolidinyl]-5-oxo-2-tetrahydrofurancarboxylate;

(9) sodium 2-[(4S)-4-(5-methyl-3-phenyl-2-isoxazoline -4-carboxamido)-3-oxo-2-isoxazolidinyl]-5-oxo-2-tetrahydrofurancarboxylate;

(10) sodium 2-[(4S)-4-(2-ethoxynaphthalenyl-1-carboxamido)-3-oxo-2-isoxazolidinyl]-5-oxo-2-tetrahydrofurancarboxylate;

(11) sodium 2-[(4S)-4-(2-cyanomethylthioacetamido)-3-oxo-2-isoxazolidinyl]-5-oxo-2-tetrahydrofurancarboxylate;

(12) sodium 2-[(4S)-4-(2-difluoromethylthioacetamido)-3-oxo-2-isoxazolidinyl]-5-oxo-2-tetrahydrofurancarboxylate;

(13) sodium 2-{(4S)-4-[(R)-2-(4-cyclohexyl-2,3-dioxo-1-piperazinocarboxamido-2-phenylacetamido]-3-oxo-2-isoxazolidinyl}-5-oxo-2-tetrahydrofurancarboxylate;

(14) sodium 2-((4S)-4-chloroacetamido-3-oxo-2-isoxazolidinyl)-5-oxo-2-tetrahydrofurancarboxylate;

(15) sodium 2-{(4S)-4-[2-(4-pyridylthio)acetamido]-3-oxo-2-isoxazolidinyl}-5-oxo-2-tetrahydrofurancarboxylate;

(16) methyl 2-[(4S)-4-acetamido-3-oxo-2-isoxazolidinyl]-5-oxo-2-tetrahydrofurancarboxylate;

(17) pivaloyloxymethyl 2-[(4S)-4-acetamido-3-oxo-2-isoxazolidinyl]-5-oxo-2-tetrahydrofurancarboxylate;

(18) 3,5-dinitrobenzyl 2-[(4S)-4-acetamido-3-oxo-2-isoxazolidinyl]-5-oxo-2-tetrahydrofurancarboxylate;

(19) p-bromophenacyl 2-[(4S)-4-acetamido-3-oxo-2-isoxazolidinyl]-5-oxo-2-tetrahydrofurancarboxylate;

(20) p-bromobenzyl 2-[(4S)-4-acetamido-3-oxo-2-isoxazolidinyl]-5-oxo-2-tetrahydrofurancarboxylate;

(21) sodium 2-[(4S)-4-formylamino-3-oxo-2-isoxazolidinyl]-5-oxo-2-tetrahydrofurancarboxylate;

(22) sodium 2-[(4S)-4-n-butyrylamino-3-oxo-2-isoxazolidinyl]-5-oxo-2-tetrahydrofurancarboxylate;

(23) sodium 2- [(4S)-4-methoxycarbonylamino-3-oxo-2-isoxazolidinyl]-5-oxo-2-tetrahydrofurancarboxylate;

(24) sodium 2-[(4S)-4-(N-benzyloxycarbonyl)-glycylamino-3-oxo-2-isoxazolidinyl]-5-oxo-2-tetrahydrofurancarboxylate;
(25) sodium 2-[(4S)-4-benzoylamino-3-oxo-2-isoxazolidinyl]-5-oxo-2-tetrahydrofurancarboxylate;
(26) sodium 2-[(4S)-4-phenylacetylamino-3-oxo-2-isoxazolidinyl]-5-oxo-2-tetrahydrofurancarboxylate;
(27) sodium 2-[(4S)-4-nicotinylamino-3-oxo-2-isoxazolidinyl]-5-oxo-2-tetrahydrofurancarboxylate;
(28) pivaloyloxymethyl 2-[(4S)-4-phenoxyacetylamino-3-oxo-2-isoxazolidinyl]-5-oxo-2-tetrahydrofurancarboxylate;
(29) sodium 2-[(4S)-4-(p-toluenesulfonyl)amino-3-oxo-2-isoxazolidinyl]-5-oxo-2-tetrahydrofurancarboxylate;
(30) diphenylmethyl 2-[(4S)-4-(p-bromobenzoyl)-amino-3-oxo-2-isoxazolidinyl]-5-oxo-2-tetrahydrofurancarboxylate;
(31) diphenylmethyl 2-[(4S)-4(N-benzyloxy-carbonyl-D-alanyl)amino-3-oxo-2-isoxazolidinyl]-5-oxo-2-tetrahydrofurancarboxylate;
(32) 2-[(42)-4-dimethylamino-3-oxo-2-isoxazolidinyl]-5-oxo-2-tetrahydrofurancarboxylic acid;
(33) sodium 2-{(4S)-4-[2-(2-amino-4-thiazolyl)-acetamido]-3-oxo-2-isoxazolidinyl}-5-oxo-2-tetrahydrofurancarboxylate;
(34) sodium 2-[4-methoxy-4-(2-thienylacetamido)-3-oxo-2-isoxazolidinyl]-5-oxo-2-tetrahydrofurancarboxylate;
(35) disodium 2-[(4S)-4-[2-(2-amino-4-thiazolyl)-(Z)-2-(carboxymethyloxyimino)acetamido]-3-oxo-2-isoxazolidinyl}-5-oxo-2-tetrahydrofurancarboxylate;
(38) 2-{(4S)-4-[2-(2-aminomethyl)phenylacetamido]-3-oxo-2-isoxazolidinyl)-5-oxo-2-tetrahydrofurancarboxylic acid;
(39) sodium 2-[(4S)-4-(4-fluorobenzenesulfonyl-amino-3-oxo-2-isoxazolidinyl]-5-oxo-2-tetrahydrofurancarboxylate;
(40) sodium 2-{(4S)-4-{2-[1-(2-dimethylaminoethyl)-1H-5-tetrazolylthio]acetamido}-3-oxo-2-isoxazolidinyl}-5-oxo-2-tetrahydrofurancarboxylate;
(41) sodium 2-{(4S)-4-[2-(4-methyl-4H-1,2,4-triazolyl-3-ylthio)acetamido]-3-oxo-2-isoxazolidinyl}-5-oxo-2-tetrahydrofurancarboxylate;
(42) sodium 2-{(4S)-4-[(2R,3S)-2-(4-ethyl-2,3-dioxo-1-piperazinecarboxamido)-3-formyloxybutylamido]-3-oxo-2-isoxazolidinyl}-5-oxo-2-tetrahydrofurancarboxylate;
(43) disodium 2-[(4S)-4-(2-phenyl-2-sulfoacetamido)-3-oxo-2-isoxazolidinyl]-5-oxo-2-tetrahydrofurancarboxylate;
(44) sodium 2-{(4S)-4-[2-(2-amino-4-thiazolyl)-(E)-2-methoxyiminoacetamido]-3-oxo-2-isoxazolidinyl}-5-oxo-2-tetrahydrofurancarboxylate;
(45) sodium 2-[(4S)-4-(2-trimethylsilyl)-ethoxycarbonylamino-3-oxo-2-isoxazolidinyl]-5-oxo-2-tetrahydrofurancarboxylate;
(46) sodium 2-[(4S)-4-(1-aminocyclohexylcarbonylamino)-3-oxo-2-isoxazolidinyl]-5-oxo-2-tetrahydrofurancarboxylic acid;
(47) sodium 2-{(4S)-4-[3-(2,6-dichlorophenyl)-5-methyl-4-isoxazolylcarbonylamino]-3-oxo-2-isoxazolidinyl}-5-oxo-2-tetrahydrofurancarboxylate;
(48) sodium 2-{(4S)-4-[2-(2-pyridylthio)acetamido]-3-oxo-2-isoxazolidinyl}-5-oxo-2-tetrahydrofurancarboxylate;
(49) sodium 2-{(4S)-4-[2-(4-pyridylmethylthio)-acetamido]-3-oxo-2-isoxazolidinyl}-5-oxo-2-tetrahydrofurancarboxylate;
(50) sodium 2-{(4S)-4-[2-(2-pyrimidinylthio)-acetamido]-3-oxo-2-isoxazolidinyl}-5-oxo-2-tetrahydrofurancarboxylate;
(51) sodium 2-{(4S)-4-{2-[(E)-2-(acetamidovinyl)thio]acetamido}-3-oxo-2-isoxazolidinyl}-5-oxo-2-tetrahydrofurancarboxylate;
(52) sodium 2-[(4S)-4-cyclopropylcarbonylamino-3-oxo-2-isoxazolidinyl]-5-oxo-2-tetrahydrofurancarboxylate;
(53) sodium 2-[(4S)-4-methoxalylamino-3-oxo-2-isoxazolidinyl]-5-oxo-2-tetrahydrofurancarboxylate;
(54) sodium 2-[(4S)-4-acrylamido-3-oxo-2-isoxazolidinyl]-5-oxo-2-tetrahydrofurancarboxylate;
(55) sodium 2-{(4S)-4-[2-(2-chloro-4-pyridyl-thio)acetamido]-3-oxo-2-isoxazolidinyl}-5-oxo-2-tetrahydrofurancarboxylate;
(56) sodium 2-[(4S)-4-(2-acetamido-5-oxo-2-tetrahydrofurancarbonylamino)-3-oxo-2-isoxasolidinyl]-5-oxo-2-tetrahydrofurancarboxylate;
(57) sodium 2-{(4S)-4-[2-(1-pyrazolyl)acetamido]-3-oxo-2-isoxazolidinyl}-5-oxo-2-tetrahydrofurancarboxylate;

(58) sodium 2-{(4S)-4-[2-(5-amino-1,2,4-thiadiazole-3-yl)-(Z)-2-ethoxyiminoacetamido]-3-oxo-2-isoxazolidinyl}-5-oxo-2-tetrahydrofurancarboxylate;
(59) sodium 2-{(4S)-4-[2-(3-pyridazinylthio)-acetamido]-3-oxo-2-isoxazolidinyl}-5-oxo-2-tetrahydrofurancarboxylate;
(60) sodium 2-[(4S)-4-dichloroacetamido-3-oxo-2-isoxazolidinyl]-5-oxo-2-tetrahydrofurancarboxylate;
(61) sodium 2-{(4S)-4-[(2R,3S)-2-(4-ethyl-2,3-dioxo-1-piperazinecarboxamido)-3-hydroxybutyramido]-3-oxo-2-isoxazolidinyl}-5-oxo-2-tetrahydrofurancarboxylate;
(62) sodium 2-{(4S)-4-[2-(5-trifluoromethyl-1,3,4-thiadiazol-2-ylthio)acetamido]-3-oxo-2-isoxazolidinyl}-5-oxo-2-tetrahydrofurancarboxylate;
(63) sodium 2-{(4S)-4-{2-[1-ethoxy(hydroxy)phosphinylmethyl-1H-5-tetrazolylthio]acetamido}-3-oxo-2-isoxazolidinyl}-5-oxo-2-tetrahydrofurancarboxylate;
(64) sodium 2-{(4S)-4-{2-[5-(2-diethoxyphosphinylethylthio)-1,3,4-thiadiazolylthio]acetamido}-3-oxo-2-isoxazolidinyl}-5-oxo-2-tetrahydrofurancarboxylate;
(65) sodium 2-{(4S)-4-[2-(1-dimethylamino-1H-5-tetrazolylthio)acetamido]-3-oxo-2-isoxazolidinyl}-5-oxo-2-tetrahydrofurancarboxylate;
(66) sodium 2-{(4S)-4-[2-(4,5-dimethyl-2-thiazolylthio)acetamido]-3-oxo-2-isoxazolidinyl}-5-oxo-2-tetrahydrofurancarboxylate;
(67) sodium 2-{(4S)-4-[2-(4,5-dimethyl-2-oxa-zolylthio)acetamido]-3-oxo-2-isoxazolidinyl}-5-oxo-2-tetrahydrofurancarboxylate;
(68) sodium 2-{(4S)-4-[2-(5-methoxymethyl-1,3,4-thiadiazol-2-ylthio)acetamido]-3-oxo-2-isoxazolidinyl}-5-oxo-2-tetrahydrofurancarboxylate;
(69) sodium 2-{(4S)-4-[2-(5-methylsulfonyl-methyl-1,3,4-thiadiazol-2-ylthio)acetamido]-3-oxo-2-isoxazolidinyl)-5-oxo-2-tetrahydrofurancarboxylate;
(70) sodium 2-{(4S)-4-[2-(4-ethyl-4H-1,2,4-triazol-3-ylthio)acetamido]-3-oxo-2-isoxazolidinyl}-5-oxo-2-tetrahydrofurancarboxylate;
(71) disodium 2-{(4S)-4-[2-(5-carboxymethyl-1,3,4-thiadiazol-2-ylthio)acetamido]-3-oxo-2-isoxazolidinyl}-5-oxo-2-tetrahydrofurancarboxylate;
(72) sodium 2-{(4S)-4-(2-[1-(2-hydroxyethyl)-1H-5-tetrazolylthio]acetamido}-3-oxo-2-isoxazolidinyl}-5-oxo-2-tetrahydrofurancarboxylate;
(73) sodium 2-{(4S)-4-{2-[1-(3-dimethylamino-propyl)-1H-5-tetrazolylthio]acetamido}-3-oxo-2-isoxazolidinyl}-5-oxo-2-tetrahydrofurancarboxylate;
(74) 2-[(4S)-4-(2-amino-3-sulfamoylpropionamido)-3-oxo-2-isoxazolidinyl]-5-oxo-2-tetrahydrofurancarboxylic acid;
(75) sodium 2-{(4S)-4-[2-(4-cyano-3-hydroxy-5-isothiazolylthio)acetamido]-3-oxo-2-isoxazolidinyl}-5-oxo-2-tetrahydrofurancarboxylate;
(76) sodium 2-{(4S)-4-[2-(2-amino-4-thiazolyl)-2-oxoacetamido]-3-oxo-2-isoxazolidinyl}-5-oxo-2-tetrahydrofurancarboxylate;
(77) sodium 2-{(4S)-4-[2-(2-amino-4-thiazolyl)-(Z)-2-isopropoxyiminoacetamido]-3-oxo-2-isoxazolidinyl}-5-oxo-2-tetrahydrofurancarboxylate;
(78) sodium 2-[(4S)-4-trifluoroacetamido-3-oxo-2-isoxazolidinyl]-5-oxo-2-tetrahydrofurancarboxylate;
(79) disodium 2-{(42)-4-[2-(2-amino-4-thiazolyl)-(Z)-2-(1-carboxy-1-methylethoxyimino)acetamido]-3-oxo-2-isoxazolidinyl)-5-oxo-2-tetrahydrofurancarboxylate;
(80) sodium 2-{(4S)-4-[2-(2-amino-4-thiazolyl)-(Z)-2-(hydroxyimino)acetamido]-3-oxo-2-isoxazolidinyl}-5-oxo-2-tetrahydrofurancarboxylate;
(81) sodium 2-[(4S)-4-isobutyloxycarbonylamino-3-oxo 2-isoxazolidinyl]-5-oxo-2-tetrahydrofurancarboxylate;
(82) diphenylmethyl 2-{(42)-4-[N-(4-nitrobenzyloxycarbonyl)glycylamino]-3-oxo-2-isoxazolidinyl)-5-oxo-2-tetrahydrofurancarboxylate;
(83) diphenylmethyl 2-{(4S)-4-[N-(2,2,2-trichloroethoxycarbonyl)phenylglycylamino]-3-oxo-2-isoxazolidinyl}-5-oxo-2-tetrahydrofurancarboxylate;
(84) sodium 2-{(4S)-4-[N-(benzyloxycarbonyl)-D-phenylglycylamino]-3-oxo-2-isoxazolidinyl}-5-oxo-2-tetrahydrofurancarboxylate;
(85) 2-[(4S)-4-L-phenylglycylamino-3-oxo-2-isoxazolidinyl]-5-oxo-2-tetrahydrofurancarboxylic acid;
(86) 2-{(4S)-4-D-phenylglycylamino-3-oxo-2-isoxazolidinyl}-5-oxo-2-tetrahydrofurancarboxylic acid;
(87) sodium 2-{(4S)-4-[(2S)-(2-hydroxy-2-phenyl)acetamido]-3-oxo-2-isoxazolidinyl}-5-oxo-2-tetrahydrofurancarboxylate;
(88) sodium 2-{(4S)-4-[(2R)-(2-hydroxy-2-phenyl)acetamido]-3-oxo-2-isoxazolidinyl}-5-oxo-2-tetrahydrofurancarboxylate;

(89) sodium 2-{(4S)-4-[2-(4-chlorophenyl)-2-hydroxyacetamido]-3-oxo-2-isoxazolidinyl}-5-oxo-2-tetrahydrofurancarboxylate;
(90) sodium 2-{(4S)-4-[2-hydroxy-2-(4-hydroxyphenyl)-acetamido]-3-oxo-2-isoxazolidinyl}-5-oxo-2-tetrahydrofurancarboxylate;
(91) sodium 2-[(4S)-4-(2-hydroxypropionamido)-3-oxo-2-isoxazolidinyl]-5-oxo-2-tetrahydrofurancarboxylate;
(92) disodium 2-[(4S)-4-(2-carboxy-2-phenylacetamido)-3-oxo-2-isoxazolidinyl]-5-oxo-2-tetrahydrofurancarboxylate;
(93) sodium 2-[(4S)-4-benzyloxycarbonylamino-3-oxo-2-isoxazolidinyl]-5-oxo-2-tetrahydrofurancarboxylate;
(94) sodium 2-((4S)-4-pyruvamido-3-oxo-2-isoxazolidinyl)-5-oxo-2-tetrahydrofurancarboxylate;
(95) pivaloyloxymethyl 2-{(4S)-4-[(2R)-(2-hydroxy-2-phenyl)acetamido]-3-oxo-2-isoxazolidinyl)-5-oxo-2-tetrahydrofurancarboxylate;
(96) diphenylmethyl 2-[(4S)-4-(4-nitrobenzylidene)amino-3-oxo-2-isoxazolidinyl]-5-oxo-2-tetrahydrofurancarboxylate;
(97) sodium 2-{(4S)-4-[2-(3-chloro-6-pyridazinylthio)acetamido]-3-oxo-2-isoxazolidinyl}-5-oxo-2-tetrahydrofurancarboxylate;
(98) sodium 2-[(4S)-4-(2-phenylthioacetamido)-3-oxo-2-isoxazolidinyl]-5-oxo-2-tetrahydrofurancarboxylate;
(99) sodium 2-[(4S)-4-(2-ethoxydithiocarbonylacetamido)-3-oxo-2-isoxazolidinyl]-5-oxo-2-tetrahydrofurancarboxylate;
(100) sodium 2-{(4S)-4-[2-(2-amino-4-thiazolyl)-(Z)-2-(ethoxyimino)acetamido]-3-oxo-2-isoxazolidinyl}-5-oxo-2-tetrahydrofurancarboxylate;
(101) sodium 2-{(4S)-4-[2-(2-amino-5-chloro-4-thiazolyl)-(Z)-2-(methoxyimino)acetamido]-3-oxo-2-isoxazolidinyl}-5-oxo-2-tetrahydrofurancarboxylate;
(102) sodium 2-{(42)-4-[2-4-ethyl-2,3-dioxo-1-piperazinocarboxamido)-2-thienylacetamido]-3-oxo-2-isoxazolidinyl}-5-oxo-2-tetrahydrofurancarboxylate;
(103) sodium 2-((4S)-4-ureido-3-oxo-2-isoxazolidinyl)-5-oxo-2-tetrahydrofurancarboxylate;
(104) sodium 2-((4S)-4-phenylureylene-3-oxo-2-isoxazolidinyl)-5-oxo-2-tetrahydrofurancarboxylate;
(105) sodium 2-{(4S)-4-[2-(pyridyl)acetamido]-3-oxo-2-isoxazolidinyl}-5-oxo-2-tetrahydrofurancarboxylate;
(106) sodium 2-{(4S)-4-[2-(4-chlorophenyl)acetamido]-3-oxo-2-isoxazolidinyl}-5-oxo-2-tetrahydrofurancarboxylate;
(107) sodium 2-{(4S)-4-[(2-chloro-2-phenyl)acetamido]-3-oxo-2-isoxazolidinyl)-5-oxo-2-tetrahydrofurancarboxylate;
(108) sodium 2-{(4S)-4-[2-(3-pyridylthio)acetamido]-3-oxo-2-isoxazolidinyl)-5-oxo-2-tetrahydrofurancarboxylate;
(109) sodium 2-{(4S)-4-[2-(4-chlorophenylthio)acetamido]-3-oxo-2-isoxazolidinyl}-5-oxo-2-tetrahydrofurancarboxylate;
(110) sodium 2-[(4S)-4-(2-isopropylphenoxy-carbonylamino)-3-oxo-2-isoxazolidinyl]-5-oxo-2-tetrahydrofurancarboxylate;
(111) sodium 2-((4S)-4-difluoroacetamido-3-oxo-2-isoxazolidinyl-5-oxo-2-tetrahydrofurancarboxylate;
(112) sodium 2-[(4S)-4-(3-chloro)propionamido-3-oxo-2-isoxazolidinyl]-5-oxo-2-tetrahydrofurancarboxylate;
(113) pivaloyloxymethyl 2-((4S)-4-chloroacetamido-3-oxo-2-isoxazolidinyl)-5-oxo-2-tetrahydrofurancarboxylate;
(114) pivaloyloxymethyl 2-[(4S)-4-(2-phenylthioacetamido)-3-oxo-2-isoxazolidinyl]-5-oxo-2-tetrahydrofurancarboxylate;
(115) sodium 2-((4S)-4-propionamido-3-oxo-2-isoxazolidinyl)-5-oxo-2-tetrahydrofurancarboxylate;
(116) pivaloyloxymethyl 2-((4S)-4-benzyloxy-carbonylamino-3-oxo-2-isoxazolidinyl)-5-oxo-2-tetrahydrofurancarboxylate;
(117) pivaloyloxymethyl 2-((4S)-4-bromoacetamido-3-oxo-2-isoxazolidinyl)-5-oxo-2-tetrahydrofurancarboxylate;
(118) pivaloyloxymethyl 2-[(4S)-4-(2,4-hexadiene)amido-3-oxo-2-isoxazolidinyl]-5-oxo-2-tetrahydrofurancarboxylate;
(119) pivaloyloxymethyl 2-[(4S)-4-(2-thienyl)acetamido-3-oxo-2-isoxazolidinyl]-5-oxo-2-tetrahydrofurancarboxylate;
(120) pivaloyloxymethyl 2-[(4S)-4-(hexahydro-1H-azepin-1-yl)methyleneamino-3-oxo-2-isoxazolidinyl]-

5-oxo-2-tetrahydrofurancarboxylate;
(121) diphenylmethyl 2-[(4S)-4-(tert-butyl-dimethylsilyl)amino-3-oxo-2-isoxazolidinyl]-5-oxo-2-tetrahydrofurancarboxylate;
(122) diphenylmethyl 2-[(4S)-4-tert-butyl-diphenylsilyl)amino-3-oxo-2-isoxazolidinyl]-5-oxo-2-tetrahydrofurancarboxylate;
(123) sodium 2-[(4S)-3-diphenylphosphorylamino-3-oxo-2-isoxazolidinyl]-5-oxo-2-tetrahydrofurancarboxylate;
(124) diphenylmethyl 2-[(4S)-4-dimethyl-phosphorylamino-3-oxo-2-isoxazolidinyl]-5-oxo-2-tetrahydrofurancarboxylate;
(125) sodium 2-[(4S)-4-ethylsulfonylamino-3-oxo-2-isoxazolidinyl]-5-oxo-2-tetrahydrofurancarboxylate;
(126) sodium 2-[(4S)-4-vinylsulfonylamino-3-oxo-2-isoxazolidinyl]-5-oxo-2-tetrahydrofurancarboxylate;
(127) 2-[(4S)-4-thienylacetamido-3-oxo-2-isoxazolidinyl]-5-oxo-2-tetrahydrofurancarbonylanilide;
(128) 2-[(4S)-4-benzyloxycarbonylamino-3-oxo-2-isoxazolidinyl]-5-oxo-2-tetrahydrofurancarbonylanilide;
(129) 2-[(4S)-4-benzyloxycarbonylamino-3-oxo-2-isoxazolidinyl]-5-oxo-2-tetrahydrofurancarbonylpyrrolidinamide;
(130) 2-[(4S)-4-thienylacetamido-3-oxo-2-isoxazolidinyl]-5-oxo-2-tetrahydrofurancarbonylpyrrolidinamide;
(131) 2-[(4S)-4-thienylacetamido-3-oxo-2-isoxazolidinyl]-5-oxo-2-tetrahydrofurancarbonylpropylamine;
(132) sodium 2-[(4S)-4-γ-D-glutamylamino-3-oxo-2-isoxazolidinyl]-5-oxo-2-tetrahydrofurancarboxylate; and
(133) sodium 2-[(4S)-4-(2-hydroxy)isobutyryl-amino-3-oxo-2-isoxazolidinyl]-5-oxo-2-tetrahydrofurancarboxylate;

(b) A pharmaceutical composition for inhibiting the growth of microorganisms which contains an effective amount of a compound (I'), or a pharmaceutically acceptable salt thereof, as defined at (a) above, together with a pharmaceutically acceptable carrier or diluent therefor.
(c) A compound (I'), or a pharmaceutically acceptable salt thereof, as defined at (a) above, for use in the preparation of a pharmaceutical composition for inhibiting the growth of microorganisms.

In preparing the compounds of the present invention, the antibiotic TAN-588, i.e. 2-[(4S)-4-acetamido-3-oxo-2-isoxazolidinyl]-5-oxo-2-tetrahydrofurancarboxylic acid, its salts, its p-nitrobenzyl ester, its benzhydryl ester, or their N-deacetyl derivative can be employed as starting materials.

The antibiotic TAN-588, which is useful as a starting compound in the processes of this invention, can be produced by cultivating an antibiotic TAN-588 producing microorganism belonging to the genus Empedobacter or Lysobacter in a culture medium to have the antibiotic TAN-588 elaborated and accumulated in the culture broth, followed by harvesting. The said producing microorganism includes, for example, the strains Empedobacter lactamgenus YK-256 and Lysobacter albus sp. nov. YK-422. The said microorganisms have been deposited at the Institution for Fermentation, Osaka (IFO, 17-85, Juso-honmachi 2-chome, Yodogawa-ku, Osaka, Japan) as described below.

| Microorganism | IFO Accession Number Date of Deposition |
|---|---|
| YK-258<br>YK-422 | IFO 14322 20th February, 1984<br>IFO 14384 5th October, 1984 |

Also the said microorganisms have been deposited at the Fermentation Reseach Institute, Agency of Industrial Science and Technology, Ministry of International Trade and Industry, Japan, (FRI, 1-3, Yatabe-cho higashi 1-chome, Tsukuba-gun, Ibaragi Prefecture, Japan) under the accession number of FERM P-number as described below, and the deposits were converted to deposits under the Budapest Treaty and have been stored at FRI under the accession numbers of FERM BP given.

| Microorganism | FRI | |
|---|---|---|
| | Accession Number Date of Deposition | Accession Number under the Budapest Treaty |
| YK-258<br>YK-422 | FERM P-7558 26th March, 1984<br>FERM P-7938 14th November, 1984 | FERM BP-699<br>FERM BP-698 |

As concretly shown in the below mentioned Reference Examples, N-deacetyl derivatives of TAN-588 can be produced by introducing a p-nitrobenzyl or benzhydryl group into TAN-588 or its salt to prepare the

p-nitrobenzyl ester or benzhydryl ester derivative, deacetylating the said ester derivative to prepare the p-nitrobenzyl ester or benzhydryl ester derivative of N-deacetyl TAN-588, and eliminating such ester groups.

The objective compound (I') thus obtained can be isolated and purified by the per se known means, such as concentration, solvent extraction, lyophilization, crystallization, recrystallization, fractional distillation and chromatography.

The presence of two asymmetric carbons in the basic skeleton allows theoretically the objective compound (I') to exist in four kinds of stereoisomers, and their individual stereoisomers and mixtures thereof fall into the scope of this invention. Similarly, the occurence of any asymmetric carbon in the groups represented by $R^1$ and $R^2$ results in existence of stereoisomers, and their individual stereoisomers and mixtures thereof are also included in the scope of this invention. In cases in which the above-described reaction produces these stereoisomers as a mixture, their individual stereoisomers can be isolated by the conventional methods, such as various chromatographic procedures and recrystallization, if necessary.

The compound (I') of this invention can in some instances act on bases to form salts. The said base includes, for example, inorganic bases, such as sodium, potassium, lithium, calcium, magnesium and ammonia, and organic bases, such as pyridine, collidine, triethylamine and triethanolamine.

The compound (I'), when produced in the free form, may beallowed to form salts by use of the conventional means, and the compound (I') obtained in the form of a salt may be converted into the free form by use of the conventional means.

Also, the compound (I') in some instances forms the intramolecular salt and such a salt falls within the scope of this invention, as well.

The stereoisomers of the compound (I'), either alone or as a mixture, can be used as a drug.

The compound (I') thus obtained is useful as a drug, having antimicrobial activity against some species of gram-positive and gram-negative bacteria.

Biological properties of the compound (I') are described in the following. Typical compounds of the compound (I') demonstrate antimicrobial spectra against different microorganisms as shown in Table 1.

Table 1

| Test microorganism | Minimum growth inhibitory concentration (µg/ml) Compound** | | | | | |
|---|---|---|---|---|---|---|
| | 21b | 6b | 1c | 1b | 28c | R-4 |
| Staphylococcus aureus FDA 209P | 6.25 | 1.56 | 12.5 | 12.5 | 3.18 | 100 |
| Escherichia coli NIHJ JC-2 | 25 | 25 | 1.56 | 3.13 | >100 | >100 |
| Klebsiella pneumonae DT | 50 | 50 | 0.78 | 1.56 | 100 | >100 |
| Pseudomonas aeruginosa IFO 3455 | >100 | >100 | 50 | >100 | >100 | >100 |

* : Medium, Trypticase soy agar Amount of a bacterium inoculated, $10^8$ CFU/ml

** : The compound number designates a number as referred to in the examples. The compound (R-4) means the compound as obtained in Reference Example 4.

The compound of the compound (I') of this invention wherein $R^1$ = -NHCOH, $R^2$ = -COONa and X = H, as obtained in Example 21 (b), when administered subcutaneously to mice in the amount of 400 mg/kg, was observed to cause no lethal case. Therefore, the compound (I') or its salts are considered to be of low toxicity.

As described above, the compound (I') of this invention and its salts have antimicrobial activity against some species of gram-positive and gram-negative bacteria, and in addition, are of low toxicity. Consequently, they can be used, as an antimicrobial agent or as a therapeutic agent for bacterial infections, for the treatment of bacterial infections (e.g., respiratory tract infections, urinary tract infections, suppurative diseases, bile duct infections, intratestinal infections, gynecological infections, surgical infections, etc.) in mammals (e.g., mouse, rat, dog, cattle, pig, human) being caused by infections with bacteria.

The daily dosage of the compound (I') or its salt is in an amount of about 2 to 100 mg/kg as the compound (I'), more preferably about 5 to 40 mg/kg.

For administration of the compound (I'), the compound (I') or its pharmacologically acceptable salt can be formulated by the conventional means with a suitable, pharmacologically acceptable carrier, excipient and diluent into such dosage form as a tablet, granule, capsule or elixir to administer orally, and also can be processed into an injectable solution by the conventional means, followed by incorporation into a sterile carrier prepared by the conventional means to administer parenterally.

In producing the above-described oral pharmaceutical preparations, such as tablets, there can suitably

be formulated binding agents (e.g., hydroxylpropylcellulose, hydroxypropylmethyl cellulose, macrogol, etc.), disintegrating agents (e.g., starch, carboxymethylcellulose calcium, etc.), excipients (e.g., lactose, starch, etc.), lubricants (e.g., magnesium stearate, talc, etc.) and the like.

In manufacturing non-oral or parenteral pharmaceutical preparations, such as injectable solutions, there can suitably be formulated isotonizing agents (e.g., glucose, D-sorbitol, D-mannitol, sodium chloride, etc.), preservatives (e.g., benzyl alcohol, chlorobutanol, methyl p-oxybenzoate, propyl p-oxybenzoate, etc.), buffering agents (e.g., phosphate buffers, sodium acetate buffer, etc.) and the like.

The Reference Example and Examples are described in the following to illustrate this invention in more detail,. The term "percent" means "weight/volume %", unless specified otherwise.

In the resins to be used, the ones designated by the abbreviations are defined as follows.

CHP-20: Diaion CHP-20 (produced by Mitsubishi Chemical Industries, Ltd., Japan)
HP-20 : Diaion HP-20 (produced by Mitsubishi Chemical Industries, Ltd., Japan)
LH-20 : Sephadex LH-20 (produced by Pharmacia Co., Sweden)
XAD-2 : Amberlite XAD-2 (produced by Rohm & Haas Co., U.S.A.)
SP-207: Diaion SP-207 (produced by Mitsubishi Chemical Industries, Ltd., Japan)

The abbreviations used in the examples are as defined as follows.

rt : Room temperature
h : Hour
Me : Methyl
Ac : Acetyl
DMA : Dimethylacetamide

Reference Example 1

(1) The strain Empedobacter lactamgenus YK-258 (IFO 14322, FERM BP-699) grown on a slant of nutrient agar was inoculated into a 2-ℓ Sakaguchi flask containing 500 ml of a medium comprising an aqueous solution (pH 7.0) containing 2 % of glucose, 3 % of soluble starch, 1 % of raw soybean flour, 0.5 % of Polypepton (produced by Daigo Nutritive Chemicals, Japan) and 0.3 % of sodium chloride being admixed with 0.5 % precipitating calcium carbonate, and shake culture was carried out on a reciprocating shaker at 24° C for 48 hours. The whole volume of the resulting culture broth was inoculated into a 50-ℓ capacity tank containing 30 ℓ of the medium comprising the above medium admixed with 0.05 % of Actcol (produced by Takeda Chemical Industries, Ltd., Japan), and culture was conducted at 24° C under the conditions of aeration of 50 ℓ/min and agitation of 200 r.p.m. for 48 hours. 6 ℓ of this culture broth was inoculated into a 200-ℓ capacity tank containing 120 ℓ of a medium comprising an aqueous solution (pH 6.5) containing 3 % of dextrin, 1.5 % of raw soybean flour, 1.5 % of corn gluten meal, 0.2 % of Polypepton and 0.1 % of sodium thiosulfate being admixed with 0.05 % of Actcol, and culture was carried out at 17° C under the conditions of aeration of 200 ℓ/min and agitation of 150 r.p.m. for 66 hours.

This cultivation was repeated twice, and the culture broth (230 ℓ) was adjusted to pH 8 and filtered with the use of 9kg of Hyflo Supercel (produced by Johns Manville Co. of U.S.A.). The filtrate (200 ℓ) was adjusted to pH 6 and chromatographed on a column of Amberlite IRA-402 (Cℓ type, 10 ℓ, produced by Rohm & Haas Co. of U.S.A.). The antibiotic was eluted with 2 % aqueous sodium chloride solution, and the eluate (53 ℓ) was adjusted to pH 6 and chromatographed on activated carbon (5 ℓ, produced by Takeda Chemical Industries, Ltd. of Japan). The antibiotic was eluted with 8 % aqueous isobutanol, and the eluate (14 ℓ) was concentrated under reduced pressure to 5 ℓ of the total volume. The concentrate was adjusted to pH 6, followed by extraction with 2 % tri-n-octylammonium chloride/dichloromethane solution (2.5 ℓ x 2). The extract was treated with 1.6 % aqueous sodium iodide solution (2.5 ℓ), and the antibiotic was transferred into the aqueous phase. The aqueous layer was concentrated, and the concentrate was chromatographed on activated carbon (500 ml), followed by elution with 8 % aqueous isobutanol. The eluate was concentrated and lyophilized, to give 1.41 g of a crude powder. The crude powder (1.4 g) was dissolved in water (100 ml), and the solution was chromatographed on a column of 200 ml of QAE-Sephadex A-25 (Cl type, produced by Pharmacia Co. of Sweden), followed by elution and fractionation with 0.03M aqueous sodium chloride solution. The fractions were collected (600 ml), adjusted to pH 5.1 and desalted through chromatography on activated carbon, and the eluate was concentrated and lyophilized to give a powder (384 mg). The powder was dissolved in water, and the solution was subjected to preparative HPLC
using YMC-Pack SH-343 (produced by Yamamura Chemical Laboratory of Japan), followed by elution with 0.01M phosphate buffer. The eluates containing the antibiotic were collected and desalted through

chromatography on activated carbon, and the eluate was concentrated and lyophilized to give a white powder (141 mg) of TAN-588 sodium salt.

The TAN-588 sodium salt (an equilibrium mixture of isomers A and B) as obtained above shows physico-chemical properties as illustrated below.

1) Appearance: White powder

2) Specific rotation: $[\alpha]_D^{23}$ -19.0° ± 10° (c = 0.5, in water)

3) Elemental analysis (%) for the compound consisting of the elements, C, H, N, O and Na: for a sample dried over phosphorus pentoxide at 40°C for 6 hours.

| Found | | Calculated* | |
|---|---|---|---|
| C, | 38.5 ± 2.0 | C, | 39.61 |
| H, | 4.5 ± 1.0 | H, | 3.99 |
| N, | 9.1 ± 1.5 | N, | 9.24 |
| | | O, | 39.58 |
| Na, | 6.9 ± 1.5 | Na, | 7.58 |

(*; calculated assuming that 0.5 mole of adhesive water is contained)

4) Content of adhesive water: 3.0 ± 1.5% (by the thermogravimetric method)

5) Molecular ion peaks obtained by means of the SIMS method: m/z 611 $(2M + Na)^+$, 317 $(M + Na)^+$, 295 $(M + H)^+$

6) Molecular formula:

$C_{10}H_{11}N_2O_7Na$

7) Ultraviolet absorption (UV) spectrum (in water):

$$\lambda_{max}\ 216\ nm\ (E_{1cm}^{1\%} = 130,\ shoulder)$$

8) Infrared absorption (IR) spectrum (by the KBr tablet method): The major absorptions (wave number) as measured in KBr tablets are as follows:

3450, 1780, 1730, 1660, 1550, 1385, 1320, 1290, 1260, 1200, 1120, 1040, 980, 910, 810, 770, 690, 600, 540 $cm^{-1}$

9) $^{13}C$-nuclear magnetic resonance (NMR) spectrum (100 MHz, in heavy water):

The following signals are observed:

182.02(s), 177.30(s), 173.79(s), 173.30(s), 173.25(s), 172.58(s), 96.97(s), 96.92(s), 74.27(t), 72.63(t), 55.57(d) 55.34(d), 31.92(t), 31.08(t),30.98(t), 24.59(q) ppm (where the abbreviations denote the following: s; singlet, d; doublet, t; triplet, q; quartet).

10) Circular dichroism (CD) spectrum (in water): The negative Cotton effect is exhibited at 232 ± 3 nm.

11) Solubility:

Soluble in: water, dimethylsulfoxide

Sparingly soluble in: ethyl acetate, chloroform, diethyl ether.

12) Color reactions:

Positive to: ninhydrin reaction

Negative to: Greig-Leaback reaction, Sakaguchi reaction, Ehrlich reaction, Barton reaction and Dragendorff reaction.

13) Amino acid analysis: Hydrolysis in 6N hydrochloric acid at 105°C for 20 hours produces serine detected as the known amino acid.

14) Stability:

Stable in an aqueous solution at pH 5; slightly stable at pH 3 and pH 7; unstable at pH 9.

15) Thin-layer chromatography (TLC) (cellulose f, produced by Tokyo Kasei Co. of Japan).

| Solvent system | Rf |
|---|---|
| Acetonitrile:water (4:1) | 0.33 |
| Butanol:acetic acid:water (1:1:1) | 0.77 |
| Acetonitrile:3 % ammonium sulfate (4:1) | 0.28 |

16) Distinction between acidity, neutrality and basicity: Neutral substance.

17) High-performance liquid chromatography (HPLC) (carrier: YMC A-312, produced by Yamamura Chemical Laboratories, Japan; Mobile phase: 4 % methanol/0.01M phosphate buffer (pH 6.3), 2 ml/min.):

Rt = 4.3 and 4.8 (min).

(2) Using Lysobacter albus sp. nov. YK-422 (IFO 14383, FERM BP-698), cultivation and purification by a procedure similar to the above mentioned procedure produced Antibiotic TAN-588 sodium salt (620 g) a mixture of two isomers A and B).

In the examples to be described in the following, the TAN-588 sodium salt (a mixture of two isomers A and B) as obtained in the above is in some instances referred to as "Compound (R-1)".

Reference Example 2

In 500 ml of dichloromethane were dissolved 58.8 g of benzophenone hydrazone, 42 ml of 1,1,3,3-tetramethylguanidine and 150 mg of iodine, and after the mixed solution was cooled to 0°C to -5°C, 74 g of m-chloroperbenzoic acid (with a purity of 70 %) was added, followed by stirring at 0°C for 40 minutes. The reaction solution was washed with water and dried over sodium sulfate, and the solvent was distilled off to give diphenyldiazomethane.

31 g of TAN-588 sodium salt (a mixture of two isomers A and B) was suspended in tetrahydrofurane, and the whole amount of diphenyldiazomethane as obtained in the above was dissolved in 150 ml of tetrahydrofurane and added to the suspension. The mixed solution was cooled at 0°C, and 60 ml of 2N HCl was added dropwise, followed by stirring at room temperature for 1 hour. 10 ml of 2N HCl was added, followed by stirring for another 1 hour, and 3 ℓ of dichloromethane was added. The resulting solution was washed with water and concentrated, and ether was added to the residue to give 28 g of a white crystalline powder of TAN-588 benzhydryl ester (a mixture of isomers A and B).

The above mixture (1.8 g) was chromatographed on a column of silica gel (180 ml), and elution was performed with the solvent system of chloroform:methanol (97:3), whereby the compound isomer B eluted firstly and then the compound of isomer A eluted. Each of the fractions was concentrated to give the isomer A (433 mg), isomer B (400 mg) and a mixture of the isomer A and B (476 mg) of TAN-588 benzhydryl ester in the form of colorless crystals.

In the examples to be described in the following, the TAN-588 benzhydryl ester (a mixture of isomers A and B) as obtained in the above is in some instances referred to as "Compound (R-2)".

The TAN-588 benzhydryl ester (a mixture of isomers A and B) as obtained in the above shows physico-chemical properties as illustrated in the following.

1) Appearance:
Colorless crystals

2) Melting point:
153 to 155°C (decomposition)

3) Specific rotation:
$[\alpha]_D^{23}$ + 9.2° ± 5° (c=0.52, in $CHCl_3$)

4) Molecular weight:
m/z, 438 ($M^+$) (EI-MS method)

5)

| Elemental analysis: | | | | |
|---|---|---|---|---|
| Calcd:<br>Found: | C, 63.01;<br>C, 62.83; | H, 5.06;<br>H, 5.32; | N, 6.39;<br>N, 6.28 | O, 25.54 |

6) Molecular formula:

$C_{23}H_{22}N_2O_7$

7) UV spectrum: in methanol

$$\lambda_{max}\ 220\pm 2\ nm\ (E^{1\%}_{1cm} = 285\pm 50,\ shoulder)\ and\ 250\text{-}260\ nm\ (E^{1\%}_{1cm} = 28 \pm 10,\ shoulder)$$

8) IR spectrum: KBr method
3380, 3080, 3050, 2960, 1800, 1780, 1750, 1705, 1690, 1600, 1590, 1540, 1500, 1460, 1380, 1310, 1280, 1190, 1110, 1060, 980, 920, 880, 750, 710, 700, 650, 630, 610, 570, 550, 470 $cm^{-1}$.
9) $^1$H-NMR spectrum: 90 MHz, in $CDCl_3$. δ ppm J (Hz)
1.97(3H,s), 2.1-3.5(4H,m), 3.8-4.2(1H,m), 4.5-5.1(2H,m), 6.1-6.4(1H,b), 6.97(1H,s), 7.3-7.4(10H,m).
(where the abbreviations denote the following: m; multiplet, b; broad, H; proton)
10) TLC: the same conditions as those to be described below, Rf value, 0.58 and 0.65
11) Distinction between acidity, neutrality and basicity: Neutral substance.

The TAN-588 benzhydryl ester (isomer A) and TAN-588 benzhydryl ester (isomer B) as obtained in the above show the properties as illustrated in the following.

Isomer A

1) Appearance:
Colorless crystals
2) Melting point:
97-135°C (the compound undergoes gradual bubbling or foaming and decomposition).
3) Specific rotation:
$[\alpha]_D^{21}$ +44.2° ± 10° (c = 0.505, in $CHCl_3$)
4) Molecular weight: Molecular ion peak by means of the EI-MS method.
m/z 438($M^+$)
5)

| Elemental analysis: | | | | |
|---|---|---|---|---|
| Calcd.<br>Found | C, 63.01;<br>C, 62.62; | H, 5.06;<br>H, 5.06; | N, 6.39;<br>N, 6.32 | O, 25.54 |

6) Molecular formula:

$C_{23}H_{22}N_2O_7$

7) UV spectrum (in methanol):

$$\lambda_{max}\ 220\pm 2\ nm\ (E^{1\%}_{1cm} = 290\pm 50,\ shoulder)\ and\ 250\text{-}260\ nm\ (E^{1\%}_{1cm} = 30\pm 10,\ shoulder).$$

8) IR spectrum : KBr method
3380, 3080, 3050, 1800, 1780, 1760, 1685, 1540, 1500, 1450, 1380, 1310, 1280, 1190, 1110, 1050, 980, 920, 880, 750, 710, 650, 610, 550 $cm^{-1}$.
9) Nuclear magnetic resonance ($^1$H-NMR) spectrum: 100MHz, in $CDCl_3$-$d_6$-DMSO mixture, δ ppm J(Hz).
1.98(3H,s), 2.2-3.4(4H,m), 4.10(1H,dd,J = 8.10), 4.4-5.0(2H,m), 6.93(1H,s), 7.3-7.5(10H,m), 8.27-(1H,d,J = 7)
10) Thin-layer chromatography (TLC):
Carrier, silica gel (produced by Merck & Co. of West Germany). Developping solvent, ethyl acetate

Rf value, 0.58
11) Distinction between acidity, neutrality and basicity: Neutral substance.

Isomer B

1) Appearance:
Colorless crystals
2) Melting point:
157-160 ° C (decomposition)
3) Specific rotation:
$[\alpha]_D^{21}$ -28.8 ° ± 10 ° (c = 0.5, in $CHCl_3$)
4) Molecular weight: m/z 438($M^+$) (EI-MS method)
5)

| Elemental analysis: | | | | |
|---|---|---|---|---|
| Calcd.<br>Found | C, 63.01;<br>C, 63.11; | H, 5.06;<br>H, 5.13; | N, 6.39;<br>N, 6.30 | O, 25.54 |

6) Molecular weight: $C_{23}H_{22}N_2O_7$
7) UV spectrum: in methanol

$\lambda_{max}$ 220$\pm$2 nm ($E^{1\%}_{1cm}$ = 300$\pm$50, shoulder) and 250-260 nm ($E^{1\%}_{1cm}$ = 26$\pm$10, shoulder).

8) IR spectrum: KBr method
3400, 3080, 3050, 1815, 1780, 1735, 1705, 1540, 1460, 1380, 1290, 1265, 1190, 1060, 980, 920, 880, 760, 715, 610, 550 $cm^{-1}$
9) $^1H$-NMR spectrum: 100 MHz, in $CDCl_3$, δppm J(Hz)
1.98(3H,s), 2.2-2.3(4H,m), 4.03(1H,dd,J = 8,10), 4.6-5,2(2H,m), 6.32(1H,d,J = 5), 6.96(1H,s), 7.2-7.5-(10H,m)
10) TLC: (the same conditions as described for isomer A):
Rf value, 0.65
11) Distinction between acidity, neutrality and basicity:
Neutral substance.

Reference Example 3

In 1.2 ℓ of dichloromethane was suspended 26 g (59 mmole) of TAN-588 benzhydryl ester (a mixture of isomers A and B), and the suspension was cooled to -20 ° C. 49 ml of pyridine and 37.6 g of phosphorus chloride were added to it, followed by stirring at -10 ° to -15 ° C for 50 minutes. The mixed solution was cooled to -30 ° C, and 180 ml of methanol was added, followed by stirring at -5 ° to -15 ° C for 30 minutes and then at room temperature for 1 hour. 100 ml of 1N HCl was added to the mixture, followed by stirring at room temperature for 45 minutes, and 100 ml of 50 % aqueous sodium phosphate and 2N sodium hydroxide (ca. 500 ml) were added to the reaction solution to adjust the pH of the aqueous layer to 8.0. The aqueous layer was separated from the dichloromethane layer and extracted with dichloroemthane (600 ml), and the organic layers were combined and concentrated, followed by addition of ether to the residue to give 17.9 g of a white powder of a benzhydryl ester of the N-deacetyl TAN-588 (a mixture of isomers A and B).

In the examples to be described below, the benzhydryl ester of the N-deacetyl TAN-588 (a mixture of isomers A and B) as obtained in the above is in some instances referred to as "Compound (R-3)".

The benzhydryl ester (a mixture of isomers A and B) of the N-deacetyl TAN-588 as obtained in the above shows physico-chemical properties as illustrated below.
1) Appearance:
White powder
2) Specific rotation:
$[\alpha]_D^{25}$ -15.2±5 ° (c = 0.5, in $CHCl_3$)

3) Molecular weight:
m/z, 396($M^+$)(EI-MS method)
4)

| Elemental analysis: | | | | |
|---|---|---|---|---|
| Calcd.<br>Found | C, 63.63;<br>C, 63.63; | H, 5.09;<br>H, 5.05; | N, 7.07;<br>N, 7.02 | O, 24.22 |

5) Molecular weight:

$C_{21}H_{20}N_2O_6$

6) UV spectrum: in methanol

$\lambda_{max}$ 220$\pm$2 nm ($E^{1\%}_{1cm}$ = 336$\pm$50, in shoulder) and 250-260 nm ($E^{1\%}_{1cm}$ = 32$\pm$10, shoulder)

7) IR spectrum: KBr method
3400, 3050, 2970, 1800, 1780, 1740, 1600, 1500, 1460, 1305, 1270, 1190, 1110, 1060, 980, 920, 880, 850, 750, 710, 650, 620, 605 $cm^{-1}$.
8) $^1$H-NMR spectrum: 90 MHz, in $CDCl_3$ $\delta$ ppm J(Hz)
2.2-3.5(4H,m), 3.7-4.0(2H,m), 4.4-4.6(1H,m), 6.97(1H,s), 7.2-7.4(10H,m).
9) HPLC:
Apparatus, Model 6000A/660/440 (Waters Assoc. of U.S.A.), Carrier, YMC-Pack A-312 (Produced by Yamamura Chemical Laboratories of Japan).
Mobile phase, 65 % methanol/0.01M phosphate buffer (pH 6.3).
Rate of flow, 2 ml/min, Rt, 5.3 and 5.6 min.
10) Color reactions:
Positive to: Nynhydrin reaction.
Negative to: Ferric chloride.
11) Distinction between acidity, neutrality and basicity: Basic substance.

Reference Example 4

In 10 ml of dichloromethane was suspended 396 mg of benzhydryl ester (a mixture of isomers A and B) of the N-deacetyl TAN-588, and the suspension was cooled to -20 °C. 434 µl of anisole and 924 µl of trifluoroacetic acid were added to the suspension , followed by stirring at -20 ° to -10 °C for 40 minutes. 280 ml of dichloromethane was added to the reaction solution, followed by extraction with 0.1M $H_3PO_4$-$Na_2HPO_4$ solution (pH 7.3) (420 ml). The extract was adjusted to pH 5.5 and concentrated, and the concentrate was passed through a column packed with Diaion HP-20 (50 to 100 mesh, 100 ml). The column was washed with water, and elution was effected with 40 % aqueous methanol. The fractions exhibiting antimicrobial activity were collected, concentrated and lyophilized to give 143 mg of a white powder of the N-deacetyl TAN-588 (a mixture) of isomers A and B).

The N-deacetyl TAN-588 (a mixture of isomers A and B) as obtained in the above shows physico-chemical properties as illustrated in the following.
1) Appearance:
White powder.
2) Specific rotation: $[a]_D^{25}$ -11 ° ±5 ° (c = 0.1, in water)
3) Molecular weight:
m/z, 231$(M+H)^+$ (FD-MS method)
4)

| Elemental analysis: | | | |
|---|---|---|---|
| Found | | Calcd.* | |
| C,<br>H,<br>N, | 40.42<br>4.36<br>11.65 | C,<br>H,<br>N,<br>O, | 40.17<br>4.64<br>11.71<br>43.48 |

(*; calculated assuming that 0.5 mole of adhesive water is contained).

5) Molecular formula:

$C_8H_{10}N_2O_6$ $(0.5H_2O)$

6) UV spectrum: in water

$$\lambda_{max}\ 221\pm2\ nm\ (E^{1\%}_{1cm} = 154\pm20)$$

7) IR spectrum: KBr method
Major peaks are as follows:
3450, 3220, 2960, 2900, 1800, 1760, 1740, 1670, 1580, 1420, 1390, 1370, 1310, 1250, 1200, 1120, 1050, 1030, 980, 950, 920, 810, 770, 720, 690, 610, 540 $cm^{-1}$.
8) $^1H$-NMR spectrum: 400 MHz, in $D_2O$. The following signals are observed. δ ppm J (Hz)
2.52(1H,m), 2.27(1H,m), 2.91(1H,m), 3.08(1H,m), 4.35(1H,m), 4.56(1H,m), 4.80(1H,m).
9) Circular dichroism (CD) spectrum: in water
The negative Cotton effect is exhibited at 233±3 nm.
10) Solubility:
Soluble in: water
Sparingly soluble in: dimethylsulfoxide, ethyl acetate, diethyl ether.
11) HPLC: The apparatus, carrier and rate of flow are as described above for the benzhydryl ester (a mixture of isomers A and B) of the N-deacetyl TAN-588.
Mobile phase, 0.01M phosphate buffer (pH 6.3)
Rt: 3.1 and 3.3 min.
12) Color reactions:
Positive to: Nynhydrin, Iodine
Negative to: Ferric chloride
13) Distinction between acidity, neutrality and basicity: Amphoteric substance.

In the examples to be described below, the N-deacetyl TAN-588 (a mixture of isomers A and B) is in some instances referred to as "Compound (R-4)".

Reference Example 5

In water was dissolved 100 mg of a white powder of the N-deacetyl TAN-588 (a 1:1 mixture of isomers A and B), and the solution was allowed to stand overnight at 7° C, whereby colorless crystals separated out. The crystals which separated out were recovered by filtration to give 40 mg of the N-deacetyl TAN-588 (isomer A) in the form of crystals.

The crystals of the N-deacetyl TAN-588 as obtained in the above show physico-chemical properties as illustrated in the following.

1) Appearance:
Colorless crystals.
2)Melting point:
177-181° C (decomposition)
3) Specific rotation:
$[\alpha]^{25}_{D}$ + 124° ± 20° )c=0.1, in water)
4) Molecular weight: m/z 231$(M+H)^+$ (FD-MS method)

5)

| Elemental analysis: | | | |
|---|---|---|---|
| Found | | Calcd. | |
| C, | 41.57 | C, | 41.75 |
| H, | 4.39 | H, | 4.38 |
| N, | 12.11 | N, | 12.17 |
| | | O, | 41.71 |

6) Molecualr formula:

$C_8H_{10}N_2O_6$

7) UV spectrum: in water

$$\lambda_{max}\ 221 \pm 2\ nm\ (E^{1\%}_{1cm} = 151 \pm 20)$$

8) IR spectrum: KBr method
Major absorption peaks are as follows. 3450, 3220, 2950, 2900, 1800, 1735, 1660, 1580, 1440, 1420, 1400, 1360, 1340, 1310, 1280, 1200, 1160, 1110, 1050, 1025, 980, 940, 920, 810, 770, 710, 690, 600, 540 $cm^{-1}$
9) $^1$H-NMR spectrum: 400 MHz, in $D_2O$, the following signals are observed. δppm J(Hz)
2.52(1H,m), 2.72(1H,m), 2.91(1H,m), 3.08(1H,m), 4.34(1H,m), 4.55(1H,m), 4.78(1H,m).
10) CD spectrum: in water
The negative Cotton effect is exhibited at 238±3 nm.
11) Solubility:
Soluble in: water
Sparingly soluble in: dimethylsulfoxide, ethyl acetate, chloroform, diethyl ether.
12) HPLC: The conditions are as described above for the mixture of isomers A and B
Rt, 3.3 min.
13) Distinction between acidity, neutrality and basicity: Amphoteric substance.

Reference Example 6

By following the procedure of Reference Example 3, 657 mg of TAN-588 benzhydryl ester (isomer B) was reacted and treated to give 200 mg of a benzhydryl ester (isomer B) of the N-deacetyl TAN-588. 180 mg of the said compound was dissolved in 18 ml of aqueous tetrahydrofurane (1:1), and 90 mg of 10 % palladium-carbon was added to the solution, followed by stirring under a stream of hydrogen. After the catalyst was filtered out, the filtrate was concentrated, and the aqueous layer was washed with diethyl ether, concentrated and lyophilized to give 77 mg of a powder of the N-deacetyl TAN-588 (isomer B).

The powder of the N-deacetyl TAN-588 as obtained in the above shows physico-chemical properties as illustrated in the following.

1) Appearance:
White powder
2) Molecular weight:
m/z 231 $(M+H)^+$ (FD-MS method)
3)

| Elemental analysis: | | | |
|---|---|---|---|
| Found | | Calcd.* | |
| C, | 40.98 | C, | 40.17 |
| H, | 4.88 | H, | 4.64 |
| N, | 12.17 | N, | 11.71 |
| | | O, | 43.48 |

(*; calculated assuming that 0.5 mole of adhesive water is contained).

4) Molecular formula:

$C_8H_{10}N_2O_6$

5) UV spectrum: in water

$$\lambda_{max}\ 221 \pm 2\ nm\ (E^{1\%}_{1cm} = 133 \pm 20).$$

6) IR spectrum: KBr method
Major peaks are as follows.
3440, 2980, 1800, 1760, 1670, 1570, 1520, 1390, 1290, 1250, 1190, 1090, 1050, 990, 920, 810, 760, 720, 690 $cm^{-1}$.
7) $^1$H-NMR spectrum: 400MHz, in $D_2O$, the following signals are observed. ppm J(Hz).
2.52(1H,m), 2.72(1H,m), 2.90(1H,m), 3.08(1H,m), 4.44(1H,m), 4.68(1H,m), 4.86(1H,m).
8) CD spectrum: in water
The negative Cotton effect is exhibited at 224±2 nm.
9) Solubility:
Soluble in: water
Sparingly soluble in: dimethylsulfoxide, ethyl acetate, chloroform, diethyl ether.
10) HPLC: The conditions are as described above for the mixture of isomers A and B.
Rt, 3.1 min.
11) Distinction between acidity, neutrality and basicity: Amphoteric substance.

Reference Example 7

In DMF (4 ml) was dissolved TAN-588 sodium salt (400 mg), and triethylamine (10 µl) and p-nitrobenzyl bromide (800 mg) were added to the solution, followed by stirring at room temperature for 3 hours. 0.1M phosphate buffer (pH 6.3, 50 ml) was added to the reaction solution, and extraction was effected twice with ethyl acetate (50 ml). The extract was washed with water, and concentrated, and the resultant oily material was treated with ethyl acetate-petroleum benzin to convert into powder (507 mg), thereby giving a mixture of TAN-588 p-nitrobenzyl ester (isomer A) and TAN-588 p-nitrobenzyl ester (isomer B). The resultant powder was chromatographed on a column of Sephadex LH-20 using ethyl acetate:methanol = 19:1 as a mobile phase to give TAN-588 p-nitrobenzyl ester (isomer A) (105 mg), TAN-588 p-nitrobenzyl ester (isomer B)(67 mg) and a mixture thereof (280 mg).

The p-nitrobenzyl ester of TAN-588 as obtained in the above (a mixture of isomers A and B) shows physico-chemical properties as illustrated in the following.
1) Appearance:
White powder.
2) Specific rotation:
$[\alpha]_D^{23}$ +16.3° ± 5° (c=0.485, in $CHCl_3$)
3) Molecular weight:
407 (by means of the SIMS method)
4)

| Elemental analysis: | | | | |
|---|---|---|---|---|
| Calcd.<br>Found | C, 50.13;<br>C, 50.26; | H, 4.21;<br>H, 4.32; | N, 10.32;<br>N, 10.31 | O, 35.35 |

5) Molecular formula:

$C_{17}H_{17}N_3O_9$

6) UV spectrum:

$$\lambda_{max}^{MeOH}\ nm\ (E_{1cm}^{1\%}) = 262\pm2\ (281\pm20),\ 214\pm2\ (278\pm20,\ shoulder)$$

7) IR spectrum: KBr method
3400, 3080, 2960, 1805, 1760, 1680, 1610, 1520, 1450, 1380, 1350, 1270, 1180, 1105, 1050, 1015, 970, 905, 850, 740, 690, 600, 540 $cm^{-1}$.
8) $^1$H-NMR spectrum; 90MHz, in $CDCl_3$. $\delta$ ppm J(Hz).
2.05(3H,s), 2.3-3.3(4H,m), 4.10(1H,m), 4.5-5.1(2H,m), 5.35 (2H,s), 6.25(1H,d,like), 7.55(2H,dd,like), 8.27-(2H,d,like)
9) TLC:
Carrier: silica gel (produced by Merck & Co. of West Germany) Developping solvent: chloroform:methanol (19:1)
Rf value, 0.25 and 0.32
10) Distinction between acidity, neutrality and basicity: Neutral substance.

The TAN-588 p-nitrobenzyl ester (isomer A) as obtained in the above shows physico-chemical properties as illustrated in the following.

1) Appearance:
White powder.
2) Specific rotation:
$[\alpha]_D^{20}$ +97.3°±15° (c=0.48, in $CHCl_3$)
3) Molecular weight:
407 (by means of the SIMS method)
4)

| Elemental analysis: | | | | |
|---|---|---|---|---|
| Calcd.<br>Found | C, 50.13;<br>C, 50.20; | H, 4.21;<br>H, 4.22; | N, 10.32;<br>N, 10.13 | O, 35.35 |

5) Molecular formula:

$C_{17}H_{17}N_3O_9$

6) UV spectrum:

$$\lambda_{max}^{MeOH}\ (E_{1cm}^{1\%}) = 262\pm2\ nm\ (280\pm30),\ 214\pm2\ nm\ (276\pm30,\ shoulder)$$

7) $^{13}$C-NMR spectrum: (100MHz, $CDCl_3$)
173.70(s), 171.53(s), 170.72(s), 165.09(s), 148.06(s), 141.38(s), 128.86(d), 123.91(d), 91.82(s), 71.60(t), 67.29(t), 53.00(d), 29.09(t), 27.49(t), 22.64(q) ppm.
8) IR spectrum: KBr method
3400, 3080, 2950, 1805, 1775, 1760, 1680, 1610, 1530, 1450, 1380, 1350, 1300, 1275, 1190, 1105, 1060, 1020, 980, 910, 850, 740, 700, 600, 540 $cm^{-1}$.

9) TLC:
Carrier: silica gel (produced by Merck & Co. of West Germany)
Developing solvent: chloroform:methanol (19:1)
Rf value, 0.25
10) Distinction between acidity, neutrality and basicity: Neutral substance.

The TAN-588 p-nitrobenzyl ester (isomer B) as obtained in the above shows physico-chemical properties as illustrated in the following.

1) Appearance:
White powder
2) Specific rotation:
$[\alpha]_D^{20}$ -64.5° +15° (c=0.50, in $CHCl_3$)
3) Molecular weight:
407 (by means of the SIMS method)
4)

| Elemental analysis: | | | | |
|---|---|---|---|---|
| Calcd.<br>Found | C, 50.13;<br>C, 50.10; | H, 4.21;<br>H, 4.21; | N, 10.32;<br>N, 10.15 | O, 35.35 |

5) Molecular formula:

$C_{17}H_{17}N_3O_9$

6) UV spectrum:

$$\lambda_{max}^{MeOH} (E_{1cm}^{1\%}) = 262\pm2 \text{ nm } (282\pm30),\ 214\pm2 \text{ nm } (280\pm30, \text{ shoulder})$$

7) $^{13}$C-NMR spectrum: (100MHz, $CDCl_3$)
173.59(s), 170.86(s), 170.61(s), 165.06(s), 148.12(s), 141.24(s), 128.96(d), 123.96(d), 91.69(s), 74.60(t), 67.39(t), 51.94(d), 29.11(t), 27.38(t), 22.67(q), ppm.
8) IR spectrum: KBr method
3400, 3090, 2950, 1805, 1760, 1680, 1610, 1530, 1450, 1380, 1355, 1270, 1180, 1105, 1055, 1015, 965, 910, 855, 740, 695, 600, 540 $cm^{-1}$.
9) TLC: The conditions are as described above for the TAN-588 p-nitrobenzyl ester (Isomer A).
Rf value, 0.32
10) Distinction between acidity, neutrality and basicity: Neutral substance.

Example 1

Production of Sodium 2-{(4S)-4-[2-(2-amino-4-thiazolyl)-(Z)-2-(methoxyimino)acetamido]-3-oxo-2-isoxazolidinyl}-5-oxo-2-tetrahydrofurancarboxylate [Compound (1c)]:

(a) Production of diphenylmethyl 2-{(4S)-4-[2-(2-chloroaoetamido-4-thiazolyl)-(Z)-2-(methoxyimino)-acetamido]-3-oxo-2-isoxazolidinyl}-5-oxo-2-tetrahydrofurancarboxylate [Compound (1a)]:

Diphenylmethyl 2-[(4S)-4-amino-3-oxo-2-isoxazolidinyl]-5-oxo-2-tetrahydro furancarboxylate Compound (R-3)] as obtained in Reference Example 3 was added to a solution in dimethylformamide (DMF) of 2-(2-chloroacetamido-4-thiazolyl)-(Z)-2-methoxyiminoacetic acid, 1-hydroxybenzotriazole (HOBT) and dicyclohexylcarbodiimide (DCC) under ice-cooling, followed by stirring for 1 hour. Ethyl acetate was added to the reaction solution, and the precipitate which separated out was filtered off, and the filtrate was washed with aqueous sodium hydrogencarbonate solution and water, successively, and dried over anhydrous sodium sulfate. Then, the solvent was distilled off, and the residue was chromatographed on silica gel, followed by elution with ethyl acetate-hexane (2:1) to give Compound (1a) in the form of a colorless foamed

substance.

(b) Production of diphenylmethyl 2-{(4S)-4-[2-(2-amino-4-thiazolyl)-(Z)-2-(methoxyimino)acetamido]-3-oxo-2-isoxazolidinyl}-5-oxo-2-tetrahydrofurancarboxylate [Compound (1b)]:

Compound (1a) as obtained in the above was dissolved in a mixed solution of tetrahydrofuran and water, and sodium N-methyldithiocarbamate was added little by little to the solution, followed by stirring at room temperature for 1 hour. THF was distilled off, and the residue was extracted with ethyl acetate. The extract was washed with saturated aqueous sodium chloride solution and dried over anhydrous sodium sulfate. The extract was freed of solvent, and the residue was chromatographed on silica gel, followed by elution with ethyl acetate to give Compound (1b) in the form of a colorless foamed substance.

(c) Production of the subject Compound (1c):

Anisole and trifluoroacetic acid were added to a dichloromethane solution of the Compound (1b) under an atmosphere of nitrogen at -10 ° C to -15 ° C, followed by stirring for 5.5 hours. The reaction solution was poured into a phosphate buffer of pH 7.0 (8 mℓ) containing sodium hydrogencarbonate (220 mg) under cooling, and the aqueous layer was separated, while the organic layer was extracted with a phosphate buffer of pH 7.0 (4 mℓ). The aqueous layers were combined, washed with dichloromethane and concentrated under reduced pressure. The concentrate was chromatographed on HP-20, and elution was performed with water, followed by lyophilization of the eluate to give the subject Compound (1c) in the form of a colorless powder.

Example 2

Production of sodium 2-{(4S)-4-[D-2-(4-ethyl-2,3-dioxo-1-piperazinocarboxamido)-2-phenylacetamido]-3-oxo-2-isoxazolidinyl}-5-oxo-2-tetrahydrofurancarboxylate [Compound (2b)]:

(a) Production of Compound (2a), a diphenylmethyl ester of the subject Compound (2b):

From Compound (R-3) and N-(4-ethyl-2,3-dioxo-1-piperizinylcarbonyl)-D-phenylglycine, there was obtained Compound (2a), a diphenylmethyl ester of the subject Compound (2b), by the procedure of Example 1 (a).

(b) Production of the subject Compound (2b):

Compound (2a) as obtained above was dissolved in THF (10 ml) and a buffer of pH 6.86 (5 ml), and 5 % palladium-carbon (141 mg) was added to the solution followed by stirring under a hydrogen atmosphere and under ice-cooling for 20 minutes. The catalyst was filtered off, and the filtrate was freed of THF under reduced pressure. The residual solution was washed with ethyl acetate, and the aqueous layer was concentrated under reduced pressure and chromatographed on a column of XAD-2, followed by elution with 20 % aqueous ethanol. The eluate was concentrated under reduced pressure, and the concentrate was lyophilized to give the subject Compound (2b) in the form of a colorless powder.

Example 3

Production of sodium 2-{(4S)-4-[D-2-(3-methylsulfonyl-2-oxoimidazolidine-1-carboxamido)-2-phenylacetamido]-3-oxo-2-isoxazolidinyl}-5-oxo-2-tetrahydrofurancarboxylate [Compound (3b)]:

From Compound (R-3) and N-(3-methylsulfonyl-2-oxoisoxazolidinyl-1-carbonyl)-D-phenylglycine, there was obtained Compound (3a),a diphenylmethyl ester of the subject Compound (3b) by the procedure of Example 1 (a), and by following the procedure of Example 2, there was obtained the subject Compound (3b).

Example 4

Production of sodium 2-{(45)-4-[D-2-(3-furfurylideneamino-2-oxoimidazolidine-1-carboxamido)-2-phenylacetamido]-3-oxo-2-isoxazolidinyl}-5-oxo-2-tetrahydrofurancarboxylate [Compound (4b)]:

By the procedure of Example 1 (a), Compound (R-3) was acylated to give Compound (4a), a diphenylmethyl ester of the subject Compound (4b), and by the procedure of Example 2, there was obtained the subject Compound (4b).

Example 5

Production of sodium 2-[(4S)-4-(2-thienylacetamido)-3-oxo-2-isoxazolidinyl]-5-oxo-2-tetrahydrofurancarboxylate [Compound (5b)]:

Dimethylformamide and 2-thiopheneacetyl chloride were added to a dichloromethane solution of Compound (R-3) under ice-cooling, followed by stirring for 10 minutes and then at room temperature for 20 minutes. Ethyl acetate was added to the reaction solution, and the organic layer was washed with aqueous sodium hydrogencarbonate solution and water, successively, and dried over anhydrous magnesium sulfate. The solvent was distilled off under reduced pressure, and ether was added to the residue to give Compound (5a), a diphenylmethyl ester of the subject Compound (5a) in the form of a colorless powder. Then, the Compound (5a) was treated by the procedure of Example 1 (c) to give the subject Compound (5b).

Example 6

Production of sodium 2-[(4S)-4-(2-cyanoacetamido)-3-oxo-2-isoxazolidinyl]-5-oxo-2-tetrahydrofurancarboxylate [Compound (6b)]:

Using Compound (R-3) and cyanoacetic acid, there was obtained Compound (6a), a diphenylmethyl ester of the subject Compound (6b), by the procedure of Example 1 (a), and then, the procedure of Example 2 (b) was carried out to give the subject Compound (6b).

Example 7

Production of sodium 2-{[4S)-4-[(1H)-tetrazolylacetamido]-3-oxo-2-isoxazolidinyl}-5-oxo-2-tetrahydrofurancarboxylate [Compound (7b)]:

Using Compound (R-3) and (1H)-tetrazolylacetic acid, there was obtained Compound (7a), a diphenylmethyl ester of the subject Compound (7b), by the procedure of Example 1 (a), and then the procedure of Example 2 (b) was carried out to give the subject Compound (7b).

Example 8

Production of sodium 2-[(4S)-4-(2,6-dimethoxybenzamido)-3-oxo-2-isoxazolidinyl]-5-oxo-2-tetrahydrofurancarboxylate [Compound (8b)]:

Using Compound (R-3) and 2,6-dimethoxybenzoyl chloride, there was obtained Compound (8a), a diphenylmethyl ester of the subject Compound (8b), by the procedure of Example 5 and 2 (b), and then, the subject Compound (8b) was produced.

Example 9

Production of sodium 2-[(4S)-4-(5-methyl-3-phenyl-2-isoxazoline-4-carboxamido)-3-oxo-2-isoxazolidinyl)-5-oxo-2-tetrahydrofurancarboxylate [Compound (9b)]:

Using Compound (R-3) and 5-methyl-3-phenyl-2-isoxazolyl-4-carbonyl chloride, there was obtained Compound (9a), a diphenylmethyl ester of the subject Compound (9b), by the procedure of Example 5, and then, the procedure of Example 2 (b) was conducted to give the subject Compound (9b).

Example 10

Production of sodium 2-[(4S)-4-(2-ethoxynaphthalenyl-1-carboxamido)-3-oxo-2-isoxazolidinyl]-5-oxo-2-tetrahydrofurancarboxylate[Compound (10b)]:

Using Compound (R-3) and 2-ethoxynaphthalenyl-1-carbonyl chloride, there was obtained Compounds (10a), a diphenylmethyl ester of the subject Compound (10b), by the procedure of Example 5, and then, the procedure of Example 2 (b) was carried out to give the subject Compound (10b).

Below are given the reaction conditions of Examples 1 to 10 as well as the yields and typical physicochemical properties of the compounds obtained.

| Product | Reaction conditions | Yield (mg) St.mat → Prod. | KBr IR $\nu_{max}$ $cm^{-1}$ | NMR(90MHz)$\delta$ |
|---|---|---|---|---|
| 1a | 2-(2-chloroacetamido-4-thiazolyl)-(Z)-2-methoxyiminoacetic acid (155mg), HOBT (75mg), DCC (115mg), DMF(0.5mℓ) 0℃, 1h reacted, extraction with ethyl acetate, silica gel chromatography(ethyl actate-hexane=2:1) | 198 → 300 | 3250, 1800, 1760, 1680 | 2.2-3.3(4H,m), 3.98(3H,s), 4.0-5.3(3H,m), 4.13(2H,s), 6.95, 6.98(each 0.5H,s), 7.20(1H,s), 7.3-7.4(10H,m), 7.7(1H,b) (solvent: $CDCl_3$) |
| 2a | N-(4-ethyl-2,3-dioxo-1-piperazinylcarbonyl)-D-phenylglycine (81mg), HOBT(10mg), DCC (58mg), DMF(2mℓ) rt, 1h reacted, extraction with ethyl acetate, silica gel chromatography(ethyl acete) | 100 → 145 | 3300, 1805, 1760, 1720, 1685, 1500, 1180 | 1.09, 1.12(each 1.5H,t,J=7Hz), 3.2-3.6(2H,m), 3.8-4.1(2H,m), 5.53, 5.75(each 0.5H,d,J=7Hz), 6.87, 6.90 (each 0.5H,s), 7.1-7.6(15H,m), 7.86, 7.92(each 0.5H, d,J=7Hz), 9.75, 9.81(each 0.5H,d,J=7Hz) (solvent: $CDCl_3$) |
| 3a | N-(3-methylsulfonyl-2-oxoimidazolidinyl-1-carbonyl)-D-phenylglycine(116mg), HOBT(15mg), DCC (71mg), DMF(3mℓ) rt, 2h reacted, extraction with | 120 → 178 | 3325, 1800, 1735, 1680, 1525, 1390, 1355, 1255, 1170 | 1.5-3.1(4H,m), 3.18, 3.20 (each 1.5H,s), 3.72, 3.76 (each 0.5H,b), 4.42, 4.53 (each 1H,t,J=9Hz), 4.4-5.2(1H,m), 5.51, 5.67(each 0.5H,d,J=7Hz), 6.90, 6.91 (each 0.5H,s), 7.1-7.5 (15H,m), 7.50(0.5H,d,J=6Hz), 8.77, 8.90(each 0.5H, |

| | | | | |
|---|---|---|---|---|
| 3a | ethyl acetate, silica gel chromatography(ethyl acetate) | | | d,J=7Hz) (solvent:$CDCl_3$) |
| 4a | N-(3-furfurylideneamino-2-oxo imidazolidinyl-1-carbonyl)-D-phenylglycine(178mg), HOBT(30mg),DCC (103mg),DMF(5mℓ), rt,3h reacted, extraction with ethyl acetate,silica gel chromatography(ethyl acetate) | 150 → 282 | 3310,1805, 1765(sh), 1735,1675, 1530,1480, 1410,1390, 1270,1230 | 1.9-3.4(4H,m),3.5-5.3(3H, m),3.68(4H,s),5.57,5.74 (each 0.5H,d,J=7Hz), 6.87,6.89(each 0.5H,s), 6.3-7.5(18H,m),7.57(1H, s),7.78,7.95(each 0.5H,d, J=7Hz),9.10,9.23(each 0.5H,d,J=7Hz) (solvent:$CDCl_3$) |
| 5a | 2-thiopheneacetyl chloride(60 mg),DMA(0.16mℓ), $CH_2Cl_2$(3mℓ), 0℃,10min→ r.t.20min extraction with ethyl acetate, crystallized from ether | 100 → 110 | 3400,1780, 1760,1680, 1510,1180 | 2.2-3.3(4H,m),4.76(2H,s), 3.5-4.2(1H,m),4.63-4.9 (2H,m),6.2-6.5(1H,m),7.0-7.61(13H,m) (solvent:$CDCl_3$) |
| 6a | cyanoacetic acid (47mg),HOBT(37mg) ,DCC(128mg),DMF (2mℓ),rt.60min extraction with ethyl acetate, silica gel chromatography (ethyl acetate-hexane=2:1) | 100 → 76 | 3320,2250, 1790,1760, 1720,1685, 1620,1530, 1180,1050 | 3.66(2H,s),3.91-4.47(1H, m),4.55-5.09(1H,m),5.41-5.60(1H,m),6.91(1H,s), 7.3-7.4(10H,m) (solvent:$d_6$-DMSO) |
| 7a | (1H)-tetrazolylacetic acid(484 mg),HOBT(51mg), | 100 → 88 | 3320,2930, 2850,1790, 1765,1690, | 3.95-4.35(1H,m),4.51-5.09 (1H,m),5.32(2H,s),5.43-5.61(1H,m),6.91(1H,s), |

| | | | | |
|---|---|---|---|---|
| 7a | DCC(78mg), DMF(2 mℓ), rt, 40min extraction with ethyl acetate, silica gel chromatography(ethyl acetate) | | 1620, 1570, 1180, 1050 | 7.3-7.4(10H, m), 9.24(1H, s) (solvent: $d_6$ - DMSO) |
| 8a | 2,6-dimethoxy-benzoyl chloride (101mg), DMA(0.2mℓ), $CH_2Cl_2$(3mℓ), rt, 24h extraction with ethyl acetate, silica gel chromatography(ethyl acetate) | 100 → 100 | 3350, 2990, 1800, 1740, 1660, 1600, 1480, 1180, 1050 | 2.25-3.32(4H, m), 3.78(6H, s), 3.97-4.29(1H, m), 3.75-4.99(1H, m), 6.50(2H, d, J=8Hz), 6.92(1H, s), 7.13-7.40 (11H, m) (solvent: $CDCl_3$) |
| 9a | 5-methy-3-phenyl-2-isoxazolyl-4-carbonyl chloride (112mg), DMA (0.2mℓ), $CH_2Cl_2$ (5mℓ), 0℃, 5min, rt. 30min, extraction with ethyl acetate, silica gel chromatography(ethyl acetate-hexane=1:1) | 100 → 124 | 3400, 3040, 1800, 1760, 1660, 1420, 1180, 1050 | 2.19-3.30(4H, m), 2.67(3H, s), 3.35-4.00(1H, m), 4.41-4.78(1H, m), 5.81-6.09(1H, m), 6.87, 6.90(1H, sX2), 7.20-7.60(15H, m) (solvent: $CDCl_3$) |
| 10a | 2-ethoxynaphthalenyl-1-carbonyl chloride (177mg), DMA (0.4mℓ), $CH_2Cl_2$ (10mℓ), 0℃, 10min, rt, 120min extraction with ethyl acetate, | 200 → 160 | 3400, 2900, 1800, 1750, 1660, 1595, 1510, 1250, 1180, 1050 | 1.42(3H, t, J=7Hz), 2.31-3.46(4H, m), 3.18(2H, q), 3.02-3.61(1H, m), 4.83-5.22 (1H, m), 6.70-6.84(1H, m), 6.97, 7.01(1H, sX2), 7.18-8.15(16H, m), (solvent: $CDCl_3$) |

| | | | | |
|---|---|---|---|---|
| 10a | silica gel chromatography(ethyl acetate-hexane=1:1) | | | |
| 1b | $CH_3NHCS_2Na \cdot 2H_2O$ (120mℓ) THF(8mℓ)−$H_2O$ (17mℓ) r.t.1h extraction with ethyl acetate, silica gel chromatography(ethyl acetate) | 300 → 250 | 3330,1800, 1750,1680, 1610 | 2.2-3.3(4H,m),3.90(3H,s), 4.0-4.9(3H,m),5.7(2H,b), 6.70,6.77(each 0.5H,s), 6.93(1H,s),7.2-7.4(10H, m),8.2(1H,b) (solvent:$CDCl_3$) |
| 1c | $CF_3COOH$(0.2mℓ) anisole(0.15mℓ) $CH_2Cl_2$(8mℓ) −10℃～−15℃, 5.5h HP-20column(water),lyophilization | 120 → 45 | 3430,1780, 1730,1660, 1540 | 2.4-3.3(4H,m),4.03(3H,s), 4.3-4.8(3H,m),7.03(1H,s) (solvent:$D_2O$) |
| 2b | $H_2$/5%Pd-C(141mg) THF-buffer(pH6.86) 0℃,20min XAD-2 column (20%ethanol) lyophilization | 141 → 89 | 1780,1720, 1690-1650, 1510,1370, 1190 | 1.14(3H,t,J=7Hz),3.3-3.6 (2H,m),3.44(2H,q,J=7Hz), 3.8-4.1(2H,m),4.2-4.6(2H, m),4.7-5.1(1H,m),5.55(1H, d,J=7Hz),7.2-7.6(5H,m), 9.26(1H,b),9.82(1H,d,J=7Hz) (solvent:$D_2O$) |
| 3b | $H_2$/5%Pd-C(98mg) THF-buffer(pH7.0) 0℃,20min XAD-2 column (10%ethanol) lyophilization | 176 → 89.5 | 1785,1740, 1670,1535, 1400,1360, 1175 | 3.35(3H,s),3.82(4H,s), 4.32,4.42(each 1H,t,J=8Hz),4.6-5.1(1H,m),5.48, 5.53(each 0.5H,d,J=7Hz), 7.1-7.6(5H,m),8.77(1H,d, J=7Hz),9.17,9.26(each 0.5H,d,J=8Hz) (solvent:$D_2O$) |
| 4b | $H_2$/5%Pd-C(120mg) THF-buffer(pH7.0) 0℃,20min XAD-2 column | 120 → 64 | 1780,1725, 1660,1530, 1480,1415, 1390,1270, | 3.7-4.7(2H,m),3.86(4H,s), 4.7-5.7(1H,m),5.59(1H,d, J=7Hz),6.4-7.8(8H,m), 7.72(1H,s),8.9-9.3(1H,b), |

| | | | | |
|---|---|---|---|---|
| 4b | (20% ethanol) lyophilization | | 1235, 1190 | 9.10(1H, d, J=7Hz) (solvent: $D_2O$) |
| 5b | $CF_3COOH$(0.18mℓ) anisole(0.092mℓ) $CH_2Cl_2$ 8mℓ -10℃~-15℃, 3h XAD-2 column (10% ethanol) lyophilization | 105 → 35 | 3420, 1780, 1720, 1650, 1540, 1380, 1190 | 2.41-3.42(4H, m), 4.11(2H, s), 4.29-4.54(1H, m), 4.79-5.05(1H, m), 5.13-5.43(1H, m), 7.24-7.81(3H, m) (solvent: $D_2O$) |
| 6b | $H_2$/5% Pd-C(100mg) THF-buffer(pH7.0) 0℃, 20min HP-20column(water), lyophilization | 100 → 50 | 3420, 2250, 1775, 1720, 1660, 1540, 1380, 1190 | 2.41-3.41(4H, m), 4.00(2H, s), 4.31-4.60(1H, m), 4.81-5.09(1H, m), 5.19-5.48(1H, m) (solvent: $D_2O$) |
| 7b | $H_2$/5% Pd-C(133mg) THF-buffer(pH7.0) 0℃, 20min HP-20column(water), lyophilization | 133 → 49 | 3420, 1780, 1720, 1650, 1550, 1380, 1190 | 2.42-3.42(4H, m), 4.32-4.59 (1H, m), 4.81-5.09(1H, m), 5.21-5.49(1H, m), 5.77(1H, s), 9.58(1H, s) (solvent: $D_2O$) |
| 8b | $H_2$/5% Pd-C(100mg) THF-buffer(pH7.0) 0℃, 25min XAD-2 column (10% ethanol) lyophilization | 100 → 54 | 3420, 1780, 1730, 1650, 1595, 1530, 1375, 1250, 1105 | 2.41-3.42(4H, m), 4.05(6H, s), 4.38-4.64(1H, m), 4.85-5.16(1H, m), 5.27-5.58(1H, m), 7.04(2H, d, J=8Hz), 7.70 (1H, d, J=8Hz) (solvent: $D_2O$) |
| 9b | $H_2$/5% Pd-C(124mg) THF-buffer(pH7.0) 0℃, 40min XAD-2 column (20% ethanol) lyophilization | 124 → 53 | 3420, 1780, 1730, 1660, 1380, 1190 | 2.42-3.41(4H, m), 3.87(3H, s), 4.31-4.55(1H, m), 4.81-5.07(1H, m), 5.20-5.48(1H, m), 7.82(5H, s) (solvent: $D_2O$) |
| 10b | $H_2$/5% Pd-C(134mg) THF-buffer(pH7.0) 0℃, 1h XAD-2 column (20% ethanol) lyophilization | 134 → 62 | 3420, 1780, 1730, 1650, 1590, 1520, 1380, 1250, 1190 | 1.53(3H, t, J=7Hz), 2.43-3.42(4H, m), 4.34-4.72(1H, m), 4.48(2H, q, J=7.15Hz), 4.85-5.17(1H, m), 5.35-5.58(1H, m), 7.58-8.31(6H, s) (solvent: $D_2O$) |

Example 11

Production of sodium 2-[(4S)-4-(2-cyanomethylthioacetamido)-3-oxo-2-isoxazolidinyl]-5-oxo-2-tetrahydrofurancarboxylate [Compound (11b)]:

Using Compound (R-3) and cyanomethylthioacetyl chloride, there was obtained Compound (11a), a diphenylmethyl ester of the subject Compound (11b), by the procedure of Example 5, and then the procedure of Example 1 (c) was carried out to give the subject Compound (11b).

Example 12

Production of sodium 2-[(4S)-4-(2-difluoromethylthioacetamido)-3-oxo-2-isoxazolidinyl]-5-oxo-2-tetrahydrofurancarboxylate [Compound (12b)]:

Using Compound (R-3) and difluoromethylthioacetyl chloride, there was ontained Compound (12a), a diphenylmethyl ester of the subject Compound (12b), by the procedure of Example 5, and then, the procedure of Example 1 (c) was carried out to give the subject Compound (12b).

Example 13

Production of sodium 2-{(4S)-4-[(R)-2-(4-cyclohexyl-2,3-dioxo-1-piperazinocarboxamido-2-phenylacetamido]-3-oxo-2-isoxazolidinyl}-5-oxo-2-tetrahydrofurancarboxylate [Compound (13b)]:

Using Compound (R-3) and N-(4-cyclohexyl-2,3-dioxo-1-piperazinylcarbonyl)-D-phenylglycine, there was obtained Compound (13a) (chromatography on silica gel permitted isolation of the stereoisomers in relation to the 2-position, A and B), a diphenylmethyl ester of the subject Compound (13b), by the procedure of Example 2 (a). Then, using the isomers, A and B, of Compound (13a), there was obtained the subject Compound (13b) by the procedure of Example 2 (b).

Example 14

Producion of sodium 2-((4S)-4-chloroacetamido-3-oxo-2-isoxazolidinyl)-5-oxo-2-tetrahydrofurancarboxylate - [Compound (14b)]:

Propylene oxide was added to a dichloromethane solution of Compound (R-3) under cooling at -10°C to -20°C, and then, chloroacetyl chloride was added dropwise to the mixture, followed by stirring at the same temperature for 1 hour. The reaction solution was poured into aqueous sodium hydrogencarbonate solution (10 ml), and the organic layer was separated, washed with saturated aqueous sodium chloride solution and dried over anhydrous magnesium sulfate. The solvent was distilled off, and the residue was chromatographed on a column of silica gel, followed by elution with dichloromethane:ethyl acetate: hexane (1:1:1) to give Compound (14a), a diphenylmethyl ester of the subject Compound (14b), in the form of crystals.

Then, a deprotection reaction was carried out by the procedure of Example 1 (c) with Compound (14a) to give the subject Compound (14b).

Example 15

Production of sodium 2-{(4S)-4-[2-(4-pyridylthio)acetamido]-3-oxo-2-isoxazolidinyl}-5-oxo-2-tetrahydrofuran-carboxylate [Compound (15b)]:

Using Compound (14a) as obtained in Example 14, a reaction was carried out with 4-pyridinethiol to give Compound (15a), a diphenylmethyl ester of the subject Compound (15b), and the procedure of Example 1 (c) was carried out to give the subject Compound (15b).
Below are given the reaction conditions of Examples 11 to 15 as well as the yields and typical physico-chemical properties of the compounds obtained.

| Product | Reaction conditions | Yield (mg) St.mat → Prod. | KBr IR ν cm⁻¹ max | NMR(90MHz)δ (solvent) |
|---|---|---|---|---|
| 11a | cyancmethylthio-acetyl chloride (176.4mg), propyleneoxide(2 mℓ), $CH_2Cl_2$(9mℓ) -20℃, 20min extraction with ethyl acetate, silica gel chromatography($CH_2Cl_2$ :ethyl acetate-hexane=1:1:1) | 230.9 → 163 | 3340, 2230, 1765, 1670, 1180, 1050, 735, 690 | ($d_6$-DMSO+$D_2O$) 3.40(2H,s), 4.5-5.8(3H,m), 6.85(1H,s), 7.3-7.5(10H,m) |
| 12a | difluoromethyl thioacetyl chloride(181mg), propyleneoxide(2 mℓ), $CH_2Cl_2$(9mℓ) -20℃, 30min extraction with ethyl acetate, silica gel chromatography($CH_2Cl_2$ -ethyl acetate-hexane=1:1:1) | 221.5 → 285 | 3350, 1790, 1765, 1735, 1510, 1180, 1050, 735, 695 | ($d_6$-DMSO+$D_2O$) 2.60-3.35(4H,m), 3.65(2H,s), 4.6-4.8(1H,m), 6.92(1H,s), 7.30(1H,t,J=55Hz), 7.35-7.50(10H,m) |
| 13a | N-(4-cyclohexyl-2,3-dioxo-1-piperazinylcarbonyl)-D-phenylglycine (233mg), DCC(125mg), HOBT(30mg), DMF(5mℓ), rt, 3h extraction with ethyl acetate, silica gel chro- | 200 → A, 64 B, 92 A+B 113 | isomerA 3310, 2940, 1810, 1765, 1720, 1680, 1520, 1175, 700 isomerB 3300, 2940, 1800, 1760, 1720, 1680, 1510, 1175, 700 | isomerA not measured because of insolubility in $CDCl_3$, $(CD_3)_2CO$ isomerB 0.9-1.9(10H,m), 2.0-3.3(4H,m), 3.2-3.5(2H,m), 3.7-4.0(2H,m), 4.38(1H,t,J=8Hz), 4.6-5.0(1H,m), 5.48(1H,d,J=6Hz), 6.88(1H,s), 7.0-7.5(15H, |

| | | | | |
|---|---|---|---|---|
| 13a | matography(hexane -ethyl acetate= 1:3) | | | m), 7.74(1H, d, J=7Hz), 9.70(1H, d, J=6Hz) |
| 14a | chloroacetyl chloride(208mg), propyleneoxide (5mℓ), $CH_2Cl_2$(40mℓ), -10℃～-20℃, 1h extraction with ethyl acetate, silica gel chromatography($CH_2Cl_2$ -ethyl acetate-hexane=1:1:1) | 1000 → 750 | 3500, 3450, 1795, 1785, 1670, 1525, 1180, 1050, 740, 700 | ($d_6$-DMSO) 2.6-3.3(4H, m), 4.12(2H, s), 4.5-5.2(3H, m), 6.90(1H, s), 7.3-7.5(10H, m), 8.91(1H, d, J=7.5Hz) |
| 15a | 4-pyridine thiol (56.4mg), NaH(19.2mg), NaI(100mg), DMF(1mℓ), r.t. 30min extraction with ethyl acetate, silica gel chromatography(ethyl acetate-acetone =2:1) | 200 → 163 | 3350, 1770, 1740, 1680, 1570, 1520, 1180, 1050 | ($CDCl_3$) 2.12-3.22(4H, m), 3.38-4.09 (1.5H, m), 4.69(2H, s), 4.46-4.93(1.5H, m), 6.91, 6.93 (each 0.5H, s), 7.02-7.40 (12H, m), 8.39(2H, d, J=7Hz) |
| 11b | $CF_3COOH$(0.12mℓ) anisole(1.0mℓ) $CH_2Cl_2$(0.8mℓ) -20℃～0℃, 6h CHP-20 column (water) lyophilization | 130 → 46 | 2240, 1780, 1660, 1200, 1030, 905, 830, 690 | ($d_6$-DMSO) 2.55-3.10(4H, m), 3.40(2H, s), 3.72(2H, s), 3.9-5.1(3H, m), 8.85(1H, d, J=7.5Hz) |
| 12b | $CF_3COOH$(0.5mℓ) anisole(0.5mℓ) $CH_2Cl_2$(4mℓ) -20℃～-10℃, 7h CHP-20 column (20% ethanol) | 240 → 82 | 3350, 3250, 1800, 1740, 1670, 1540, 1320, 1190, 1060, 1050, 900, 750 | ($d_6$-DMSO) 2.85-3.20(1H, m), 3.55(2H, s), 3.9-4.2(1H, m), 4.45-5.10(3H, m), 7.32(1H, t, J=55Hz), 8.90(1H, d, J=6Hz) |

| | | | | |
|---|---|---|---|---|
| 12b | lyophilization | | | |
| 13b | $H_2$/5%Pd-C(120mg)<br>THF(8mℓ)-pH7.0<br>buffer(4mℓ)<br>0°C,20min<br>XAD-2 column<br>(20%ethanol)<br>lyophilization | 120<br>→<br>71 | 2940,1790,<br>1720,1670,<br>1515,1190 | ($d_6$-DMSO+$CDCl_3$)<br>0.9-1.9(10H,m),2.2-3.2<br>(4H,m),3.3-3.6(2H,m),3.7-<br>4.0(2H,m),3.7-4.6(2H,m),<br>4.7-5.1(1H,m),5.53,5.55<br>(each 0.5H,d,J=7Hz),7.2-<br>7.6(5H,m),9.17,9.26(each<br>0.5H,d,J=8Hz),9.82(1H,d,<br>J=7Hz) |
| 14b | $CF_3COOH$(0.3mℓ)<br>anisole(0.5mℓ)<br>$CH_2Cl_2$(3mℓ)<br>-20°C~0°C,3.5h<br>CHP-20 column<br>(water)<br>lyophilization | 159<br>→<br>85 | 1780,1660,<br>1540,1380,<br>1200,1120,<br>1030, 910,<br>765, 680 | ($D_2O$)<br>2.6-3.6(4H,m),4.44(2H,s),<br>4.3-4.6(1H,m),4.8-5.1(1H,<br>m),5.2-5.5(1H,m) |
| 15b | $CF_3COOH$(0.228mℓ)<br>anisole(0.113mℓ)<br>$CH_2Cl_2$(10mℓ)<br>-10°C~-15°C,3h<br>XAD-2 column<br>(water)<br>lyophilization | 135<br>→<br>21 | 3420,1780,<br>1730,1660,<br>1585,1540,<br>1380,1190 | ($D_2O$)<br>2.41-3.42(4H,m),4.22(2H,<br>s),4.15-4.50(1H,m),4.73-<br>5.02(1H,m),5.12-5.48(1H,<br>m),7.59(2H,m),8.61(2H,m) |

Example 16

Production of methyl 2-[(4S)-4-acetamido-3-oxo-2-isoxazolidinyl]-5-oxo-2-tetrahydrofurancarboxylate [Compound (16)]:

Sodium 2-[(4S)-4-acetamido-3-oxo-2-isoxazolidinyl]-5-oxo-2-tetrahydrofurancarboxylate [Compound (R-1)] as obtained in Reference Example 1 was dissolved in DMF, and methyl iodide was added to the solution, followed by stirring under shield of sunlight at room temperature for 5 hours. The reaction solution was concentrated, and 50 ml of water was added to the concentrate, followed by extraction with 20 ml of dichloromethane. The extract was concentrated, and the residue was crystallized from ethyl acetate-ether to give crystals (1.36 g). The crystals (a mixture of isomers A and B, 1 g) was chromatographed on silica gel (50 g), and elution for fractionation was performed with a solvent system of chloroform-methanol (30:1). The fractions which flowed out of the column first were concentrated and crystallized to give the subject Compound (isomer B of 16), while the fraction which flowed out of the column subsequently afforded the subject Compound (isomer A of 16) in the form of a crystalline powder.

Example 17

Production of pivaloyloxymethyl 2-[(4S)-4-acetamido-3-oxo-2-isoxazolidinyl]-5-oxo-2-tetrahydrofurancarboxylate [Compound (17)]:

Compound (R-1) was dissolved in DMF, and chloromethyl pivalate was added to the solution, followed by stirring . Water was added to the reaction solution, and the mixture was extracted with 150 ml of ethyl acetate. The extract was concentrated, and the resultant residue was powdered from petroleum ether. 140 mg of the resulting powder was chromatographed on Sephadex LH-20 (500 ml), and elution was performed

with ethyl acetate-methanol (19:1). The corresponding fractions afforded the subject Compound (isomer B of 17) and Compound (isomer A of 17) in the form of crystals, respectively.

Example 18

Production of 3,5-dinitrobenzyl 2-[(4S)-4-acetamido-3-oxo-2-isoxazolidinyl]-5-oxo-2-tetrahydrofurancarboxylate [Compound (18)]:

Compound (R-1) was dissolved in DMF, and 3,5-dinitrobenzyl chloride was added to the solution, followed by stirring. Water was added to the reaction system, and the mixture was extracted with ethyl acetate. The extract was concentrated, and the residue was powdered from petroleum ether. 624 mg of the resultant powder crystallized from acetone-hexane to give crystals of Compound (isomer A of 18). The mother liquor was also crystallized from the same solvent to give crystals of the subject Compound (isomer B of 18).

Example 19

Production of p-bromophenacyl 2-[(4S)-4-acetamido-3-oxo-2-isoxazolidinyl]-5-oxo-2-tetrahydrofurancarboxylate [Compound (19)]:

Compound (R-1) was dissolved in DMF, and p-bromophenacyl bromide was added to the solution, followed by stirring. Water was added to the reaction solution, and the mixture was extracted with ethyl acetate. The extract was concentrated, and petroleum benzine was added to the residue to give 410 mg of a powder. The powder was chromatographed on Sephadex LH-20 (500 ml), and elution for fractionation was performed with a solvent system of ethyl acetate-methanol (19:1). The fraction which flowed out of the column first produced a powder of the subject Compound (isomer B of 19) and the fraction which flowed out of the column last afforded a powder of Compound (isomer A of 19), while the fraction which flowed out intermediate yielded a powder of Compound (a mixture of isomers A and B of 19).

Example 20

Production of p-bromobenzyl 2-[(4S)-4-acetamido-3-oxo-2-isoxazolidinyl]-5-oxo-2-tetrahydrofurancarboxylate [Compound (20)]:

Compound (R-1) was dissolved in DMF, and triethylamine and p-bromobenzyl bromide were added to the solution, followed by stirring. 100 ml of 0.1M phosphate buffer (pH 6.3) was added to the reaction solution, and the mixture was extracted with ethyl acetate. The extract was concentrated, and chloroform and petroleum benzine were added to the residue to convert it into powder, thereby giving 562 mg of a powder. The powder was chromatographed on a column of Sephadex LH-20, and elution was performed with ethyl acetate-methanol (19:1). The fraction which flowed out of the column first yielded crystals of the subject Compound (isomer B of 20), and the fraction which flowed out last afforded crystals of Compound (isomer A of 20), while the fraction which flowed out intermediate gave a powder of Compound (a mixture of isomers A and B of 20).

Below given are the reaction conditions of Examples 16 to 20 as well as the yields and typical physicochemical properties of the compounds obtained.

| Product | Reaction conditions of esterification | Yield (mg) St.mat → Prod. | KBr IR $\nu_{max}$ $cm^{-1}$ | NMR (90MHz) $\delta$ (solvent) |
|---|---|---|---|---|
| 16 | methyl iodide (0.8ml) DMF(10ml) rt,5h,ethyl acetate, extraction with $CH_2Cl$ A,B were separated with silica gel chromatography (chloroform-methanol) | 2000 → 1360 | isomerA 1800, 1760, 1750, 1650, 1305, 1180, 900<br>isomerB 1815, 1805, 1760, 1740, 1665, 1305 | isomerA ($CDCl_3$) 2.03(3H,s), 2.3~3.4(4H,m), 3.88(3H,s), 3.9~4.3(1H,m), 4.6~5.2(2H,m), 6.40(1H,d, J=6Hz)<br>isomerB ($CDCl_3$) 2.04(3H,s), 2.3~3.3(4H,m), 3.87(3H,s), 4.11(1H,dd,J=8,10Hz), 4.7~5.3(2H,m), 6.39(1H,d,J=6Hz) |
| 17 | chloromethyl pivalate(360μl) DMF(5ml) rt,14h extraction with ethyl acetate Sephadex LH-20 chromatography(ethyl acetate-methanol=19:1) | 500 → A,116 B,105 A+B, 173 | isomerA 3400, 1810, 1760, 1675, 1540<br>isomerB 3320, 1800, 1750, 1660, 1535 | isomerA ($CDCl_3$) 1.22(9H,s), 2.03(3H,s), 2.2-3.4(4H,m), 3.9-4.3(1H,m), 4.6-5.0(2H,m), 5.83(2H,s), 6.55(1H,d,J=5Hz)<br>isomerB ($CDCl_3$) 1.22(9H,s), 2.05(3H,s), 2.2-3.4(4H,m), 4.13(1H,dd,J=8,10Hs), 4.6-5.3(2H,m), 5.85(2H,m), 6.55(1H,d,J=6Hz) |
| 18 | 3,5-dinitrobenzyl chloride (570mg) DMF(5ml) tr,13h extraction with ethyl acetate, fractional recrystallization with acetone-hexane | 570 → A,330 B,187 | isomerA 3370, 1805, 1775, 1755, 1670, 1550, 1350<br>isomerB 3310, 1815, 1760, 1670, 1550, 1350 | isomerA ($CDCl_3$+$d_6$-DMSO) 1.93(3H,s), 2.4-3.3(4H,m), 4.0-4.4(1H,m), 4.4-5.0(2H,m), 5.6(2H,m), 8.37(1H,d,J=7Hz), 8.7-8.8(2H,m), 8.9-9.0(1H,m)<br>isomerB ($CDCl_3$+$d_6$-DMSO) 1.93(3H,s), 2.4-3.3(4H,m), 3.9-4.4(1H,m), 4.4-5.2(2H,m), 5.6(2H,m), 8.53(1H,d,J=7Hz), 8.7-8.8(2H,m), 8.9-9.0(1H,m) |
| 19 | p-bromophenacyl bromide(280mg) | 312 → | isomerA 3390, 1810, 1780, | isomerA ($CDCl_3$) 2.03(3H,s), 2.5-3.4(4H,m), |

| | | | | |
|---|---|---|---|---|
| 19 | DMF(3ml) rt,2h extraction with ethyl acetate, Sephadex LH-20 chromatography(ethyl acetate-methanol=19:1) | A,176 B,124 A+B, 76 | 1710,1540<br>isomerB3400, 1810,1780, 1745,1710, 1540 | 3.9-4.4(1H,m),4.6-5.1(2H, m),5.48(2H,s),6.37(1H,d,J =6Hz),7.67(2H,d,J=8Hz), 7.80(2H,d,J=8Hz)<br>isomerB ($CDCl_3$) 2.03(3H,s),2.5-3.5(4H,m), 4.0-4.3(1H,m),4.7-5.3(2H, m),5.5(2H,m),6.57(1H,d,J= 6Hz),7.67(2H,d,J=8Hz), 7.80(2H,d,J=8Hz) |
| 20 | p-bromobenzyl bromide(800mg) $Et_3N$(250μl) DMF(5ml) rt,3h extraction with ethyl acetate powdered with chloroform-petroleum benzine | 500 → 562 | 3400,1810, 1760,1680, 1540 | ($CDCl_3$) 2.02(3H,s),2.3-3.4(4H,m), 3.9-4.3(1H,m),4.6-5.1(2H, m),5.25(2H,s),6.18(0.5H,d, J=6Hz),6.32(0.5H,d,J=6 Hz),7.23(1H,d,J=8Hz), 7.27(1H,d,J=8Hz),7.53(2H, d,J=8Hz) |

Example 21

Production of sodium 2-[(4S)-4-formylamino-3-oxo-2-isoxazolidinyl]-5-oxo-2-tetrahydrofurancarboxylate [Compound (21b)]:

(a) Production of diphenylmethyl 2-[(4S)-4-formylamino-3-oxo-2-isoxazolidinyl]-5-oxo-2-tetrahydrofurancarboxylate [Compound (21a)]:

Compound (R-3) was dissolved in dichloromethane, and pyridine and a mixed solution of acetic anhydride and formic acid were added to the solution, followed by stirring. The reaction solution was washed with N-hydrochloric acid and 2 % aqueous sodium hydrogencarbonate solution, successively, and the organic layer was dried and concentrated, followed by adding ether to the residue to give a powder of Compound (21a).

(b) Producion of the subject Compound (21b):

10 % palladium-carbon was suspended in THF, and Compound (21a) was added to the suspension, followed by stirring under a stream of hydrogen. After the catalyst was filtered off, the filtrate was concentrated, and water was added to the concentrated residue, followed by adjustment to pH 6. The aqueous layer was washed with ether, and chromatographed on activated carbon. Elution for fractionation was performed with a solvent system of 8 % isobutanol/0.02N aqueous ammonia, and the eluate fraction was concentrated. The residue was treated with acetone and converted into powder to give a powder of the subject Compound (21b).

Example 22

Production of sodium 2-[(4S)-4-n-butyrylamino-3-oxo-2-isoxazolidinyl]-5-oxo-2-tetrahydrofurancarboxylate [Compound (22b)]:

(a) Production of diphenylmethyl 2-[(4S)-4-n-butyrylamino-3-oxo-2-isoxazolidinyl]-5-oxo-2-tetrahydrofurancarboxylate [Compound (22a)]:

Compound (R-3) was dissolved in dichloromethane, and N,N-dimethylacetamide (DMA) and n-butyryl chloride were added to the solution, followed by stirring. The reaction solution was treated by the procedure of Example 21 (a) to give a powder of Compound (22a).

(b) Production of the subject Compoud (22b):

10 % Palladium-carbon was suspended in THF and 0.1M phosphate buffer (pH 7.0), and Compound (22a) was added to the suspension, followed by stirring under a stream of hydrogen. After the catalyst was filtered off, the THF was distilled off, and the aqueous layer was washed with ether and chromatographed on a column of Diaion HP-20. Elution was performed with water, and the effective fraction was concentrated and lyophilized to give a powder of the subject Compound (22b).

Example 23

Production of sodium 2-[(4S)-4-methoxycarbonylamino-3-oxo-2-isoxazolidinyl]-5-oxo-2-tetrahydrofurancarboxylate [Compound (23b)]:

(a) Production of diphenylmethyl 2-[(4S)-4-methoxycarbonylamino-3-oxo-2-isoxazolidinyl]-5-oxo-2-tetrahydrofurancarboxylate [Compound (23a)]:

Compound (R-3) was dissolved in THF, and triethylamine and methyl chlorocarbonate were added to the solution, followed by stirring. The reaction solution was treated by the procedure of Example 21 (a) to give a powder of Compound (23a).

(b) Production of the subject Compound (23b):

Compound (23a) was subjected to a reaction analogous to Example 22 (b), and the reaction solution was treated by the procedure of Example 21 (b) to give a powder of the subject Compound (23b).

Example 24

Production of sodium 2-[(4S)-4-(N-benzyloxycarbonyl)glycylamino-3-oxo-2-isoxazolidinyl]-5-oxo-2-tetrahydrofurancarboxylate [Compound [24b)]:

(a) Production of diphenylmethyl 2-[(4S)-4-(N-benzyloxycarbonyl)glycylamino-3-oxo-2-isoxazolidinyl]-5-oxo-2-tetrahydrofurancarboxylate [Compound (24a)]:

Methyl chlorocarbonate, N-benzyloxycarbonylglycine (Z-glycine) and triethylamine were added to THF, and a suspension of Compound (R-3) in THF was added to the said solution, followed by stirring. The reaction solution was treated by the procedure of Example 21 (a) to give crystals of Compound (24a).

(b) Production of the subject Compound(24b):

Compound (24a) was subjected to a reaction analogous to Example 22 (b), followed by treatment to give a powder of the subject Compound (24b).

Example 25

Production of sodium 2-[(4S)-4-benzoylamino-3-oxo-2-isoxazolidinyl]-5-oxo-2-tetrahydrofurancarboxylate [Compound (25b)]:

(a) Production of diphenylmethyl 2-[(4S)-4-benzoylamino-3-oxo-2-isoxazolidinyl]-5-oxo-2-tetrahydrofurancar-

boxylate [Compound (25a)]:

Compound (R-3) was dissolved in dichloromethane, and DMA and benzoyl chloride were added to the solution, followed by stirring. The reaction solution was treated by the procedure of Example 21 (a) to give crystals of Compound (25a).

(b) Production of the subject Compound (25b):

Compound (25a) was subjected to a reaction analogous to Example 22 (b), and after treatment was conducted, the residue was chromatographed on a column of Diaion HP-20, followed by elution with 40 % aqueous methanol to give a powder of the subject Compound (25b).

Example 26

Production of sodium 2-[(4S)-4-phenylacetylamino-3-oxo-2-isoxazolidinyl]-5-oxo-2-tetrahydrofurancarboxylate [Compound (26b)]:

(a) Production of diphenylmethyl 2-[(4S)-4-phenylacetylamino-3-oxo-2-isoxazolidinyl]-5-oxo-2-tetrahydrofurancarboxylate [Compound (26b)]:

Compound (R-3) was dissolved in dichloromethane, and DMA and phenylacetyl chloride were added to the solution, followed by stirring. The reaction solution was treated by the procedure of Example 21 (a) to give crystals of Compound (26a).

(b) Production of the subject Compound (26b):

Compound (26a) was subjected to a reaction analogous to Example 25 (b), followed by treatment to give a powder of the subject Compound (26b).

Example 27

Production of sodium 2-[(4S)-4-nicotinylamino-3-oxo-2-isoxazolidinyl]-5-oxo-2-tetrahydrofurancarboxylate [Compound (27b)]:

(a) Production of diphenylmethyl 2-[(4S)-4-nicotinylamino-3-oxo-2-isoxazolidinyl]-5-oxo-2-tetrahydrofurancarboxylate [Compound (27a)]:

Compound (R-3) was dissolved in dichloromethane, and DMA and nicotinyl chloride hydrochloride were added to the solution, followed by stirring. The reaction solution was treated by the procedure of Example 21 (a) to give crystals of Compound (27a).

(b) Production of the subject Compound (27b):

Compound (27a) was subjected to a reaction analogous to Example 22, followed by treatment to give the subject Compound (27b) in the form of powder.

Example 28

Production of pivaloyloxymethyl 2-[(4S)-4-phenoxyacetylamino-3-oxo-2-isoxazolidinyl]-5-oxo-2-tetrahydrofurancarboxylate [Compound (28c)]:

(a) Production of diphenylmethyl 2-[(4S)-4-phenoxyacetylamino-3-oxo-2-isoxazolidinyl]-5-oxo-2-tetrahydrofurancarboxylate [Compound (28a)]:

Compound (R-3) was dissolved in dichloromethane, and DMA and phenoxyacetyl chloride were added to the solution, followed by stirring. The reaction solution was treated by the procedure of Example 21 (a) to give crystals of Compound (28a).

(b) Production of sodium 2-[(4S)-4-phenoxyacetylamino-3-oxo-2-isoxazolidinyl]-5-oxo-2-tetrahydrofurancarboxylate [Compound (28b)]:

Compound (28a) was subjected to a reaction analogous to Example 25 (b), followed by a work up to give a powder of the subject Compound (28b).

(c) Production of the subject Compound (28c):

Compound (28b) was dissolved in DMF, and chloromethyl pivalate was added to the solution, followed by stirring. Water was added to the reaction solution, and the mixture was extracted with ethyl acetate. The extract was concentrated, and hexane was added to the residue. The mixture was subjected to decantation twice and to further decantation (twice) with petroleum benzine. The residue was dried through a vacuum pump to give the subject Compound (28c).

Example 29

Production of sodium 2-[(4S)-4-(p-toluenesulfonyl)amino-3-oxo-2-isoxazolidinyl]-5-oxo-2-tetrahydrofurancarboxylate [Compound (29b)]:

(a) Production of diphenylmethyl 2-[(4S)-4-p-toluenesulfonylamino-3-oxo-2-isoxazolidinyl]-5-oxo-2-tetrahydrofurancarboxylate [Compound (29a)]:

Compound (R-3) was dissolved in pyridine, and p-toluenesulfonyl chloride was added to the solution, followed by stirring. Dichloromethane and water were added to the reaction solution, and the organic layer was washed with dilute hydrochloric acid and dilute aqueous sodium hydrogencarbonate solution, successively, and concentrated. Ether was added to the concentrate to give a powder of Compound (29a).

(b) Production of the subject Compound (29b):

Compound (29a) was subjected to the reaction similar to Example 25 (b), followed by work up to give a powder of the subject Compound (29b).

Example 30

Production of diphenylmethyl 2-[(4S)-4-(p-bromobenzoyl)amino-3-oxo-2-isoxazolidinyl]-5-oxo-2-tetrahydrofurancarboxylate [Compound (30a)]:

Compound (R-3) was dissolved in dichloromethane, and DMA and p-bromobenzoyl chloride were added to the solution, followed by stirring. The reaction solution was subjected to a work up analogous to Example 25 (b) to give 528 mg of a crude substance of Compound (30a). This product was chromatographed on silica gel (50 g), and elution was performed with a solvent system of ethyl acetate-hexane (2:3). The eluate was concentrated to give crystals of the subject Compound (30a).

Example 31

Production of diphenylmethyl 2-[(4S)-4-(N-benzyloxycarbonyl-D-alanyl)amino-3-oxo-2-isoxazolidinyl]-5-oxo-2-tetrahydrofurancarboxylate [Compound (31a)]:

N-Benzyloxycarbonyl-D-alanine and triethylamine were added to a THF solution of methyl chlorocarbonate, and a solution of Compound (R-3) in THF was added gradually to the solution, followed by stirring. The work up was carried out according to the procedure of Example 21 (a) to give a powder of the subject Compound (31a).

Below are given the reaction conditions of Examples 21 to 31 as well as the yields and typical physicochemical properties of the compounds obtained.

| Product | Reaction conditions | Yield (mg) St.mat → Prod. | KBr IR ν max cm$^{-1}$ | NMR δ (solvent)(MHz) |
|---|---|---|---|---|
| 21a | HCOOH-$Ac_2O$(1.0ml)<br>Py(1.6ml)<br>$CH_2Cl_2$(40ml)<br>0℃, 1.5h<br>extraction with $CH_2Cl_2$, powdered with ether | 800 → 720 | 3400, 1800, 1775, 1745, 1735, 1700, 1530 | ($d_6$-DMSO)(90MHz)<br>2.4-3.3(4H, m), 3.8-4.2(1H, m), 4.4-5.2(2H, m), 6.90(1H, s), 7.2-7.4(10H, m), 8.13(1H, s), 8.70(1H, d, J=7Hz) |
| 22a | n-butyryl chloride(0.265ml),<br>DMA(1.0ml)<br>$CH_2Cl_2$(20ml)<br>rt, 30min<br>extraction with $CH_2Cl_2$, powdered with ether | 1000 → 860 | 3370, 2970, 1805, 1785, 1750, 1685, 1185, 1055 | ($CDCl_3$)(90MHz)<br>0.91(3H, t, J=7Hz), 1.63(2H, sextet, J=7Hz), 1.9-3.4(6H, m), 4.00(1H, m), 4.5-5.1(2H, m), 7.00(1H, s), 7.36(10H, m) |
| 23a | methyl chlorocarbonate(0.204ml)<br>$Et_3N$(0.368mℓ)<br>THF20mℓ<br>-10℃～-5℃, 3h<br>extraction with ethyl acetate, powdered by ether | 1000 → 1010 | 3430, 1805, 1775, 1740, 1260, 1190, 1060 | ($CDCl_3$)(90MHz)<br>2.1-3.5(4H, m), 3.69(3H, s), 3.8-4.3(1H, m), 4.4-5.0(2H, m), 5.29(1H, m), 6.99(1H, s), 7.38(10H, m) |
| 24a | methyl chlorocarbonate(0.196mℓ)<br>N-benzyloxycarbonyl glycine (531mg)<br>$Et_3N$(0.357mℓ)<br>THF(50mℓ)<br>-10℃～-5℃, 2h<br>extraction with ethyl acetate, powdered by ether | 1000 → 1238 | 3360, 1800, 1780, 1740, 1700, 1530, 1190, 1060 | ($CDCl_3$)(90MHz)<br>2.1-3.4(4H, m), 3.6-4.3(3H, m), 4.4-5.2(2H, m), 5.11(2H, s), 5.61(1H, m), 6.93, 7.10(each0.5H, d, J=7Hz), 7.00(1H, s), 7.36(15H, m) |
| 25a | benzoyl chloride (0.28ml) | 800 → | 3420, 1810, 1780, 1675, | ($d_6$-DMSO)(90MHz)<br>2.4-3.4(4H, m), 4.1-5.5(3H, |

| | | | | |
|---|---|---|---|---|
| 25a | DMA(0.8ml) $CH_2Cl_2$(20ml) rt,30min extraction with $CH_2Cl_2$,powdered with ether | 557 | 1190,1055 | m),6.95(1H,s),7.2-7.7(13H, m),7.90(2H,m),9.12(1H,d,J =8Hz) |
| 26a | phenylacetyl chloride(0.26ml) DMA(0.64ml) $CH_2Cl_2$(20ml) rt,30min extraction with $CH_2Cl_2$,powdered with ether | 800 → 694 | 3430,1805, (sh),1775, 1685,1185, 1050 | ($d_6$-DMSO)(90MHz) 2.3-3.4(4H,m),3.50(2H,s), 3.7-4.3(1H,m),4.4-5.2(2H, m),6.90(1H,s),7.1-7.6(15H, m),8.77(1H,d,J=7Hz) |
| 27a | nicotinyl chloride hydro-chloride(356mg) DMA(0.8ml) $CH_2Cl_2$(20ml) rt,30min extraction with $CH_2Cl_2$,powdered with ether | 800 → 762 | 3400,1810, 1780,1750, 1680,1190, 1060 | ($d_6$-DMSO)(90MHz) 2.3-3.5(4H,m),4.0-5.5(3H, m),6.95(1H,b),7.2-7.7(11H, m),8.28(1H,dt,J=8,2Hz), 8.76(1H,d,J=4Hz),9.09(1H, b),9.35(1H,d,J=7Hz) |
| 28a | phenoxyacetyl chloride(0.62ml) DMA(1.0ml) $CH_2Cl_2$(40ml) rt,30min | 1200 → 975 | 3400,1815, 1775,1700, 1500,1190, 1055 | ($d_6$-DMSO)(90MHz) 2.3-3.3(4H,m),3.9-5.3(3H, m),4.56(2H,s),6.92(1H,s), 6.99(3H,m),7.37(12H,m), 8.78(1H,d,J=8Hz) |
| 29a | p-toluensulfonyl chloride(457mg) Pyridine(195μℓ) $CH_2Cl_2$(30ml) rt,3h extraction with $CH_2Cl_2$, powdered with hexane-ether | 800 → 745 | 3270,1805, 1775,1750, 1340,1300, 1275,1185, 1170 | ($d_6$-DMSO+$CDCl_3$)(90MHz) 2.40(3H,s),2.3-3.4(4H,m), 3.9-4.7(3H,m),6.93(1H,s), 7.2-7.4(12H,m),7.78(1H,d, J=8Hz),7.80(1H,d,J=8Hz) |
| 30a | p-bromobenzoyl chloride(530mg) DMA(0.8ml) $CH_2Cl_2$(20ml) | 800 → 232 | 3400,1810, 1775,1675, 1490,1190, 1060 | ($d_6$-DMSO)(90MHz) 2.3-3.3(4H,m),4.0-5.5(3H, m),6.93(1H,s),7.38(10H,m), 7.77(4H,m),9.17(1H,d,J=7 |

| | | | | |
|---|---|---|---|---|
| 30a | rt, 30min silica gel chromatography (ethyl acetate-hexane) | | | Hz) |
| 31a | methyl chlorocarbonate (232 μℓ) N-benzyloxycarbonyl D-alanine (677mg) $Et_3N$ (417 μℓ) THF (30ml) -10°C, 2.5h THF (30mℓ) extraction with $CH_2Cl_2$, powdered with ethyl acetate-ether | 990 → 1170 | 3350, 1800, 1780, 1760, 1700, 1520, 1500 | ($CDCl_3$)(90MHz) 1.35(3H, d, J=7Hz), 2.3-3.4 (4H, m), 3.8-5.0(4H, m), 5.10 (2H, s), 5.40(0.5H, d, J=7 Hz), 5.48(0.5H, d, J=7Hz), 7.00(1H, s), 6.9-7.1(1H, m), 7.3-7.5(15H, m) |
| 21b | $H_2$/10% Pd-C(650mg), THF-buffer(pH 7.0), rt, 1h column of carbon powder (8% isobutanol-0.02N aqueous ammonia) powdered with acetone | 650 → 306 | 1780, 1730, 1670, 1540 | ($D_2O$)(100MHz) 2.4-3.4(4H, m), 4.1-4.4(1H, m), 4.6-4.9(1H, m), 5.0-5.3(1H, m), 8.22(1H, s) |
| 22b | $H_2$/10% Pd-C(760mg), THF-buffer(pH 7.0), rt, 50min HP-20column($H_2O$) lyophilization | 760 → 295 | 2980, 1790, 1730, 1660, 1540, 1385, 1200 | ($D_2O$)(100MHz) 0.94(3H, t, J=7Hz), 1.64(2H, sextet, J=7Hz), 2.32(2H, t, J=7Hz), 2.4-3.4(2H, m), 4.22 (1H, m), 4.5-5.3(2H, m) |
| 23b | $H_2$/10% Pd-C(900mg), THF-buffer(pH 6.3), rt, 40min column of carbon powder (8% isobutanol-0.02N ammonia), powdered with acetone | 900 → 343 | 1795, 1725, 1660, 1550, 1280, 1200 | ($D_2O$)(100MHz) 2.3-3.4(4H, m), 3.75(3H, s), 4.1-4.4(1H, m), 4.4-5.2(2H, m) |

| | | | | |
|---|---|---|---|---|
| 24b | $H_2$/10%Pd-C(1000 mg), THF-buffer(pH7.0), rt, 45min HP-20 column (40% methanol), lyophilization | 1000 → 270 | 1790, 1730, 1670, 1540, 1390, 1260, 1200 | ($D_2O$)(100MHz) 2.3-3.4(4H, m), 3.8-5.2(3H, m), 3.91(2H, s), 5.17(2H, s), 7.45(5H, s) |
| 25b | $H_2$/10%Pd-C(450mg), THF-buffer(pH 7.0), rt, 45min HP-20 column (40% methanol), lyophilization | 450 → 163 | 1785, 1730, 1660, 1540, 1380, 1200 | ($D_2O$)(100MHz) 2.4-3.3(4H, m), 4.1-5.6(3H, m), 7.4-7.9(5H, m) |
| 26b | $H_2$/10%Pd-C(690mg), THF-buffer(pH 7.0), rt, 40min HP-20 column (40% methanol), lyophilization | 690 → 325 | 1790, 1730, 1660, 1540, 1385, 1195 | ($D_2O$)(100MHz) 2.3--3.3(4H, m), 3.69(2H, s), 4.18(1H, m), 4.5-5.3(2H, m), 7.38(5H, m) |
| 27b | $H_2$/10%Pd-C(650mg), THF-buffer(pH 7.0), rt, 60min HP-20 column ($H_2O$), lyophilization | 650 → 160 | 1790, 1630, 1665, 1380, 1200 | ($D_2O$)(100MHz) 2.4-3.4(4H, m), 4.2-5.5(3H, m), 7.59(1H, dd, J=8, 5Hz), 8.24(1H, dt, J=8, 2Hz), 8.72(1H, b, J=5Hz), 8.92(1H, b) |
| 28b | $H_2$/10%Pd-C(900mg), THF-buffer(pH 7.0), rt, 40min HP-20 column (40% methanol), lyophilization | 900 → 464 | 1790, 1730, 1665, 1540, 1495 | ($d_6$-DMSO)(100MHz) 2.1-3.2(4H, m), 3.7-5.2(3H, m), 4.54(2H, s), 7.00(3H, m), 7.31(2H, m), 8.67, 8.85(each 0.5H, d, J=8Hz) |
| 29b | $H_2$/10%Pd-C(650mg), THF-buffer(pH 7.0), rt, 30min HP-20 column (40% methanol), lyophilization | 650 → 238 | 1785, 1740, 1660, 1390, 1340, 1160 | ($D_2O$)(100MHz) 2.46(3H, s), 2.4-3.2(4H, m), 3.98(1H, t, J=9Hz), 4.3-4.8(2H, m), 7.46(2H, d, J=8Hz), 7.80(2H, d, J=8Hz) |
| 28c | chloromethyl pivalate(185μl) DMF(1ml) rt, 8.5h | 260 → 182 | 3400, 1815, 1770, 1700, 1540 | ($CDCl_3$)(90MHz) 1.02(9H, s), 2.3-3.1(4H, m), 4.0-4.4(1H, m), 4.53(2H, s), 4.6-5.2(2H, m), 5.87(2H, s), |

| | | | | |
|---|---|---|---|---|
| 28c | extraction with ethyl acetate powdered with petroleum benzine | | | 6.8-7.5(6H,m) |

Example 32

Production of 2-[(4S)-4-dimethylamino-3-oxo-2-isoxazolidinyl]-5-oxo-2-tetrahydrofurancarboxylic acid [Compound (32b)]:

(a) Production of diphenylmethyl 2-[(4S)-4-dimethylamino-3-oxo-2-isoxazolidinyl]-5-oxo-2-tetrahydrofurancarboxylate [Compound (32a)]:

Compound (R-3) was dissolved in acetonitrile, and formaldehyde, sodium cyanoborohydride and acetic acid were added to the solution, followed by stirring. Water was added to the reaction solution, and the acetonitrile was distilled off, followed by extraction of the aqueous layer with ethyl acetate. The extract was washed with 2 % aqueous sodium hydogencarbonate solution and concentrated, and the concentrate was admixed with THF and then 2N hydrochloric acid, followed by stirring at room temperature overnight. The organic layer was concentrated, and water was added to the concentrate, followed by adjustment with aqueous dilute sodium hydroxide solution to pH 10. The water layer was extracted with ethyl acetate, and the extract was concentrated to give a powder of Compound (32a).

(b) Production of the subject Compound (32b):

Compound (32a) was subjected to a reaction analogous to Example 25 (b), followed by a work up to give a powder of the subject Compound (32b).

Below are given the reaction conditions of Example 32 as well as the yield and typical physico-chemical properties of the compound obtained.

| Product | Reaction conditions | Yield (mg) St.mat → Prod. | KBr IR: $\nu_{max}$ $cm^{-1}$ | NMR(90MHz) δ (solvent) |
|---|---|---|---|---|
| 32a | i)37% $CH_2O$(2.05 ml), $NaBH_3CN$(480 mg), AcOH(0.30ml) $CH_3CN$(20ml) 0℃, 40min extraction with ethyl acetate ii)THF－2NHCl rt, one overnight extraction with ethyl acetate | 1000 → 809 | 1800, 1775, 1750, 1730 | ($CDCl_3$) 2.1-3.5(4H, m), 2.40, 2.41 (each 3H, s), 3.5-4.6(3H, m), 7.00(1H, s), 7.2-7.6(10H, m) |
| 32b | $H_2$, 10% Pd－C THF－water rt, 40min HP－20 column (water) lyophilization | 650 → 194 | 1790, 1720, 1665 | ($D_2O$) 2.3-3.4(4H, m), 2.90(6H, s) 4.2-5.4(3H, m) |

Example 33

Production of sodium 2-{(4S)-4-[2-(2-amino-4-thiazolyl)acetamido]-3-oxo-2-isoxazolidinyl}-5-oxo-2-tetrahydrofurancarboxylate [Compound (33c)]:

(a) Production of diphenylmethyl 2-{(4S)-4-[2-(2-chloroacetamido-4-thiazolyl)acetamido]-3-oxo-2-isoxazolidinyl}-5-oxo-2-tetrahydrofurancarboxylate [Compound (33a)]:

Using Compound (R-3) and 2-(2-chloroacetamido-4-thiazolyl)acetyl chloride, there was obtained Compound (33a) by the procedure of Example 5.

(b) Production of diphenylmethyl 2-{(4S)-4-[2-(2-amino-4-thiazolyl)acetamido]-3-oxo-2-isoxazolidinyl}-5-oxo-2-tetrahydrofurancarboxylate [Compound (33b)]:

The Compound (33a) as obtained above was subjected to a reaction analogous to Example 1 (b) to give Compound (33b).

(c) Production of the subject Compound (33c):

The Compound (33b) as obtained above was subjected to a reaction analogous to Example 1 (c) to give the subject Compound (33c).

Below are given the reaction conditions of Example 33 as well as the yield and physico-chemical

properties of the compound obtained.

| Product | Reaction conditions | Yield (mg) St.mat → Prod. | KBr IR ν $cm^{-1}$ max | NMR ($d_6$-DMSO, 90MHz) |
|---|---|---|---|---|
| 33a | 2-(2-chloroacetoamide-4-thiazolyl)acetyl chloride(380mg) butylene oxide (0.8ml) $CH_2Cl_2$(12ml) 0℃, 1h | 396 → 595 | 3350, 1770, 1670, 1540, 1185, 1190, 1060 | 2.40-3.55(4H, m), 3.56(2H, s), 3.70-5.10(3H, m), 6.90 (1H, s), 6.97(1H, s), 7.25-7.50(10H, m), 8.71(1H, d, J=7Hz) |
| 33b | $CH_3NHCSSNa \cdot 2H_2O$ (129mg) THF(4ml)-$H_2O$(4ml) rt, 1h extraction with ethyl acetate, silica gel chromatography(ethyl acetate) | 400 → 225 | 3330, 1770, 1745, 1670, 1525, 1180, 1055 | 2.30-3.50(4H, m), 3.80-5.10 (3H, m), 6.26(1H, s), 6.83 (2H, b), 6.90(1H, s), 7.20-7.55(10H, m), 8.57(1H, d, J=7Hz) |
| 33c | $CF_3COOH$(0.30ml) anisole(0.15ml) $CH_2Cl_2$(12ml) -10° ~ -15°, 4h XAD-2 column (water), lyophilization | 160 → 71 | 3430, 1780, 1720, 1660, 1520, 1380, 1200, 1120, 1030 | 2.10-3.50(4H, m), 3.60-5.05 (3H, m), 6.23(1H, s), 6.83 (2H, b), 8.46, 8.60(each 0.5 H, d, J=7Hz) |

Example 34

Production of sodium 2-[4-methoxy-4-(2-thienylacetamido)-3-oxo-2-isoxazolidinyl]-5-oxo-2-tetrahydrofuran-carboxylate [Compound (34b)]:

The Compound (5a) as obtained in Example 5 was dissolved in THF, and a methanol solution of t-butyl hypochloride and lithium methoxide was added to the solution under cooling with dry ice-acetone, followed by stirring for 5 minutes. Then, after one drop of acetic acid was added, the reaction solution was diluted with ethyl acetate, and the organic layer was washed with aqueous sodium thiosulfate solution and saturated aqueous sodium chloride solution, successively, and dried over anhydrous magnesium sulfate. The solvent was distilled off, and the residue was chromatographed on a column of silica gel to give Compound (34a), a diphenylmethyl ester of the subject Compound (349). Subsequently, the Compound (34a) was dissolved in THF, and a buffer of pH 7.0, palladium-black and palladium oxide were added to the solution, followed by stirring under a stream of hydrogen. After the catalyst was filtered off, the filtrate was concentrated under reduced pressure, and the aqueous layer was washed with ether and concentrated. The concentrate was chromatographed on a column of XAD-2, and elution was performed with 10 % ethanol. The eluate was concentrated under reduced pressure, and lyophilized to give the subject Compound (34b)in the form of a grey-white powder.

Example 35

Production of disodium 2-{(4S)-4-[2-(2-amino-4-thiazolyl)-(Z)-2-(carboxymethyloxyimino)acetamido]-3-oxo-2-isoxazolidinyl}-5-oxo-2-tetrahydrofurancarboxylate [Compound (35d)]:

(a) Production of diphenylmethyl 2-{(4S)-4-[2-(2-chloroacetamido-4-thiazolyl)-(Z)-2-(4-nitrobenzyloxycarbonylmethyloxyimino)acetamido]-3-oxo-2-isoxazolidinyl}-5-oxo-2-tetrahydrofurancarboxylate [Compound (35a)]:

Compound (R-3) was suspended in dichloromethane, and butylene oxide and 2-(2-chloroacetamido-4-thiazolyl)-(Z)-2-(4-nitrobenzyloxycarbonylmethoxyimino)acetyl chloride hydrochloride were added to the suspension under ice-cooling to allow the reaction to proceed for 1 hour. Ethyl acetate was added, and the organic layer was washed with aqueous sodium hydrogencarbonate solution and aqueous sodium chloride solution, successively, and dried over anhydrous sodium sulfate. The solvent was distilled off under reduced pressure to give Compound (35a) in the form of a foamed substance.

(b) Production of diphenylmethyl 2-{(4S)-4-[2-(2-amino-4-thiazolyl)-(Z)-2-(4-nitrobenzyloxycarbonylmethoxyimino)acetamido]-3-oxo-2-isoxazolidinyl}-5-oxo-2-tetrahydrofurancarboxylate [Compound (35b)]:

The Compound (35a) as obtained above was subjected to a reaction and treatment similar to those of Example 1 (b) to give Compound (35b).

(c) Production of sodium 2-{(4S)-4-[2-(2-amino-4-thiazolyl)-(Z)-2-(4-nitrobenzyloxycarbonylmethoxyimino)-acetamido]-3-oxo-2-isoxazolidinyl}-5-oxo-2-tetrahydrofurancarboxylate [Compound (35c)]:

The Compound (35b) as obtained above was subjected to a reaction and treatment analogous to Example 1 (c) to give Compound (35c).

(d) Production of the subject Compound (35d):

The Compound (35c) as obtained above was subjected to a reaction and treatment analogous to Example 2 (b) to give the subject Compound (35d).

Below are given the reaction conditions of Example 34 and 35 as well as the yields and typical physico-chemical properties of the compounds obtained.

| Product | Reaction conditions | Yield (mg) St.mat → Prod. | KBr IR ν max cm⁻¹ | NMR (90MHz) δ |
|---|---|---|---|---|
| 34a | THF(10mℓ) t-BuOCl(30 μℓ) MeOLi in MeOH (0.22μℓ of a solution of 1.368mmol/mℓ), −78°C, 5min, extraction with ethyl acetate, silica gel chromatography (hexane-ethyl acetate=3:2) | 210→144 | 3350, 1800, 1795(sh), 1770, 1690, 1175, 1055 | 2.3-3.2(4H,m), 3.17, 3.21 (each1.5H,s), 3.81(2H,s), 4.32, 4.96(each0.5H,d,J=10Hz), 4.41, 4.92(each0.5H, d,J=10Hz), 6.69, 6.72(each 0.5H,s), 6.9-7.5(3H,m), 7.29, 7.32(each5H,s) (solvent:$CDCl_3$) |
| 34b | THF(10mℓ) pH7.0buffer(10mℓ) Pd-black(150mg) PdO(74mg), rt, 1h XAD-2 column (10%ethanol), lyophilization | 144→75.5 | 1785, 1730, 1695(sh), 1660, 1520, 1380, 1250, 1195, 1105, 1040 | 2.7-3.8(4H,m), 3.79(3H,s), 4.36(2H,s), 5.08(2H,ABq, 10,34Hz), 7.4-7.9(3H,m) (solvent:$D_2O$) |
| 35a | $CH_2Cl_2$(12mℓ) butylene oxide (0.8mℓ) 2-(2-chloroacetamide-4-thiazolyl)-(Z)-2-(4-nitrobenzyloxycarbonylmethoxyimino)-acetyl acid chloride hydrochloride(665mg) 0°C, 1 h extraction with ethyl acetate | 396→835 | 1800, 1750, 1690, 1520, 1190, 1050 | 2.15-3.50(4H,m), 3.60-5.35 (3H,m), 4.26(2H,s), 4.90(2H, s), 5.27(2H,s), 6.98(1H,b), 7.30-7.36(10H,s), 7.40-8.30 (5H,m) (solvent:$CDCl_3$) |
| 35b | THF(8mℓ)-$H_2O$(8mℓ) $CH_3NHCS_2Na$(233mg) rt, 2h | 835→541 | 3330, 1800, 1750, 1680, 1605, 1520, | 1.66(2H,b), 2.10-3.50(4H, m), 3.60-5.50(3H,m), 4.87 (2H,s), 5.25(2H,s), 6.96(1H, |

| | | | | |
|---|---|---|---|---|
| 35b | extraction with ethyl acetate, silica gel chromatography(ethyl acetate-hexane=7:3) | | 1195, 1050 | s), 7.20(1H, s), 7.30, 7.33 (each5H, s), 7.40-8.30(4H, m), 7.80-8.00(1H, m) (solvent: $d_6$-DMSO) |
| 35c | $CH_2Cl_2$(24mℓ) $CF_3COOH$(0.66mℓ) anisole(0.33mℓ) -15℃, 5h decantation after adding hexane XAD-2 column (20% ethanol), lyophilization | 500→ 275 | 3420, 2930, 1770, 1740, 1660, 1520, 1350, 1200, 1030 | 2.10-3.50(4H, m), 3.60-5.50 (3H, m), 4.76(2H, s), 5.33 (2H, s), 6.96, 7.00(each0.5H, s), 7.20(2H, b), 7.56-8.27 (4H, m), 9.00-9.30(1H, m) (solvent: $d_6$-DMSO) |
| 35d | $H_2O$(10mℓ) 10% Pd-C(100mg) rt, 2h, XAD-2 column($H_2O$), then CHP-20 column($H_2O$) lyophilization | 200→ 85 | 3400, 1780, 1730, 1660, 1610, 1540, 1390, 1320, 1190, 1040 | 2.10-3.50(4H, m), 3.60-5.50 (3H, m), 4.30(2H, s), 6.85, 6.86(each0.5H, s), 7.20 (2H, b) (solvent: $d_6$-DMSO) |

Example 36

Using the following ingredients, tablets are produced by the conventional means:

| | |
|---|---|
| Compound (1c) as obtained in Example 1 | 300 mg |
| Corn starch | 50 mg |
| Lactose | 28 mg |
| Hydroxypropylcellulose L | 20 mg |
| Magnesium stearate | 2 mg |
| | 400 mg per tablet |

4 to 8 tablets are to be administered to an adult daily after each meal (three times per day).

Example 37

Using the following ingredients, tablets are produced by the conventional means:

| | |
|---|---|
| Compound (35d) as obtained in Example 35<br>Corn starch<br>Lactose<br>Hydroxypropylcellulose L<br>Magnesium stearate | 300 mg<br>50 mg<br>28 mg<br>20 mg<br>2 mg |
| | 400 mg per tablet |

4 to 8 tablets are to be administered to an adult daily after each meal (three times per day).

Example 38

Production of 2-{(4S)-4-[2-(2-aminomethyl)phenylacetamido]-3-oxo-2-isoxazolidinyl}-5-oxo-2-tetrahydrofurancarboxylic acid [Compound (38b)]:

(a) Production of diphenylmethyl 2-{(4S)-4-[2-(2-benzyloxycarbonylaminomethyl)phenylacetamido]-3-oxo-2-isoxazolidinyl}-5-oxo-2-tetrahydrofurancarboxylate [Compound (38a)]:

Using Compound (R-3) and 2-benzyloxycarbonylaminomethylphenylacetic acid, there was obtained Compound (38a) by the procedure of Example 1 (a).

(b) Production of the subject Compound (38b):

Compound (38a) was subjected to a reaction and treatment analogous to Example 2 (b) to give the subject Compound (38b).

Example 39

Production of sodium 2-[(4S)-4-(4-fluorobenzenesulfonylamino)-3-oxo-2-isoxazolidinyl]-5-oxo-2-tetrahydrofurancarboxylate [Compound (39b)]:

(a) Production of diphenylmethyl 2-[(4S)-4-(4-fluorobenzenesulfonylamino)-3-oxo-2-isoxazolidinyl]-5-oxo-2-tetrahydrofurancarboxylate [Compound (39a)]:

Compound (R-3), 4-fluorobenzenesulfonyl chloride and propylene oxide were stirred in DMA under ice-cooling for 1 hour, followed by a treatment analogous to Example 1 (a) to give Compound (39a).

b) Production of the subject Compound (39b):

Compound (39a) was catalytically reduced with use of palladium black, followed by treatment by the procedure of Example 2 (b) to give the subject compound.

Example 40

Production of sodium 2-{(4S)-4-{2-[1-(2-dimethylaminoethyl)-1H-5-tetrazolylthio]acetamido}-3-oxo-2-isoxazolidinyl}-5-oxo-2-tetrahydrofurancarboxylate [Compound (40b)]:

Using Compound (14a) and 1-(2-dimethylaminoethyl)-5-mercapto-1H-tetrazole, there was obtained Compound (40a), a diphenylmethyl ester of the subject Compound (40b), by the procedure of Example 15, and then, the subject Compound (40b) was formed.

Example 41

Production of sodium 2-{(4S)-4-[2-(4-methyl-4H-1,2,4-triazolyl-3-ylthio)acetamido]-3-oxo-2-isoxazolidinyl}-5-oxo-2-tetrahydrofurancarboxylate [Compound (41b)]:

Using Compound (14a) and 3-mercapto-4-methyl-4H-1,2,4-triazole, there was obtained Compound (41a), a diphenylmethyl ester of the subject Compound (41b), by the procedure of Example 15, and then,

the subject Compound (41b) was formed.

Example 42

Production of sodium 2-{(4S)-4-[(2R,3S)-2-(4-ethyl-2,3-dioxo-1-piperazinecarboxamido) -3-formyloxybutylamido]-3-oxo-2-isoxazolidinyl}-5-oxo-2-tetrahydrofurancarboxylate [Compound (42b)]:

Using Compound (R-3) and (2R,3S)-2-(4-ethyl-2,3-dioxo-1-piperazinecarboxamido)-3-formyloxybutyric acid, there was obtained Compound (42a), a diphenylmethyl ester of the subject compound, by the procedure of Example 5, and then by following the procedure of Example 2(b), there was formed the subject Compound (42b).

Example 43

Production of disodium 2-[(4S)-4-(2-phenyl-2-sulfoacetamido)-3-oxo-2-isoxazolidinyl]-5-oxo-2-tetrahydrofurancarboxylate [Compound (43)]:

Compound (R-4) as obtained in Reference Example 4 was dissolved in water, and sodium hydrogencarbonate and an ether solution of phenylsulfoacetyl chloride were added to the solution under ice-cooling with stirring. After the reaction was allowed to proceed for 1 hour, the aqueous layer was separated, washed with ethyl acetate and concentrated, and the concentrate was chromatographed on a column of XAD-2. The fraction eluted with water was lyophilized to give the subject Compound (43) in the form of a colorless powder.

Example 44

Production of sodium 2-{(4S)-4-[2-(2-amino-4-thiazolyl)-(E)-2-methoxyiminoacetamido]-3-oxo-2-isoxazolidinyl}-5-oxo-2-tetrahydrofurancarboxylate [Compound (44c)]:

(a) Production of diphenylmethyl 2-{(4S)-4-[2-(2-chloroacetamido-4-thiazolyl)-(E)-2-methoxyiminoacetamido]-3-oxo-2-isoxazolidinyl}-5-oxo-2-tetrahydrofurancarboxylate [Compound (44a)]:

Compound (R-3) and 2-(2-chloroacetamido-4-thiazolyl)-(E)-2-methoxyiminoacetic acid, there was obtained Compound (44a) by conducting a reaction and treatment analogous to Example 1 (a).

(b) Production of diphenylmethyl 2-{(4S)-4-[2-(2-amino-4-thiazolyl)-(E)-2-methoxyiminoacetamido] 3-oxo-2-isoxazolidinyl}-5-oxo-2-tetrahydrofurancarboxylate [Compound (44b)]:

Using Compound (44a), there was obtained Compound (44b) by conducting a treatment by the procedure of Example 1 (b).

(c) Production of the subject Compound (44c):

Using Compound (44b), there was obtained the subject Compound (44c) by conducting a treatment by the procedure of Example 1 (c).

Example 45

Production of sodium 2-[(4S)-4-(2-trimethylsilyl)ethoxycarbonylamino-3-oxo-2-isoxazolidinyl]-5-oxo-2-tetrahydrofurancarboxylate [Compound (45b)]:

Butylene oxide and trimethylsilylethoxycarbonyl chloride were added to a dichloromethane suspension of Compound (R-3), followed by stirring for 2 hours. The solvent was distilled off, and a powder which separated out upon addition of hexane was collected by filtration to give Compound (45a), a diphenylmethyl ester of the subject compound. This product was treated by the procedure of Example 2 (b) to give the subject Compound (45b).

Example 46

Production of 2-[(4S)-4-(1-aminocyclohexylcarbonylamino)-3-oxo-2-isoxazolidinyl]-5-oxo-2-tetrahydrofurancarboxylic acid [Compound (46b)]:

Using Compound (R-3) and 1-benzyloxycarbonylaminocyclohexanecarboxylic acid, a treatment was carried out by the procedure of Example 1 (a) to give diphenylmethyl 2-[(4S)-4-(1-benzyloxycarbonylaminocyclohexylcarbonylamino)-3-oxo-2-isoxazolidinyl]-5-oxo-2-tetrahydrofurancarboxylate [Compound (46a)]. This product was treated by the procedure of Example 2 (b) to give the subject Compound (46b).

Example 47

Production of sodium 2-{(4S)-4-[3-(2,6-dichlorophenyl)-5-methyl-4-isoxazolylcarbonylamino} -3-oxo-2-isoxazolidinyl}-5-oxo-2-tetrahydrofurancarboxylate [Compound (47b)]:

Using Compound (R-3) and 3-(2,6-dichlorophenyl)-5-methyl-4-isoxazolylcarbonyl chloride, there was obtained Compound (47a), a diphenylmethyl ester of the subject Compound (47b), by the procedure of Example 5. This product was treated by the procedure of Example 1 (c) to give the subject Compound (47b).

Example 48

Production of sodium 2-{(4S)-4-[2-(2-pyridylthio)acetamido]-3-oxo-2-isoxazolidinyl}-5-oxo-2-tetrahydrofurancarboxylate [Compound (48b)]:

Using Compound (14a) and 2-pyridinethiol , there was obtained Compound (48a), a diphenylmethyl ester of the subject Compound (48b), by the procedure of Example 15, and then, there was formed the subject Compound (48b).

Example 49

Production of sodium 2-{(4S)-4-[2-(4-pyridylmethylthio)acetamido]-3-oxo-2-isoxazolidinyl}-5-oxo-2-tetrahydrofurancarboxylate[Compound (49b)]:

Using Compound (14a) and 4-pyridylmethanethiol, there was obtained Compound (49a), a diphenylmethyl ester of the subject Compound (49b), by the procedure of Example 15, and then, there was formed the subject Compound (49b).

Example 50

Production of sodium 2-{(4S)-4-[2-(2-pyrimidinylthio)acetamido]-3-oxo-2-isoxazolidinyl]-5-oxo-2-tetrahydrofurancarboxylate [Compound (50b)]:

Using Compound (14a) and 2-pyrimidinethiol, there was obtained Compound (50a), a diphenylmethyl ester of the subject Compound (50b), by the procedure of Example 15, and then, there was formed the subject Compound (50b).

Example 51

Production of sodium 2-{(4S)-4-{2-[(E)-2-(acetamidovinyl)thio]acetamido}-3-oxo-2-isoxazolidinyl}-5-oxo-2-tetrahydrofurancarboxylate [Compound (51b)]:

Using Compound (14a) and (E)-2-acetamidovinylthiol silver salt, there was obtained Compound (51a), a diphenylmethyl ester of the subject Compound (51b), by the procedure of Example 15, and then, there was formed the subject Compound (51b)

Example 52

Production of sodium 2-[(4S)-4-cyclopropylcarbonylamino-3-oxo-2-isoxazolidinyl]-5-oxo-2-tetrahydrofurancarboxylate [Compound (52b)]:

Using Compound (R-3) and cyclopropanecarboxylic acid, there was obtained Compound (52a), a diphenylmethyl ester of the subject Compound (52b), by the procedure of Example 1 (a), and then, there was formed the subject Compound (52b) by the procedure of Example 2 (b).

Example 53

Production of sodium 2-[(4S)-4-methoxalylamino-3-oxo-2-isoxazolidinyl]-5-oxo-2-tetrahydrofurancarboxylate [Compound (53b)]:

Using Compound (R-3) and methyl oxalyl chloride, there was obtained Compound (53a), a dimethyl ester of the subject Compound (53b), by the procedure of Example 2(b), and then, there was formed the subject Compound (53b) by the procedure of Example 2 (b).

Example 54

Production of sodium 2-[(4S)-4-acrylamido-3-oxo-2-isoxazolidinyl]-5-oxo-2-tetrahydrofurancarboxylate [Compound (54)]:

Compound (R-3) was suspended in tetrahydrofuran, and water and 5 % palladium-carbon were added to the suspension, followed by stirring under ice-cooling and under a stream of hydrogen. After the catalyst was filtered off, the filtrate was washed with water, and the filtrate and washings were combined and concentrated under reduced pressure. The concentrate was washed with ethyl acetate, and the aqueous layer was separated out and admixed, under ice-cooling with stirring, with sodium hydrogencarbonate and a tetrahydrofuran solution of acryloyl chloride. The reaction solution was washed with ethyl acetate, and the aqueous layer was concentrated. The concentrate was chromatographed on a column of HP-20, and the fraction eluted with water was lyophilized to give the subject Compound (54).

Example 55

Production of sodium 2-{(4S)-4-[2-(2-chloro-4-pyridylthio)acetamido]-3-oxo-2-isoxazolidinyl}-5-oxo-2-tetrahydrofurancarboxylate [Compound (55b)]:

Using Compound (14a) and 2-chloro-4-pyridinethiol, there was obtained Compound (55a), a diphenylmethyl ester of the subject Compound (55b), by the procedure of Example 15, and then, there was formed the subject Compound (55b).

Example 56

Production of sodium 2-[(4S)-4-(2-acetamido-5-oxo-2-tetrahydrofurancarbonylamino)-3-oxo-2-isoxazolidinyl]-5-oxo-2-tetrahydrofurancarboxylate [Compound (56b)]:

Using Compound (R-3) and 2-acetamido-5-oxo-2-tetrahydrofurancarboxylate, there was obtained Compound (56a), a diphenylmethyl ester of the subject Compound (56b), by the procedure of Example 1 (a), and then, there was formed the subject Compound (56b) by the procedure of Example 2 (b).

Example 57

Production of sodium 2-{(4S)-4-[2-(1-pyrazolyl)acetamido]-3-oxo-2-isoxazolidinyl}-5-oxo-2-tetrahydrofurancarboxylate [Compound (57b)]:

Using Compound (R-3) and 1-pyrazolylacetic acid, there was obtained Compound (57a), a diphenylmethyl ester of the subject Compound (57b), by the procedure of Example 1 (a), and then, there was formed the subject Compound (57b) by the procedure of Example 2 (b).

Example 58

Production of sodium 2-{(4S)-4-[2-(5-amino-1,2,4-thiadiazole-3-yl)-(Z)-2-ethoxyiminoacetamido]-3-oxo-2-isoxazolidinyl}-5-oxo-2-tetrahydrofurancarboxylate [Compound (58b)]:

Using Compound (R-3) and 2-(5-amino-1,2,4-thidiazole-3-yl)-(Z)-2-ethoxyiminoacetic acid, there was obtained Compound (58a), a diphenylmethyl ester of the subject Compound (58b), by the procedure of Example 1 (a), and then, there was formed the subject Compound (58b)by the procedure of Example 1 (c).

Example 59

Production of sodium 2-{(4S)-4-[2-(3-pyridazinylthio)acetamido]-3-oxo-2-isoxazolidinyl}-5-oxo-2-tetrahydrofurancarboxylate [Compound (59b)]:

Using Compound (14a) and 3-pyridazinethiol lithium salt, there was obtained Compound (59a), a diphenylmethyl ester of the subject Compound (59b), by the procedure of Example 15, and then, there was formed the subject Compound (59b).

Example 60

Production of sodium 2-[(4S)-4-dichloroacetamido-3-oxo-2-isoxazolidinyl]-5-oxo-2-tetrahydrofurancarboxylate [Compound (60b)]:

Using Compound (R-3) and dichloroacetyl chloride, there was obtained Compound (60a), a diphenylmethyl ester of the subject Compound (60b), by the procedure of Example 14, and then, there was formed the subject Compound (60b).

Example 61

Production of sodium 2-{(4S)-4-[(2R,3S)-2-(4-ethyl-2,3-dioxo-1-piperazinecarboxamido)-3-hydroxybutyramido]-3-oxo-2-isoxazolidinyl}-5-oxo-2-tetrahydrofurancarboxylate [Compound (61b)]:

Using Compound (R-3) and (2R,3S)-2-(4-ethyl-2,3-dioxo-1-piperazinecarboxamido)-3-hydroxybutyric acid, there was obtained Compound (61a), a diphenylmethyl ester of the subject Compound (61b), through a reaction analogous to Example 1 (a), and there was formed the subject Compound (61b) through catalytic reduction with use of palladium black.

Example 62

Production of sodium 2-{(4S)-4-[2-(5-trifluoromethyl}-1,3,4-thiadiazol -2-ylthio)acetamido]-3-oxo-2-isoxazolidinyl}-5-oxo-2-tetrahydrofurancarboxylate [Compound (62b)]:

Using Compound 14 (a) and 5-trifluoromethyl-1,3,4-thiadiazole-2-thiol, there was obtained Compound (62a), a diphenylmethyl ester of the subject Compound (62b), by the procedure of Example 15, and then, there was formed the subject Compound (62b).

Example 63

Production of disodium 2-{(4S)-4-{2-[1-ethoxy(hydroxy)phosphinylmethyl-1H-5-tetrazolylthio]acetamido}-3-oxo-2-isoxazolidinyl}-5-oxo-2-tetrahydrofurancarboxylate [Compound (63b)]:

Using Compound (14a) and 1-[ethoxy(hydroxy)phosphinylmethyl]-1H-tetrazole-5-thiol O,S-disodium salt, there was obtained Compound (63a), a diphenylmethyl ester of the subject Compound (63b), by the procedure of Example 15, and then, there was formed the subject Compound (63b).

Example 64

Production of sodium 2-{(4S)-4-{2-[5-(2-diethoxyphosphinylethylthio)-1,3,4-thiadiazolylthio]acetamido}-3-oxo-2-isoxazolidinyl}-5-oxo-2-tetrahydrofurancarboxylate [Compound (64b)]:

Using Compound (14a) and 5-(2-diethoxyphosphinylethylthio)-1,3,4-thiadiazole-2-thiol, there was obtained Compound (64a), a diphenylmethyl ester of the subject Compound (64b), by the procedure of Example 15, and then, there was formed the subject Compound (64b).

Example 65

Production of sodium 2-{(4S)-4-[2-(1-dimethylamino-1H-5-tetrazolylthio)acetamido]-3-oxo-2-isoxazolidinyl}-5-oxo-2-tetrahydrofurancarboxylate [Compound (65b)]:

Using Compound (14a) and 1-dimethylamino-1H-tetrazole-5-thiol, there was obtained Compound (65a), a diphenylmethyl ester of the subject Compound (65b), by the procedure of Example 15, and then, there was formed the subject Compound (65b).

Example 66

Production of sodium 2-{(4S)-4-[2-(4,5-dimethyl-2-thiazolylthio)acetamido]-3-oxo-2-isoxazolidinyl}-5-oxo-2-tetrahydrofurancarboxylate [Compound (66b)];

Using Compound 14 (a) and 4,5-dimethyl-2-thiazolethiol, there was obtained Compound (66a), a diphenylmethyl ester of the subject Compound (66b), by the procedure of Example 15, and then, there was formed the subject Compound (66b).

Example 67

Production of sodium 2-{(4S)-4-[2-(4,5-dimethyl-2-oxazolylthio)acetamido]-3-oxo-2-isoxazolidinyl}-5-oxo-2-tetrahydrofurancarboxylate [Compound (67b)]:

Using Compound (14a) and 4,5-dimethyl-2-oxazolethiol, there was obtained Compound (67a), a diphenylmethyl ester of the subject Compound (67b), by the procedure of Example 15, and then, there was formed the subject Compound (67b).

Example 68

Production of sodium 2-{(4S)-4-[2-(5-methoxymethyl-1,3,4-thiadiazol-2-ylthio)acetamido]-3-oxo-2-isoxazolydinyl}-5-oxo-2-tetrahydrofurancarboxylate [Compound 68b)[:

Using Compound (14a) and 5-methoxymethyl-1,3,4-thiadiazole-2-thiol, there was obtained Compound (68a), a diphenylmethyl ester of the subject Compound (68b), by the procedure of Example 15, and then, there was formed the subject Compound (68b).

Example 69

Production of sodium 2-{(4S)-4-[2-(5-methylsulfonylmethyl-1,3,4-thiadiazol -2-ylthio)acetamido]-3-oxo-2-isoxazolidinyl}-5-oxo-2-tetrahydrofurancarboxylate [Compound (69b)]:

Using Compound (14a) and 5-methylsulfonylmethyl-1,3,4-thiadiazole-2-thiol, there was obtained Compound (69a), a diphenylmethyl ester of the subject Compound (69a), by the procedure of Example 15, and then, there was formed the subject Compound (69b).

Example 70

Production of sodium 2-{(4S)-4-[2-(4-ethyl-4H-1,2,4-triazol-3-ylthio)acetamido]-3-oxo-2-isoxazolidinyl)-5-oxo-2-tetrahydrofurancarboxylate [Compound (70b)]:

Using Compound (14a) and 4-ethyl-4H-1,2,4-triazole-3-thiol, there was obtained Compound (70a), a diphenylmethyl ester of the subject Compound (70b), by the procedure of Example 15, and then, there was formed the subject Compound (70b).

Example 71

Production of disodium 2-{(4S)-4-[2-(5-carboxymethyl-1,3,4-thiadiazol -2-ylthio)acetamido]-3-oxo-2-isoxazolidinyl}-5-oxo-2-tetrahydrofurancarboxylate [Compound (71b)]:

Using Compound (14a) and sodium 2-mercapto-1,3,4-thiadiazol -5-ylacetate, there was obtained Compound (71a], a diphenylmethyl ester of the subject Compound (71b), by the procedure of Example 15, and then, there was formed the subject Compound (71b).

Example 72

Production of sodium 2-{(4S)-4-{2-[1-(2-hydroxyethyl)-1H-5-tetrazolylthio]acetamido}-3-oxo-2-isoxazolidinyl-5-oxo-2-tetrahydrofurancarboxylate [Compound (72b)]:

Using Compound(14a) and 1-(2-hydroxyethyl)-1H-tetrazole-5-thiol, there was obtained Compound (72a), a diphenylmethyl ester of the subject Compound (72b), by a the procedure of Example 15, and then, there was formed the subject Compound (72b).

Example 73

Production of sodium 2-{(4S)-4-{2-[1-(3-dimethylaminopropyl)-1H-5-tetrazolylthio]acetamido}-3-oxo-2-isoxazolidinyl}-5-oxo-2-tetrahydrofurancarboxylate [Compound (73b)]:

Using Compound (14a) and 1-(3-dimethylaminopropyl)-1H-tetrazole-5-thiol, there was obtained Compound (73a), a diphenylmethyl ester of the subject Compound (73b), by the procedure of Example 15, and then, there was formed the subject Compound (73b).

Example 74

Production of 2-[(4S)-4-(2-amino-3-sulfamoylpropionamido)-3-oxo-2-isoxazolidinyl]-5-oxo-2-tetrahydrofurancarboxylic acid [Compound (74b)]:

Using Compound (R-3) and 2-benzyloxycarbonylamino-3-sulfamoylpropionic acid, there was obtained Compound (74a), a diphenylmethyl ester of the subject Compound (74b), through a reaction analogous to Example 1 (a), and then, there was formed the subject Compound (74b) through catalytic reduction with use of palladium-balck as a catalyst.

Example 75

Production of sodium 2-{(4S)-4-[2-(4-cyano-3-hydroxy-5-isothiazolylthio)acetamido]-3-oxo-2-isoxazolidinyl}-5-oxo-2-tetrahydrofurancarboxylate [Compound (75b)]:

Using Compound (R-3) and 4-cyano-3-hydroxy-5-isothiazolylacetic acid, there was obtained Compound (75a), a diphenylmethyl ester of the subject Compound (75b), through a reaction analogous to Example 1 (a), and then there was obtained the subject Compound (75b).

Example 76

Produciton of sodium 2-{(4S)-4-[2-(2-amino-4-thiazolyl)-2-oxoacetamido]-3-oxo-2-isoxazolidinyl}-5-oxo-2-terahydrofurancarboxylate [Compound (76c)]:

Using Compound (R-3) and 2-(2-chloroacetamido-4-thiazolyl)-2-oxoacetic acid, there was obtained diphenylmethyl (4S)-2-{4-[2-(2-chloroacetamido-4-thiazolyl)-2-oxoacetamido]-3-oxo-2-isoxazolidinyl}-5-oxo-2-tetrahydrofurancarboxylate [Compound (76a)] through a reaction analogous to Example 1, and after this product was converted into Compound (76b), a diphenylmethyl ester of the subject Compound (76c), there was formed the subject Compound (76c).

Example 77

Production of sodium 2-{(4S)-4-[2-(2-amino-4-thiazolyl)-(Z)-2-isopropoxyiminoacetamido]-3-oxo-2-isoxazolidinyl}-5-oxo-2-tetrahydrofurancarboxylate [Compound (77c)]:

Using Compound (R-3) and 2-(2-chloroacetamido-4-thiazolyl-(Z)-2-isopropoxyiminoacetic acid, there was

obtained diphenylmethyl 2-{(4S)-4-[2-(2-chloroacetamido-4-thiazolyl)-(Z)-2-isopropoxyiminoacetamido]-3-oxo-2-isoxazolidinyl}-5-oxo-2-tetrahydrofurancarboxylate [Compound (77a)] through a reaction analogous to Example 1, and after this product was converted into Compound (77b), a diphenylmethyl ester of the subject Compound (77c), there was obtained the subject Compound (77c).

Example 78

Production of sodium 2-[(4S)-4-trifluoroacetamido-3-oxo-2-isoxazolidinyl]-5-oxo-2-tetrahydrofurancarboxylate [Compound (78b)]:

Compound (R-3) and trifluoroacetic anhydride were stirred in dichloromethane under ice cooling, and the reaction solution was poured into water, followed by extraction with ethyl acetate. The organic layer was washed with aqueous sodium hydrogencarbonate solution, water and saturated aqueous sodium chloride solution, successively, and dried ($MgSO_4$). The solvent was distilled off, and the residue was chromatographed on a column of silica gel to give Compound (78a), a diphenylmethyl ester of the subject Compound (78b) and then, a reaction and treatment analogous to Example 2 (b) yielded the subject Compound (78b).

Example 79

Production of disodium 2-{(4S)-4-[2-(2-amino-4-thiazolyl)-(Z)-2-(1-carboxy-1-methylethoxyimino)acetamido]-3-oxo-2-isoxazolidinyl}-5-oxo-2-tetrahydrofurancarboxylate [Compound (79d)]:

(a) Production of diphenylmethyl 2-{(4S)-4-{2-(2-chloroacetamido-4-thiazolyl)-(Z)-2-[1-methyl-1-(4-nitrobenzyloxycarbonyl)ethoxyimino]acetamido}-3-oxo-2-isoxazolidinyl}-5-oxo-2-tetrahydrofurancarboxylate [Compound (79a)]:

Using Compound (R-3) and 2-(2-chloroacetamido-4-thiazolyl)-(Z)-2-[1-methyl-1-(4-nitrobenzyloxycarbonyl)ethoxyimino]acetic acid, there was obtained Compound (79a) by the procedure of Example 1 (a).

(b) Production of diphenylmethyl 2-{(4S)-4-{2-(2-amino-4-thiazolyl)-(Z)-2-[1-methyl-1-(4-nitrobenzyloxycarbonyl)ethoxyimino]acetamido]-3-oxo-2-isoxazolidinyl}-5-oxo-2-tetrahydrofurancarboxylate [Compound (79b)]:

Compound (79a) was treated by the procedure of Example 1 (b), there was obtained Compound (79b).

(c) Production of sodium 2-{(4S)-4-{2-(2-amino-4-thiazolyl)-(Z)-2-[1-methyl-1-(4-nitrobenzyloxycarbonyl)-ethoxyimino]acetamido)- 3-oxo-2-isoxazolidinyl)-5-oxo-2-tetrahydrofurancarboxylate [Compound (79c)]:

Compound(79b) was treated in accordance with Example 1 (c) to give Compound (79c).

(d) Production of the subject Compound (79d):

Compound (79c) was treated the procedure of Example 2 (b) to give the subject compound (79d).

Example 80

Production of sodium 2-{(4S)-4-[2-(2-amino-4-thiazolyl)-(Z)-2-(hydroxyimino)acetamido]-3-oxo-2-isoxazolidinyl}-5-oxo-2-tetrahydrofurancarboxylate [Compound (80b)]:

(a) Production of diphenylmethyl 2-{(4S)-4-{2-(2-tritylamino-4-thiazolyl)-(Z)-2-(trityloxyimino)acetamido}-3-oxo-2-isoxazolidinyl}-5-oxo-2-tetrahydrofurancarboxylate [Compound (80a)]:

A solution of Compound (R-3) in N,N-dimethylformamide was added to a solution of 2-(2-tritylamino-4-thiazolyl)-(Z)-2-(trityloxyimino)acetic acid 2-benzothiazolylthio ester in tetrahydrofuran, followed by stirring at room temperature for 16 hours. The reaction solution was diluted with ethyl acetate, washed with aqueous sodium hydrogencarbonate solution and water, successively, and dried ($Na_2SO_4$). Then, the solvent was distilled off, and the residue was chromatographed on a column of silica gel to give Compound (80a).

(b) Production of the subject Compound (80b):

Formic acid (>98 %) was added to a dichloromethane solution of Compound (80a), followed by stirring at room temperature for 1 hour. After the solvent was distilled off under reduced pressure, the residue was dissolved in dichloromethane, and the solution was shaken after the addition of phosphate buffer of pH 7.0 (10 mℓ) containing sodium hydrogencarbonate (400 mg), followed by separating the aqueous layer. The organic layer was further extracted with a phosphate buffer of pH 7.0 (5 mℓ), and the aqueous layers were combined and concentrated under reduced pressure. The concentrate was chromatographed on a column of HP-20, followed by lyophilization to give the subject Compound (80b).

Example 81

Production of sodium 2-[(4S)-4-isobutyloxycarbonylamino-3-oxo-2-isoxazolidinyl]-5-oxo-2-tetrahydrofurancarboxylate [Compound (81b)]:

(a) Production of sodium diphenylmethyl 2-[(4S)-4-isobutyloxycarbonylamino-3-oxo-2-isoxazolidinyl -5-oxo-2-tetrahydrofurancarboxylate [Compound (81a)];

Using Compound (R-3) and isobutyl chlorocarbonate, there was obtained Compound (81a) by the procedure of Example 23 (a).

(b) Production of the subject Compound (81b):

Compound (81a) was treated by the procedure of Example 23 (b) to give the subject Compound (81b).

Example 82

Production of diphenylmethyl 2-{(4S)-4-[N-(4-nitrobenzyloxycarbonyl)glycylamino]-3-oxo-2-isoxazolidinyl}-5-oxo-2-tetrahydrofurancarboxylate [Compound (82)]:

Using Compound (R-3), methyl chlorocarbonate and N-(4-nitrobenzyloxycarbonyl)glycine, there was obtained the subject Compound (82) by the procedure of Example 24 (a).

Example 83

Production of diphenylmethyl 2-{(4S)-4-[N-(2,2,2-trichloroethoxycarbonyl)phenylglycylamino]-3-oxo-2-isoxazolidinyl}-5-oxo-2-tetrahydrofurancarboxylate [Compound (83)]:

Using Compound (R-3), methyl chlorocarbonate and N-(2,2,2-trichloroethoxycarbonyl)phenylglycine, there was obtained the subject Compound (83) by the procedure of Example 24 (a).

Example 84

Production of sodium 2-{(4S)-4-[N-(benzyloxycarbonyl)-D-phenylglycylamino]-3-oxo-2-isoxazolidinyl}-5-oxo-2-tetrahydrofurancarboxylate [Compound (84b)]:

(a) Production of diphenylmethyl 2-{(4S)-4-[N-(benzyloxycarbonyl)D-phenylglycylamino]-3-oxo-2-isoxazolidinyl}-5-oxo-2-tetrahydrofurancarboxylate [Compound (84a)]:

Using Compound (R-3) and N-benzyloxycarbonyl-D-phenylglycine, there was obtained Compound (84a) by the procedure of Example 1 (a).

(b) Production of the subject Compound (84b):

Compound (84a) was treated by the procedure of Example 2 (b) to give the subject Compound (84b).

Example 85

Production of 2-[(4S)-4-L-phenylglycylamino-3-oxo-2-isoxazolidinyl]-5-oxo-2-tetrahydrofurancarboxylic acid [Compound (85b)]:

(a) Production of diphenylmethyl 2-{(4S)-4-[N-(4-methoxybenzyloxycarbonyl)-L-phenylglycylamino]-3-oxo-2-isoxazolidinyl}-5-oxo-2-tetrahydrofurancarboxylate [Compound (85a)]:

Using Compound (R-3) and N-(4-methoxybenzyloxycarbonyl)-L-phenylglycine, there was obtained Compound (85a) by the procedure of Example 1 (a).

(b) Production of the subject Compound (85b):

Compound (85a) was treated by the procedure of Example 1 (c) to give the subject Compound (85b).

Example 86

Production of 2-{(4S)-4-D-phenylglycylamino-3-oxo-2-isoxazolidinyl)-5-oxo-2-tetrahydrofurancarboxylic acid [Compound (86b)]:

(a) Production of diphenylmethyl 2-{(4S)-4-[N-(4-methoxybenzyloxycarbonyl)-D-phenylglycylamino]-3-oxo-2-isoxazolidinyl}-5-oxo-2-tetrahydrofurancarboxylate [Compound (86a)]:

Using Compound (R-3) and N-(4-methoxybenzyloxycarbonyl)-D-phenylglycine, there was obtained Compound (86a) by the procedure of Example 1 (a).

(b) Production of the subject Compound (86b):

Compound (86a) was treated by the procedure of Example 1 (c) to give the subject Compound (86b).

Example 87

Production of sodium 2-{(4S)-4-[(2S)-(2-hydroxy-2-phenyl)acetamido]-3-oxo-2-isoxazolidinyl]-5-oxo-2-tetrahydrofurancarboxylate [Compound (87b)]:

(a) Production of diphenylmethyl 2-{(4S)-4-[(2S)-(2-hydroxy-2-phenyl)acetamido]-3-oxo-2-isoxazolidinyl}-5-oxo-2-tetrahydrofuran carboxylate [Compound (87a)]:

Using Compound (R-3) and S-mandelic acid, there was obtained Compound (87a) by the procedure of Example 1 (a).

(b) Production of the subject Compound (87b):

Compound (87a) was treated by the procedure of Example 2 (b) to give the subject Compound (87b).

Example 88

Production of sodium 2-{(4S)-4-[(2R)-(2-hydroxy-2-phenyl)acetamido]-3-oxo-2-isoxazolidinyl}-5-oxo-2-tetrahydrofurancarboxylate [Compound (88b)]:

Using Compound (R-3) and R-mandelic acid, there was obtained Compound (88a), a diphenylmethyl ester of the subject Compound (88b), by the procedure of Example 87, and then, there was formed the subject Compound (88b).

Example 89

Production of sodium 2-{(4S)-4-[2-(4-chlorophenyl)-2-hydroxyacetamido]-3-oxo-2-isoxazolidinyl}-5-oxo-2-tetrahydrofurancarboxylate [compound (89b)]:

Using Compound (R-3) and 4-chloro-DL-mandelic acid, there was obtained Compound (89a), a diphenylmethyl ester of the subject Compound (89b), by the procedure of Example 87, and then, there was formed the subject Compound (89b).

Example 90

Production of sodium 2-{(4S)-4-[2-hydroxy-2-(4-hydroxyphenyl)-acetamido]-3-oxo-2-isoxazolidinyl}-5-oxo-2-tetrahydrofurancarboxylate [Compound (90b)]:

Using Compound (R-3) and 4-hydroxy-DL-mandelic acid, there was obtained Compound (90a), a diphenylmethyl ester of the subject Compound (90b), by the procedure of Example 87, and then, there was formed the subject Compound (90b).

Example 91

Production of sodium 2-[(4S)-4-(2-hydroxypropionamido)-3-oxo-2-isoxazolidinyl]-5-oxo-2-tetrahydrofurancarboxylate [Compound (91b)]:

Using Compound (R-3) and DL-lactic acid, there was obtained Compound (91a), a diphenylmethyl ester of the subject Compound (91b), by the procedure of Example 87, and then, there was formed the subject Compound (91b).

Example 92

Production of disodium 2-[(4S)-4-(2-carboxy-2-phenylacetamido)-3-oxo-2-isoxazolidinyl]-5-oxo-2-tetrahydrofurancarboxylate [Compound (92b)]:

Using Compound (R-3) and monodiphenylmethyl DL-phenylmalonate, there was obtained Compound (92a), a bis(diphenylmethyl ester) of the subject Compound (92b), by the procedure of Example 87, and then, there was formed the subject Compound (92b).

Example 93

Production of sodium 2-[(4S)-4-benzyloxycarbonylamino-3-oxo-2-isoxazolidinyl]-5-oxo-2-tetrahydrofurancarboxylate [Compound (93b)]:

Using Compound (R-3) and benzyloxycarbonyl chloride, there was obtained Compound (93a), a diphenylmethyl ester of the subject Compound (93b), by the procedure of Example 5, and then, there was formed the subject Compound (93b).

Example 94

Production of sodium 2-((4S)-4-pyruvamido-3-oxo-2-isoxazolidinyl)-5-oxo-2-tetrahydrofurancarboxylate [Compound (94b)];

Using Compound (R-3) and pyruvic acid, there was obtained Compound (94a), a diphenylmethyl ester of the subject Compound (94b), by the procedure of Example 87, and then, there was formed the subject Compound (94b).

Example 95

Production of pivaloyloxymethyl 2-{(4S)-4-[(2R)-(2-hydroxy-2-phenyl)acetamido]-3-oxo-2-isoxazolidinyl)-5-oxo-2-tetrahydrofurancarboxylate [Compound (95)]:

Using Compound (88b) and chloromethyl pivalate, there was obtained the subject Compound (95) by the procedure of Example 28 (c).

Example 96

Production of diphenylmethyl 2-[(4S)-4-(4-nitrobenzylidene)amino-3-oxo-2-isoxazolidinyl]-5-oxo-2-tetrahydrofurancarboxylate [Compound (96)]:

Molecular sieves 4A was added to a dichloromethane solution of Compound (R-3) and 4-nitrobenzaldehyde, followed by stirring at room temperature for 8 hours. Molecular sieves was filtered off, and the filtrate was concentrated to dryness under reduced pressure. Ether was added to the residue, which was converted into powder to give the subject Compound (96).

Example 97

Production of sodium 2-{(4S)-4-[2-(3-chloro-6-pyridazinylthio)acetamido]-3-oxo-2-isoxazolidinyl]-5-oxo-2-tetrahydrofurancarboxylate [Compound (97b)]:

Using Compound (14a) and 3-chloro-6-pyridazinethiol sodium salt, there was obtained Compound (97a), a diphenylmethyl ester of the subject Compound (97b), by the procedure of Example 15, and then, there was formed the subject Compound (97b).

Example 98

Production of sodium 2-[(45)-4-(2-phenylthioacetamido)-3-oxo-2-isoxazolidinyl]-5-oxo-2-tetrahydrofurancarboxylate [Compound (98b)]:

Using Compound (R-3) and phenylthioacetic acid, there was obtained Compound (98a), a diphenylmethyl ester of the subject Compound (98b), by the procedure of Example 1 (a), and then, there was formed the subject Compound (98b) by the procedure of Example 1 (c).

Example 99

Production of sodium 2-[(4S)-4-(2-ethoxydithiocarbonylacetamido)-3-oxo-2-isoxazolidinyl]-5-oxo-2-tetrahydrofurancarboxylate [Compound (99b)]:

Using Compound (14a) and potassium ethylxanthate, there was obtained Compound (99a), a diphenylmethyl ester of the subject Compound (99b), by the procedure of Example 15, and then, there was formed the subject Compound (99b).

Below are given the reaction conditions of Examples 38 to 99 as well as the yields and typical physicochemical properties of the compounds obtained.

| Product | Reaction conditions | Yield (mg) St.mat → Prod. | KBr IR ν max cm⁻¹ | NMR(90MHz)δ |
|---|---|---|---|---|
| 38a | 2-benzyloxycarbonylaminomethylphenylacetic acid(166mg) HOBT(75mg) DCC(128mg) DMF(4mℓ) rt,1h extraction with ethyl acetate, silica gel chromatography ($CH_2Cl_2$-ethyl acetate=2:1) | 200 → 200 | 3320,2920, 2840,1770, 1760,1710, 1670,1610, 1530,1260, 1180,1060 | 2.22-3.30(4H,m),3.5-4.1 (1H,m),3.62(2H,s),4.36(2H, d,J=6Hz),4.48-4.90(1H,m), 5.10(2H,s),5.61-5.80(1H, m),6.67-6.80(1H,m),6.97 (1H,s),7.17-7.40(19H,m) (solvent:$CDCl_3$) |
| 38b | $H_2$/Pd-C(200mg) THF(3mℓ)-water (1mℓ)0℃,90min XAD-2 column (20%ethanol) lyophilization | 200 → 53 | 3410,3240, 3040,1780, 1730,1640, 1540,1370, 1190 | 2.41-3.42(4H,m),4.08(2H, s),4.30-4.59(1H,m),4.48 (2H,s),4.73-5.01(1H,m), 5.05-5.45(1H,m),7.58-7.78(4H,m) (solvent:$D_2O$) |
| 39a | 4-fluorobenzenesulfonyl chloride(100mg) propylene oxide (0.5mℓ) DMA(1mℓ) 0℃,1h extraction with ethyl acetate, silica gel chromatography (hexane-ethyl acetate=1:1) | 200 → 179 | 3260,1795, 1765,1750, 1590,1490, 1340,1170, 1150,1050 | 2.2-2.9(4H,m),3.9-4.3(2H, m),4.5-4.7(1H,m),5.1-5.3 (1H,m),6.94(1H,s),7.1-7.5 (12H,m),7.7-8.0(2H,m) (solvent:$CDCl_3$) |
| 39b | $H_2$/Pd-black(80mg) THF(3mℓ)-$NaHCO_3$ (23mg) | 149 → 88 | 1780,1725, 1650,1395, 1490,1380, | 2.6-3.4(4H,m),4.1-4.4(1H, m),4.6-5.1(2H,m),7.5-7.8 (2H,m),8.1-8.4(2H,m) |

| | | | | |
|---|---|---|---|---|
| 39b | 0℃, 1h<br>HP-20 column<br>(10% ethanol)<br>lyophilization | | 1340, 1155,<br>1090 | (solvent: $D_2O$) |
| 40a | 1-(2-dimethyl-aminoethyl)-1H-tetrazole-5-thiol(77mg)<br>NaH(16mg)<br>DMF(0.5mℓ)<br>rt, 3h<br>extraction with ethyl acetate, crystallized from hexane | 200<br>→<br>234 | 3340, 1790,<br>1730, 1680,<br>1530, 1450,<br>1300, 1260,<br>1180, 1050 | 2.30(6H, s), 2.2-3.4(5H, m), 2.78(2H, t, J=8Hz), 3.95 (2H, d, J=2Hz), 4.0-4.8(2H, m), 4.35(2H, t, J=8Hz), 6.99 (1H, s), 7.2-7.4(10H, m), 7.8-7.9(1H, m)<br>(solvent: $D_2O$) |
| 40b | $CF_3COOH$(0.3mℓ)<br>anisole(0.5mℓ)<br>−20° ∼0℃, 3h<br>CHP-20 column<br>(5% ethanol)<br>lyophilization | 200<br>→<br>93 | 1770, 1730,<br>1655, 1370,<br>1190, 1110,<br>1020 | 2.6-3.4(4H, m), 3.20(6H, s), 4.00(2H, t, J=6.6Hz), 4.3-4.6(1H, m), 4.40(2H, d, J=2 Hz), 5.11(2H, t, J=6.6Hz), 4.8-5.3(2H, m)<br>(solvent: $D_2O$) |
| 41a | 1-methyl-1H-1,3,4-triazole-3-thiol(56mg)<br>NaH(18mg)<br>DMF(1mℓ)<br>rt, 3h<br>extraction with ethyl acetate, silica gel chromatography<br>(ethyl acetate-ethanol=5:1) | 209<br>→<br>140 | 3360, 1770,<br>1745, 1725,<br>1680, 1525,<br>1260, 1185,<br>1050 | 2.3-3.3(4H, m), 3.59(3H, s), 3.85(2H, s), 3.8-5.0(3H, m), 6.90(1H, s), 7.2-7.5(10H, m), 8.50(1H, s), 8.89(1H, d, J=7Hz)<br>(solvent: $d_6$−DMSO) |
| 41b | $CF_3COOH$(0.22mℓ)<br>anisole(0.37mℓ)<br>−20° ∼0℃, 3h<br>CHP-20 column<br>(water)<br>lyophilization | 140<br>→<br>72 | 1775, 1720,<br>1660, 1550,<br>1520, 1375,<br>1195, 1015,<br>1030 | 2.6-3.5(4H, m), 3.93(3H, s), 4.10(2H, s), 4.2-4.5(1H, m), 4.8-5.4(2H, m), 7.80(1H, s)<br>(solvent: $D_2O$) |
| 42a | (2R, 3S)-2-(4- | 360 | 2950, 1805, | 1.1-1.4(6H, m), 2.2-3.0(4H, |

| | | | | |
|---|---|---|---|---|
| 42a | ethyl-2,3-dioxo -1-piperazino-carboxamide)-3-formyloxybutyric acid(315mg) DCC(206.3mg) DMF(3mℓ) rt,3h extraction with ethyl acetate, silica gel chromatography (ethyl acetate) | → 239 | 1720,1680, 1520,1365, 1260,1180, 1050 | m),3.3-3.7(4H,m),3.9-4.3 (2H,m),4.4-4.8(2H,m),4.9-5.2(1H,m),5.5-5.9(1H,m), 6.93(1H,s),7.1-7.4(10H, m),7.7-8.0(1H,m),7.96(1H, s),9.4-9.6(1H,m) (solvent: $CDCl_3$) |
| 42b | $H_2$/10%Pd-C (200mg) THF(2mℓ) $NaHCO_3$(24mg) rt,2h CHP-20 column (5%ethanol) lyophilization | 198 → 88 | 2975,1775, 1710,1670, 1520,1365, 1190,1060, 1030,1005 | 1.46(3H,t,J=7Hz),1.61 (3H,d,J=6.6Hz),2.5-3.6 (4H,m),3.75(2H,q,J=7 Hz),3.9-4.1(2H,m),4.2-4.6 (3H,m),5.1-5.5(1H,m),5.7 -6.0(1H,m),7.43(1H,s) (solvent: $D_2O$) |
| 43 | water(20mℓ) $NaHCO_3$(148mg) phenylsulfoacetyl chloride (119mg) ether(10mℓ) 0℃,1h XAD-2 column (water) lyophilization | 115 → 34 | 3450,1780, 1720,1660, 1540,1380, 1200,1120, 1050 | 2.55-3.42(4H,m),4.32-4.59 (1H,m),4.80-5.09(1H,m), 5.31(1H,s),5.21-5.46(1H, m),7.61-7.92(5H,m) (solvent: $D_2O$) |
| 44a | 2-(2-chloroaceto amide-4-thiazolyl )-(E)-2-methoxy iminoacetic acid (310mg) HOBT(150mg) DCC(230mg) DMF(3mℓ) 0℃,1h | 396 → 600 | 3240,1800, 1760,1690, 1660,1550, 1260,1180, 1040 | 2.20-3.43(4H,m),4.11(3H, s),4.18(2H,s),4.05-5.10 (3H,m),6.97(1H,s),7.24-7.44(10H,m),7.83(1H,s), 8.52(1H,b) (solvent: $CDCl_3$) |

| | | | | |
|---|---|---|---|---|
| 44a | extraction with ethyl acetate, silica gel chromatography (hexane-ethyl acetate=1:2) | | | |
| 44b | $CH_3NHCS_2Na \cdot 2H_2O$ (290mg) THF(16mℓ)-water (14mℓ) rt,1h extraction with ethyl acetate, silica gel chromatography (ethyl acetate) | 600 → 440 | 3300,1800, 1755,1680, 1610,1530, 1180,1050, 1020 | 2.20-3.39(4H,m),4.00-5.12 (3H,m),4.14(3H,s),5.29 (2H,b),6.98(1H,s),7.22-7.39(10H,m),7.51(1H,s), 9.29,9.50(each 0.5H,b) (solvent: $CDCl_3$) |
| 44c | $CF_3COOH$(0.35mℓ) anisole(0.26mℓ) $CH_2Cl_2$(14mℓ) −10° ∼ −15℃, 5.5h HP-20 column (5% ethanol) lyophilization | 210 → 115 | 3430,1780, 1730,1660, 1530,1380, 1190,1010 | 2.60-3.49(4H,m),4.28(3H, s),4.41-4.67(1H,m),4.90-5.12(1H,m),5.31-5.59(1H, m),7.76(1H,s) (solvent: $D_2O$) |
| 45a | trimethylsilyl ethoxycarbonyl chloride(0.7mℓ) butylene oxide (1.6mℓ) $CH_2Cl_2$(40mℓ) 0℃,2h powdered with hexane | 792 → 1000 | (Nujol) 3330,1760, 1710,1530, 1240,1180, 1050 | 0.80-1.33(2H,m),2.25-3.40 (4H,m),3.90-5.00(5H,m), 6.90(1H,s),7.20-7.60(10H, m),7.60-7.90(1H,m) (solvent: $CDCl_3$) |
| 45b | 10% Pd-C(500mg) $NaHCO_3$(160mg) THF(10mℓ)-water (5mℓ) rt,2h CHP-20 column (20% ethanol) | 1000 → 490 | (Nujol) 3300,1780, 1720,1645, 1520,1240, 1180,1050 | 0.80-1.10(2H,m),2.20-3.40 (4H,m),3.50-4.90(5H,m), 7.40-7.70(1H,m) (solvent: $d_6$-DMSO) |

| | | | | |
|---|---|---|---|---|
| 45b | lyophilization | | | |
| 46a | 1-benzyloxy-carbonylamino cyclohexane carbonic acid (181mg) HOBT(88mg) DCC(135mg) $CH_2Cl_2$(10mℓ) rt,4days extraction with ethyl acetate, silica gel chromatography (hexane-ethyl acetate=1:1) | 199 → 240 | 3350,2940, 1800,1760, 1720,1680, 1520,1450, 1240,1180, 1050 | 1.08-2.07(10H,m),2.22-3.31(4H,m),3.79-4.78(3H,m),5.03(2H,s),6.97(1H,s),7.08-7.39(15H,m) (solvent: $CDCl_3$) |
| 46b | $H_2$/5%Pd-C (240mg) THF(3mℓ)-water (1mℓ) 0℃,90min XAD-2 column (20%ethanol) lyophilization | 240 → 35 | 3450,1780, 1730,1650, 1520,1370, 1190,1030 | 1.48-2.34(10H,m),2.41-3.47(4H,m),4.32-4.65(1H,m),4.81-5.05(1H,m),5.21 5.49(1H,m) (solvent: $D_2O$) |
| 47a | 3-(2,6-dichloro phenyl)-5-methyl -4-isoxazolyl-carbonyl chloride (192mg) DMA(0.4mℓ) $CH_2Cl_2$(10mℓ) 0℃,5min→rt, 30min extraction with ethyl acetate, silica gel chromatography (hexane-ethyl acetate=1:1) | 200 → 230 | 3400,3040, 1820,1760, 1670,1420, 1180,1050 | 2.19-3.30(4H,m),2.79(3H,s),3.69-4.02(1H,m),4.63-4.89(1H,m),5.59-5.81(1H,m),6.94(1H,s),7.14-7.59 (13H,m) (solvent: $CDCl_3$) |
| 47b | $CF_3COOH$(0.32mℓ) | 218 | 3450,1780, | 2.41-3.40(4H,m),3.90(3H, |

| | | | | |
|---|---|---|---|---|
| 47b | anisole(0.24mℓ) CH$_2$Cl$_2$(13mℓ) −10° ∼ −15℃, 4h XAD-2 column (20% ethanol) lyophilization | → 79 | 1730,1650, 1520,1380, 1190,1030 | s),4.21-4.55(1H,m),4.80-5.06(1H,m),5.21-5.48(1H,m),7.82(3H,s) (solvent: D$_2$O) |
| 48a | 2-pyridinethiol (56.4mg) NaH(19.2mg) NaI(100mg) DMF(1mℓ) rt,30min extraction with ethyl acetate, silica gel chromatography (hexane-ethyl acetate=2:3) | 200 → 157 | 3350,1770, 1730,1670, 1570,1510, 1180,1050 | 2.21-3.40(4H,m),3.68(2H,s),3.87-4.19(1.5H,m),4.46-4.82(1.5H,m),6.92(1H,s),6.93-8.81(14H,m) (solvent: CDCl$_3$) |
| 48b | CF$_3$COOH(0.28mℓ) anisole(0.21mℓ) CH$_2$Cl$_2$(11mℓ) −10° ∼ −15℃, 5h XAD-2 column (10% ethanol) lyophilization | 157 → 85 | 3450,1780, 1730,1650, 1380,1190, 1100,1030 | 2.48-3.47(4H,m),4.09-4.42(1H,m),4.13(2H,m),4.63-4.93(1H,m),5.08-5.42(1H,m),7.35-8.80(4H,m) (solvent: D$_2$O) |
| 49a | 4-pyridinemethanethiol(99mg) DMF(3mℓ) rt,1h extraction with ethyl acetate, silica gel chromatography (ethyl acetate-acetone=3:1) | 300 → 75 | 3340,1770, 1730,1670, 1590,1510, 1180,1050 | 2.20-3.49(4H,m),3.10(2H,s),3.71(2H,s),3.79-4.11(1.5H,m),4.51-4.97(1.5H,m),6.97(1H,s),7.13-8.62(14H,m) (solvent: CDCl$_3$) |
| 49b | CF$_3$COOH(0.3mℓ) anisole(0.23mℓ) CH$_2$Cl$_2$(12mℓ) | 175 → 86 | 3410,1780, 1720,1650, 1380,1190, | 2.60-3.46(4H,m),3.54(2H,s),4.17(2H,s),4.25-4.42(1H,m),4.62-5.31(2H,m), |

| | | | | |
|---|---|---|---|---|
| 49b | −10° ∼ −15℃, 4h XAD-2 column (10% ethanol) lyophilization | | 1030 | 7.80-8.02(2H,m),8.68-9.00 (2H,m) (solvent: $D_2O$) |
| 50a | 2-pyridinethiol (55mg) NaH(19.2mg) NaI(100mg) DMF(1mℓ) rt,30min extraction with ethyl acetate, crystallized from ether | 124 → 128 | 3350,1770, 1720,1670, 1550,1510, 1380,1180, 1050 | 2.30-3.45(4H,m),3.79(2H, s),3.89-4.20(1.5H,m), 4.45-4.90(1.5H,m),6.99 (1H,s),7.02-8.64(13H,m) (solvent: $CDCl_3$) |
| 50b | $CF_3COOH$(0.4mℓ) anisole(0.31mℓ) $CH_2Cl_2$(16mℓ) −10° ∼ −15℃, 3h XAD-2 column (10% ethanol) lyophilization | 228 → 88 | 3430,1780, 1720,1650, 1550,1380, 1190,1030 | 2.61-3.49(4H,m),4.22(2H, s),4.28-4.56(1H,m),4.77-5.03(1H,m),5.13-5.46(1H, m),7.53(1H,t,J=5Hz), (solvent: $D_2O$) |
| 51a | (E)-2-acetamide-vinylthiol silver salt(85mg) NaI(89mg) DMF(2mℓ) rt,4h extraction with ethyl acetate, silica gel chromatography (ethyl acetate) | 89 → 65 | 3340,1765, 1720,1670, 1610,1500, 1370,1180, 1050 | 1.91,2.00(each 1.5H,s), 2.30-3.45(4H,m),3.22(2H, s),3.89-4.29(1.5H,m), 4.41-4.95(1.5H,m),5.72 (1H,d,J=14Hz),6.82-7.49 (11H,m),6.91(1H,s),8.71 (1H,m) (solvent: $d_6$-DMSO) |
| 51b | $CF_3COOH$(0.247mℓ) anisole(0.189mℓ) $CH_2Cl_2$(10mℓ) −10° ∼ −15℃, 3h XAD-2 column | 142 → 27 | 3250∼ 3450,1780, 1720,1650, 1520,1380, 1190,1030 | 2.27,2.32(each 1.5H,s), 2.61-3.49(4H,m),3.59,3.70 (each 2H,s),4.29-4.60(1H, m),4.77-5.02(1H,m),5.13-5.49(1H,m),5.69-6.20(1H, m),7.14-7.40(1H,m) |

| | | | | |
|---|---|---|---|---|
| 51b | (water) lyophilization | | | (solvent: $D_2O$) |
| 52a | cyclopropanecarboxylic acid(86mg) HOBT(134mg) DCC(206mg) DMF(4mℓ) rt, 1h extraction with ethyl acetate, silica gel chromatography (hexane-ethyl acetate=1:2) | 200 → 183 | 3350, 1760, 1720, 1680, 1540, 1180, 1050 | 0.91-1.15(4H, m), 1.42-1.80 (1H, m), 2.21-3.40(4H, m), 3.87-4.21(1.5H, m), 4.61-4.90(1.5H, m), 6.27-6.51 (1H, m), 6.93(1H, s), 7.25-7.45(10H, m) (solvent: $CDCl_3$) |
| 52b | $H_2$/5%Pd-C (180mg) THF-buffer (pH7.0) 0℃, 30min HP-20 column (water) lyophilization | 180 → 60 | 3450, 1780, 1720, 1650, 1540, 1380, 1200, 1030 | 1.02-1.15(4H, m), 1.70-2.02 (1H, m), 2.59-3.42(4H, m), 4.28-4.57(1H, m), 4.81-5.05 (1H, m), 5.13-5.42(1H, m) (solvent: $D_2O$) |
| 53a | methyl oxalyl chloride(0.65mℓ) DMA(0.4mℓ) $CH_2Cl_2$(10mℓ) rt, 30min extraction with ethyl acetate, crystallized from ether | 200 → 200 | 3350, 1760, 1730, 1700, 1530, 1450, 1180, 1050 | 2.31-3.40(4H, m), 3.81(3H, s), 3.91-4.46(1H, m), 4.55-5.09(1H, m), 5.43-5.60(1H, m), 6.92(1H, s), 7.31-7.55 (10H, m) (solvent: $d_6$-DMSO) |
| 53b | $H_2$/5%Pd-C(150mg) THF-buffer(pH7.0) 0℃, 30min HP-20 column (water) lyophilization | 150 → 91 | 3400, 1780, 1760-1740, 1700, 1650, 1540, 1380, 1190, 1030 | 2.62-3.52(4H, m), 4.18(3H, s), 4.40-4.68(1H, m), 4.88-5.10(1H, m), 5.28-5.61(1H, m) (solvent: $D_2O$) |
| 54 | $H_2$/5%Pd-C(300mg) THF(20mℓ)-(water)(10mℓ) | 300 → 19 | 3450, 1780, 1730, 1660, 1540, 1380, | 2.53-3.41(4H, m), 4.35-4.63 (1H, m), 4.81-5.09(1H, m), 5.21-5.51(1H, m), 6.00-6.21 |

| | | | | |
|---|---|---|---|---|
| 54 | $NaHCO_3$(222mg) acryloyl chloride(81mg) THF(6mℓ) HP-20 column (water) lyophilization | | 1200,1120, 1040 | (1H,m),6.47-6.60(2H,m) (solvent: $D_2O$) |
| 55a | 2-chloro-4-pyridinethiol (215mg) DMF(4mℓ) rt,1h extraction with ethyl acetate, silica gel chromatography (hexane-ethyl acetate=1:2) | 300 → 170 | 3350,1770, 1720,1680, 1570,1520, 1180,1050 | 2.20-3.51(4H,m),3.69(2H,s),3.81-4.19(1.5H,m), 4.48-4.88(1.5H,m),6.98 (1H,s),7.02-8.23(13H,m) (solvent: $CDCl_3$) |
| 55b | $CF_3COOH$(0.28mℓ) anisole(0.22mℓ) $CH_2Cl_2$(14mℓ) −10° ～−15℃, 5h XAD-2 column (10%ethanol) lyophilization | 170 → 82 | 3450,1780, 1720,1650, 1560,1380, 1190,1020, 1040 | 2.60-3.43(4H,m),4.12-4.53 (1H,m),4.20(2H,s),4.75-5.02(1H,m),5.13-5.40(1H,m),7.42-7.61(2H,m),8.40 (1H,d,J=5Hz) (solvent: $D_2O$) |
| 56a | 2-acetamide-5-oxo-2-tetrahydrofurancarboxylic acid(187mg) HOBT(134mg) DCC(206mg) DMF(6mℓ) rt,1h extraction with ethyl acetate, silica gel chromatography (ethyl acetate-acetone=20:1) | 300 → 370 | 3350,1800, 1780,1720, 1670,1530, 1180,1050 | 1.91(3H,s),2.21-3.29(8H,m),3.82-4.20(1.5H,m),4.50-4.87(1.5H,m),6.97(1H,s), 7.28-7.49(10H,m) (solvent: $d_6$-DMSO) |

| | | | | |
|---|---|---|---|---|
| 56b | $H_2$/5%Pd-C (250mg) THF-buffer (pH7.0) 0℃,25min HP-20 column (water) lyophilization | 250 → 123 | 3450,1780, 1730, 1680-1640, 1540,1380, 1190,1040 | 2.26(3H,s),2.59-3.48(8H, m),4.32-4.62(1H,m),4.80-5.06(1H,m),5.13-5.50(1H, m) (solvent: $D_2O$) |
| 57a | 1-pyrazolylacetic acid(126mg) HOBT(134mg) DCC(206mg) DMF(4mℓ) rt,10min extraction with ethyl acetate, silica gel chromatography (ethyl acetate) | 200 → 230 | 3350,1770, 1730,1680, 1580,1520, 1180,1050 | 2.22-3.51(4H,m),3.87-4.21 (1.5H,m),4.48-4.88(1.5H, m),4.83(2H,s),6.34(1H,m), 6.98(1H,s),7.05-7.68(12H, m) (solvent: $CDCl_3$) |
| 57b | $H_2$/5%Pd-C (230mg) THF-buffer (pH7.0) 0℃,40min XAD-2 column (water) lyophilization | 230 → 96 | 3400,1780, 1720,1650, 1400,1190, 1040 | 2.59-3.43(4H,m),4.30-4.59 (1H,m),4.81-5.08(1H,m), 5.21-5.51(1H,m),5.31(2H, s),6.65-6.74(1H,m),7.91-8.07(2H,m) (solvent: $D_2O$) |
| 58a | 2-(5-amino-1,2,4-thiadiazol-3-yl)-(Z)-2-ethoxyiminoacetic acid(235mg) HOBT(145mg) DCC(222mg) rt,1h extraction with ethyl acetate, silica gel chromatography (ethyl acetate- | 280 → 299 | 3320,1800, 1760,1660, 1520,1180, 1050 | 1.23(3H,t,J=7Hz),2.23-3.28(4H,m),3.93-4.32 (3.5H,m),4.58-4.96(1.5H, m),6.63(2H,bs),6.91(1H, s),7.19-7.34(10H,m) (solvent: $CDCl_3$) |

| | | | | |
|---|---|---|---|---|
| 58a | $CHCl_3$ = 2:1) | | | |
| 58b | $CF_3COOH$(0.48ml) anisole(0.36ml) $CH_2Cl_2$(20ml) −10° ∼ −15°C, 4h XAD-2 column (water) lyophilization | 299 → 62 | 3400, 1780, 1720, 1650, 1520, 1390, 1190, 1050 | 1.53(3H, t, J=7Hz), 2.59-3.53(4H, m), 4.39-4.71(3H, m), 4.98-5.17(1H, m), 5.32-5.61(1H, m) (solvent: $D_2O$) |
| 59a | lithium 3-pyridazinethiol (90mg) NaI(100mg) DMF(60ml) rt, 1h extraction with ethyl acetate, silica gel chromatography (ethyl acetate-acetone=9:1) | 300 → 263 | 3340, 1770, 1740, 1680, 1420, 1180, 1050 | 2.21-3.32(4H, m), 3.98(2H, s), 3.83-4.27(1.5H, m), 4.58-4.94(1.5H, m), 6.98 (1H, s), 7.19-7.52(11H, m), 7.91-8.10(1H, m), 8.89-9.00 (1H, m) (solvent: $CDCl_3$) |
| 59b | $CF_3COOH$(0.45ml) anisole(0.35ml) $CH_2Cl_2$(20ml) −10° ∼ −15°C, 4h HP-20 column (water) lyophilization | 263 → 93 | 3450, 1780, 1720, 1650, 1410, 1380, 1190, 1040 | 2.58-3.51(4H, m), 4.21-4.52 (1H, m), 4.32(2H, s), 4.74-5.00(1H, m), 5.12-5.43(1H, m), 7.77-8.08(2H, m), 9.17-9.30(1H, m) (solvent: $D_2O$) |
| 60a | dichloroacetic chloride(111mg) propyleneoxide (5ml) $CH_2Cl_2$(15ml) −20°C, 5 min extraction with ethyl acetate, silica gel chromatography | 300 → 301 | 3480, 1780, 1765, 1720, 1690, 1520, 1490, 1450, 1300, 1260, 1180, 1050 | 2.4-3.6(4H, m), 3.9-5.3(3H, m), 6.51(1H, d, J=3.3Hz), 6.91(1H, s), 7.2-7.5(10H, m), 9.30(1H, d, J=8.3Hz) (solvent: $d_6$-DMSO) |

| | | | | |
|---|---|---|---|---|
| 60a | (hexane-$CH_2Cl_2$-ethyl acetate=1:2:1) | | | |
| 60b | $CF_3COOH$(0.44ml) anisole(0.74ml) $CH_2Cl_2$(4ml) 0℃,2h CHP-20 column (water) lyophilization | 274 → 167 | 3400,1780, 1690,1650, 1525,1380, 1190,1115, 1030 | 2.6-3.5(4H,m),4.4-4.7(1H, m),4.8-5.1(1H,m),5.2-5.5 (1H,m),6.64(1H,s) (solvent:$D_2O$) |
| 61a | (2R,3S)-2-(4-ethyl-2,3-dioxo-1-piperazine-carboxamide)-3-hydroxybutyric acid(238mg) DCC(156.5mg) DMF(3ml) rt,5h extraction with ethyl acetate, silica gel chromatography (ethyl acetate-methanol=5:1) | 300 → 350 | 3320,1810, 1715,1680, 1520,1370, 1250,1195, 1050 | 1.0-1.4(6H,m),2.1-3.1(4H, m),3.1-4.8(11H,m),4.9-5.4 (1H,m),6.88(1H,d,J=3Hz), 7.1-7.5(10H,m),7.8-8.2 (1H,m),9.4-9.6(1H,m) (solvent:$CDCl_3$) |
| 61b | $H_2$/10%Pd-black (150mg) $NaHCO_3$(44mg) THF(5ml)-water (1 ml) 0℃,5h CHP-20 column (10%ethanol) lyophilization | 349 → 102 | 3310,1780, 1720,1670, 1520,1370, 1195,1105, 1005 | 1.44(3H,t,J=8Hz),1.49 (3H,d,J=8.3Hz),2.5-3.5 (4H,m),3.6-4.1(4H,m),4.2-5.1(7H,m),5.2-5.6(1H,m) (solvent:$D_2O$) |
| 62a | 5-trifluoromethyl-1,3,4-thiadiazole-2-thiol (137.8mg) NaH(29.6mg) DMF(3ml) | 350 → 391 | 3400,3350, 1800,1770, 1730,1680, 1520,1325, 1300,1180, 1050 | 2.5-3.4(4H,m),3.8-5.2(3H, m),4.26(2H,s),6.90(1H,s), 7.2-7.6(10H,m),9.09(1H,d, J=8.3Hz) (solvent:$d_6$-DMSO) |

| | | | | |
|---|---|---|---|---|
| 62a | 0℃,4h<br>extraction with ethyl acetate, silica gel chromatography (hexane-$CH_2Cl_2$-ethyl acetate(1:1:1) | | | |
| 62b | $CF_3COOH$(0.48mℓ)<br>anisole(0.8mℓ)<br>$CH_2Cl_2$(4mℓ)<br>0℃,4h<br>CHP-20 column (10%ethanol)<br>lyophilization | 350 → 190 | 3380,1780, 1720,1650, 1540,1480, 1380,1325, 1190,1150, 1225 | 2.6-3.6(4H,m),4.49(2H,s), 4.3-4.6(1H,m),4.8-5.5(2H, m)<br>(solvent:$D_2O$) |
| 63a | 1-[ethoxy(hydroxy)phosphinyl methyl]-5-mercapto-1H-tetrazole O,S-disodium salt<br>(208mg)<br>DMF(3mℓ)<br>rt,4h<br>CHP-20 column (50%ethanol)<br>lyophilization | 350 → 460 | 3350,1765, 1730,1675, 1540,1450, 1390,1300, 1250,1180, 1050 | 0.9-1.2(3H,m),2.3-3.3(4H, m),3.4-4.2(4H,m),3.98(2H, s),4.2-4.8(3H,m),6.91(1H, s),7.2-7.6(10H,m),9.3-9.7 (1H,m)<br>(solvent:$d_6$-DMSO) |
| 63b | $CF_3COOH$(0.35mℓ)<br>anisole(0.6mℓ)<br>$CH_2Cl_2$(4mℓ)<br>0℃,2h<br>CHP-20 column (water)<br>lyophilization | 296 → 210 | 3420,1780, 1650,1540, 1440,1380, 1220,1190, 1040 | 1.48(3H,t,J=8.6Hz),2.4-3.5(4H,m),4.0-4.6(3H,m), 4.35(2H,s),5.2-5.5(1H,m)<br>(solvent:$D_2O$) |
| 64a | 5-(2-diethoxy phosphinylethyl thio)-1,3,4-thiadiazole-2-thiol<br>(219.4mg)<br>NaH(27.8mg) | 300 → 375 | 3360,1770, 1740,1680, 1550,1390, 1300,1240, 1180,1050 | 1.32(6H,t,J=8.3Hz),2.3-3.7(8H,m),3.9-4.4(6H,m), 3.95(2H,s),4.6-4.8(1H,m), 6.97(1H,s),7.2-7.5(10H, m),7.5-7.8(1H,m)<br>(solvent:$CDCl_3$) |

| | | | | |
|---|---|---|---|---|
| 64a | DMF(3mℓ) rt,3h→40℃,2h extraction with silica gel chromatography (ethyl acetate) | | | |
| 64b | $CF_3COOH$(0.37mℓ) anisole(0.62mℓ) $CH_2Cl_2$(2mℓ) 0℃,4h CHP-20 column (20%ethanol) lyophilization | 340 → 110 | 3440,1780, 1720,1650, 1540,1380, 1230,1190, 1030 | 1.54(3H,t,J=7Hz),2.4-3.9 (8H,m),4.2-4.6(6H,m),4.31 (2H,s),5.2-5.4(1H,m) (solvent:$D_2O$) |
| 65a | 1-dimethylamino -1H-tetrazole -5-thiol (110.5mg) NaH(30mg) DMF(3mℓ) rt,2h→40℃,1h extraction with ethyl acetate, silica gel chromatography (hexane-$CH_2Cl_2$-ethyl acetate=2:1:2) | 300 → 352 | 3360,1800, 1760,1685, 1530,1450, 1250,1180, 1050 | 2.5-3.4(4H,m),2.90(6H,s), 3.8-5.2(3H,m),4.15(2H,s), 6.91(1H,s),7.2-7.6(10H, m),9.05(1H,d,J=8.3Hz) (solvent:$d_6$-DMSO) |
| 65b | $CF_3COOH$(0.42mℓ) anisole(0.7mℓ) $CH_2Cl_2$(2mℓ) 0℃,3h CHP-20 column (10%ethanol) lyophilization | 289 → 125 | 3430,1790, 1730,1660, 1530,1450, 1390,1195, 1115,1030 | 2.6-3.5(4H,m),3.23(6H,s), 4.3-4.6(1H,m),4.40(2H,s), 4.8-5.5(2H,m) (solvent:$D_2O$) |
| 66a | 4,5-dimethyl-2-thiazolethiol (79mg) NaH(25.4mg) DMF(3mℓ) 0℃,1h→rt,1h | 300 → 291 | 3340,1770, 1680,1510, 1300,1260, 1180,1050 | 2.2-3.5(4H,m),2.29(6H,s), 3.73(2H,s),3.9-4.2(1H,m), 4.6-5.0(2H,m),6.97(1H,s), 7.2-7.5(10H,m),8.9-9.2 (1H,m) (solvent:$CDCl_3$) |

| | | | | |
|---|---|---|---|---|
| 66a | extraction with ethyl acetate, silica gel chromatography (hexane-$CH_2Cl_2$-ethyl acetate=2:1:2) | | | |
| 66b | $CF_3COOH$(0.36ml) anisole(0.6ml) $CH_2Cl_2$(3ml) 0℃,4h CHP-20 column (10%ethanol) lyophilization | 240 → 173 | 3350,1780, 1720,1650, 1500,1380, 1190,1115, 1035 | 2.26(3H,s),2.45(2H,s), 2.6-3.5(4H,m),4.14(2H,s), 4.25-4.55(1H,m),4.8-5.4 (2H,m) (solvent:$D_2O$) |
| 67a | 4,5-dimethyl-2-oxazolethiol (90.1mg) NaH(25.4mg) DMF(3ml) rt,1.5h extraction with ethyl acetate, silica gel chromatography (hexane-$CH_2Cl_2$-ethyl acetate=2:1:2) | 300 → 285 | 3340,1765, 1680,1500, 1450,1300, 1180,1050 | 2.04(3H,d,J=2Hz),2.20 (3H,s),2.2-3.4(4H,m), 3.69(2H,s),3.9-4.2(1H,m), 4.5-5.0(2H,m),6.99(1H, s),7.2-7.5(10H,m),8.6-8.9(1H,m) (solvent:$CDCl_3$) |
| 67b | $CF_3COOH$(0.36ml) anisole(0.6ml) $CH_2Cl_2$(3ml) 0℃,4h CHP-20 column (10%ethanol) lyophilization | 240 → 173 | 3350,1780, 1720,1650, 1500,1380, 1190,1115, 1035 | 2.26(3H,s),2.45(2H,s), 2.6-3.5(4H,m),4.14(2H,s), 4.3-4.6(1H,m),4.8-5.4 (2H,m) (solvent:$D_2O$) |
| 68a | 5-methoxymethyl-1,3,4-thiadiazole-2-thiol (113.2mg) NaH(25.4mg) NaI(10mg) | 300 → 276 | 3325,1810, 1770,1765, 1680,1530, 1180,1070, 1055 | 2.4-3.5(4H,m),3.21(3H,s), 4.14(2H,s),3.9-4.9(3H,m), 4.78(2H,s),6.94(1H,s), 7.2-7.5(10H,m),8.9-9.1 (1H,m) (solvent:$CDCl_3$+$d_6$-DMSO) |

| | | | | |
|---|---|---|---|---|
| 68a | DMF(4mℓ)<br>rt,2h<br>extraction with ethyl acetate, silica gel chromatography ($CH_2Cl_2$-ethyl acetate=3:1) | | | |
| 68b | $CF_3COOH$(0.33mℓ)<br>anisole(0.56mℓ)<br>$CH_2Cl_2$(3mℓ)<br>0℃,5h<br>CHP-20 column (10%ethanol)<br>lyophilization | 222<br>→<br>81 | 3400,1780,<br>1720,1650,<br>1530,1370,<br>1190,1110,<br>1070,1030 | 2.6-3.5(4H,m),3.70(3H,s),<br>4.36(2H,s),4.3-4.6(1H,m),<br>4.8-5.5(2H,m),5.13(2H,s)<br>(solvent:$D_2O$) |
| 69a | 5-methylsulfonylmethyl-1,3,4-thiadiazole-2-thiol(146.7mg)<br>NaH(25.4mg)<br>NaI(10mg)<br>DMF(4mℓ)<br>rt, 2 h<br>extraction with ethyl acetate, silica gel chromatography ($CH_2Cl_2$-ethyl acetate =3:1) | 300<br>→<br>330 | 3360,1770,<br>1735,1685,<br>1540,1310,<br>1190,1060 | 2.4-3.3(4H,m),2.09(3H,s),<br>3.9-4.3(1H,m),4.19(2H,s),<br>4.4-5.3(2H,m),5.18(2H,s),<br>6.91(1H,s),7.2-7.6(10H,m),9.05(1H,d,J=8.3Hz)<br>(solvent:$d_6$-DMSO) |
| 69b | $CF_3COOH$(0.36mℓ)<br>anisole(0.6mℓ)<br>$CH_2Cl_2$(3mℓ)<br>0℃,4h<br>CHP-20 column (10%ethanol)<br>lyophilization | 274<br>→<br>115 | 3400,1780,<br>1720,1650,<br>1530,1370,<br>1300,1190,<br>1140,1040 | 2.6-3.6(4H,m),3.46(3H,s),<br>4.3-4.6(1H,m),4.40(2H,s),<br>4.3-4.6(1H,m),4.8-5.5<br>(2H,m),5.39(2H,s)<br>(solvent:$D_2O$) |
| 70a | 4-ethyl-4H-1,2,4-triazole-3-thiol(90.1mg)<br>NaH(25.4mg) | 300<br>→<br>304 | 3380,3320,<br>1765,1720,<br>1670,1510,<br>1450,1360, | 1.34(3H,t,J=7Hz),2.3-3.4<br>(4H,m),3.88(2H,s),3.8-4.3<br>(3H,m),4.4-5.2(2H,m),6.92<br>(1H,s),7.2-7.6(10H,m), |

| | | | | |
|---|---|---|---|---|
| 70a | NaI(10mg)<br>DMF(1mℓ)<br>rt,1h<br>extraction with ethyl acetate, silica gel chromatography<br>($CH_2Cl_2$-ethyl acetate=1:1) | | 1260,1180, 1050 | 8.47(1H,s),8.86(1H,d,J=8.3Hz)<br>(solvent:$d_6$-DMSO) |
| 70b | $CF_3COOH$(0.37mℓ)<br>anisole(0.63mℓ)<br>$CH_2Cl_2$(3mℓ)<br>0℃,3h<br>CHP-20 column<br>(10%ethanol)<br>lyophilization | 250<br>→<br>142 | 3440,1790, 1730,1660, 1520,1380, 1270,1190, 1120,1030 | 1.67(3H,t,J=7Hz),2.6-3.6 (4H,m),4.10(2H,s),4.2-4.6 (3H,m),4.7-5.4(2H,m)<br>(solvent:$D_2O$) |
| 71a | 2-mercapto-1,3,4-thiadiazol-5-ylacetic acid sodium salt<br>(123mg)<br>NaH(50.8mg)<br>DMF(1mℓ)<br>rt,1h<br>CHP-20 column<br>(50%ethanol)<br>lyophilization | 300<br>→<br>265 | 3380,1765, 1720,1670, 1600,1370, 1185,1055 | 2.3-3.2(4H,m),3.59(2H,s), 4.10(2H,s),4.4-5.2(2H,m), 6.89(1H,s),7.2-7.5(10H,m),9.0-9.2(1H,m)<br>(solvent:$d_6$-DMSO) |
| 71b | $CF_3COOH$(0.3mℓ)<br>anisole(0.5mℓ)<br>$CH_2Cl_2$(3mℓ)<br>0℃,5h<br>CHP-20 column<br>(water)<br>lyophilization | 230<br>→<br>117 | 3420,1780, 1660,1600, 1370,1195, 1120,1025 | 2.6-3.6(4H,m),4.23(2H,s), 4.34(2H,s),4.2-4.6(1H,m), 4.8-5.5(2H,m)<br>(solvent:$D_2O$) |
| 72a | 1-(2-hydroxyethyl)-1H-tetrazole-5-thiol<br>(92.7mg)<br>NaH(25.4mg)<br>DMF(1mℓ) | 300<br>→<br>263 | 3340,1795, 1765,1725, 1670,1520, 1450,1295, 1260,1180, 1050 | 2.5-3.4(4H,m),3.7-4.9(6H,m),4.9-5.2(1H,m),6.91(1H,s),7.2-7.5(10H,m),8.99 (1H,d,J=7Hz)<br>(solvent:$d_6$-DMSO) |

| | | | | |
|---|---|---|---|---|
| 72a | rt, 3h<br>extraction with ethyl acetate, crystallization | | | |
| 72b | $CF_3COOH$(0.31mℓ)<br>anisole(0.52mℓ)<br>$CH_2Cl_2$(2mℓ)<br>0℃, 2.5h<br>CHP-20 column (5% ethanol)<br>lyophilization | 215<br>→<br>104 | 3410, 1780, 1720, 1660, 1540, 1380, 1195, 1120, 1030 | 2.6-3.5(4H, m), 4.2-4.6(3H, m), 3.37(2H, s), 4.7-5.4(4H, m)<br>(solvent: $D_2O$) |
| 73a | 1-(3-dimethyl aminopropyl)-1H-tetrazole-5-thiol(118.8mg)<br>NaH(25.4mg)<br>DMF(1mℓ)<br>rt, 2h<br>extraction with ethyl acetate, crystallization | 300<br>→<br>350 | 3340, 1800, 1765, 1730, 1680, 1520, 1450, 1300, 1260, 1180, 1100, 1050 | 1.8-3.7(9H, m), 2.14(6H, s), 3.8-5.2(5H, m), 6.90(1H, s), 7.2-7.5(10H, m), 9.01(1H, d, J=7Hz)<br>(solvent: $d_6$-DMSO) |
| 73b | $CF_3COOH$(0.38mℓ)<br>anisole(0.63mℓ)<br>$CH_2Cl_2$(2mℓ)<br>0℃, 4h<br>CHP-20 column (20% ethanol)<br>lyophilization | 279<br>→<br>147 | 3420, 1780, 1730, 1640, 1540, 1465, 1370, 1195, 1110, 1030 | 2.4-3.7(8H, m), 3.14(6H, s), 4.37(2H, s), 4.3-4.6(1H, m), 4.7-5.4(4H, m)<br>(solvent: $D_2O$) |
| 74a | 2-benzyloxycarbonylamino-3-sulfamoylpropionic acid (270.2mg)<br>DCC(206.3mg)<br>DMF(3mℓ)<br>rt, 3h<br>extraction with ethyl acetate, silica gel chromatography | 396.4<br>→<br>645 | 3370, 3070, 3050, 2950, 1805, 1760, 1730, 1690, 1520, 1450, 1340, 1260, 1180, 1050 | 2.2-3.2(4H, m), 3.4-3.7(2H, m), 3.9-5.0(4H, m), 5.3-5.7(2H, m), 6.2-6.5(1H, m), 6.94(1H, s), 7.1-7.5(10H, m), 7.5-7.8(1H, m)<br>(solvent: $CDCl_3$) |

| | | | | |
|---|---|---|---|---|
| 74a | (hexane-ethyl acetate=1:3) | | | |
| 74b | $H_2$/10%Pd-black (300mg) THF(3mℓ)-water (1 mℓ) 0℃, 3h CHP-20 column (water) lyophilization | 200 → 34 | 3240, 1780, 1695, 1645, 1560, 1380, 1330, 1195, 1155, 1030 | 2.6-3.4(4H,m), 4.16(2H,s), 3.9-4.2(2H,m), 4.4-5.5 (4H,m) (solvent: $D_2O$) |
| 75a | 4-cyano-3-hydroxy-5-isothiazolyl-acetic acid (163mg) DCC(155.9mg) DMF(2mℓ) rt, 5h extraction with thyl acetate, silica gel chromatography (ethyl acetate) | 300 → 270 | 3330, 2120, 1800, 1765, 1740, 1650, 1520, 1495, 1390, 1300, 1260, 1180, 1050 | 2.5-3.3(4H,m), 4.04(2H,s), 3.9-4.3(1H,m), 4.5-5.2(2H,m), 6.91(1H,s), 7.2-7.7 (10H,m), 9.09(1H,d,J=8.3Hz) (solvent: $d_6$-DMSO) |
| 75b | $CF_3COOH$(0.32mℓ) anisole(0.54mℓ) $CH_2Cl_2$(3mℓ) CHP-20 column (water) lyophilization | 235 → 105 | 2120, 1780, 1720, 1665, 1645, 1520, 1460, 1380, 1190, 1120, 1030 | 2.6-3.5(4H,m), 4.18(2H,s), 4.3-4.6(1H,m), 5.2-5.5 (1H,m) (solvent: $D_2O$) |
| 76a | 2-(2-chloroacetoamide-4-thiazolyl)-2-oxoacetic acid(273mg) DCC(206.3mg) DMF(2mℓ) rt, 1h extraction with ethyl acetate, silica gel chromatography (hexane-ethyl | 396.4 → 372 | 3340, 1810, 1760, 1630, 1550, 1270, 1180, 1050 | 2.3-3.5(4H,m), 4.1-5.2(3H,m), 4.36(2H,s), 6.96(1H,s), 7.2-7.5(10H,m), 8.78(1H,s), 8.90(1H,d,J=8.3Hz) (solvent: $CDCl_3$ + $d_6$-DMSO) |

| | | | | |
|---|---|---|---|---|
| 76a | acetate=1:2) | | | |
| 76b | $CH_3NHCS_2Na$ (64.9mg) DMF(2mℓ)−water (1mℓ) rt,5h extraction with ethyl acetate, silica gel chromatography (ethyl acetate) | 314.9 → 180 | 3420,3325, 3140,1800, 1750,1660, 1520,1480, 1380,1260, 1175,1045 | 2.2-3.5(4H,m),4.0-5.2(3H, m),5.8-6.3(2H,m),6.99 (1H,s),7.2-7.5(10H,m), 8.01(1H,s),8.1-8.5(1H,m), (solvent: $CDCl_3$) |
| 76c | $CF_3COOH$(0.23mℓ) anisole(0.39mℓ) $CH_2Cl_2$(4mℓ) 0℃,3h CHP-20 column (water) lyophilization | 155 → 71 | 3270,1780, 1720,1650, 1520,1480, 1370,1190, 1110,1020 | 2.6-3.5(4H,m),4.3-4.7(1H, m),4.8-5.1(1H,m),5.3-5.6 (1H,m),8.55(1H,s) (solvent: $D_2O$) |
| 77a | 2-(2-chloroacetamide-4-thiazolyl-(Z)-2-isopropoxyiminoacetic acid (305.74mg) DCC(206.3mg) DMF(2mℓ) rt,1h extraction with ethyl acetate, silica gel chromatography (hexane-ethyl acetate=1:1) | 396.4 → 462 | 3280,1800, 1750,1675, 1540,1450, 1365,1260, 1175,1045 | 1.28(6H,d,J=6.6Hz),2.2-3.4(4H,m),4.20(2h,s),4.0-5.0(4H,m),6.96(1H,d,J=3Hz),7.1-7.5(10H,m),7.40 (1H,d,J=2Hz) (solvent: $CDCl_3$) |
| 77b | $CH_3NHCS_2Na$ (72.5mg) DMF(2mℓ)−water (1mℓ) rt,9h extraction with ethyl acetate silica gel chro- | 355 → 342 | 3400,1810, 1740,1670, 1630,1530, 1295,1180, 1120,1050 | 1.30(6H,d,J=6.7Hz),2.2-3.5(4H,m),4.0-5.0(4H,m), 5.2-5.4(2H,m),6.94(1H,s), 6.98(1H,d,J=3Hz),7.2-7.5 (10H,m) (solvent: $CDCl_3$) |

| | | | | |
|---|---|---|---|---|
| 77b | matography (hexane-ethyl acetate=1:2) | | | |
| 77c | $CF_3COOH$(0.42mℓ) anisole(0.71mℓ) $CH_2Cl_2$(2mℓ) 0℃,5h CHP-20 column (10%ethanol) lyophilization | 314 → 75 | 3430,1780, 1730,1660, 1530,1380, 1200,1105 | 1.46(6H,d,J=6.6Hz), 2.6-3.4(4H,m),4.4-5.2 (3H,m),5.3-5.6(1H,m), 7.24(1H,s) (solvent:$D_2O$) |
| 78a | trifluoroacetic anhydride (159mg) $CH_2Cl_2$(15mℓ) 0℃,15min extraction with ethyl acetate, silica gel chromatography ($CH_2Cl_2$-ethyl acetate=1:1) | 300 → 220 | 3440,1765, 1715,1540, 1445,1295, 1170,1050 | 2.4-3.3(4H,m),4.0-5.3(3H, m),6.90(1H,s),7.2-7.6 (10H,m) (solvent:$d_6$-DMSO) |
| 78b | $H_2$/10%Pd-C (200mg) $NaHCO_3$(32mg) THF(3mℓ)-water (1 mℓ) rt,20min CHP-20 column (water) lyophilization | 189 → 130 | 3420,1780, 1720,1650, 1560,1380, 1190,1160, 1120,1030 | 2.6-3.6(4H,m),4.4-4.6(1H, m),4.9-5.1(1H,m),5.3-5.6 (1H,m) (solvent:$D_2O$) |
| 79a | 2-(2-chloroacetamide-4-thiazolyl)-(Z)-2-[1-methyl-1-(4-nitrobenzyloxycarbonyl)-ethoxyimino]-acetic acid (533mg) HOBT(150mg) DCC(230mg) | 396 → 683 | 3320,1800, 1760,1690, 1520,1350, 1180,1050 | 1.61(6H,s),2.20-3.33(4H, m),3.91-4.18(1.5H,m),4.21 (2H,s),4.59-4.92(1.5H,m), 5.20(2H,s),6.97(1H,s), 7.19(1H,s),7.20-7.59(12H, m),7.92-8.11(2H,m) (solvent:$CDCl_3$) |

| | | | | |
|---|---|---|---|---|
| 79a | DMF(3mℓ)<br>rt,1h<br>extraction with ethyl acetate, silica gel chromatography (hexane-ethyl acetate=3:4) | | | |
| 79b | $CH_3NHCS_2Na$ (206mg)<br>THF(18mℓ)－water (16mℓ)<br>rt,90min<br>extraction with ethyl acetate, silica gel chromatography (hexane-ethyl acetate=2:1 →ethyl acetate) | 683 → 474 | 3420,3350, 1800,1760, 1690,1520, 1350,1180, 1050 | 1.61(6H,s),2.28-3.32(4H, m),3.97-4.36(1.5H,m),4.71-4.98(1.5H,m),5.26(2H,s), 6.41(2H,bs),6.77(1H,s), 7.01(1H,s),7.21-7.51(12H, m),7.90-8.07(2H,m) (solvent:$CDCl_3$) |
| 79c | $CF_3COOH$(0.64mℓ)<br>anisole(0.48mℓ)<br>$CH_2Cl_2$(28mℓ)<br>－10° ～－15℃, 5h<br>XAD-2 column (30%ethanol)<br>lyophilization | 474 → 213 | 3420,1780, 1740,1660, 1530,1350, 1180,1150, 1050, 980, 910 | 1.81(6H,s),2.57-3.42(4H, m);4.38-4.70(1H,m),4.81-5.11(1H,m),5.29-5.51(1H, m),5.52(2H,s),7.19(1H,s), 7.73(2H,d,J=8Hz),8.28 (2H,d,J=8Hz) (solvent:$D_2O$) |
| 79d | $H_2$/10%Pd-C (183mg)<br>$NaHCO_3$(26mg)<br>ethyl acetate(8mℓ)－water(8mℓ)<br>0℃,90min<br>HP-20 column (water)<br>lyophilization | 183 → 111 | 3400,1780, 1730,1650, 1580,1190, 1040, 980, 910 | 1.71(6H,s),2.57-3.51(4H, m),4.32-4.71(1H,m),4.91-5.17(1H,m),5.31-5.59(1H, m),7.27(1H,s) (solvent:$D_2O$) |
| 80a | 2-(2-tritylamino-4-thiazolyl)-(Z)-2-(trityl | 200 → 488 | 3890,1800, 1740,1680, 1520,1490, | 2.31-3.35(4H,m),3.93-4.26 (1.5H,m),4.67-4.95(1.5H, m),6.52(1H,s),6.89(1H,s), |

| | | | | |
|---|---|---|---|---|
| 80a | oxyimino)acetic acid 2-benzo-thiazolylthiol ester(497mg) THF(4mℓ) DMF(2mℓ) rt,16h extraction with ethyl acetate, silica gel chro-matography (hexane-ethyl acetate=2:1 →3:2) | | 1440,1190, 1050 | 6.98(1H,s),7.11-7.42(40H, m) (solvent:$CDCl_3$) |
| 80b | HCOOH(3mℓ) $CH_2Cl_2$(1 mℓ) 0℃→rt,1h HP-20 column (water) lyophilization | 488 → 48 | 3400,1780, 1720,1650, 1530,1390, 1190,1030 | 2.61-3.53(4H,m),4.31-4.68 (1H,m),4.83-5.12(1H,m), 5.31-5.62(1H,m),7.20(1H, s) (solvent:$D_2O$) |
| 81a | isobutyl chloro-carbonate(82mg) $Et_3N$(60.6mg) THF(6mℓ) DMF(2mℓ) −10℃,60min→ rt,30min extraction with ethyl acetate, silica gel chro-matography (hexane-ethyl acetate=3:2) | 200 → 150 | 3350,1770, 1730,1530, 1180,1050 | 0.91(6H,d,J=6Hz),1.81-2.13(1H,m),2.34-3.39(4H, m),3.87(2H,d,J=6Hz), 3.86-4.13(1.5H,m),4.43-4.85(1.5H,m),6.98(1H,s), 7.21-7.43(10H,m) (solvent:$CDCl_3$) |
| 81b | $H_2$/5%Pd-C (150mg) THF−water $NaHCO_3$(25mg ) 0℃,60min XAD-2 column (water) | 150 → 17 | 3400,1780, 1720,1650, 1390,1190, 1030 | 1.11(6H,d,J=6Hz),1.91-2.37(1H,m),2.61-3.49(4H, m),4.13(2H,d,J=6Hz), 4.31-4.59(1H,m),4.80-5.02 (1H,m),5.11-5.42(1H,m) (solvent:$D_2O$) |

| | | | | |
|---|---|---|---|---|
| 81b | lyophilization | | | |
| 82 | methyl chloro-carbonate(196 μℓ) $Et_3N$(357 μℓ) N-(4-nitrobenzyl-oxycarbonyl)-glycine (645mg) THF(50mℓ) −10° ∼ −5℃, 60min extraction with ethyl acetate, after concentration, the product was powdered with ethyl acetate | 1000 → 1470 | 1805, 1775, 1740, 1700, 1610 | 2.1-3.3(4H,m), 3.6-4.2(3H,m), 4.3-5.3(2H,m), 5.17(2H,s), 5.73(1H,m), 6.93(1H,s), 6.87, 7.11(each 0.5H,d,J=7Hz), 7.2-7.5(10H,m), 7.45(2H,d,J=9Hz), 8.16(2H,d,J=9Hz) (solvent: $CDCl_3$) |
| 83 | methyl chloro-carbonate(98 μℓ) $Et_3N$(179 μℓ) N-(2,2,2-trichloroethoxycarbonyl-)phenylglycine (414mg) THF(25mℓ) −10℃ ∼ −5℃, 60min silica gel chromatography (hexane-ethyl acetate=1:1) | 500 → 250 | 1810, 1750, 1700, 1500 | 2.1-3.4(4H,m), 3.6-5.2(3H,m), 4.68(2H,s), 5.24, 5.31(each 0.5H,s), 6.25(1H,m), 6.50(0.5H,m), 6.73(0.5H,d,J=5Hz), 6.97(1H,s), 7.2-7.5(10H,m) (solvent: $CDCl_3$) |
| 84a | N-benzyloxy-carbonyl-D-phenylglycine (723mg) HOBT(350mg) DCC(530mg) DMF(10mℓ) 0℃, 40min extraction with | 1000 → 1170 | 3350, 1810, 1775, 1735, 1700, 1500 | 2.1-3.4(4H,m), 3.7-4.0(1H,m), 4.4-5.0(2H,m), 5.04(2H,s), 5.25(1H,d,J=7Hz), 5.90(1H,d,J=7Hz), 6.4-6.8(1H,m), 6.95(1H,s), 7.2-7.5(20H,m) (solvent: $CDCl_3$) |

| | | | | |
|---|---|---|---|---|
| 84a | ethyl acetate, silica gel chromatography (hexane-ethyl acetate=1:1) | | | |
| 84b | $H_2$/10%Pd-C (500mg) THF(30mℓ)-pH7.0 buffer(20mℓ) 0℃,40min HP-20 column (50%methanol) lyophilization | 1000 → 250 | 3400,1795, 1730,1670, 1500,1390 | 2.3-3.4(4H,m),3.9-5.3(3H,m),5.15(2H,s),5.29(1H,s),7.46(10H,m) (solvent: $D_2O$) |
| 85a | N-(4-methoxy-benzyloxycarbonyl)-L-phenylglycine (800mg) HOBT(350mg) DCC(528mg) DMF(10mℓ) 0℃,30min extraction with ethyl acetate, silica gel chromatography (hexane-ethyl acetate=1:1) | 1000 → 1100 | 3400,1815, 1760,1735, 1700,1520 | 2.2-3.3(4H,m),3.77(3H,s),3.8-4.2(1H,m),4.4-4.9(2H,m),4.99(2H,s),5.21(1H,d,J=7Hz),5.87(1H,d,J=7Hz),6.54(1H,m),6.84(2H,d,J=8Hz),6.94(1H,s),7.1-7.4(17H,m) (solvent: $CDCl_3$) |
| 85b | $CF_3COOH$(1.6mℓ) anisole(752μℓ) $CH_2Cl_2$(40mℓ) -10℃,6.5h HP-20 column (30%methanol) lyophilization | 800 → 315 | 3400,1790, 1740,1670 | 2.3-3.3(4H,m),4.0-5.2(3H,m),5.25(1H,s),7.4-7.7(5H,m) (solvent: $D_2O$) |
| 86a | N-(4-methoxy-benzyloxycarbonyl)-D-phenyl-glycine (1200mg) HOBT(525mg) | 1500 → 1770 | 1815,1765, 1740,1705 | 2.1-3.4(4H,m),3.78(3H,s),3.85(1H,m),4.4-5.1(2H,m),4.98(2H,s),5.25(1H,d,J=7Hz),5.88(1H,d,J=7Hz),6.5-6.9(3H,m),6.94(1H,s),7.1-7.5(17H,m) |

| | | | | |
|---|---|---|---|---|
| 86a | DCC(792mg) DMF(15mℓ) 0℃,30min extraction with ethyl acetate, silica gel chromatography (hexane-ethyl acetate=1:1) | | | (solvent: $CDCl_3$) |
| 86b | $CF_3COOH$(2mℓ) anisole(940μℓ) $CH_2Cl_2$(50mℓ) −10℃,6.5h HP-20 column (20%methanol→ 50%methanol) lyophilization | 1000 → 410 | 1790,1730, 1700,1665 | 2.3-3.3(4H,m),3.9-5.3(3H, m),5.25(1H,s),7.4-7.7(5H, m) (solvent: $D_2O$) |
| 87a | S-mandelic acid (387mg) HOBT(350mg) DCC(530mg) DMF(10mℓ) 0℃,1h extraction with ethyl acetate, silica gel chromatography (hexane-ethyl acetate=1:2) | 1000 → 628 | 3400,1805, 1775,1690 | 2.1-3.5(4H,m),3.5-5.2(4H, m),5.10(1H,s),6.95(1H,s), 7.2-7.6(16H,m) (solvent: $CDCl_3$) |
| 87b | $H_2$/10%Pd-C (450mg) THF(12mℓ)-pH7.0buffer(8mℓ) rt,30min HP-20 column (20%methanol) lyophilization | 450 → 230 | 3400,1785, 1730,1670 | 2.3-3.3(4H,m),4.28(1H,t, J=9Hz),4.5-5.3(2H,m), 5.29(1H,s),7.50(5H,s) (solvent: $D_2O$) |
| 88a | R-mandelic acid (387mg) HOBT(350mg) | 1000 → 485 | 3400,1810, 1770,1680 | 2.1-3.5(4H,m),3.50(1H, br),4.03(1H,m),4.60(2H, m),5.07(1H,s),6.96(1H,s), |

| | | | | |
|---|---|---|---|---|
| 88a | DCC(530mg)<br>DMF(10mℓ)<br>0℃,1h<br>extraction with ethyl acetate, silica gel chromatography (hexane-ethyl acetate=2:3) | | | 7.05(1H,m),7.2-7.5(15H,m)<br>(solvent:$CDCl_3$) |
| 88b | $H_2$/10%Pd-C (180mg)<br>THF(12mℓ)-pH7.0buffer(8mℓ)<br>rt,30min<br>HP-20 column(water-20%methanol)<br>lyophilization | 350<br>→<br>150 | 1785,1730,<br>1665 | 2.4-3.3(4H,m),4.23(1H,t, J=9Hz),4.5-5.3(2H,m), 5.25(1H,s),7.50(5H,s)<br>(solvent:$D_2O$) |
| 89a | 4-chloro-DL-mandelic acid(711mg)<br>HOBT(525mg)<br>DCC(792mg)<br>DMF(15mℓ)<br>0℃,30min<br>extraction with ethyl acetate, silica gel chromatography (hexane-ethyl acetate=1:1) | 1500<br>→<br>1410 | 3400,1810,<br>1775,1690 | 2.1-3.3(4H,m),3.8-4.3(2H, m),4.4-5.2(3H,m),6.96(1H, s),7.2-7.7(15H,m)<br>(solvent:$CDCl_3$) |
| 89b | $H_2$/10%Pd-C (500mg)<br>THF(30mℓ)-pH7.0 buffer(20mℓ)<br>rt,50min<br>HP-20 column (20%methanol→50%methanol)<br>lyophilization | 1000<br>→<br>330 | 3420,1780,<br>1730,1660 | 2.3-3.3(4H,m),4.1-5.3(3H, m),5.25,5.28(each 0.5H, s),7.49(4H,s)<br>(solvent:$D_2O$) |
| 90a | 4-hydroxy-DL-mandelic acid | 1500<br>→ | 3380,1805,<br>1775,1750, | 2.2-3.5(4H,m),3.9-5.2(4H, m),6.10(1H,m),6.70(2H,d, |

| | | | | |
|---|---|---|---|---|
| 90a | (711mg) HOBT(525mg) DCC(795mg) DMF(15mℓ) 0℃, 1h extraction with ethyl acetate, silica gel chromatography (hexane-ethyl acetate=1:2) | 898 | 1665 | J=8Hz), 6.92(1H, s), 7.1-7.6(12H, m), 8.60(1H, d, J=8 Hz), 9.33(1H, s) (solvent: $d_6$-DMSO) |
| 90b | $H_2$/10%Pd-C (350mg) THF(21mℓ)-pH7.0 buffer(14mℓ) rt, 40min SP-207 column (water→20% methanol) lyophilization | 700 → 354 | 3430, 1790, 1730, 1660 | 2.3-3.4(4H, m), 4.1-4.5(1H, m), 4.5-5.3(2H, m), 5.20, 5.22(each 0.5H, s), 6.97 (2H, d, J=8Hz), 7.38, 7.40 (each 1H, d, J=8Hz) (solvent: $D_2O$) |
| 91a | DL-lactic acid (222μℓ) HOBT(350mg) DCC(636mg) 0℃, 1.5h extraction with ethyl acetate, silica gel chromatography (hexane-ethyl) acetate=1:2) | 1000 → 597 | 3420, 1800, 1700, 1680 | 1.1-1.5(3H, m), 2.3-3.3 (4H, m), 3.9-5.2(4H, m), 5.39 (0.25H, d, J=5Hz), 5.63 (0.75H, m), 6.91(1H, s), 7.2-7.5(10H, m), 8.37(0.75H, d, J=7Hz), 8.67(0.25H, d, J=6Hz) (solvent: $d_6$-DMSO) |
| 91b | $H_2$/10%Pd-C (300mg) THF(21mℓ) pH7.0 buffer (14mℓ) rt, 40min SP-207 column (water) lyophilization | 700 → 292 | 3430, 1795, 1730, 1660 | 1.40(3H, d, J=7Hz), 2.3-3.4 (4H, m), 4.2-4.5(2H, m), 4.6-5.3(2H, m) (solvent: $D_2O$) |

| | | | | |
|---|---|---|---|---|
| 92a | DL-phenylmalonic acid monodi-phenylmethyl ester(880mg) HOBT(350mg) DCC(530mg) DMF(13mℓ) 0℃,30min extraction with ethyl acetate, silica gel chromatography (hexane-ethyl acetate=1:1) | 1000 → 570 | 3370,1810, 1760,1700, 1530,1500, 1455,1270, 1185 | 2.1-3.0(3H,m),3.0-3.5 (1H,m),3.7-4.2(1H,m),4.5-4.9(2H,m),4.64(1H,s),6.87 (1H,s),6.97(1H,s),6.9-7.8 (26H,m) (solvent: $CDCl_3$) |
| 92b | $H_2$/10%Pd-C (400mg) THF(12mℓ) pH7.0buffer(8mℓ) rt,30min HP-20 column (water) lyophilization | 400 → 153 | 1790,1730, 1670,1615 | 2.4-3.4(4H,m),4.1-5.3 (3H,m),4.60(1H,s),7.41 (5H,s) (solvent: $D_2O$) |
| 93a | benzyloxy-carbonyl chloride (360μℓ) DMA(1.0mℓ) $CH_2Cl_2$(40mℓ) rt,30min washed with 2% $NaHCO_3$ aqueoeus solution silica gel chromatography (hexane-ethyl acetate=1:4) | 1000 → 740 | 3360,1805, 1780,1730 | 2.1-3.5(4H,m),4.05(1H,m), 4.3-5.0(2H,m),5.10(2H,s), 5.28(1H,m),6.97(1H,s), 7.2-7.5(15H,m) (solvent: $CDCl_3$) |
| 93b | $CF_3COOH$(462μℓ) anisole(217μℓ) $CH_2Cl_2$(10mℓ) −10℃, 1 h HP-20 column | 265 → 127 | 1790,1730, 1670 | 2.3-3.4(4H,m),4.0-5.2(3H, m),5.18(2H,s),7.48(5H,s) (solvent: $D_2O$) |

| | | | | |
|---|---|---|---|---|
| 93b | (20% methanol) lyophilization | | | |
| 94a | pyruvic acid (177 μℓ) HOBT(350mg) DCC(530mg) DMF(10mℓ) 0℃, 30min extraction with ethyl acetate, silica gel chromatography (hexane-ethyl acetate = 1:1) | 1000 → 495 | 3350, 1810, 1775, 1700 | 2.37(3H,s), 2.3-3.3(4H,m), 4.0-5.1(3H,m), 6.92(1H,s), 7.2-7.5(10H,m), 9.22(1H, d, J=8Hz) (solvent: $d_6$-DMSO) |
| 94b | $H_2$/10% Pd-C (350mg) THF(12mℓ)-pH7.0 buffer(8mℓ) rt, 20min SP-207 column (water→20% methanol) lyophilization | 700 → 300 | 1780, 1730, 1660 | 1.59, 2.49(total 3H,s), 2.4-3.3(4H,m), 4.30(1H,m), 4.76(1H,m), 5.11(1H,m) (solvent: $D_2O$) |
| 95 | chloromethyl pivalate(182 μℓ) DMF(1.0mℓ) 50℃, 4h extraction with ethyl acetate, after concentrated, the product was powdered with petroleum benzine | (88b) 250 → 219 | 3400, 1810, 1760, 1680 | 1.18(9H,s), 2.2-3.3(4H,m), 4.10(1H,m), 4.5-5.2(2H,m), 5.09(1H,s), 5.83(2H,s), 7.1-7.6(6H,m) (solvent: $CDCl_3$) |
| 96 | 4-nitrobenzaldehyde(30mg) Molecular Sieves 4A $CH_2Cl_2$(5mℓ) rt, 8h filtration conce- | 80 → 46 | 3420, 1800, 1765, 1730, 1640, 1600, 1520, 1350 | 2.2-3.3(4H,m), 4.4-4.8(3H, m), 7.00(1H,s), 7.3-7.4 (10H,m), 7.92(2H,d,J=8Hz), 8.28(2H,d,J=8Hz), 8.47(0.5H,s), 8.50(0.5H,s) (solvent: $CDCl_3$) |

| | | | | |
|---|---|---|---|---|
| 96 | ntration,drying powdered with ether | | | |
| 97a | 3-chloro-6-pyridazinethiol sodium salt(143mg) NaI(100mg) DMF(4mℓ) rt,30min extraction with ethyl acetate, silica gel chromatography (hexane-ethyl acetate=3:7) | 200 → 180 | 3350,1770, 1740,1680, 1390,1180, 1140,1050 | 2.20-3.38(4H,m),3.94(2H, s),3.83-4.25(1.5H,m),4.56-4.94(1.5H,m),6.96(1H,s), 7.20-7.71(12H,m) (solvent:$CDCl_3$) |
| 97b | $CF_3COOH$(0.3mℓ) anisole (0.24mℓ) $CH_2Cl_2$(15mℓ) -10° ~-15℃, 4h XAD-2 column (10%ethanol) lyophilization | 180 → 86 | 3400,1780, 1720,1650, 1390,1150, 1030 | (100MHz) 2.41-3.39(4H,m),4.03-4.30 (1H,m),4.11(2H,s),4.49-4.78(1H,m),4.90-5.20(1H, m),7.65-7.72(2H,m) (solvent:$D_2O$) |
| 98a | phenylthioacetic acid(118mg) HOBT(94mg) DCC(144mg) DMF(3mℓ) rt,1h extraction with ethyl acetate, silica gel chromatography (hexane-ethyl acetate=1:3) | 200 → 106 | 3350,1770, 1730,1670, 1510,1180, 1050 | 2.21-3.37(4H,m),3.61(2H, s),3.72-4.09(1.5H,m), 4.48-5.03(1.5H,m),6.91 (1H,s),7.17-7.71(15H,m) (solvent:$CDCl_3$) |
| 98b | $CF_3COOH$(0.2mℓ) anisole (0.16mℓ) $CH_2Cl_2$(10mℓ) | 105 → 41 | 3400,1780, 1720,1650, 1390,1190, 1030 | (100MHz) 2.41-3.29(4H,m),3.73(2H, s),3.84-4.05(1H,m),4.40-4.73(1H,m),4.87-5.13(1H, |

| | | | | |
|---|---|---|---|---|
| 98b | −10° ～−15℃, 4h XAD-2 column (10% ethanol) lyophilization | | | m), 7.32-7.58(5H, m) (solvent: $D_2O$) |
| 99a | potassiu methyl xanthate (156mg) DMF(2ml) rt, 1h extraction with ethyl acetate, silica gel chromatography (hexane−ethyl acetate=1:1) | 250 → 174 | 3350, 1770, 1740, 1680, 1180, 1050 | 1.25(3H, t, J=7Hz), 2.21-3.59(4H, m), 3.88(2H, s), 3.92-4.32(3.5H, m), 4.51-5.02(1.5H, m), 7.01(1H, s), 7.29-7.58(10H, m) (solvent: $CDCl_3$) |
| 99b | $CF_3COOH$(0.3ml) anisole (0.24ml) $CH_2Cl_2$(10ml) −10° ～−15℃, 4h XAD-2 column (10% ethanol) lyophilization | 174 → 72 | 3400, 1780, 1720, 1650, 1390, 1240, 1190, 1050 | (100MHz) 1.39(3H, t, J=7Hz), 2.40-3.29(4H, m), 3.98(2H, s), 4.12-4.34(1H, m), 4.49-4.78 (1H, m), 4.90-5.21(1H, m) ·(solvent: $D_2O$) |

Example 100

Production of sodium 2-{(4S)-4-[2-(2-amino-4-thiazolyl)-(Z)-2-(ethoxyimino)acetamido]-3-oxo-2-isoxazolidinyl} -5-oxo-2-tetrahydrofurancarboxylate [Compound (100)]:

(a) Compound (R-3) was dissolved in a solvent mix of ethyl acetate anaphosphate buffer (pH 7.0), with 10% palladium-carbon added. The resulting solution was stirred in hydrogen stream under ice-cooling for 1.5 hours. After catalyst removal by filtration and water rinsing, the filtrate and the rinsing liquid were combined and the aqueous layer separated. Tetrahydrofuran was added to the aqueous layer, with sodium bicarbonate and 2-(2-chloroacetamido-4-thiazolyl)-(Z)-2-ethoxyiminoacetyl chloride hydrochloride added; the resulting solution was stirred under ice-cooling for 30 minutes. Tetrahydrofuran was vaporized under reduced pressure and the resulting aqueous layer rinsed with ethyl acetate.

(b) Tetrahydrofuran and sodium N-methyldithiocarbamate were next added to the aqueous layer; the resulting solution was stirred at room temperature for 30 minutes. Tetrahydrofuran was vaporized under reduced pressure. After rinsing with ethyl acetate, the resulting concentrate was subjected to column chromatography using HP-20. The fraction eluted with 10% ethanol, after concentration, was subjected to freeze-drying, yielding Compound(100).

Example 101

Production of sodium 2-{(4S)-4-[2-(2-amino-5-chloro-4-thiazolyl)-(Z)-2-(methoxyimino)acetamido]-3-oxo-2-isoxazolidinyl}-5-oxo-2-tetrahydrofurancarboxylate [Compound (101)]:

Compound (101) was obtained via reaction by a method analogous to Example 100, using Compound

(R-3) and 2-(5-chloro-2-chloroacetamido-4-thiazolyl)-(Z)-2-methoxyiminoacetyl chloride hydrochloride.

Example 102

Production of sodium 2-{(4S)-4-[2-4-ethyl-2,3-dioxo-1-piperazinocarboxamido) -2-thienylacetamido]-3-oxo-2-isoxazolidinyl}-5-oxo-2-tetrahydrofurancarboxylate [Compound (102b)]:

(a) Production of a diphenylmethyl ester of Compound (102b) [Compound (102a)]:

Compound (102a) was obtained via reaction by a method analogous to Example 1 (a), using Compound (R-3) and 2-(4-ethyl-2,3-dioxo-1-piperazinylcarboxamido)-2-thienylacetic acid.

(b) Production of Compound (102b):

Compound (102b) was obtained via reaction on Compound (102a) by a method analogous to Example 1 (c).

Example 103

Production of sodium 2-((4S)-4-ureido-3-oxo-2-isoxazolidinyl)-5-oxo-2-tetrahydrofurancarboxylate [Compound (103b)]:

(a) Production of a diphenylmethyl ester of Compound (103b) [Compound (103a)]:

An aqueous solution of potassium isocyanate was added to an acetic solution of Compound (R-3), the obtained solution being stirred at room temperature for 2 hours. The reaction solution obtained was added to water and eluted with ethyl acetate. The organic layer, after rinsing with water, an aqueous solution of sodium bicarbonate and saturated saline solution, was in turn dried ($Na_2SO_4$). After the solvent was vaporized off, the residue was refined by column chromatography using silica gel, yielding Compound (103a).

(b) Production of Compound (103b):

Compound (103b) was obtained via reaction with Compound (103a) by a method analogous to Example 2 (b).

Example 104

Production of sodium 2-((4S)-4-phenylureylene-3-oxo-2-isoxazolidinyl)-5-oxo-2-tetrahydrofurancarboxylate [Compound (104b)]:

(a) Production of a diphenylmethyl ester of Compound (104b) [Compound (104a)]:

Phenylisocyanate was added to a dichloromethane solution of Compound (R-3), and stirred at room temperature for 10 minutes. The solvent was then vaporized off. The residue was refined by column chromatography using silica gel, yielding Compound (104a).

(b) Production of Compound (104b):

Compound (104b) was obtained via reaction with Compound (104a) by a method analogous to Example 2 (b).

Example 105

Production of sodium 2-{(4S)-4-[2-(pyridyl)acetamido] -3-oxo-2-isoxazolidinyl}-5-oxo-2-tetrahydrofurancarboxylate [Compound (105)]:

Compound (105) was obtained by a method analogous to Example 100 (a), using Compound (R-3) and

2-pyridylacetyl chloride hydrochloride.

Example 106

Production of sodium 2-{(4S)-4-[2-(4-chlorophenyl) acetamido]-3-oxo-2-isoxazolidinyl}-5-oxo-2-tetrahydrofurancarboxylate [Compound (106)]:

Compound (106) was obtained by a method analogous to Example 100 (a), using Compound (R-3) and 4-chlorophenyl acetyl chloride.

Example 107

Production of sodium 2-{(4S)-4-[(2-chloro-2-phenyl) acetamido]-3-oxo-2-isoxazolidinyl}-5-oxo-2-tetrahydrofurancarboxylate [Compound (107)]:

Compound (107) was obtained by a method analogous to Example 100 (a), using Compound (R-3) and 2-chloro-2-phenylacetyl chloride.

Example 108

Production of sodium 2-{(4S)-4-[2-(3-pyridylthio) acetamido]-3-oxo-2-isoxazolidinyl}-5-oxo-2-tetrahydrofurancarboxylate [Compound (108b)]:

A diphenylmethyl ester of Compound (108b) [Compound (108a)] was first obtained by a method analogous to Example 15, using Compound (14a) and sodium 3-pyridinethiol, and then by a method analogous to Example 1 (c).

Example 109

Production of sodium 2-{(4S)-4-[2-(4-chlorophenylthio) acetamido]-3-oxo-2-isoxazolidinyl}-5-oxo-2-tetrahydrofurancarboxylate [Compound (109b)]:

Diphenylmethyl ester of Compound (109b) [Compound (109a)] was obtained by a method analogous to Example 15, using Compound (14a) and sodium 4-chlorophenylthiol, Compound (109b) was then obtained.

Example 110

Production of sodium 2-[(4S)-4-(2-isopropylphenoxycarbonylamino)-3-oxo-2-isoxazolidinyl]-5-oxo-2-tetrahydrofurancarboxylate [Compound (110b)]:

Compound (110a), a diphenylmethyl ester of Compound (110b), was obtained by a method analogous to Example 5, using Compound (R-3) and 2-isopropylphenoxycarbonyl chloride, Compound (110b) was then obtained by a method analogous to Example 2 (b).

Example 111

Production of sodium 2-((4S)-4-difluoroacetamido-3-oxo-2-isoxazolidinyl)-5-oxo-2-tetrahydrofurancarboxylate [Compound (111b)]:

A diphenylmethyl ester of Compound (111b) [Compound (111a)] was obtained by a method analogous to Example 1 (a),using Compound (R-3) and difluoroacetic acid; Compound (111b) was then obtained by a method analogous to Example 2 (b).

Example 112

Production of sodium 2-[(4S)-4-(3-chloro)propionamido-3-oxo-2-isoxazolidinyl]-5-oxo-2-tetrahydrofurancarboxylate [Compound (112)]:

Compound (112) was obtained by a method analogous to Example 100 (a), using Compound (R-3) and 3-chloropropionyl chloride.

Example 113

Production of pivaloyloxymethyl 2-((4S)-4-chloroacetamido-3-oxo-2-isoxazolidinyl)-5-oxo-2-tetrahydrofuran-carboxylate [Compound (113)]:

Compound (113) was obtained by a method analogous to Example 17, using Compound (14b) and chloromethyl pivalate.

Example 114

Production of pivaloyloxymethyl 2-[(4S)-4-(2-phenylthioacetamido)-3-oxo-2-isoxazolidinyl]-5-oxo-2-tetrahydrofurancarboxylate [Compound (114)]:

Compound (114) was obtained by a method analogous to Example 17, using Compound (98b) and chloromethyl pivalate.

Example 115

Production of sodium 2-((4S)-4-propionamido-3-oxo-2-isoxazolidinyl)-5-oxo-2-tetrahydrofurancarboxylate [Compound (115b)]:

A diphenylmethyl ester of Compound (115b) [Compound (115a)] was obtained by a method similar to that shown in Example 5, using Compound (R-3) and propyonyl chloride Compound (115b) was then obtained by a method analogous to Example 2 (b).

Example 116

Production of pivaloyloxymethyl 2-((4S)-4-benzyloxycarbonylamino-3-oxo-2-isoxazolidinyl)-5-oxo-2-tetrahydrofurancarboxylate [Compound (116)]:

Compound (116) was obtained by a method analogous to Example 17, using Compound (93b) and chloromethyl pivalate.

Example 117

Production of pivaloyloxymethyl 2-((4S)-4-bromoacetamido-3-oxo-2-isoxazolidinyl)-5-oxo-2-tetrahydrofuran-carboxylate [Compound (117)]:

Compound (116) was dissolved in ethyl acetate with 5% palladium-carbon added. The solution obtained was stirred in hydrogen stream under ice-cooling for 1 hour. After the catalyst was removed by filtration, DMA and bromoacetyl bromide were added and the solution stirred under ice-cooling for 0.5 hour. The organic layer, after rinsing with water, was dried ($Na_2SO_4$) to vaporize off the solvent. The residue was refined by column chromatography using silica gel, yielding Compound (117).

Example 118

Production of pivaloyloxymethyl 2-[(4S)-4-(2,4-hexadiene)amido-3-oxo-2-isoxazolidinyl]-5-oxo-2-tetrahydrofurancarboxylate [Compound (118)]:

Compound (116) was subjected to catalytic reduction by a method analogous to Example 117. After the catalyst was removed by filtration, the filtrate was concentrated to dryness. The residue obtained was dissolved in DMF; sorbic acid, DCC and HOBT were then added and the solution stirred at room temperature for 1 hour. Ethyl acetate was added to the reaction mixture obtained, the crystals separated being removed by filtration. The organic layer, after rinsing with water, was dried ($Na_2SO_4$). The solvent was vaporized off. The residue was refined by column chromatography using silica gel, yielding Compound

(118).

Example 119

Production of pivaloyloxymethyl 2-[(4S)-4-(2-thienyl)acetamido-3-oxo-2-isoxazolidinyl]-5-oxo-2-tetrahydrofurancarboxylate [Compound (119)]:

Compound (119) was obtained by a method analogous to of Example 117, using Compound (116) and thienylacetyl chloride.

Hereinafter are listed the reaction conditions, yields and physico-chemical properties of the compounds produced in Examples 100 to 119.

| Product | Reaction conditions | Yield (mg) St.mat. → Prod. | KBr IR $\nu_{max}$ $cm^{-1}$ | NMR (90MHz) δ |
|---|---|---|---|---|
| 100 | 1) $H_2$/10% Pd-C (290mg) ethyl acetate(7mℓ)-pH 7.0 phosphate buffer(10mℓ) 0 ℃, 1.5h<br>2) 2-(2-chloroacetamide-4-thiazolyl)-(Z)-2-ethoxyiminoacetyl chloride hydrochloride (233mg), THF(7mℓ) $NaHCO_3$(145mg) 0℃, 30min<br>3) sodium N-methyldithiocarbamic acid (132mg) rt, 30min<br>4) HP-20 column (10% ethanol) lyophilization | 290 → 107 | 1780, 1720, 1650 | 1.48(3H, t, J=7Hz), 2.5-3.6(4H, m), 4.45(2H, q, J=7Hz), 4.3-5.6(3H, m), 7.20(1H, s) (solvent: $D_2O$) |
| 101 | 1) $H_2$/10% Pd-C (225mg) ethyl acetate(7mℓ) pH 7.0 phosphate buffer(7.5mℓ) 0℃, 1h<br>2) 2-(5-chloro-2-chloroacetamide-4-thiazolyl-(Z)-2-methoxyimino acetyl chloride · hydrochloride (197mg) THF(5mℓ) | 225 → 97.6 | 3400, 1780, 1770, 1710, 1660, 1530, 1200, 1130 | 2.3-3.4(4H, m), 4.1(3H, s), 4.2-4.6(1H, m), 5.0-5.4(2H, m) (solvent: $D_2O$) |

| | | | | |
|---|---|---|---|---|
| 101 | $NaHCO_3$(110mg) 0℃, 30min<br>3) sodium N-methyl-dithiocarbamic acid (103mg) rt, 50min<br>4) XAD-2 column (water) lyophilization | | | |
| 102a | 2-(4-ethyl-2,3-dioxo-1-piperazinyl carboxamide-2-thienylacetic acid (390mg)<br>DCC(248mg)<br>HOBT(184mg)<br>DMF(5mℓ)<br>rt, 3.5h<br>extraction with ethyl acetate, silica gel chromatography ($CH_2Cl_2$-ethyl acetate=1:2) | 396<br>→<br>299 | 3300, 1800, 1720, 1680, 1510, 1180 | 1.16(3H,t,J=7Hz), 1.77-2.82(4H,m), 3.33-3.65(4H,m), 3.89-4.24(3H,m), 4.38-4.85(2H,m), 5.73-5.88(1H,m), 6.88-7.03(3H,m), 7.14-7.58(11H,m), 7.68-7.90(1H,m)<br>(solvent: $CDCl_3$) |
| 102b | $CF_3COOH$(0.26mℓ)<br>anisole (0.20mℓ)<br>-15° ~ -10℃, 3.5h<br>XAD-2 column (10% ethanol)<br>lyophilization | 191<br>→<br>48 | 3450, 1780, 1710, 1670, 1510, 1370, 1190 | 1.27(3H,t,J=7Hz), 2.43-3.27(4H,m), 3.44-3.90 (4H,m), 4.02-4.35(3H,m), 4.55-5.24(2H,m), 5.91 (1H,s), 7.14-7.42(2H,m), 7.55-7.70(1H,m)<br>(solvent: $D_2O$) |
| | KNCO(160mg)<br>acetic acid(4mℓ)<br>water(1mℓ) | 400<br>→<br>220 | 3300, 1765, 1720, | 2.20-5.00(7H,m), 5.73(2H,bs), 6.60(1H,d,J=6Hz), |
| 103a | rt, 2h<br>extraction with ethyl acetate, silica gel chromatography ($CHCℓ_3$-ethyl acetate- | | 1680, 1650, 1180, 1050 | 6.86(1H,s), 7.20-7.52(10H,m)<br>(solvent: $d_6$-DMSO) |

| | | | | |
|---|---|---|---|---|
| 103a | methanol=85:5:10) | | | |
| 103b | $H_2$/5% Pd-C (220mg) THF(5mℓ) water(5mℓ) $NaHCO_3$(43mg) rt, 1 h CHP-20 column (water) lyophilization | 220 → 100 | 1770, 1718, 1580, 1400 | 2.00-3.20(4H,m), 3.60-4.50(3H,m) (solvent:$D_2O$) |
| 104a | phenylisocyanate (0.1mℓ) $CH_2Cl_2$(10mℓ) rt,10min silica gel chromatography ($CH_2Cl_2$-ethyl acetate-methanol=85:5:10) | 198 → 138 | 3300, 1760, 1720, 1670, 1180, 1055 | 2.20-5.20(7H,m), 6.73-7.50(16H,m), 8.73(1H,d,J=7Hz) (solvent:$d_6$-DMSO) |
| 104b | $H_2$/5% Pd-C (140mg) THF(4mℓ) water (4mℓ) $NaHCO_3$(42mg) rt, 1 h CHP-20 column (water) lyophilization | 138 → 70 | 3400, 1770, 1720, 1590, 1500, 1420 | 2.10-2.95(4H,m), 4.35-4.90(3H,m), 7.25-7.80(5H,m) (solvent:$D_2O$) |
| 105 | 1) $H_2$/5% Pd-C (380mg) ethyl acetate(10mℓ) water(10mℓ) 0℃,1h 2) 2-pyridylacetyl chloride・hydrochloride (292mg) $NaHCO_3$ aqueous layer (mentioned above) -THF (10mℓ) 0℃,20min | 380 → 30 | 3400, 1770, 1710, 1675, 1645, 1190, 1105 | 2.3-3.3(4H,m), 4.1-4.4(1H,m), 4.50-5.25(4H,m), 7.30-7.55(2H,m), 7.80-8.05(1H,m), 8.4-8.6(1H,m) |

| | | | | |
|---|---|---|---|---|
| 105 | →rt,40min<br>3) XAD-2 column<br>(10% ethanol)<br>lyophilization | | | |
| 106 | 1) $H_2$/5%Pd-C<br>(1.8g)<br>ethyl acetate(20mℓ)<br>pH7.0 phosphate<br>buffer(20mℓ)<br>rt,1h<br>2) 4-chlorophenyl<br>acetyl chloride<br>(1.05g)<br>$NaHCO_3$(940mℓ)<br>phosphate buffer<br>mentioned above,<br>THF(30mℓ)<br>0℃,30min<br>3) lyophilization | 1900<br>→<br>471 | 1780,<br>1720,<br>1660 | 2.40-3.25(4H,m),<br>3.60(2H,s),<br>4.04-4.78(2H,m),<br>4.84-5.14(1H,m),<br>7.15-7.36(4H,m)<br>(solvent:$D_2O$) |
| 107 | 1) $H_2$/5%Pd-C<br>(350mg)<br>ethyl acetate(10mℓ)<br>pH7.0 phosphate<br>buffer(5mℓ)<br>rt,1.5h<br>2) 2-chloro-2-pheny-<br>acetyl chloride<br>(199mg)<br>$NaHCO_3$ aqueous<br>solution<br>the above phosphate<br>buffer THF(10mℓ)<br>0℃,20min→<br>rt,40min<br>3) XAD-2 column<br>(10% ethanol)<br>lyophilization | 350<br>→<br>25 | 3430,<br>1775,<br>1725,<br>1650,<br>1540,<br>1380,<br>1200 | 2.2-3.3(4H,m),<br>4.05-4.50(2H,m),<br>4.8-5.3(2H,m),<br>5.70(1H,s),<br>7.4-7.7(4H,m),<br>7.74-7.90(1H,m)<br>(solvent:$D_2O$) |
| 108a | sodium 3-phridine-<br>thiol (113mg)<br>NaI(100mg) | 200<br>→<br>128 | 3350,<br>1770,<br>1740, | 2.21-3.40(4H,m),<br>3.62(2H,s),<br>3.84-4.09(1.5H,m), |

| | | | | |
|---|---|---|---|---|
| 108a | DMF(4mℓ) rt,30min extraction with ethyl acetate silica gel chromatography (hexane-ethyl acetate=1:4) | | 1680, 1510, 1180, 1050 | 4.54-5.07(1.5H,m), 6.97(1H,s), 7.11-8.71(14H,m) (solvent: $CDCl_3$) |
| 108b | $CF_3COOH$(0.25mℓ) anisole(0.2mℓ) $CH_2Cl_2$(10mℓ) -10° ~-15℃,5h XAD-2 column (10% ethanol) lyophilization | 128 → 53 | 3440, 1780, 1720, 1650, 1380, 1190, 1120, 1040 | 2.41-3.28(4H,m), 3.78(2H,s), 3.92-4.13(1H,m), 4.46-4.71(1H,m), 4.86-5.10(1H,m), 7.42-8.10(4H,m) (solvent: $D_2O$) |
| 109a | sodium 4-chlorophenylthiol (211mg) NaI(150mg) DMF(4mℓ) rt,1h extraction with ethyl acetate silica gel chromatography (hexane-ethyl acetate=3:4) | 300 → 160 | 3350, 1770, 1740, 1670, 1510, 1480, 1180, 1050 | 2.19-3.38(4H,m), 3.60(2H,s), 3.73-4.11(1.5H,m), 4.50-5.02(1.5H,m), 6.93(1H,s), 7.01-7.70(14H,m) (solvent: $CDCl_3$) |
| 109b | $CF_3COOH$(0.31mℓ) anisole (0.25mℓ) $CH_2Cl_2$(15mℓ) -10° ~-15℃,4h XAD-2 column (30% ethanol) lyophilization | 160 → 76 | 3350, 1780, 1720, 1650, 1380, 1190, 1100, 1030 | 2.40-3.91(4H,m), 3.71(2H,s), 3.87-4.19(1H,m), 4.42-4.73(1H,m), 4.81-5.14(1H,m), 7.43(4H,s) (solvent: $D_2O$) |
| 110a | 2-isopropylphenoxycarbonyl chloride (149mg) DMA(0.4mℓ) $CH_2Cl_2$(10mℓ) rt,30min | 200 → 202 | 3350, 2940, 1770, 1740, 1720, 1490, | 1.20(3H,s),1.31(3H,s), 2.21-3.50(5H,m), 4.01-4.39(1H,m), 4.53-5.07(1H,m), 5.58-5.82(1H,m), 7.0(1H,s),7.07-7.51(14 |

| | | | | |
|---|---|---|---|---|
| 110a | extraction with ethyl acetate, crystallization with hexane-ether (1:1) | | 1180, 1050 | H,m) (solvent: $CDCl_3$) |
| 110b | $H_2$/5% Pd-C (202mg) THF-buffer(pH7.0) 0℃, 30min XAD-2 column (30% ethanol) lyophilization | 202 → 83 | 3400, 1780, 1730, 1650, 1380, 1210, 1190, 1050 | 1.18(3H,s), 1.27(3H,s), 2.42-3.27(5H,m), 4.18-4.39(1H,m), 4.54-5.09(2H,m), 7.07-7.53(4H,m) (solvent: $D_2O$) |
| 111a | difluoroacetic acid (96mg) DCC(206mg) HOBT(134mg) DMF(4 mℓ) rt, 1h extraction with ethyl acetate, silicagel chromatography (hexan-ethyl acetate=1:1) | 250 → 175 | 3350, 2930, 1770, 1740, 1700, 1540, 1180, 1050 | 2.22-3.48(4H,m), 3.73-4.30(1.5H,m), 4.55-5.01(1.5H,m), 5.91(1H,t,J=54Hz), 7.01(1H,s), 7.25-7.40(10H,m) (solvent: $CDCl_3$) |
| 111b | $H_2$/5% Pd-C (175mg) THF-water $NaHCO_3$(34mg) 0℃, 25min HP-20 column (water) lyophilization | 175 → 59 | 3400, 1780, 1720, 1650, 1540, 1380, 1190, 1105, 1030 | 2.35-3.32(4H,m), 4.20-4.41(1H,m), 4.55-4.87(1H,m), 5.07-5.21(1H,m), 6.25(1H,t,J=48Hz) (solvent: $D_2O$) |
| 112 | 1) $H_2$/5% Pd-C (300mg) THF(20mℓ)-water(10mℓ) 0℃, 40min 2) 3-chloropropionyl chloride | 300 → 21 | 3450, 1780, 1720, 1650, 1380, 1190, 1030 | 2.22-3.31(4H,m), 2.83(2H,t,J=7Hz), 3.86(2H,t,J=7Hz), 4.10-4.35(1H,m), 4.51-4.82(1H,m), 4.98-5.23(1H,m) (solvent: $D_2O$) |

| | | | | |
|---|---|---|---|---|
| 112 | (0.08mℓ)<br>aqueous layer<br>(mentioned above)-<br>THF (6mℓ)<br>$NaHCO_3$(222mg)<br>0℃, 60min<br>3) XAD-2 column<br>(water)<br>lyophilization | | | |
| 113 | chloromethyl<br>pivalate(0.1mℓ)<br>DMF(3mℓ)<br>rt, 24h<br>extraction with<br>ethyl acetate,<br>silica gel chromato-<br>graphy (hexane-ethyl<br>acetate=1:2) | 160<br>→<br>111 | 3350,<br>1800,<br>1750,<br>1675,<br>1535 | 1.22(9H,s),<br>2.29-3.40(4H,m),<br>3.9-4.3(1H,m),<br>4.11(2H,s),<br>4.62-5.13(2H,m),<br>5.87(2H,s), 7.30(1H,m)<br>(solvent: $D_2O$) |
| 114 | chloromethyl<br>pivalate(0.11mℓ)<br>DMF(4mℓ)<br>rt, 24h<br>extraction with<br>ethyl acetate,<br>silica gel chromato-<br>graphy (hexane- ethyl<br>acetate=2:3) | 200<br>→<br>172 | 3300,<br>1800,<br>1750,<br>1660,<br>1520 | 1.21(9H,s),<br>2.29-3.32(4H,m),<br>3.61(2H,s),<br>3.81-4.10(1H,m),<br>4.49-5.12(2H,m),<br>5.81(2H,s),<br>7.11-7.50(5H,m)<br>(solvent: $CDCl_3$) |
| 115a | propionyl acid<br>chloride (0.13mℓ)<br>DMA(1mℓ)<br>$CH_2Cl_2$(20mℓ)<br>0℃, 1h<br>extraction with ethyl<br>acetate, silica gel<br>chromatography<br>(hexane-ethyl acetate<br>=1:2) | 500<br>→<br>396 | 3350,<br>1770,<br>1740,<br>1680,<br>1530,<br>1190,<br>1050 | 1.10(3H,t,J=8Hz),<br>1.98-3.59(6H,m),<br>3.85-4.16(1H,m),<br>4.57-5.09(2H,m),<br>6.95(1H,s),<br>7.21-7.52(10H,m)<br>(solvent: $CDCl_3$) |
| 115b | $H_2$/5% Pd-C<br>(396mg)<br>THF − water | 396<br>→<br>140 | 3450,<br>1790,<br>1730, | 1.23(3H,t,J=8Hz),<br>2.23-3.31(6H,m),<br>4.11-4.31(1H,m), |

| | | | | |
|---|---|---|---|---|
| 115b | $NaHCO_3$(73mg)<br>0℃,30min<br>HP-20 column<br>(water)<br>lyophilization | | 1650,<br>1380,<br>1190,<br>1050 | 4.59-4.82(1H,m),<br>4.93-5.18(1H,m)<br>(solvent:$D_2O$) |
| 116 | chloromethyl<br>pivalate(0.1mℓ)<br>DMF(4mℓ)<br>rt,24h<br>extraction with<br>ethyl acetate,<br>silica gel chromato-<br>graphy ($CH_2Cl_2$-ethyl<br>acetate=5:1) | 900<br>→<br>620 | 3330,1800,<br>1750,1720,<br>1520,1230,<br>1180,1120,<br>1050,1020 | 1.20(9H,s),<br>2.30-3.35(4H,m),<br>3.90-4.40(1H,m),<br>4.50-4.90(2H,m),<br>5.14(2H,s),5.68(1H,bs),<br>5.86(2H,s),7.35(5H,s)<br>(solvent:$CDCl_3$) |
| 117 | 1) $H_2$/5%Pd-C<br>(215mg)<br>ethyl acetate(6mℓ)<br>0℃,1h<br>2)bromoacetyl bromide<br>(90mg)<br>ethyl acetate<br>(mentioned above)<br>DMA(0.35mℓ)<br>0℃,0.5h<br>silica gel chromato-<br>graphy ($CH_2Cl_2$-ethyl<br>acetate=5:1) | 215<br>→<br>103 | 3340,<br>1800,<br>1740,<br>1670,<br>1520 | 1.20(9H,s),<br>2.30-3.49(4H,m),<br>3.83(2H,s),<br>4.02-4.30(1H,m),<br>4.59-5.21(2H,m),<br>5.79(2H,m),<br>7.32(1H,m)<br>(solvent:$CDCl_3$) |
| 118 | 1) $H_2$/5%Pd-C<br>(146mg)<br>ethyl acetate(5mℓ)<br>0℃,1h<br>2)sorbic acid<br>(45mg)<br>DCC(83mg)<br>HOBT(54mg)<br>DMF(2mℓ)<br>rt,1h<br>silica gel chromato-<br>graphy ($CH_2Cl_2$-<br>ethyl acetate=4:1) | 146<br>→<br>60 | 3320,<br>1800,<br>1740,<br>1660,<br>1520 | 1.20(9H,s),<br>1.82(3H,d,J=3Hz),<br>2.30-3.57(4H,m),<br>4.00-4.31(1H,m),<br>4.69-5.19(2H,m),<br>5.80(2H,s),<br>5.71-6.50(3H,m),<br>7.04-7.38(1H,m)<br>(solvent:$CDCl_3$) |

| | | | | |
|---|---|---|---|---|
| 119 | 1) $H_2$/5%Pd-C (185mg) 0℃, 90min ethyl acetate(6mℓ) 2) thienylacetyl chloride(62mg) ethyl acetate (mentioned above) DMA(0.3mℓ) 0℃, 1h silica gel chromatography ($CH_2Cl_2$-ethyl acetate=5:1) | 185 → 88 | 3340, 1800, 1740, 1660, 1520 | 1.20(9H,s), 2.29-3.30(4H,m), 3.78(2H,s), 3.93-4.20(1H,m), 4.58-5.28(2H,m), 5.81(2H,m), 6.60-7.29(3H,m) (solvent: $CDCl_3$) |

Example 120

Production of pivaloyloxymethyl 2-[(4S)-4-(hexahydro-1H-azepin-1-yl)methyleneamino-3-oxo-2-isoxazolidinyl]-5-oxo-2-tetrahydrofurancarboxylate [Compound (120)]:

Compound (116) was subjected to catalytic reduction by a method analogous to Example 117. After removing the catalyst by filtration, to the filtrate were added 1-hexamethyleneiminecarboxyaldehyde dimethylacetal and a catalytic amount of boron trifluoride under ice-cooling and the mixture was stirred at room temperature for 20 hours. The solvent was removed by evaporation under reduced pressure, and then the residue was subjected to purification by column chromatography using Florisil to give the subject Compound (120).

Example 121

Production of diphenylmethyl 2-[(4S)-4-(tert-butyldimethylsilyl)amino-3-oxo-2-isoxazolidinyl]-5-oxo-2-tetrahydrofurancarboxylate[Compound (121)]:

To a dimethylformamide solution of Compound (R-3) was added tert-butyldimethylsilyl chloride and triethylamine, and then the mixture was stirred at room temperature for one hour. The reaction solution was added to water-ethyl acetate and the mixture was shaken. The organic layer was recovered, washed with water and dried ($Na_2SO_4$). The solvent was removed by evaporation under reduced pressure to give the subject Compound (121).

Example 122

Production of diphenylmethyl 2-[(4S)-4-tert-butyldiphenylsilyl)amino-3-oxo-2-isoxazolidinyl]-5-oxo-2-tetrahydrofurancarboxylate [Compound (122)]:

Using Compound (R-3) and tert-butyl-diphenylsilyl chloride, there was obtained the subject Compound (122) by the procedure of Example 121.

Example 123

Production of sodium 2-[(4S)-3-diphenylphosphorylamino-3-oxo-2-isoxazolidinyl]-5-oxo-2-tetrahydrofurancarboxylate [Compound (123b)]:

To a dimethylformamide solution of Compound (R-3) was added diphenyl phosphorochloridate under ice-cooling, and the mixture was stirred for one hour. The reaction solution was added to water, and the mixture was subjected to extraction with ethyl acetate. The ethyl acetate layer was washed with water and

then dried ($Na_2SO_4$). The solvent was removed by evaporation under reduced pressure, and then the residue was subjected to purification by silica gel column chromatography to give Compound (123a), a diphenylmethyl ester of the subject Compound (123b). Then, the thus obtained Compound (123a) was treated by the procedure of Example 2(b) to give the subject Compound (123b).

Example 124

Production of diphenylmethyl 2-[(4S)-4-dimethylphosphorylamino-3-oxo-2-isoxazolidinyl]-5-oxo-2-tetrahydrofurancarboxylate [Compound (124)]:

Using Compound (R-3) and dimethyl phosphorochloridate, there was obtained the subject Compound (124) by the procedure of Example 123.

Example 125

Production of sodium 2-[(4S)-4-ethylsulfonylamino-3-oxo-2-isoxazolidinyl]-5-oxo-2-tetrahydrofurancarboxylate [Compound (125b)]:

To a dichloromethane solution of Compound (R-3) were added ethanesulfonylchloride and triethylamine under ice-cooling. The mixture was stirred at 0°C for 30 minutes, and then at room temperature for 90 minutes. The reaction mixture was washed with water and then dried ($Na_2SO_4$). The solvent was removed by evaporation under reduced pressure, and then the residue was subjected to purification by silica gel chromatography to give Compound (125a), a diphenyl ester of the subject Compound (125b). Then, the thus obtained Compound (125a) was treated by the procedure of Example 2(b) to give the subject Compound (125b).

Example 126

Production of sodium 2-[(4S)-4-vinylsulfonylamino-3-oxo-2-isoxazolidinyl]-5-oxo-2-tetrahydrofurancarboxylate [Compound (126)]:

Using Compound (R-3) and 2-chloroethanesulfonyl chloride, there was obtained the subject Compound (126) by the procedure of Example 125 and then by the procedure of Example 1(c).

Example 127

Production of 2-[(4S)-4-thienylacetamido-3-oxo-2-isoxazolidinyl]-5-oxo-2-tetrahydrofurancarbonylanilide [Compound (127)]:

To the suspension of Compound (5b) in dichloromethane was added trimethylacetyl chloride at 0°C, and then the mixture was stirred at 0°C for one hour. To the resultant was added aniline, and then the mixture was stirred at room temperature for one hour. The reaction mixture was washed with water and dried ($Na_2SO_4$). The solvent was removed by evaporation under reduced pressure, and the residue was subjected to purification by column chromatography using Florisil to give Compound (127) as a colourless oily product.

Example 128

Production of 2-[(4S)-4-benzyloxycarbonylamino-3-oxo-2-isoxazolidinyl]-5-oxo-2-tetrahydrofurancarbonylanilide [Compound (128)]:

Using Compound (93b) and aniline, there was obtained the subject Compound (128) as colorless crystals by the procedure of Example 127.

Example 129

Production of 2-[(4S)-4-benzyloxycarbonylamino-3-oxo-2-isoxazolidinyl]-5-oxo-2-tetrahydrofurancarbonylpyrrolidinamide [Compound (129)]:

Using Compound (93b) and pyrrolidine, thus was obtained the subject Compound (129) as a colorless oily product by the procedure of Example 127.

Example 130

Production of 2-[(4S)-4-thienylacetamido-3-oxo-2-isoxazolidinyl]-5-oxo-2-tetrahydrofurancarbonylpyrrolidinamide [Compound (13O)].

Using Compound (5b) and pyrrolidine, there was obtained the subject Compound (130) as acolorless oily product by the procedure of Example 127.

Example 131

Production of 2-[(4S)-4-thienylacetamido-3-oxo-2-isoxazolidinyl]-5-oxo-2-tetrahydrofurancarbonylpropylamine [Compound (131)]:

Using Compound (5b) and propylamine, there was obtained the subject Compound (130) as a colorless oily product by the procedure of Example 127.

Example 132

Production of sodium 2-[(4S)-4-γ-D-glutamylamino-3-oxo-2-isoxazolidinyl]-5-oxo-2-tetrahydrofurancarboxylate [Compound (132)]:

Using Compound (R-3) and α-benzyl N-carbobenzoxy-D-glutamate, there was obtained the subject Compound (132) by the procedure of Example 1(a) and then by the procedure of Example 2(b).

Example 133

Production of sodium 2-[(4S)-4-(2-hydroxy)isobutyrylamino-3-oxo-2-isoxazolidinyl]-5-oxo-2-tetrahydrofurancarboxylate [Compound (133)]:

Using Compound (R-3) and 2-hydroxyisobutyric acid, there was obtained the subject Compound (133) by the procedure of Example 1 (a) and then by the procedure of Example 2 (b).

Below are given the reaction conditions of Examples 120 to 133 as well as the yields and typical physico-chemical properties of the compounds obtained.

| Product | Reaction conditions | Yield (mg) St.mat. → Prod. | KBr IR $\nu_{max}$ $cm^{-1}$ | NMR(90MHz) $\delta$ |
|---|---|---|---|---|
| 120 | 1) $H_2$/5%Pd-C (274mg) ethyl acetate (10mℓ1) 2) 1-hexamethyleneiminecarboxyaldehyde dimethylacetal (119mg) $BF_3 \cdot Et_2O$(4 drops) rt,20h Florisil chromatography (ethyl acetate) | 274 → 180 | 1800,1740, 1630,1520, 1240,1040 | 1.19(9H,s), 1.40-1.81(8H,m), 2.29-3.29(4H,m), 3.30-3.47(4H,m), 3.92-4.20(1H,m), 4.42-4.89(2H,m), 5.82(2H,m), 8.03(1H,s) (solvent:$CDCl_3$) |
| 121 | tert-butyldimethylsilyl chloride(115mg), triethylamine (78mg), DMF(4 mℓ), rt, 1h extraction with ethyl acetate | 202 → 255 | 1800,1730, 1250,1180, 1090 | 0.89(9H,s), 2.21-3.32(4H,m), 3.57-3.90(1H,m), 3.98-4.39(2H,m), 6.96(1H,s), 7.21-7.40(10H,m) (solvent:$CDCl_3$) |
| 122 | tert-butyldiphenylsilyl chloride(211mg) triethylamine (78mg) DMF(4 mℓ) rt, 1h extraction with ethyl acetate | 202 → 317 | 1800,1730, 1240,1180, 1100 | 1.09(9H,s), 2.21-3.31(4H,m), 3.57-3.90(1H,m), 3.96-4.42(2H,m), 7.01(1H,s), 7.20-7.96(20H,m) (solven:$CDCl_3$) |
| 123a | diphenyl phosphorochloridate (134mg) | 198 → 83 | 3350,1800, 1750,1590, 1490,1180, | 2.20-3.35(4H,m), 3.71-4.14(1.5H,m), 4.31-4.70(1.5H,m), |

| | | | | |
|---|---|---|---|---|
| 123a | pyridine(0.04mℓ) DMF(4mℓ) 0℃, 1h extraction with ethyl acetate silica gel chromatography ($CH_2Cl_2$-ethyl acetate=1:1) | | 940 | 6.96(1H,s), 7.03-7.49(20H,m), (solvent:$CDCl_3$) |
| 123b | $H_2$/10%Pd-C (74mg) THF(7mℓ)-pH7.0 phosphate buffer(3.5mℓ) 0℃,25min XAD-2 column (20%ethanol) lyophilization | 74 → 28 | 3450,1780, 1730,1640, 1490,1380, 1190 | 2.21-3.27(4H,m), 3.83-4.08(1H,m), 4.31-4.90(2H,m), 7.18-7.57(10H,m), (solvent:$D_2O$) |
| 124 | dimethyl phosphorochloridate (71mg) pyridine(0.04mℓ) DMF(4mℓ) 0℃,1h extraction with ethyl acetate silica gel chromatography(ethyl acetate) | 198 → 30 | 3320,1800, 1760,1490, 1250,1180, 1050,1020 | 2.20-3.25(4H,m), 3.65(3H,s), 3.77(3H,s), 3.75-4.10(1.5H,m), 4.29-4.66(1.5H,m), 6.98(1H,s), 7.22-7.45(10H,m) (solvent:$CDCl_3$) |
| 125a | ethanesulfonyl chloride(56μℓ) triethylamine (84μℓ) $CH_2Cl_2$(10mℓ) 0℃,30min→ rt,90min silica gel chromatography(hexane-ethyl acetate =1:1) | 198 → 150 | 3300,1800, 1770,1550, 1330,1180, 1160,1050 | 1.39(3H,t,J=8Hz), 2.31-3.59(6H,m), 3.89-4.20(1H,m), 4.51-4.69(2H,m), 6.98(1H,s), 7.31-7.50(10H,m), (solvent:$CDCl_3$) |

| | | | | |
|---|---|---|---|---|
| 125b | $H_2$/10%Pd-C (115mg) $NaHCO_3$(19.7mg) THF(6mℓ)-water (3mℓ) 0℃,30min XAD-2 column (water) lyophilization | 115 → 54 | 3450,1780, 1720,1640, 1380,1320, 1200,1140 | 1.40(3H,t,J=8Hz), 2.26-3.44(6H,m), 4.07-4.41(1H,m), 4.63-4.94(2H,m) (solvent:$D_2O$) |
| 126 | 1) 2-chloro-ethanesulfonyl chloride(196mg) triethylamine (0.169mℓ) $CH_2Cl_2$(20mℓ) 0℃,60min silica gel chromatography ($CH_2Cl_2$-ethyl acetate=5:1) 2) $CF_3COOH$(0.63 mℓ), anisole (0.24mℓ) $CH_2Cl_2$(24mℓ) -10~-15℃,4h XAD-2 column (water) lyophilization | 396 → 64 | 3400,1780, 1720,1380, 1330,1200, 1140,1020 | 2.43-3.22(4H,m), 4.08-4.32(1H,m), 4.57-5.20(2H,m), 6.10-6.42(2H,m), 6.60-6.98(1H,m) (solvent:$D_2O$) |
| 127 | trimethylacetyl chloride (32mℓ) $CH_2Cl_2$(4 mℓ) 0℃,1h aniline(24μℓ) rt, 1h Florisil column (hexane-ethyl acetate=2:3) | 100 → 11 | 3300,1800, 1730,1690, 1540,1180, 1040 | 2.37-3.46(4H,m), 3.80(2H,s), 3.81-4.08(1H,m), 4.57-5.01(2H,m), 6.58-6.83(1H,m), 6.83-7.61(8H,m), 8.41-8.62(1H,m), (solvent:$CDCl_3$) |
| 128 | trimethylacetyl chloride | 103 → | 3320,1800, 1730,1690, | 2.37-3.54(4H,m), 4.01-4.29(1H,m), |

| | | | | |
|---|---|---|---|---|
| 128 | (32 μℓ)<br>$CH_2Cl_2$(4 mℓ)<br>0°C, 1h<br>aniline(24 μℓ)<br>rt, 1h<br>silica gel chromatography<br>(hexane–ethyl acetate=1:1) | 31 | 1530,1250,<br>1180,1050 | 4.52-4.79(2H,m),<br>5.10(2H,s),<br>5.51-5.70(1H,m),<br>7.09-7.60(10H,m),<br>8.31-8.44(1H,m),<br>(solvent:$CDCl_3$) |
| 129 | trimethylacetyl chloride<br>(32 μℓ)<br>$CH_2Cl_2$(4 mℓ)<br>0°C, 1h<br>pyrrolidine(22 μℓ)<br>rt, 1h<br>silica gel chromatography<br>(hexane–ethyl acetate=50:80:1) | 103<br>→<br>18 | 3320,1800,<br>1730,1640,<br>1530,1250,<br>1180,1020 | 1.62-2.01(4H,m),<br>2.39-3.26(4H,m),<br>3.38-3.70(4H,m),<br>4.02-4.29(1H,m),<br>4.51-4.80(2H,m),<br>5.10(2H,s),<br>5.79-6.02(1H,m),<br>7.15-7.42(5H,m),<br>(solvent:$CDCl_3$) |
| 130 | trimethylacetyl chloride<br>(32 μℓ)<br>$CH_2Cl_2$(4 mℓ)<br>0°C, 1h<br>pyrrolidine(22 μℓ)<br>rt, 1h<br>silica gel chromatography<br>(hexane–ethyl acetate=3:6:0.1→0:10:0.1) | 100<br>→<br>21 | 3270,1790,<br>1720,1680,<br>1540,1180,<br>1040 | 1.73-2.01(4H,m),<br>2.41-3.11(4H,m),<br>3.40-3.71(4H,m),<br>3.76,3.78(2H,each s), 4.01-4.29(1H, m), 4.53-4.99(2H, m), 6.88-7.28(3H, m)<br>(solvent:$CDCl_3$) |
| 131 | trimethylacetyl chloride<br>(32 μℓ)<br>$CH_2Cl_2$(4mℓ)<br>0°C, 1h<br>propylamine<br>(22 μℓ)<br>rt, 1h | 100<br>→<br>18 | 3270,1800,<br>1720,1680,<br>1540,1180,<br>1040 | 0.81-1.00(3H,each t,J=6Hz),<br>1.38-1.68(2H,m),<br>2.25-3.42(6H,m),<br>3.70-4.09(1H,m),<br>3.77(2H,s),<br>4.59-4.98(2H,m),<br>6.87-7.29(3H,m), |

| | | | | |
|---|---|---|---|---|
| 131 | silica gel chromatography (hexane−ethyl acetate=25:75:1) | | | (solvent:$CDCl_3$) |
| 132 | 1) α−benzyl N-carbobenzoxy-D-glutamate ester(373mg) DCC(206mg) HOBT(68mg) DMF(5 mℓ) 20℃,1.5h silica gel chromatography (hexane−ethyl acetate=1:1) 2) $H_2$/10%Pd-C (150mg) THF(10mℓ)−water(3 mℓ) $NaHCO_3$(17mg) 20℃,1.5h CHP-20 chromatography (water) lyophilization | 317 → 41 | 1780,1720, 1650,1520, 1380,1200 | 1.23(6H,s), 2.00-2.70(3H,m), 2.70-3.20(1H,m), 3.80-4.10(1H,m), 4.20-4.50(1H,m), 4.60-4.80(1H,m), 8.40(1H,d,J=7Hz) (solvent:$d_6$-DMSO) |
| 133 | 1) 2-hydroxyisobutyric acid (53mg) DCC(103mg) HOBT(34mg) DMF(3mℓ) 20℃,1.5h silica gel chromatography (hexane−ethyl acetate=1:3) 2) $H_2$/10%Pd-C (70mg) THF(6mℓ)-water (2mℓ) | 200 → 30 | 1780,1720, 1660,1640, 1540,1400 1380,1200, | 1.70-3.20(8H,m), 3.70-4.10(1H,m), 4.20-4.60(1H,m) 4.60-5.00(2H,m), 8.40(1H,d,J=7Hz) (solvent:$d_6$-DMSO) |

| | | | | |
|---|---|---|---|---|
| 133 | $NaHCO_3$(12mg)<br>20℃,0.5h<br>CHP-20 column<br>(water)<br>lyophilization | | | |

## Claims

### Claims for the following Contracting States : BE, CH, DE, FR, GB, IT, LI, LU, NL, SE

**1.** A 2-((4S)-amino-3-oxo-2-isoxasolidinyl)-5-oxo-2-tetrahydrofurancarboxylic acid derivative of the formula (I') or a pharmaceutically acceptable salt thereof, selected from:

(1) sodium 2-{(4S)-4-[2-(2-amino-4-thiazolyl)-(Z)-2-(methoxyimino)acetamido]-3-oxo-2-isoxazolidinyl}-5-oxo-2-tetrahydrofurancarboxylate;
(2) sodium 2-{(4S)-4-[D-2-(4-ethyl-2,3-dioxo-1-piperazinocarboxamido)-2-phenylacetamido]-3-oxo-2-isoxazolidinyl}-5-oxo-2-tetrahydrofurancarboxylate;
(3) sodium 2-{(4S)-4-[D-2-(3-methylsulfonyl-2-oxoimidazolidine-1-carboxamido)-2-phenylacetamido]-3-oxo-2-isoxazolidinyl}-5-oxo-2-tetrahydrofurancarboxylate;
(4) sodium 2-{(4S)-4-[D-2-(3-furfurylidene-amino-2-oxoimidazolidine-1-carboxamido-2-phenylacetamido]-3-oxo-2-isoxazolidinyl}-5-oxo-2-tetrahydrofurancarboxylate;
(5) sodium 2-[(4S)-4-(2-thienylacetamido)-3-oxo-2-isoxazolidinyl]-5-oxo-2-tetrahydrofurancarboxylate;
(6) sodium 2-[(4S)-4-(2-cyanoacetamido)-3-oxo-2-isoxazolidinyl]-5-oxo-2-tetrahydrofurancarboxylate;
(7) sodium 2-{(4S)-4-[(1H)-tetrazolylacetamido]-3-oxo-2-isoxazolidinyl}-5-oxo-2-tetrahydrofurancarboxylate;
(8) sodium 2-[(4S)-4-(2-6-dimethoxybenzamido)-3-oxo-2-isoxazolidinyl]-5-oxo-2-tetrahydrofurancarboxylate;
(9) sodium 2-[(4S)-4-(5-methyl-3-phenyl-2-isoxazoline -4-carboxamido)-3-oxo-2-isoxazolidinyl]-5-oxo-2-tetrahydrofurancarboxylate;
(10) sodium 2-[(4S)-4-(2-ethoxynaphthalenyl-1-carboxamido)-3-oxo-2-isoxazolidinyl]-5-oxo-2-tetrahydrofurancarboxylate;
(11) sodium 2-[(4S)-4-(2-cyanomethylthioacetamido)-3-oxo-2-isoxazolidinyl]-5-oxo-2-tetrahydrofurancarboxylate;
(12) sodium 2-[(4S)-4-(2-difluoromethylthioacetamido)-3-oxo-2-isoxazolidinyl]-5-oxo-2-tetrahydrofurancarboxylate;
(13) sodium 2-{(4S)-4-[(R)-2-(4-cyclohexyl-2,3-dioxo-1-piperazinocarboxamido-2-phenylacetamido]-3-oxo-2-isoxazolidinyl}-5-oxo-2-tetrahydrofurancarboxylate;
(14) sodium 2-((4S)-4-chloroacetamido-3-oxo-2-isoxazolidinyl)-5-oxo-2-tetrahydrofurancarboxylate;
(15) sodium 2-{(4S)-4-[2-(4-pyridylthio)acetamido]-3-oxo-2-isoxazolidinyl)-5-oxo-2-tetrahydrofurancarboxylate;
(16) methyl 2-[(4S)-4-acetamido-3-oxo-2-isoxazolidinyl]-5-oxo-2-tetrahydrofurancarboxylate;
(17) pivaloyloxymethyl 2-[(4S)-4-acetamido-3-oxo-2-isoxazolidinyl]-5-oxo-2-tetrahydrofurancarboxylate;
(18) 3,5-dinitrobenzyl 2-[(4S)-4-acetamido-3-oxo-2-isoxazolidinyl]-5-oxo-2-tetrahydrofurancarboxylate;
(19) p-bromophenacyl 2-[(4S)-4-acetamido-3-oxo-2-isoxazolidinyl]-5-oxo-2-tetrahydrofurancarboxylate;
(20) p-bromobenzyl 2-[(4S)-4-acetamido-3-oxo-2-isoxazolidinyl]-5-oxo-2-tetrahydrofurancarboxylate;
(21) sodium 2-[(4S)-4-formylamino-3-oxo-2-isoxazolidinyl]-5-oxo-2-tetrahydrofurancarboxylate;
(22) sodium 2-[(4S)-4-n-butyrylamino-3-oxo-2-isoxazolidinyl]-5-oxo-2-tetrahydrofurancarboxylate;
(23) sodium 2-[(4S)-4-methoxycarbonylamino-3-oxo-2-isoxazolidinyl]-5-oxo-2-tetrahydrofurancarboxylate;
(24) sodium 2-[(4S)-4-(N-benzyloxycarbonyl)-glycylamino-3-oxo-2-isoxazolidinyl]-5-oxo-2-tetrahydrofurancarboxylate;
(25) sodium 2-[(4S)-4-benzoylamino-3-oxo-2-isoxazolidinyl]-5-oxo-2-tetrahydrofurancarboxylate;
(26) sodium 2-[(4S)-4-phenylacetylamino-3-oxo-2-isoxazolidinyl]-5-oxo-2-tetrahydrofurancarboxylate;

(27) sodium 2-[(4S)-4-nicotinylamino-3-oxo-2-isoxazolidinyl]-5-oxo-2-tetrahydrofurancarboxylate;
(28) pivaloyloxymethyl 2-[(4S)-4-phenoxyacetylamino-3-oxo-2-isoxazolidinyl]-5-oxo-2-tetrahydrofurancarboxylate;
(29) sodium 2-[(4S)-4-(p-toluenesulfonyl)amino-3-oxo-2-isoxazolidinyl]-5-oxo-2-tetrahydrofurancarboxylate;
(30) diphenylmethyl 2-[(4S)-4-(p-bromobenzoyl)-amino-3-oxo-2-isoxazolidinyl]-5-oxo-2-tetrahydrofurancarboxylate;
(31) diphenylmethyl 2-[(4S)-4-(N-benzyloxy-carbonyl-D-alanyl)amino-3-oxo-2-isoxazolidinyl]-5-oxo-2-tetrahydrofurancarboxylate;
(32) 2-[(4S)-4-dimethylamino-3-oxo-2-isoxazolidinyl]-5-oxo-2-tetrahydrofurancarboxylic acid;
(33) sodium 2-{(4S)-4-[2-(2-amino-4-thiazolyl)acetamido]-3-oxo-2-isoxazolidinyl}-5-oxo-2-tetrahydrofurancarboxylate;
(34) sodium 2-[4-methoxy-4-(2-thienylacetamido)-3-oxo-2-isoxazolidinyl]-5-oxo-2-tetrahydrofurancarboxylate;
(35) disodium 2-[(4S)-4-[2-(2-amino-4-thiazolyl)-(Z)-2-(carboxymethyloxyimino)acetamido]-3-oxo-2-isoxazolidinyl}-5-oxo-2-tetrahydrofurancarboxylate;
(38) 2-{(4S)-4-[2-(2-aminomethyl)phenylacetamido]-3-oxo-2-isoxazolidinyl}-5-oxo-2-tetrahydrofurancarboxylic acid;
(39) sodium 2-[(4S)-4-(4-fluorobenzenesulfonyl-amino-3-oxo-2-isoxazolidinyl]-5-oxo-2-tetrahydrofurancarboxylate;
(40) sodium 2-{(4S)-4-(2-[1-(2-dimethylaminoethyl)-1H-5-tetrazolylthio]acetamido}-3-oxo-2-isoxazolidinyl}-5-oxo-2-tetrahydrofurancarboxylate;
(41) sodium 2-{(4S)-4-[2-(4-methyl-4H-1,2,4-triazolyl-3-ylthio)acetamido]-3-oxo-2-isoxazolidinyl}-5-oxo-2-tetrahydrofurancarboxylate;
(42) sodium 2-{(4S)-4-[(2R,3S)-2-(4-ethyl-2,3-dioxo-1-piperazinecarboxamido)-3-formyloxybutylamido]-3-oxo-2-isoxazolidinyl}-5-oxo-2-tetrahydrofurancarboxylate;
(43) disodium 2-[(4S)-4-(2-phenyl-2-sulfoacetamido)-3-oxo-2-isoxazolidinyl]-5-oxo-2-tetrahydrofurancarboxylate;
(44) sodium 2-{(4S)-4-[2-(2-amino-4-thiazolyl)-(E)-2-methoxyiminoacetamido]-3-oxo-2-isoxazolidinyl}-5-oxo-2-tetrahydrofurancarboxylate;
(45) sodium 2-[(4S)-4-(2-trimethylsilyl)-ethoxycarbonylamino-3-oxo-2-isoxazolidinyl]-5-oxo-2-tetrahydrofurancarboxylate;
(46) sodium 2-[(4S)-4-(1-aminocyclohexylcarbonylamino)-3-oxo-2-isoxazolidinyl]-5-oxo-2-tetrahydrofurancarboxylic acid;
(47) sodium 2-{(4S)-4-[3-(2,6-dichlorophenyl)-5-methyl-4-isoxazolylcarbonylamino]-3-oxo-2-isoxazolidinyl}-5-oxo-2-tetrahydrofurancarboxylate;
(48) sodium 2-{(4S)-4-[2-(2-pyridylthio)acetamido]-3-oxo-2-isoxazolidinyl}-5-oxo-2-tetrahydrofurancarboxylate;
(49) sodium 2-{(4S)-4-[2-(4-pyridylmethylthio)-acetamido]-3-oxo-2-isoxazolidinyl}-5-oxo-2-tetrahydrofurancarboxylate;
(50) sodium 2-{(4S)-4-[2-(2-pyrimidinylthio)-acetamido]-3-oxo-2-isoxazolidinyl}-5-oxo-2-tetrahydrofurancarboxylate;
(51) sodium 2-{(4S)-4-{2-[(E)-2-(acetamidovinyl)thio]acetamido}-3-oxo-2-isoxazolidinyl}-5-oxo-2-tetrahydrofurancarboxylate;
(52) sodium 2-[(4S)-4-cyclopropylcarbonylamino-3-oxo-2-isoxazolidinyl]-5-oxo-2-tetrahydrofurancarboxylate;
(53) sodium 2-[(4S)-4-methoxalylamino-3-oxo-2-isoxazolidinyl]-5-oxo-2-tetrahydrofurancarboxylate;
(54) sodium 2-[(4S)-4-acrylamido-3-oxo-2-isoxazolidinyl]-5-oxo-2-tetrahydrofurancarboxylate;
(55) sodium 2-{(4S)-4-[2-(2-chloro-4-pyridylthio)acetamido]-3-oxo-2-isoxazolidinyl}-5-oxo-2-tetrahydrofurancarboxylate;
(56) sodium 2-[(4S)-4-(2-acetamido-5-oxo-2-tetrahydrofurancarbonylamino)-3-oxo-2-isoxazolidinyl]-5-oxo-2-tetrahydrofurancarboxylate;
(57) sodium 2-{(4S)-4-[2-(1-pyrazolyl)acetamido]-3-oxo-2-isoxazolidinyl}-5-oxo-2-tetrahydrofurancarboxylate;
(58) sodium 2-{(4S)-4-[2-(5-amino-1,2,4-thiadiazole-3-yl)-(Z)-2-ethoxyiminoacetamido]-3-oxo-2-isoxazolidinyl}-5-oxo-2-tetrahydrofurancarboxylate;
(59) sodium 2-{(4S)-4-[2-(3-pyridazinylthio)-acetamido]-3-oxo-2-isoxazolidinyl}-5-oxo-2-tetrahydrofurancarboxylate;

(60) sodium 2-[(4S)-4-dichloroacetamido-3-oxo-2-isoxazolidinyl]-5-oxo-2-tetrahydrofurancarboxylate;
(61) sodium 2-{(4S)-4-[(2R,3S)-2-(4-ethyl-2,3-dioxo-1-piperazinecarboxamido)-3-hydroxybutyramido]-3-oxo-2-isoxazolidinyl}-5-oxo-2-tetrahydrofurancarboxylate;
(62) sodium 2-{(4S)-4-[2-(5-trifluoromethyl-1,3,4-thiadiazol-2-ylthio)acetamido]-3-oxo-2-isoxazolidinyl}-5-oxo-2-tetrahydrofurancarboxylate;
(63) sodium 2-{(4S)-4-{2-[1-ethoxy(hydroxy)phosphinylmethyl-1H-5-tetrazolylthio]acetamido}-3-oxo-2-isoxazolidinyl}-5-oxo-2-tetrahydrofurancarboxylate;
(64) sodium 2-{(4S)-4-{2-[5-(2-diethoxyphosphinylethylthio)-1,3,4-thiadiazolylthio]acetamido}-3-oxo-2-isoxazolidinyl}-5-oxo-2-tetrahydrofurancarboxylate;
(65) sodium 2-{(4S)-4-[2-(1-dimethylamino-1H-5-tetrazolylthio)acetamido]-3-oxo-2-isoxazolidinyl}-5-oxo-2-tetrahydrofurancarboxylate;
(66) sodium 2-{(4S)-4-[2-(4,5-dimethyl-2-thiazolylthio)acetamido]-3-oxo-2-isoxazolidinyl}-5-oxo-2-tetrahydrofurancarboxylate;
(67) sodium 2-{(4S)-4-[2-(4,5-dimethyl-2-oxa-zolylthio)acetamido]-3-oxo-2-isoxazolidinyl}-5-oxo-2-tetrahydrofurancarboxylate;
(68) sodium 2-{(4S)-4-[2-(5-methoxymethyl-1,3,4-thiadiazol-2-ylthio)acetamido]-3-oxo-2-isoxazolidinyl}-5-oxo-2-tetrahydrofurancarboxylate;
(69) sodium 2-{(4S)-4-[2-(5-methylsulfonyl-methyl-1,3,4-thiadiazol-2-ylthio)acetamido]-3-oxo-2-isoxazolidinyl}-5-oxo-2-tetrahydrofurancarboxylate;
(70) sodium 2-{(4S)-4-[2-(4-ethyl-4H-1,2,4-triazol-3-ylthio)acetamido]-3-oxo-2-isoxazolidinyl}-5-oxo-2-tetrahydrofurancarboxylate;
(71) disodium 2-{{4S)-4-[2-(5-carboxymethyl-1,3,4-thiadiazol-2-ylthio)acetamido]-3-oxo-2-isoxazolidinyl}-5-oxo-2-tetrahydrofurancarboxylate;
(72) sodium 2-{(4S)-4-{2-[1-(2-hydroxyethyl)-1H-5-tetrazolylthio]acetamido}-3-oxo-2-isoxazolidinyl}-5-oxo-2-tetrahydrofurancarboxylate;
(73) sodium 2-{(4S)-4-{2-[1-(3-dimethylamino-propyl)-1H-5-tetrazolylthio]acetamido}-3-oxo-2-isoxazolidinyl}-5-oxo-2-tetrahydrofurancarboxylate;
(74) 2-[(4S)-4-(2-amino-3-sulfamoylpropionamido)-3-oxo-2-isoxazolidinyl]-5-oxo-2-tetrahydrofurancarboxylic acid;
(75) sodium 2-{(4S)-4-[2-(4-cyano-3-hydroxy-5-isothiazolylthio)acetamido]-3-oxo-2-isoxazolidinyl}-5-oxo-2-tetrahydrofurancarboxylate;
(76) sodium 2-{(4S)-4-[2-(2-amino-4-thiazolyl)-2-oxoacetamido]-3-oxo-2-isoxazolidinyl}-5-oxo-2-tetrahydrofurancarboxylate;
(77) sodium 2-{(4S)-4-[2-(2-amino-4-thiazolyl)-(Z)-2-isopropoxyiminoacetamido]-3-oxo-2-isoxazolidinyl}-5-oxo-2-tetrahydrofurancarboxylate;
(78) sodium 2-[(4S)-4-trifluoroacetamido-3-oxo-2-isoxazolidinyl]-5-oxo-2-tetrahydrofurancarboxylate;
(79) disodium 2-{(4S)-4-[2-(2-amino-4-thiazolyl)-(Z)-2-(1-carboxy-1-methylethoxyimino)acetamido]-3-oxo-2-isoxazolidinyl}-5-oxo-2-tetrahydrofurancarboxylate;
(80) sodium 2-{(4S)-4-[2-(2-amino-4-thiazolyl)-(Z)-2-(hydroxyimino)acetamido]-3-oxo-2-isoxazolidinyl}-5-oxo-2-tetrahydrofurancarboxylate;
(81) sodium 2-[(4S)-4-isobutyloxycarbonylamino-3-oxo-2-isoxazolidinyl]-5-oxo-2-tetrahydrofurancarboxylate;
(82) diphenylmethyl 2-{(4S)-4-[N-(4-nitrobenzyloxycarbonyl)glycylamino]-3-oxo-2-isoxazolidinyl}-5-oxo-2-tetrahydrofurancarboxylate;
(83) diphenylmethyl 2-{(4S)-4-[N-(2,2,2-trichloroethoxycarbonyl)phenylglycylamino]-3-oxo-2-isoxazolidinyl}-5-oxo-2-tetrahydrofurancarboxylate;
(84) sodium 2-{(4S)-4-[N-(benzyloxycarbonyl)-D-phenylglycylamino]-3-oxo-2-isoxazolidinyl}-5-oxo-2-tetrahydrofurancarboxylate;
(85) 2-[(4S)-4-L-phenylglycylamino-3-oxo-2-isoxazolidinyl]-5-oxo-2-tetrahydrofurancarboxylic acid;
(86) 2-{(4S)-4-D-phenylglycylamino-3-oxo-2-isoxazolidinyl}-5-oxo-2-tetrahydrofurancarboxylic acid;
(87) sodium 2-{(4S)-4-[(2S)-(2-hydroxy-2-phenyl)acetamido]-3-oxo-2-isoxazolidinyl}-5-oxo-2-tetrahydrofurancarboxylate;
(88) sodium 2-{(4S)-4-[(2R)-(2-hydroxy-2-phenyl)acetamido]-3-oxo-2-isoxazolidinyl}-5-oxo-2-tetrahydrofurancarboxylate;
(89) sodium 2-{(4S)-4-[2-(4-chlorophenyl)-2-hydroxyacetamido]-3-oxo-2-isoxazolidinyl}-5-oxo-2-tetrahydrofurancarboxylate;
(90) sodium 2-{(4S)-4-[2-hydroxy-2-(4-hydroxyphenyl)-acetamido]-3-oxo-2-isoxazolidinyl}-5-oxo-2-tetrahydrofurancarboxylate;

(91) sodium 2-[(4S)-4-(2-hydroxypropionamido)-3-oxo-2-isoxazolidinyl]-5-oxo-2-tetrahydrofurancarboxylate;
(92) disodium 2-[(4S)-4-(2-carboxy-2-phenylacetamido)-3-oxo-2-isoxazolidinyl]-5-oxo-2-tetrahydrofurancarboxylate;
(93) sodium 2-[(4S)-4-benzyloxycarbonylamino-3-oxo-2-isoxazolidinyl]-5-oxo-2-tetrahydrofurancarboxylate;
(94) sodium 2-((4S)-4-pyruvamido-3-oxo-2-isoxazolidinyl)-5-oxo-2-tetrahydrofurancarboxylate;
(95) piveloyloxymethyl 2-{(4S)-4-[(2R)-(2-hydroxy-2-phenyl)acetamido]-3-oxo-2-isoxazolidinyl}-5-oxo-2-tetrahydrofurancarboxylate;
(96) diphenylmethyl 2-[(4S)-4-(4-nitrobenzylidene)amino-3-oxo-2-isoxazolidinyl]-5-oxo-2-tetrahydrofurancarboxylate;
(97) sodium 2-{(4S)-4-[2-(3-chloro-6-pyridazinylthio)acetamido]-3-oxo-2-isoxazolidinyl}-5-oxo-2-tetrahydrofurancarboxylate;
(98) sodium 2-[(4S)-4-(2-phenylthioacetamido)-3-oxo-2-isoxazolidinyl]-5-oxo-2-tetrahydrofurancarboxylate;
(99) sodium 2-[(4S)-4-(2-ethoxydithiocarbonylacetamido)-3-oxo-2-isoxazolidinyl]-5-oxo-2-tetrahydrofurancarboxylate;
(100) sodium 2-{(4S)-4-[2-(2-amino-4-thiazolyl)-(Z)-2-(ethoxyimino)acetamido]-3-oxo-2-isoxazolidinyl}-5-oxo-2-tetrahydrofurancarboxylate;
(101) sodium 2-{(4S)-4-[2-(2-amino-5-chloro-4-thiazolyl)-(Z)-2-(methoxyimino)acetamido]-3-oxo-2-isoxazolidinyl}-5-oxo-2-tetrahydrofurancarboxylate;
(102) sodium 2-{(4S)-4-[2-4-ethyl-2,3-dioxo-1-piperazinocarboxamido)-2-thienylacetamido]-3-oxo-2-isoxazolidinyl}-5-oxo-2-tetrahydrofurancarboxylate;
(103) sodium 2-((4S)-4-ureido-3-oxo-2-isoxazolidinyl)-5-oxo-2-tetrahydrofurancarboxylate;
(104) sodium 2-((4S)-4-phenylureylene-3-oxo-2-isoxazolidinyl)-5-oxo-2-tetrahydrofurancarboxylate;
(105) sodium 2-{(4S)-4-[2-(pyridyl)acetamido]-3-oxo-2-isoxazolidinyl}-5-oxo-2-tetrahydrofurancarboxylate;
(106) sodium 2-{(4S)-4-[2-(4-chlorophenyl)acetamido]-3-oxo-2-isoxazolidinyl}-5-oxo-2-tetrahydrofurancarboxylate;
(107) sodium 2-{(4S)-4-[(2-chloro-2-phenyl)acetamido]-3-oxo-2-isoxazolidinyl}-5-oxo-2-tetrahydrofurancarboxylate;
(108) sodium 2-{(4S)-4-[2-(3-pyridylthio)acetamido]-3-oxo-2-isoxazolidinyl}-5-oxo-2-tetrahydrofurancarboxylate;
(109) sodium 2-{(4S)-4-[2-(4-chlorophenylthio)acetamido]-3-oxo-2-isoxazolidinyl}-5-oxo-2-tetrahydrofurancarboxylate;
(110) sodium 2-[(4S)-4-(2-isopropylphenoxy-carbonylamino)-3-oxo-2-isoxazolidinyl]-5-oxo-2-tetrahydrofurancarboxylate;
(111) sodium 2-((4S)-4-difluoroacetamido-3-oxo-2-isoxazolidinyl)-5-oxo-2-tetrahydrofurancarboxylate;
(112) sodium 2-[(4S)-4-(3-chloro)propionamido-3-oxo-2-isoxazolidinyl]-5-oxo-2-tetrahydrofurancarboxylate;
(113) pivaloyloxymethyl 2-((4S)-4-chloroacetamido-3-oxo-2-isoxazolidinyl)-5-oxo-2-tetrahydrofurancarboxylate;
(114) pivaloyloxymethyl 2-[(4S)-4-(2-phenylthioacetamido)-3-oxo-2-isoxazolidinyl]-5-oxo-2-tetrahydrofurancarboxylate;
(115) sodium 2-((4S)-4-propionamido-3-oxo-2-isoxazolidinyl)-5-oxo-2-tetrahydrofurancarboxylate;
(116) pivaloyloxymethyl 2-((4S)-4-benzyloxy-carbonylamino-3-oxo-2-isoxazolidinyl)-5-oxo-2-tetrahydrofurancarboxylate;
(117) pivaloyloxymethyl 2-((4S)-4-bromoacetamido-3-oxo-2-isoxazolidinyl)-5-oxo-2-tetrahydrofurancarboxylate;
(118) pivaloyloxymethyl 2-[(4S)-4-(2,4-hexadiene)amido-3-oxo-2-isoxazolidinyl]-5-oxo-2-tetrahydrofurancarboxylate;
(119) pivaloyloxymethyl 2-[(4S)-4-(2-thienyl)-acetamido-3-oxo-2-isoxazolidinyl]-5-oxo-2-tetrahydrofurancarboxylate;
(120) pivaloyloxymethyl 2-[(4S)-4-(hexahydro-1H-azepin-1-yl)methyleneamino-3-oxo-2-isoxazolidinyl]-5-oxo-2-tetrahydrofurancarboxylate;
(121) diphenylmethyl 2-[(4S)-4-(tert-butyl-dimethylsilyl)amino-3-oxo-2-isoxazolidinyl]-5-oxo-2-tetrahydrofurancarboxylate;
(122) diphenylmethyl 2-[(4S)-4-tert-butyl-diphenylsilyl)amino-3-oxo-2-isoxazolidinyl]-5-oxo-2-tetrahydrofurancarboxylate;
(123) sodium 2-[(4S)-3-diphenylphosphorylamino-3-oxo-2-isoxazolidinyl]-5-oxo-2-tetrahydrofurancarboxylate;
(124) diphenylmethyl 2-[(4S)-4-dimethyl-phosphorylamino-3-oxo-2-isoxazolidinyl]-5-oxo-2-tetrahydrofurancarboxylate;
(125) sodium 2-[(4S)-4-ethylsulfonylamino-3-oxo-2-isoxazolidinyl]-5-oxo-2-tetrahydrofuran-carboxylate;
(126) sodium 2-[(4S)-4-vinylsulfonylamino-3-oxo-2-isoxazolidinyl]-5-oxo-2-tetrahydrofurancarboxylate;
(127) 2-[(4S)-4-thienylacetamido-3-oxo-2-isoxazolidinyl]-5-oxo-2-tetrahydrofurancarbonylanilide;
(128) 2-[(4S)-4-benzyloxycarbonylamino-3-oxo-2-isoxazolidinyl]-5-oxo-2-tetrahydrofurancarbonylanilide;
(129) 2-[(4S)-4-benzyloxycarbonylamino-3-oxo-2-isoxazolidinyl]-5-oxo-2-tetrahydrofurancarbonylpyrrolidinamide;
(130) 2-[(4S)-4-thienylacetamido-3-oxo-2-isoxazolidinyl]-5-oxo-2-tetrahydrofurancarbonylpyrrolidinamide;
(131) 2-[(4S)-4-thienylacetamido-3-oxo-2-isoxazolidinyl]-5-oxo-2-tetrahydrofurancarbonylpropylamine;
(132) sodium 2-[(4S)-4-γ-D-glutamylamino-3-oxo-2-isoxazolidinyl]-5-oxo-2-tetrahydrofurancarboxylate; and
(133) sodium 2-[(4S)-4-(2-hydroxy)isobutyryl-amino-3-oxo-2-isoxazolidinyl]-5-oxo-2-tetrahydrofurancarboxylate;

**2.** A pharmaceutical composition for inhibiting the growth of microorganisms which contains an effective amount of a compound (I'), or a pharmaceutically acceptable salts thereof, as defined in Claim 1, together with a pharmaceutically acceptable carrier or diluent therefor.

**3.** A compound (I'), or a pharmaceutically acceptable salt thereof, as defined in Claim 1, for use in the preparation of a pharmaceutical composition for inhibiting the growth of microorganisms.

**Claims for the following Contracting State : AT**

**1.** A pharmaceutical composition for inhibiting the growth of microorganisms, which contains an effective amount of a 2-((4S)-amino-3-oxo-2-isoxazolidinyl)-5-oxo-2-tetrahydrofurancarboxylic acid derivative, compound (I'), or a pharmaceutically acceptable salt thereof, selected from:
(1) sodium 2-{(4S)-4-[2-(2-amino-4-thiazolyl)-(Z)-2-(methoxyimino)acetamido]-3-oxo-2-isoxazolidinyl}-5-oxo-2-tetrahydrofurancarboxylate;
(2) sodium 2-{(4S)-4-(D-2-(4-ethyl-2,3-dioxo-1-piperazinocarboxamido)-2-phenylacetamido]-3-oxo-2-isoxazolidinyl}-5-oxo-2-tetrahydrofurancarboxylate;
(3) sodium 2-{(4S)-4-[D-2-(3-methylsulfonyl-2-oxoimidazolidine-1-carboxamido)-2-phenylacetamido]-3-oxo-2-isoxazolidinyl}-5-oxo-2-tetrahydrofurancarboxylate;
(4) sodium 2-{(4S)-4-[D-2-(3-furfurylidene-amino-2-oxoimidazolidine-1-carboxamido-2-phenylacetamido]-3-oxo-2-isoxazolidinyl}-5-oxo-2-tetrahydrofurancarboxylate;
(5) sodium 2-[(4S)-4-(2-thienylacetamido)-3-oxo-2-isoxazolidinyl]-5-oxo-2-tetrahydrofurancarboxylate;
(6) sodium 2-[(4S)-4-(2-cyanoacetamido)-3-oxo-2-isoxazolidinyl]-5-oxo-2-tetrahydrofurancarboxylate;
(7) sodium 2-{(4S)-4-[(1H)-tetrazolylacetamido]-3-oxo-2-isoxazolidinyl}-5-oxo-2-tetrahydrofurancarboxylate;
(8) sodium 2-[(4S)-4-(2-6-dimethoxybenzamido)-3-oxo-2-isoxazolidinyl]-5-oxo-2-tetrahydrofurancarboxylate;
(9) sodium 2-[(4S)-4-(5-methyl-3-phenyl-2-isoxazoline -4-carboxamido)-3-oxo-2-isoxazolidinyl]-5-oxo-2-tetrahydrofurancarboxylate;
(10) sodium 2-[(4S)-4-(2-ethoxynaphthalenyl-1-carboxamido)-3-oxo-2-isoxazolidinyl]-5-oxo-2-tetrahydrofurancarboxylate;
(11) sodium 2-[(4S)-4-(2-cyanomethylthioacetamido)-3-oxo-2-isoxazolidinyl]-5-oxo-2-tetrahydrofurancarboxylate;
(12) sodium 2-[(4S)-4-(2-difluoromethylthioacetamido)-3-oxo-2-isoxazolidinyl]-5-oxo-2-tetrahydrofurancarboxylate;
(13) sodium 2-{(4S)-4-[(R)-2-(4-cyclohexyl-2,3-dioxo-1-piperazinocarboxamido-2-phenylacetamido]-3-oxo-2-isoxazolidinyl}-5-oxo-2-tetrahydrofurancarboxylate;
(14) sodium 2-((4S)-4-chloroacetamido-3-oxo-2-isoxazolidinyl)-5-oxo-2-tetrahydrofurancarboxylate;

(15) sodium 2-{(4S)-4-[2-(4-pyridylthio)acetamido]-3-oxo-2-isoxazolidinyl}-5-oxo-2-tetrahydrofurancarboxylate;
(16) methyl 2-[(4S)-4-acetamido-3-oxo-2-isoxazolidinyl]-5-oxo-2-tetrahydrofurancarboxylate;
(17) pivaloyloxymethyl 2-[(4S)-4-acetamido-3-oxo-2-isoxazolidinyl]-5-oxo-2-tetrahydrofurancarboxylate;
(18) 3,5-dinitrobenzyl 2-[(4S)-4-acetamido-3-oxo-2-isoxazolidinyl]-5-oxo-2-tetrahydrofurancarboxylate;
(19) p-bromophenacyl 2-[(4S)-4-acetamido-3-oxo-2-isoxazolidinyl]-5-oxo-2-tetrahydrofurancarboxylate;
(20) p-bromobenzyl 2-[(4S)-4-acetamido-3-oxo-2-isoxazolidinyl]-5-oxo-2-tetrahydrofurancarboxylate;
(21) sodium 2-[(4S)-4-formylamino-3-oxo-2-isoxazolidinyl]-5-oxo-2-tetrahydrofurancarboxylate;
(22) sodium 2-[(4S)-4-n-butyrylamino-3-oxo-2-isoxazolidinyl]-5-oxo-2-tetrahydrofurancarboxylate;
(23) sodium 2-[(4S)-4-methoxycarbonylamino-3-oxo-2-isoxazolidinyl]-5-oxo-2-tetrahydrofurancarboxylate;
(24) sodium 2-[(4S)-4-(N-benzyloxycarbonyl)-glycylamino-3-oxo-2-isoxazolidinyl]-5-oxo-2-tetrahydrofurancarboxylate;
(25) sodium 2-[(4S)-4-benzoylamino-3-oxo-2-isoxazolidinyl]-5-oxo-2-tetrahydrofurancarboxylate;
(26) sodium 2-[(4S)-4-phenylacetylamino-3-oxo-2-isoxazolidinyl]-5-oxo-2-tetrahydrofurancarboxylate;
(27) sodium 2-[(4S)-4-nicotinylamino-3-oxo-2-isoxazolidinyl]-5-oxo-2-tetrahydrofurancarboxylate;
(28) pivaloyloxymethyl 2-[(4S)-4-phenoxyacetylamino3-oxo-2-isoxazolidinyl]-5-oxo-2-tetrahydrofurancarboxylate;
(29) sodium 2-[(4S)-4-(p-toluenesulfonyl)amino-3-oxo-2-isoxazolidinyl]-5-oxo-2-tetrahydrofurancarboxylate;
(30) diphenylmethyl 2-[(4S)-4-(p-bromobenzoyl)-amino-3-oxo-2-isoxazolidinyl]-5-oxo-2-tetrahydrofurancarboxylate;
(31) diphenylmethyl 2-[(4S)-4-(N-benzyloxy-carbonyl-D-alanyl)amino-3-oxo-2-isoxazolidinyl]-5-oxo-2-tetrahydrofurancarboxylate;
(32) 2-[(4S)-4-dimethylamino-3-oxo-2-isoxazolidinyl]-5-oxo-2-tetrahydrofurancarboxylic acid;
(33) sodium 2-{(4S)-4-[2-(2-amino-4-thiazolyl)acetamido]-3-oxo-2-isoxazolidinyl}-5-oxo-2-tetrahydrofurancarboxylate;
(34) sodium 2-[4-methoxy-4-(2-thienylacetamido)-3-oxo-2-isoxazolidinyl]-5-oxo-2-tetrahydrofurancarboxylate;
(35) disodium 2-[(4S)-4-[2-(2-amino-4-thiazolyl)-(Z)-2-(carboxymethyloxyimino)acetamido]-3-oxo-2-isoxazolidinyl}-5-oxo-2-tetrahydrofurancarboxylate;
(38) 2-{(4S)-4-[2-(2-aminomethyl)phenylacetamido]-3-oxo-2-isoxazolidinyl}-5-oxo-2-tetrahydrofurancarboxylic acid;
(39) sodium 2-[(4S)-4-(4-fluorobenzenesulfonyl-amino-3-oxo-2-isoxazolidinyl]-5-oxo-2-tetrahydrofurancarboxylate;
(40) sodium 2-{(4S)-4-{2-[1-(2-dimethylaminoethyl)-1H-5-tetrazolylthio]acetamido}-3-oxo-2-isoxazolidinyl)-5-oxo-2-tetrahydrofurancarboxylate;
(41) sodium 2-{(4S)-4-[2-(4-methyl-4H-1,2,4-triazolyl-3-ylthio)acetamido]-3-oxo-2-isoxazolidinyl}-5-oxo-2-tetrahydrofurancarboxylate;
(42) sodium 2-{(4S)-4-[(2R,3S)-2-(4-ethyl-2,3-dioxo-1-piperazinecarboxamido)-3-formyloxybutylamido]-3-oxo-2-isoxazolidinyl}-5-oxo-2-tetrahydrofurancarboxylate;
(43) disodium 2-[(4S)-4-(2-phenyl-2-sulfoacetamido)-3-oxo-2-isoxazolidinyl]-5-oxo-2-tetrahydrofurancarboxylate;
(44) sodium 2-{(4S)-4-[2-(2-amino-4-thiazolyl)-(E)-2-methoxyiminoacetamido]-3-oxo-2-isoxazolidinyl}-5-oxo-2-tetrahydrofurancarboxylate;
(45) sodium 2-[(4S)-4-(2-trimethylsilyl)-ethoxycarbonylamino-3-oxo-2-isoxazolidinyl]-5-oxo-2-tetrahydrofurancarboxylate;
(46) sodium 2-[(4S)-4-(1-aminocyclohexylcarbonylamino)-3-oxo-2-isoxazolidinyl]-5-oxo-2-tetrahydrofurancarboxylic acid;
(47) sodium 2-{(4S)-4-[3-(2,6-dichlorophenyl)-5-methyl-4-isoxazolylcarbonylamino]-3-oxo-2-isoxazolidinyl}-5-oxo-2-tetrahydrofurancarboxylate;
(48) sodium 2-{(4S)-4-[2-(2-pyridylthio)acetamido]-3-oxo-2-isoxazolidinyl}-5-oxo-2-tetrahydrofurancarboxylate;
(49) sodium 2-{(4S)-4-[2-(4-pyridylmethylthio)-acetamido]-3-oxo-2-isoxazolidinyl}-5-oxo-2-tetrahydrofurancarboxylate;
(50) sodium 2-{(4S)-4-[2-(2-pyrimidinylthio)-acetamido]-3-oxo-2-isoxazolidinyl}-5-oxo-2-

tetrahydrofurancarboxylate;
(51) sodium 2-{(4S)-4-{2-[(E)-2-(acetamidovinyl)thio]acetamido}-3-oxo-2-isoxazolidinyl}-5-oxo-2-tetrahydrofurancarboxylate;
(52) sodium 2-[(4S)-4-cyclopropylcarbonylamino-3-oxo-2-isoxazolidinyl]-5-oxo-2-tetrahydrofurancarboxylate;
(53) sodium 2-[(4S)-4-methoxalylamino-3-oxo-2-isoxazolidinyl]-5-oxo-2-tetrahydrofurancarboxylate;
(54) sodium 2-[(4S)-4-acrylamido-3-oxo-2-isoxazolidinyl]-5-oxo-2-tetrahydrofurancarboxylate;
(55) sodium 2-{(4S)-4-[2-(2-chloro-4-pyridylthio)acetamido]-3-oxo-2-isoxazolidinyl}-5-oxo-2-tetrahydrofurancarboxylate;
(56) sodium 2-[(4S)-4-(2-acetamido-5-oxo-2-tetrahydrofurancarbonylamino)-3-oxo-2-isoxazolidinyl]-5-oxo-2-tetrahydrofurancarboxylate;
(57) sodium 2-{(4S)-4-[2-(1-pyrazolyl)acetamido]-3-oxo-2-isoxazolidinyl}-5-oxo-2-tetrahydrofurancarboxylate;
(58) sodium 2-{(4S)-4-[2-(5-amino-1,2,4-thiadiazole-3-yl)-(Z)-2-ethoxyiminoacetamido]-3-oxo-2-isoxazolidinyl}-5-oxo-2-tetrahydrofurancarboxylate;
(59) sodium 2-{(4S)-4-[2-(3-pyridazinylthio)-acetamido]-3-oxo-2-isoxazolidinyl}-5-oxo-2-tetrahydrofurancarboxylate;
(60) sodium 2-[(4S)-4-dichloroacetamido-3-oxo-2-isoxazolidinyl]-5-oxo-2-tetrahydrofurancarboxylate;
(61) sodium 2-{(4S)-4-[(2R,3S)-2-(4-ethyl-2,3-dioxo-1-piperazinecarboxamido)-3-hydroxybutyramido]-3-oxo-2-isoxazolidinyl}-5-oxo-2-tetrahydrofurancarboxylate;
(62) sodium 2-{(4S)-4-[2-(5-trifluoromethyl-1,3,4-thiadiazol-2-ylthio)acetamido]-3-oxo-2-isoxazolidinyl)-5-oxo-2-tetrahydrofurancarboxylate;
(63) sodium 2-{(4S)-4-{2-[1-ethoxy(hydroxy)phosphinylmethyl-1H-5-tetrazolylthio]acetamido}-3-oxo-2-isoxazolidinyl}-5-oxo-2-tetrahydrofurancarboxylate;
(64) sodium 2-{(4S)-4-{2-[5-(2-diethoxyphosphinylethylthio)-1,3,4-thiadiazolylthio]acetamido}-3-oxo-2-isoxazolidinyl}-5-oxo-2-tetrahydrofurancarboxylate;
(65) sodium 2-{(4S)-4-[2-(1-dimethylamino-1H-5-tetrazolylthio)acetamido]-3-oxo-2-isoxazolidinyl}-5-oxo-2-tetrahydrofurancarboxylate;
(66) sodium 2-{(4S)-4-[2-(4,5-dimethyl-2-thiazolylthio)acetamido]-3-oxo-2-isoxazolidiny1}-5-oxo-2-tetrahydrofurancarboxylate;
(67) sodium 2-{(4S)-4-[2-(4,5-dimethyl-2-oxa-zolylthio)acetamido]-3-oxo-2-isoxazolidinyl}-5-oxo-2-tetrahydrofurancarboxylate;
(68) sodium 2-{((4S)-4-[2-(5-methoxymethyl-1,3,4-thiadiazol-2-ylthio)acetamido]-3-oxo-2-isoxazolidinyl}-5-oxo-2-tetrahydrofurancarboxylate;
(69) sodium 2-{(4S)-4-[2-(5-methylsulfonyl-methyl-1,3,4-thiadiazol-2-ylthio)acetamido]-3-oxo-2-isoxazolidinyl}-5-oxo-2-tetrahydrofurancarboxylate;
(70) sodium 2-{(4S)-4-[2-(4-ethyl-4H-1,2,4-triazol-3-ylthio)acetamido]-3-oxo-2-isoxazolidinyl}-5-oxo-2-tetrahydrofurancarboxylate;
(71) disodium 2-{(4S)-4-[2-(5-carboxymethyl-1,3,4-thiadiazol-2-ylthio)acetamido]-3-oxo-2-isoxazolidinyl}-5-oxo-2-tetrahydrofurancarboxylate;
(72) sodium 2-{(4S)-4-{2-[1-(2-hydroxyethyl)-1H-5-tetrazolylthio]acetamido}-3-oxo-2-isoxazolidinyl}-5-oxo-2-tetrahydrofurancarboxylate;
(73) sodium 2-{(4S)-4-{2-[1-(3-dimethylamino-propyl)-1H-5-tetrazolylthio]acetamido}-3-oxo-2-isoxazolidinyl}-5-oxo-2-tetrahydrofurancarboxylate;
(74) 2-[(4S)-4-(2-amino-3-sulfamoylpropionamido)-3-oxo-2-isoxazolidinyl]-5-oxo-2-tetrahydrofurancarboxylic acid;
(75) sodium 2-{(4S)-4-[2-(4-cyano-3-hydroxy-5-isothiazolylthio)acetamido]-3-oxo-2-isoxazolidinyl}-5-oxo-2-tetrahydrofurancarboxylate;
(76) sodium 2-{[(4S)-4-[2-(2-amino-4-thiazolyl)-2-oxoacetamido]-3-oxo-2-isoxazolidinyl}-5-oxo-2-tetrahydrofurancarboxylate;
(77) sodium 2-{(4S)-4-[2-(2-amino-4-thiazolyl)-(Z)-2-isopropoxyiminoacetamido]-3-oxo-2-isoxazolidinyl}-5-oxo-2-tetrahydrofurancarboxylate;
(78) sodium 2-[(4S)-4-trifluoroacetamido-3-oxo-2-isoxazolidinyl]-5-oxo-2-tetrahydrofurancarboxylate;
(79) disodium 2-{(4S)-4-[2-(2-amino-4-thiazolyl)-(Z)-2-(1-carboxy-1-methylethoxyimino)acetamido]-3-oxo-2-isoxazolidinyl}-5-oxo-2-tetrahydrofurancarboxylate;
(80) sodium 2-{(4S)-4-[2-(2-amino-4-thiazolyl)-(Z)-2-(hydroxyimino)acetamido]-3-oxo-2-isoxazolidinyl}-5-oxo-2-tetrahydrofurancarboxylate;
(81) sodium 2-[(4S)-4-isobutyloxycarbonylamino-3-oxo-2-isoxazolidinyl]-5-oxo-2-tetrahydrofurancar-

boxylate;
(82) diphenylmethyl 2-{(4S)-4-[N-(4-nitrobenzyloxycarbonyl)glycylamino]-3-oxo-2-isoxazolidinyl}-5-oxo-2-tetrahydrofurancarboxylate;
(83) diphenylmethyl 2-{(4S)-4-[N-(2,2,2-trichloroethoxycarbonyl)phenylglycylamino]-3-oxo-2-isoxazolidinyl}-5-oxo-2-tetrahydrofurancarboxylate;
(84) sodium 2-{(4S)-4-[N-(benzyloxycarbonyl)-D-phenylglycylamino]-3-oxo-2-isoxazolidinyl}-5-oxo-2-tetrahydrofurancarboxylate;
(85) 2-[(4S)-4-L-phenylglycylamino-3-oxo-2-isoxazolidinyl]-5-oxo-2-tetrahydrofurancarboxylic acid;
(86) 2-{(4S)-4-D-phenylglycylamino-3-oxo-2-isoxazolidinyl}-5-oxo-2-tetrahydrofurancarboxylic acid;
(87) sodium 2-{(4S)-4-[(2S)-(2-hydroxy-2-phenyl)acetamido]-3-oxo-2-isoxazolidinyl}-5-oxo-2-tetrahydrofurancarboxylate;
(88) sodium 2-{(4S)-4-[(2R)-(2-hydroxy-2-phenyl)acetamido]-3-oxo-2-isoxazolidinyl}-5-oxo-2-tetrahydrofurancarboxylate;
(89) sodium 2-{(4S)-4-[2-(4-chlorophenyl)-2-hydroxyacetamido]-3-oxo-2-isoxazolidinyl}-5-oxo-2-tetrahydrofurancarboxylate;
(90) sodium 2-{(4S)-4-[2-hydroxy-2-(4-hydroxyphenyl)-acetamido]-3-oxo-2-isoxazolidinyl}-5-oxo-2-tetrahydrofurancarboxylate;
(91) sodium 2-[(4S)-4-(2-hydroxypropionamido)-3-oxo-2-isoxazolidinyl]-5-oxo-2-tetrahydrofurancarboxylate;
(92) disodium 2-[(4S)-4-(2-carboxy-2-phenylacetamido)-3-oxo-2-isoxazolidinyl]-5-oxo-2-tetrahydrofurancarboxylate;
(93) sodium 2-[(4S)-4-benzyloxycarbonylamino-3-oxo-2-isoxazolidinyl]-5-oxo-2-tetrahydrofurancarboxylate;
(94) sodium 2-((4S)-4-pyruvamido-3-oxo-2-isoxazolidinyl)-5-oxo-2-tetrahydrofurancarboxylate;
(95) pivaloyloxymethyl 2-{(4S)-4-[(2R)-(2-hydroxy-2-phenyl)acetamido]-3-oxo-2-isoxazolidinyl}-5-oxo-2-tetrahydrofurancarboxylate;
(96) diphenylmethyl 2-[(4S)-4-(4-nitrobenzylidene)amino-3-oxo-2-isoxazolidinyl]-5-oxo-2-tetrahydrofurancarboxylate;
(97) sodium 2-{(4S)-4-[2-(3-chloro-6-pyridazinylthio)acetamido]-3-oxo-2-isoxazolidinyl}-5-oxo-2-tetrahydrofurancarboxylate;
(98) sodium 2-[(4S)-4-(2-phenylthioacetamido)-3-oxo-2-isoxazolidinyl]-5-oxo-2-tetrahydrofurancarboxylate;
(99) sodium 2-[(4S)-4-(2-ethoxydithiocarbonylacetamido)-3-oxo-2-isoxazolidinyl]-5-oxo-2-tetrahydrofurancarboxylate;
(100) sodium 2-{(4S)-4-[2-(2-amino-4-thiazolyl)-(Z)-2-(ethoxyimino)acetamido]-3-oxo-2-isoxazolidinyl}-5-oxo-2-tetrahydrofurancarboxylate;
(101) sodium 2-{(4S)-4-[2-(2-amino-5-chloro-4-thiazolyl)-(Z)-2-(methoxyimino)acetamido]-3-oxo-2-isoxazolidinyl}-5-oxo-2-tetrahydrofurancarboxylate;
(102) sodium 2-{(4S)-4-[2-4-ethyl-2,3-dioxo-1-piperazinocarboxamido)-2-thienylacetamido]-3-oxo-2-isoxazolidinyl}-5-oxo-2-tetrahydrofurancarboxylate;
(103) sodium 2-((4S)-4-ureido-3-oxo-2-isoxazolidinyl)-5-oxo-2-tetrahydrofurancarboxylate;
(104) sodium 2-((4S)-4-phenylureylene-3-oxo-2-isoxazolidinyl)-5-oxo-2-tetrahydrofurancarboxylate;
(105) sodium 2-{(4S)-4-[2-(pyridyl)acetamido]-3-oxo-2-isoxazolidinyl}-5-oxo-2-tetrahydrofurancarboxylate;
(106) sodium 2-{(4S)-4-[2-(4-chlorophenyl)acetamido]-3-oxo-2-isoxazolidinyl}-5-oxo-2-tetrahydrofurancarboxylate;
(107) sodium 2-{(4S)-4-[(2-chloro-2-phenyl)acetamido]-3-oxo-2-isoxazolidinyl}-5-oxo-2-tetrahydrofurancarboxylate;
(108) sodium 2-{(4S)-4-[2-(3-pyridylthio)acetamido]-3-oxo-2-isoxazolidinyl}-5-oxo-2-tetrahydrofurancarboxylate;
(109) sodium 2-{(4S)-4-[2-(4-chlorophenylthio)acetamido]-3-oxo-2-isoxazolidinyl}-5-oxo-2-tetrahydrofurancarboxylate;
(110) sodium 2-[(4S)-4-(2-isopropylphenoxy-carbonylamino)-3-oxo-2-isoxazolidinyl]-5-oxo-2-tetrahydrofurancarboxylate;
(111) sodium 2-((4S)-4-difluoroacetamido-3-oxo-2-isoxazolidinyl)-5-oxo-2-tetrahydrofurancarboxylate;
(112) sodium 2-[(4S)-4-(3-chloro)propionamido-3-oxo-2-isoxazolidinyl]-5-oxo-2-tetrahydrofurancarboxylate;
(113) pivaloyloxymethyl 2-((4S)-4-chloroacetamido-3-oxo-2-isoxazolidinyl)-5-oxo-2-tetrahydrofurancar-

boxylate;
(114) pivaloyloxymethyl 2-[(4S)-4-(2-phenylthioacetamido)-3-oxo-2-isoxazolidinyl]-5-oxo-2-tetrahydrofurancarboxylate;
(115) sodium 2-((4S)-4-propionamido-3-oxo-2-isoxazolidinyl)-5-oxo-2-tetrahydrofurancarboxylate;
(116) pivaloyloxymethyl 2-((4S)-4-benzyloxy-carbonylamino-3-oxo-2-isoxazolidinyl)-5-oxo-2-tetrahydrofurancarboxylate;
(117) pivaloyloxymethyl 2-((4S)-4-bromoacetamido-3-oxo-2-isoxazolidinyl)-5-oxo-2-tetrahydrofurancarboxylate;
(118) pivaloyloxymethyl 2-[(4S)-4-(2,4-hexadiene)amido-3-oxo-2-isoxazolidinyl]-5-oxo-2-tetrahydrofurancarboxylate;
(119) pivaloyloxymethyl 2-[(4S)-4-(2-thienyl)-acetamido-3-oxo-2-isoxazolidinyl]-5-oxo-2-tetrahydrofurancarboxylate;
(120) pivaloyloxymethyl 2-[(4S)-4-(hexahydro-1H-azepin-1-yl)methyleneamino-3-oxo-2-isoxazolidinyl]-5-oxo-2-tetrahydrofurancarboxylate;
(121) diphenylmethyl 2-[(4S)-4-(tert-butyl-dimethylsilyl)amino-3-oxo-2-isoxazolidinyl]-5-oxo-2-tetrahydrofurancarboxylate;
(122) diphenylmethyl 2-[(4S)-4-tert-butyl-diphenylsilyl)amino-3-oxo-2-isoxazolidinyl]-5-oxo-2-tetrahydrofurancarboxylate;
(123) sodium 2-[(4S)-3-diphenylphosphorylamino-3-oxo-2-isoxazolidinyl]-5-oxo-2-tetrahydrofurancarboxylate;
(124) diphenylmethyl 2-[(4S)-4-dimethyl-phosphorylamino-3-oxo-2-isoxazolidinyl]-5-oxo-2-tetrahydrofurancarboxylate;
(125) sodium 2-[(4S)-4-ethylsulfonylamino-3-oxo-2-isoxazolidinyl]-5-oxo-2-tetrahydrofurancarboxylate;
(126) sodium 2-[(4S)-4-vinylsulfonylamino-3-oxo-2-isoxazolidinyl]-5-oxo-2-tetrahydrofurancarboxylate;
(127) 2-[(4S)-4-thienylacetamido-3-oxo-2-isoxazolidinyl]-5-oxo-2-tetrahydrofurancarbonylanilide;
(128) 2-[(4S)-4-benzyloxycarbonylamino-3-oxo-2-isoxazolidinyl]-5-oxo-2-tetrahydrofurancarbonylanilide;
(129) 2-[(4S)-4-benzyloxycarbonylamino-3-oxo-2-isoxazolidinyl]-5-oxo-2-tetrahydrofurancarbonylpyrrolidinamide;
(130) 2-[(4S)-4-thienylacetamido-3-oxo-2-isoxazolidinyl]-5-oxo-2-tetrahydrofurancarbonylpyrrolidinamide;
(131) 2-[(4S)-4-thienylacetamido-3-oxo-2-isoxazolidinyl]-5-oxo-2-tetrahydrofurancarbonylpropylamine;
(132) sodium 2-[(4S)-4-γ-D-glutamylamino-3-oxo-2-isoxazolidinyl]-5-oxo-2-tetrahydrofurancarboxylate; and
(133) sodium 2-[(4S)-4-(2-hydroxy)isobutyryl-amino-3-oxo-2-isoxazolidinyl]-5-oxo-2-tetrahydrofurancarboxylate;

together with a pharmaceutically acceptable carrier or diluent therefor.

**2.** The use of a compound (I'), or pharmaceutically acceptable salt thereof, as defined in Claim 1, in the preparation of a pharmaceutical composition for inhibiting the growth of microorganisms.

**Patentansprüche**
**Patentansprüche für folgende Vertragsstaaten : BE, CH, DE, FR, GB, IT, LU, NL, SE**

**1.** 2-[(4S)-Amino-3-oxo-2-isoxazolidinyl]-5-oxo-2-tetrahydrofurancarbonsäure-Derivat der Formel (I') oder dessen pharmazeutisch annehmbares Salz, ausgewählt aus:
(1) Natrium-2-{(4S)-4-[2-(2-amino-4-thiazolyl)-(Z)-2-(methoxyimino)acetamido]-3-oxo-2-isoxazolidinyl}-5-oxo-2-tetrahydrofurancarboxylat;
(2) Natrium-2-{(4S)-4-[D-2-(4-ethyl-2,3-dioxo-1-piperazinocarboxamido)-2-phenylacetamido]-3-oxo-2-isoxazolidinyl}-5-oxo-2-tetrahydrofurancarboxylat;
(3) Natrium-2-{(4S)-4-[D-2-(3-methylsulfonyl-2-oxoimidazolidin-1-carboxamido)-2-phenylacetamido]-3-oxo-2-isoxazolidinyl)-5-oxo-2-tetrahydrofurancarboxylat;
(4) Natrium-2-{(4S)-4-[D-2-(3-furfurylidenamino-2-oxoimidazolidin-1-carboxamido)-2-phenylacetamido]-3-oxo-2-isoxazolidinyl}-5-oxo-2-tetrahydrofurancarboxylat;
(5) Natrium-2-[(4S)-4-(2-thienylacetamido)-3-oxo-2-isoxazolidinyl]-5-oxo-2-tetrahydrofurancarboxylat;
(6) Natrium-2-[(4S)-4-(2-cyanacetamido)-3-oxo-2-isoxazolidinyl]-5-oxo-2-tetrahydrofurancarboxylat;
(7) Natrium-2-{(4S)-4-[(1H)-tetrazolylacetamido]-3-oxo-2-isoxazolidinyl]-5-oxo-2-tetrahydrofurancarboxylat;

(8) Natrium-2-[(4S)-4-(2,6-dimethoxybenzamido)-3-oxo-2-isoxazolidinyl]-5-oxo-2-tetrahydrofurancarboxylat;
(9) Natrium-2-[(4S)-4-(5-methyl-3-phenyl-2-isoxazolin-4-carboxamido)-3-oxo-2-isoxazolidinyl]-5-oxo-2-tetrahydrofurancarboxylat;
(10) Natrium-2-[(4S)-4-(2-ethoxynaphthyl-1-carboxamido)-3-oxo-2-isoxazolidinyl]-5-oxo-2-tetrahydrofurancarboxylat;
(11) Natrium-2-[(4S)-4-(2-cyanmethylthioacetamido)-3-oxo-2-isoxazolidinyl]-5-oxo-2-tetrahydrofurancarboxylat;
(12) Natrium-2-[(4S)-4-(2-difluormethylthioacetamido)-3-oxo-2-isoxazolidinyl]-5-oxo-2-tetrahydrofurancarboxylat;
(13) Natrium-2-{(4S)-4-[(R)-2-(4-cyclohexyl-2,3-dioxo-1-piperazinocarboxamido)-2-phenylacetamido]-3-oxo-2-isoxazolidinyl}-5-oxo-2-tetrahydrofurancarboxylat;
(14) Natrium-2-[(4S)-4-chloracetamido-3-oxo-2-isoxazolidinyl]-5-oxo-2-tetrahydrofurancarboxylat;
(15) Natrium-2-{(4S)-4-[2-(4-pyridylthio)acetamido]-3-oxo-2-isoxazolidinyl}-5-oxo-2-tetrahydrofurancarboxylat;
(16) 2-[(4S)-4-Acetamido-3-oxo-2-isoxazolidinyl]-5-oxo-2-tetrahydrofurancarbonsäuremethylester;
(17) 2-[(4S)-4-Acetamido-3-oxo-2-isoxazolidinyl]-5-oxo-2-tetrahydrofurancarbonsäure-pivaloyloxymethylester;
(18) 2-[(4S)-4-Acetamido-3-oxo-2-isoxazolidinyl]-5-oxo-2-tetrahydrofurancarbonsäure-3,5-dinitrobenzylester;
(19) 2-[(4S)-4-Acetamido-3-oxo-2-isoxazolidinyl]-5-oxo-2-tetrahydrofurancarbonsäure-p-bromphenacylester;
(20) 2-[(4S)-4-Acetamido-3-oxo-2-isoxazolidinyl]-5-oxo-2-tetrahydrofurancarbonsäure-p-brombenzylester;
(21) Natrium-2-[(4S)-4-formylamino-3-oxo-2-isoxazolidinyl]-5-oxo-2-tetrahydrofurancarboxylat;
(22) Natrium-2-[(4S)-4-n-butyrylamino-3-oxo-2-isoxazolidinyl]-5-oxo-2-tetrahydrofurancarboxylat;
(23) Natrium-2-[(4S)-4-methoxycarbonylamino-3-oxo-2-isoxazolidinyl]-5-oxo-2-tetrahydrofurancarboxylat;
(24) Natrium-2-[(4S)-4-(N-benzyloxycarbonyl)-glycylamino-3-oxo-2-isoxazolidinyl]-5-oxo-2-tetrahydrofurancarboxylat;
(25) Natrium-2-[(4S)-4-benzoylamino-3-oxo-2-isoxazolidinyl]-5-oxo-2-tetrahydrofurancarboxylat;
(26) Natrium-2-[(4S)-4-phenylacetylamino-3-oxo-2-isoxazolidinyl]-5-oxo-2-tetrahydrofurancarboxylat;
(27) Natrium-2-[(4S)-4-nicotinylamino-3-oxo-2-isoxazolidinyl]-5-oxo-2-tetrahydrofurancarboxylat;
(28) 2-[(4S)-4-Phenoxyacetylamino-3-oxo-2-isoxazolidinyl]-5-oxo-2-tetrahydrofurancarbonsäure-pivaloyloxymethylester;
(29) Natrium-2-[(4S)-4-(p-toluolsulfonyl)amino-3-oxo-2-isoxazolidinyl]-5-oxo-2-tetrahydrofurancarboxylat;
(30) 2-[(4S)-4-(p-Brombenzoyl)amino-3-oxo-2-isoxazolidinyl]-5-oxo-2-tetrahydrofurancarbonsaure-diphenylmethylester;
(31) 2-[(4S)-4-(N-Benzyloxycarbonyl-D-alanyl)amino-3-oxo-2-isoxazolidinyl]-5-oxo-2-tetrahydrofurancarbonsäure-diphenylmethylester;
(32) 2-[(4S)-4-Dimethylamino-3-oxo-2-isoxazolidinyl]-5-oxo-2-tetrahydrofurancarbonsäure;
(33) Natrium-2-{(4S)-4-[2-(2-amino-4-thiazolyl)acetamido]-3-oxo-2-isoxazolidinyl}-5-oxo-2-tetrahydrofurancarboxylat;
(34) Natrium-2-[4-methoxy-4-(2-thienylacetamido)-3-oxo-2-isoxazolidinyl]-5-oxo-2-tetrahydrofurancarboxylat;
(35) Dinatrium-2-{(4S)-4-[2-(2-amino-4-thiazolyl)-(Z)-2-(carboxymethyloxyimino)acetamido]-3-oxo-2-isoxazolidinyl}-5-oxo-2-tetrahydrofurancarboxylat;
(38) 2-{(4S)-4-[2-(2-Aminomethyl)phenylacetamido]-3-oxo-2-isoxazolidinyl}-5-oxo-2-tetrahydrofurancarbonsäure;
(39) Natrium-2-[(4S)-4-(4-fluorbenzolsulfonylamino)-3-oxo-2-isoxazolidinyl]-5-oxo-2-tetrahydrofurancarboxylat;
(40) Natrium-2-{(4S)-4-{2-[1-(2-dimethylaminoethyl)-1H-5-tetrazolylthio]acetamido)-3-oxo-2-isoxazolidinyl}-5-oxo-2-tetrahydrofurancarboxylat;
(41) Natrium-2-{(4S)-4-[2-(4-methyl-4H-1,2,4-triazol-3-ylthio)acetamido]-3-oxo-2-isoxazolidinyl}-5-oxo-2-tetrahydrofurancarboxylat;
(42) Natrium-2-{(4S)-4-[(2R,3S)-2-(4-ethyl-2,3-dioxo-1-piperazincarboxamido)-3-formyloxybutylamido]-3--oxo-2-isoxazolidinyl}-5-oxo-2-tetrahydrofurancarboxylat;

(43) Dinatrium-2-[(4S)-4-(2-phenyl-2-sulfoacetamido)-3-oxo-2-isoxazolidinyl]-5-oxo-2-tetrahydrofurancarboxylat;
(44) Natrium-2-{(4S)-4-[2-(2-amino-4-thiazolyl)-(E)-2-methoxyiminoacetamido]-3-oxo-2-isoxazolidinyl}-5-oxo-2-tetrahydrofurancarboxylat;
(45) Natrium-2-[(4S)-4-(2-trimethylsilyl)-ethoxycarbonylamino-3-oxo-2-isoxazolidinyl]-5-oxo-2-tetrahydrofurancarboxylat;
(46) Natrium-2-[(4S)-4-(1-aminocyclohexylcarbonylamino)-3-oxo-2-isoxazolidinyl]-5-oxo-2-tetrahydrofurancarboxylat;
(47) Natrium-2-{(4S)-4-[3-(2,6-dichlorphenyl)-5-methyl-4-isoxazolylcarbonylamino]-3-oxo-2-isoxazolidinyl}-5-oxo-2-tetrahydrofurancarboxylat;
(48) Natrium-2-{(4S)-4-[2-(2-pyridylthio)acetamido]-3-oxo-2-isoxazolidinyl}-5-oxo-2-tetrahydrofurancarboxylat;
(49) Natrium-2-{(4S)-4-[2-(4-pyridylmethylthio)acetamido]-3-oxo-2-isoxazolidinyl}-5-oxo-2-tetrahydrofurancarboxylat;
(50) Natrium-2-{(4S)-4-[2-(2-pyrimidinylthio)acetamido]-3-oxo-2-isoxazolidinyl}-5-oxo-2-tetrahydrofurancarboxylat;
(51) Natrium-2-{(4S)-4-{2-[(E)-2-(acetamidovinyl)thio]acetamido}-3-oxo-2-isoxazolidinyl}-5-oxo-2-tetrahydrofurancarboxylat;
(52) Natrium-2-[(4S)-4-cyclopropylcarbonylamino-3-oxo-2-isoxazolidinyl]-5-oxo-2-tetrahydrofurancarboxylat;
(53) Natrium-2-[(4S)-4-methoxalylamino-3-oxo-2-isoxazolidinyl]-5-oxo-2-tetrahydrofurancarboxylat;
(54) Natrium-2-[(4S)-4-acrylamido-3-oxo-2-isoxazolidinyl]-5-oxo-2-tetrahydrofurancarboxylat;
(55) Natrium-2-{(4S)-4-[2-(2-chlor-4-pyridylthio)acetamido]-3-oxo-2-isoxazolidinyl}-5-oxo-2-tetrahydrofurancarboxylat;
(56) Natrium-2-[(4S)-4-(2-acetamido-5-oxo-2-tetrahydrofurancarbonylamino)-3-oxo-2-isoxazolidinyl]-5-oxo-2-tetrahydrofurancarboxylat;
(57) Natrium-2-{(4S)-4-[2-(1-pyrazolyl)acetamido]-3-oxo-2-isoxazolidinyl}-5-oxo-2-tetrahydrofurancarboxylat;
(58) Natrium-2-{(4S)-4-[2-(5-amino-1,2,4-thiadiazol-3-yl)-(Z)-2-ethoxyiminoacetamido]-3-oxo-2-isoxazolidinyl}-5-oxo-2-tetrahydrofurancarboxylat;
(59) Natrium-2-{(4S)-4-[2-(3-pyridazinylthiol)acetamido]-3-oxo-2-isoxazolidinyl}-5-oxo-2-tetrahydrofurancarboxylat;
(60) Natrium-2-[(4S)-4-dichloracetamido-3-oxo-2-isoxazolidinyl]-5-oxo-2-tetrahydrofurancarboxylat;
(61) Natrium-2-{(4S)-4-[(2R,3S)-2-(4-ethyl-2,3-dioxo1-piperazincarboxamido)-3-hydroxybutyramido]-3-oxo-2-isoxazolidinyl}-5-oxo-2-tetrahydrofurancarboxylat;
(62) Natrium-2-{(4S)-4-[2-(5-trifluormethyl-1,3,4-thiadiazol-2-ylthio)acetamido]-3-oxo-2-isoxazolidinyl}-5-oxo-2-tetrahydrofurancarboxylat;
(63) Natrium-2-{(4S)-4-{2-[1-ethoxy(hydroxy)phosphinylmethyl-1H-5-tetrazolylthio]acetamido}-3-oxo-2-isoxazolidinyl}-5-oxo-2-tetrahydrofurancarboxylat;
(64) Natrium-2-{(4S)-4-{2-[5-(2-diethoxyphosphinylethylthio-1,3,4-thiadiazolylthio]acetamido}-3-oxo-2-isoxazolidinyl}-5-oxo-2-tetrahydrofurancarboxylat;
(65) Natrium-2-{(4S)-4-[2-(1-dimethylamino-1H-5-tetrazolylthio)acetamido]-3-oxo-2-isoxazolidinyl}-5-oxo-2-tetrahydrofurancarboxylat;
(66) Natrium-2-{(4S)-4-[2-(4,5-dimethyl-2-thiazolylthio)acetamido]-3-oxo-2-isoxazolidinyl}-5-oxo-2-tetrahydrofurancarboxylat;
(67) Natrium-2-{(4S)-4-[2-(4,5-dimethyl-2-oxazolylthio)acetamido]-3-oxo-2-isoxazolidinyl}-5-oxo-2-tetrahydrofurancarboxylat;
(68) Natrium-2-{(4S)-4-[2-(5-methoxymethyl-1,3,4-thiadiazol-2-ylthio)acetamido]-3-oxo-2-isoxazolidinyl}-5-oxo-2-tetrahydrofurancarboxylat;
(69) Natrium-2-{(4S)-4-[2-(5-methylsulfonylmethyl-1,3,4-thiadiazol-2-ylthio)acetamido]-3-oxo-2-isoxazolidinyl}-5-oxo-2-tetrahydrofurancarboxylat;
(70) Natrium-2-{(4S)-4-[2-(4-ethyl-4H-1,2,4-triazol-3-ylthio)acetamido]-3-oxo-2-isoxazolidinyl)-5-oxo-2-tetrahydrofurancarboxylat;
(71) Dinatrium-2-{(4S)-4-[2-(5-carboxymethyl-1,3,4-thiadiazol-2-ylthio)acetamido]-3-oxo-2-isoxazolidinyl}-5-oxo-2-tetrahydrofurancarboxylat;
(72) Natrium-2-{(4S)-4-{2-[1-(2-hydroxyethyl)-1H-5-tetrazolylthio]acetamido}-3-oxo-2-isoxazolidinyl}-5-oxo-2-tetrahydrofurancarboxylat;
(73) Natrium-2-{(4S)-4-{2-[1-(3-dimethylaminopropyl)-1H-5-tetrazolylthio]acetamido}-3-oxo-2-isoxazolidinyl}-5-oxo-2-tetrahydrofurancarboxylat;
(74) 2-[(4S)-4-(2-Amino-3-sulfamoylpropionamido)-3-oxo-2-isoxazolidinyl]-5-oxo-2-tetrahydrofurancarbonsäure;
(75) Natrium-2-{(4S)-4-[2-(4-cyan-3-hydroxy-5-isothiazolylthio)acetamido]-3-oxo-2-isoxazolidinyl}-5-oxo-2-tetrahydrofurancarboxylat;
(76) Natrium-2-{(4S)-4-[2-(2-amino-4-thiazolyl)-2-oxo-acetamido]-3-oxo-2-isoxazolidinyl}-5-oxo-2-tetrahydrofurancarboxylat;
(77) Natrium-2-{(4S)-4-[2-(2-amino-4-thiazolyl)-(Z)-2-isopropoxyiminoacetamido]-3-oxo-2-isoxazolidinyl}-5-oxo-2-tetrahydrofurancarboxylat;
(78) Natrium-2-[(4S)-4-trifluoracetamido-3-oxo-2-isoxazolidinyl]-5-oxo-2-tetrahydrofurancarboxylat;
(79) Dinatrium-2-{(4S)-4-[2-(2-amino-4-thiazolyl)-(Z)-2-(1-carboxy-1-methylethoxyimino)acetamido]-3-oxo-2-isoxazolidinyl}-5-oxo-2-tetrahydrofurancarboxylat;
(80) Natrium-2-{(4S)-4-[2-(2-amino-4-thiazolyl)-(Z)-2-(hydroxyimino)acetamido]-3-oxo-2-isoxazolidinyl}-5-oxo-2-tetrahydrofurancarboxylat;
(81) Natrium-2-[(4S)-4-isobutyloxycarbonylamino-3-oxo-2-isoxazolidinyl]-5-oxo-2-tetrahydrofurancarboxylat;
(82) 2-{(4S)-4-[N-(4-Nitrobenzyloxycarbonyl)glycylamino]-3-oxo-2-isoxazolidinyl}-5-oxo-2-tetrahydrofurancarbonsäure-diphenylmethylester;
(83) 2-{(4S)-4-[N-(2,2,2-Trichlorethoxycarbonyl)phenylglycylamino]-3-oxo-2-isoxazolidinyl}-5-oxo-2-tetrahydrofurancarbonsäure-diphenylmethylester;
(84) Natrium-2-{(4S)-4-[N-(benzyloxycarbonyl)-D-phenylglycylamino]-3-oxo-2-isoxazolidinyl}-5-oxo-2-tetrahydrofurancarboxylat;
(85) 2-[(4S)-4-L-Phenylglycylamino-3-oxo-2-isoxazolidinyl]-5-oxo-2-tetrahydrofurancarbonsäure;
(86) 2-[(4S)-4-D-Phenylglycylamino-3-oxo-2-isoxazolidinyl]-5-oxo-2-tetrahydrofurancarbonsäure;
(87) Natrium-2-{(4S)-4-[(2S)-(2-hydroxy-2-phenyl)acetamido]-3-oxo-2-isoxazolidinyl}-5-oxo-2-tetrahydrofurancarboxylat;
(88) Natrium-2-{(4S)-4-[(2R)-(2-hydroxy-2-phenyl)acetamido]-3-oxo-2-isoxazolidinyl}-5-oxo-2-tetrahydrofurancarboxylat;
(89) Natrium-2-{(4S)-4-[2-(4-chlorphenyl)-2-hydroxyacetamido]-3-oxo-2-isoxazolidinyl}-5-oxo-2-tetrahydrofurancarboxylat;
(90) Natrium-2-{(4S)-4-[2-hydroxy-2-(4-hydroxyphenyl)acetamido]-3-oxo-2-isoxazolidinyl}-5-oxo-2-tetrahydrofurancarboxylat;
(91) Natrium-2-[(4S)-4-(2-hydroxypropionamido)-3-oxo-2-isoxazolidinyl]-5-oxo-2-tetrahydrofurancarboxylat;
(92) Dinatrium-2-[(4S)-4-(2-carboxy-2-phenylacetamido)-3-oxo-2-isoxazolidinyl]-5-oxo-2-tetrahydrofurancarboxylat;
(93) Natrium-2-[(4S)-4-benzyloxycarbonylamino-3-oxo-2-isoxazolidinyl]-5-oxo-2-tetrahydrofurancarboxylat;
(94) Natrium-2-[(4S)-4-pyruvamido-3-oxo-2-isoxazolidinyl]-5-oxo-2-tetrahydrofurancarboxylat;
(95) 2-{(4S)-4-[(2R)-(2-Hydroxy-2-phenyl)acetamido]-3-oxo-2-isoxazolidinyl}-5-oxo-2-tetrahydrofurancarbonsäure-pivaloyloxymethylester;
(96) 2-[(4S)-4-(4-Nitrobenzyliden)amino-3-oxo-2-isoxazolidinyl]-5-oxo-2-tetrahydrofurancarbonsäurediphenylmethylester;
(97) Natrium-2-{(4S)-4-[2-(3-chlor-6-pyridazinylthio)acetamido]-3-oxo-2-isoxazolidinyl}-5-oxo-2-tetrahydrofurancarboxylat;
(98) Natrium-2-[(4S)-4-(2-phenylthioacetamido)-3-oxo-2-isoxazolidinyl]-5-oxo-2-tetrahydrofurancarboxylat;
(99) Natrium-2-[(4S)-4-(2-ethoxydithiocarbonylacetamido)-3-oxo-2-isoxazolidinyl]-5-oxo-2-tetrahydrofurancarboxylat;
(100) Natrium-2-{(4S)-4-[2-(2-amino-4-thiazolyl)-(Z)-2-(ethoxyimino)acetamido]-3-oxo-2-isoxazolidinyl}-5-oxo-2-tetrahydrofurancarboxylat;
(101) Natrium-2-{(4S)-4-[2-(2-amino-5-chlor-4-thiazolyl)-(Z)-2-(methoxyimino)acetamido]-3-oxo-2-isoxazolidinyl}-5-oxo-2-tetrahydrofurancarboxylat;
(102) Natrium-2-{(4S)-4-[2-(4-ethyl-2,3-dioxo-1-piperazinocarboxamido)-2-thienylacetamido]-3-oxo-2-isoxazolidinyl}-5-oxo-2-tetrahydrofurancarboxylat;
(103) Natrium-2-[(4S)-4-ureido-3-oxo-2-isoxazolidinyl]-5-oxo-2-tetrahydrofurancarboxylat;
(104) Natrium-2-[(4S)-4-phenylureylen-3-oxo-2-isoxazolidinyl]-5-oxo-2-tetrahydrofurancarboxylat;
(105) Natrium-2-{(4S)-4-[2-(pyridyl)acetamido]-3-oxo-2-isoxazolidinyl}-5-oxo-2-tetrahydrofurancarbox-

ylat;
(106) Natrium-2-{(4S)-4-[2-(4-chlorphenyl)acetamido]-3-oxo-2-isoxazolidinyl}-5-oxo-2-tetrahydrofurancarboxylat;
(107) Natrium-2-{(4S)-4-[(2-chlor-2-phenyl)acetamido]-3-oxo-2-isoxazolidinyl}-5-oxo-2-tetrahydrofurancarboxylat;
(108) Natrium-2-{(4S)-4-[2-(3-pyridylthio)acetamido]-3-oxo-2-isoxazolidinyl}-5-oxo-2-tetrahydrofurancarboxylat;
(109) Natrium-2-{(4S)-4-[2-(4-chlorphenylthio)acetamido]-3-oxo-2-isoxazolidinyl}-5-oxo-2-tetrahydrofurancarboxylat;
(110) Natrium-2-[(4S)-4-(2-isopropylphenoxycarbonylamino)-3-oxo-2-isoxazolidinyl]-5-oxo-2-tetrahydrofurancarboxylat;
(111) Natrium-2-[(4S)-4-difluoracetamido-3-oxo-2-isoxazolidinyl]-5-oxo-2-tetrahydrofurancarboxylat;
(112) Natrium-2-[(4S)-4-(3-chlor)propionamido-3-oxo-2-isoxazolidinyl]-5-oxo-2-tetrahydrofurancarboxylat;
(113) 2-[(4S)-4-Chloracetamido-3-oxo-2-isoxazolidinyl]-5-oxo-2-tetrahydrofurancarbonsäure-pivaloyloxymethylester;
(114) 2-[(4S)-4-(2-Phenylthioacetamido)-3-oxo-2-isoxazolidinyl]-5-oxo-2-tetrahydrofurancarbonsäure-pivaloyloxymethylester;
(115) Natrium-2-[(4S)-4-propionamido-3-oxo-2-isoxazolidinyl]-5-oxo-2-tetrahydrofurancarboxylat;
(116) 2-[(4S)-4-Benzyloxycarbonylamino-3-oxo-2-isoxazolidinyl]-5-oxo-2-tetrahydrofurancarbonsäure-pivaloyloxymethylester;
(117) 2-[(4S)-4-Bromacetamido-3-oxo-2-isoxazolidinyl]-5-oxo-2-tetrahydrofurancarbonsäure-pivaloyloxymethylester;
(118) 2-[(4S)-4-(2,4-Hexadien)amido-3-oxo-2-isoxazolidinyl]-5-oxo-2-tetrahydrofurancarbonsäure-pivaloyloxymethylester;
(119) 2-[(4S)-4-(2-Thienyl)acetamido-3-oxo-2-isoxazolidinyl]-5-oxo-2-tetrahydrofurancarbonsäure-pivaloyloxymethylester;
(120) 2-[(4S)-4-(Hexahydro-1H-azepin-1-yl)methylenamino-3-oxo-2-isoxazolidinyl]-5-oxo-2-tetrahydrofurancarbonsäure-pivaloyloxymethylester;
(121) 2-[(4S)-4-(t-Butyldimethylsilyl)amino-3-oxo-2-isoxazolidinyl]-5-oxo-2-tetrahydrofurancarbonsäure-diphenylmethylester;
(122) 2-[(4S)-4-(t-Butyldiphenylsilyl)amino-3-oxo-2-isoxazolidinyl]-5-oxo-2-tetrahydrofurancarbonsäure-diphenylmethylester;
(123) Natrium-2-[(4S)-3-diphenylphosphorylamino-3-oxo-2-isoxazolidinyl]-5-oxo-2-tetrahydrofurancarboxylat;
(124) 2-[(4S)-4-Dimethylphosphorylamino-3-oxo-2-isoxazolidinyl]-5-oxo-2-tetrahydrofurancarbonsäure-diphenylmethylester;
(125) Natrium-2-[(4S)-4-ethylsulfonylamino-3-oxo-2-isoxazolidinyl]-5-oxo-2-tetrahydrofurancarboxylat;
(126) Natrium-2-[(4S)-4-vinylsulfonylamino-3-oxo-2-isoxazolidinyl]-5-oxo-2-tetrahydrofurancarboxylat;
(127) 2-[(4S)-4-Thienylacetamido-3-oxo-2-isoxazolidinyl]-5-oxo-2-tetrahydrofurancarbonylanilid;
(128) 2-[(4S)-4-Benzyloxycarbonylamino-3-oxo-2-isoxazolidinyl]-5-oxo-2-tetrahydrofurancarbonylanilid;
(129) 2-[(4S)-4-Benzyloxycarbonylamino-3-oxo-2-isoxazolidinyl]-5-oxo-2-tetrahydrofurancarbonylpyrrolidinamid;
(130) 2-[(4S)-4-Thienylacetamido-3-oxo-2-isoxazolidinyl]-5-oxo-2-tetrahydrofurancarbonylpyrrolidinamid;
(131) 2-[(4S)-4-Thienylacetamido-3-oxo-2-isoxazolidinyl]-5-oxo-2-tetrahydrofurancarbonylpropylamin;
(132) Natrium-2-[(4S)-4-$\gamma$-D-glutamoylamino-3-oxo-2-isoxazolidinyl]-5-oxo-2-tetrahydrofurancarboxylat; und
(133) Natrium-2-[(4S)-4-(2-hydroxy)isobutyrylamino-3-oxo-2-isoxazolidinyl]-5-oxo-2-tetrahydrofurancarboxylat;

**2.** Pharmazeutische Zusammensetzung zur Hemmung des Wachstums von Mikroorganismen, die eine wirksame Menge einer Verbindung (I') oder deren pharmazeutisch annehmbare Salze wie in Anspruch 1 definiert enthält, zusammen mit einem pharmazeutisch annehmbaren Träger oder Verdünnungsmittel für diese.

**3.** Verbindung (I') oder deren pharmazeutisch annehmbares Salz wie in Anspruch 1 definiert, zur

Verwendung bei der Herstellung einer pharmazeutischen Zusammensetzung zur Hemmung des Wachstums von Mikroorganismen.

**Patentansprüche für folgenden Vertragsstaat : AT**

1. Pharmazeutische Zusammensetzung zur Hemmung des Wachstums von Mikroorganismen, die eine wirksame Menge eines 2-[(4S)-Amino-3-oxo-2-isoxazolidinyl]-5-oxo-2-tetrahydrofurancarbonsäure-Derivats, Verbindung (I'), oder dessen pharmazeutisch annehmbaren Salzes enthält, ausgewählt aus:
   (1) Natrium-2-{(4S)-4-[2-(2-amino-4-thiazolyl)-(Z)-2-(methoxyimino)acetamido]-3-oxo-2-isoxazolidinyl}-5-oxo-2-tetrahydrofurancarboxylat;
   (2) Natrium-2-{(4S)-4-[D-2-(4-ethyl-2,3-dioxo-1-piperazinocarboxamido)-2-phenylacetamido]-3-oxo-2-isoxazolidinyl}-5-oxo-2-tetrahydrofurancarboxylat;
   (3) Natrium-2-{(4S)-4-[D-2-(3-methylsulfonyl-2-oxo-imidazolidin-1-carboxamido)-2-phenylacetamido]-3-oxo-2-isoxazolidinyl}-5-oxo-2-tetrahydrofurancarboxylat;
   (4) Natrium-2-{(4S)-4-[D-2-(3-furfurylidenamino-2-oxoimidazolidin-1-carboxamido)-2-phenylacetamido]-3-oxo-2-isoxazolidinyl}-5-oxo-2-tetrahydrofurancarboxylat;
   (5) Natrium-2-[(4S)-4-(2-thienylacetamido)-3-oxo-2-isoxazolidinyl]-5-oxo-2-tetrahydrofurancarboxylat;
   (6) Natrium-2-[(4S)-4-(2-cyanacetamido)-3-oxo-2-isoxazolidinyl]-5-oxo-2-tetrahydrofurancarboxylat;
   (7) Natrium-2-{(4S)-4-[(1H)-tetrazolylacetamido]-3-oxo-2-isoxazolidinyl]-5-oxo-2-tetrahydrofurancarboxylat;
   (8) Natrium-2-[(4S)-4-(2,6-dimethoxybenzamido)-3-oxo-2-isoxazolidinyl]-5-oxo-2-tetrahydrofurancarboxylat;
   (9) Natrium-2-[(4S)-4-(5-methyl-3-phenyl-2-isoxazolin-4-carboxamido)-3-oxo-2-isoxazolidinyl]-5-oxo-2-tetrahydrofurancarboxylat;
   (10) Natrium-2-[(4S)-4-(2-ethoxynaphthyl-1-carboxamido)-3-oxo-2-isoxazolidinyl]-5-oxo-2-tetrahydrofurancarboxylat;
   (11) Natrium-2-[(4S)-4-(2-cyanmethylthioacetamido)-3-oxo-2-isoxazolidinyl]-5-oxo-2-tetrahydrofurancarboxylat;
   (12) Natrium-2-[(4S)-4-(2-difluormethylthioacetamido)-3-oxo-2-isoxazolidinyl]-5-oxo-2-tetrahydrofurancarboxylat;
   (13) Natrium-2-{(4S)-4-[(R)-2-(4-cyclohexyl-2,3-dioxo-1-piperazinocarboxamido)-2-phenylacetamido]-3-oxo-2-isoxazolidinyl}-5-oxo-2-tetrahydrofurancarboxylat;
   (14) Natrium-2-[(4S)-4-chloracetamido-3-oxo-2-isoxazolidinyl]-5-oxo-2-tetrahydrofurancarboxylat;
   (15) Natrium-2-{(4S)-4-[2-(4-pyridylthio)acetamido]-3-oxo-2-isoxazolidinyl}-5-oxo-2-tetrahydrofurancarboxylat;
   (16) 2-[(4S)-4-Acetamido-3-oxo-2-isoxazolidinyl]-5-oxo-2-tetrahydrofurancarbonsäuremethylester;
   (17) 2-[(4S)-4-Acetamido-3-oxo-2-isoxazolidinyl]-5-oxo-2-tetrahydrofurancarbonsäure-pivaloyloxymethylester;
   (18) 2-[(4S)-4-Acetamido-3-oxo-2-isoxazolidinyl]-5-oxo-2-tetrahydrofurancarbonsäure-3,5-dinitrobenzylester;
   (19) 2-[(4S)-4-Acetamido-3-oxo-2-isoxazolidinyl]-5-oxo-2-tetrahydrofurancarbonsäure-p-bromphenacylester;
   (20) 2-[(4S)-4-Acetamido-3-oxo-2-isoxazolidinyl]-5-oxo-2-tetrahydrofurancarbonsäure-p-brombenzylester;
   (21) Natrium-2-[(4S)-4-formylamino-3-oxo-2-isoxazolidinyl]-5-oxo-2-tetrahydrofurancarboxylat;
   (22) Natrium-2-[(4S)-4-n-butyrylamino-3-oxo-2-isoxazolidinyl]-5-oxo-2-tetrahydrofurancarboxylat;
   (23) Natrium-2-[(4S)-4-methoxycarbonylamino-3-oxo-2-isoxazolidinyl]-5-oxo-2-tetrahydrofurancarboxylat;
   (24) Natrium-2-[(4S)-4-(N-benzyloxycarbonyl)-glycylamino-3-oxo-2-isoxazolidinyl]-5-oxo-2-tetrahydrofurancarboxylat;
   (25) Natrium-2-[(4S)-4-benzoylamino-3-oxo-2-isoxazolidinyl]-5-oxo-2-tetrahydrofurancarboxylat;
   (26) Natrium-2-[(4S)-4-phenylacetylamino-3-oxo-2-isoxazolidinyl]-5-oxo-2-tetrahydrofurancarboxylat;
   (27) Natrium-2-[(4S)-4-nicotinylamino-3-oxo-2-isoxazolidinyl]-5-oxo-2-tetrahydrofurancarboxylat;
   (28) 2-[(4S)-4-Phenoxyacetylamino-3-oxo-2-isoxazolidinyl]-5-oxo-2-tetrahydrofurancarbonsäure-pivaloyloxymethylester;
   (29) Natrium-2-[(4S)-4-(p-toluolsulfonyl)amino-3-oxo-2-isoxazolidinyl]-5-oxo-2-tetrahydrofurancarboxylat;
   (30) 2-[(4S)-4-(p-Brombenzoyl)amino-3-oxo-2-isoxazolidinyl]-5-oxo-2-tetrahydrofurancarbonsäure-di-

phenylmethylester;
(31) 2-[(4S)-4-(N-Benzyloxycarbonyl-D-alanyl)amino-3-oxo-2-isoxazolidinyl]-5-oxo-2-tetrahydrofurancarbonsäure-diphenylmethylester;
(32) 2-[(4S)-4-Dimethylamino-3-oxo-2-isoxazolidinyl]-5-oxo-2-tetrahydrofurancarbonsäure;
(33) Natrium-2-{(4S)-4-[2-(2-amino-4-thiazolyl)acetamido]-3-oxo-2-isoxazolidinyl}-5-oxo-2-tetrahydrofurancarboxylat;
(34) Natrium-2-[4-methoxy-4-(2-thienylacetamido)-3-oxo-2-isoxazolidinyl]-5-oxo-2-tetrahydrofurancarboxylat;
(35) Dinatrium-2-{(4S)-4-[2-(2-amino-4-thiazolyl)-(Z)-2-(carboxymethyloxyimino)acetamido]-3-oxo-2-isoxazolidinyl}-5-oxo-2-tetrahydrofurancarboxylat;
(38) 2-{(4S)-4-[2-(2-Aminomethyl)phenylacetamido]-3-oxo-2-isoxazolidinyl}-5-oxo-2-tetrahydrofurancarbonsäure;
(39) Natrium-2-[(4S)-4-(4-fluorbenzolsulfonylamino)-3-oxo-2-isoxazolidinyl]-5-oxo-2-tetrahydrofurancarboxylat;
(40) Natrium-2-{(4S)-4-{2-[1-(2-dimethylaminoethyl)-1H-5-tetrazolylthio]acetamido}-3-oxo-2-isoxazolidinyl}-5-oxo-2-tetrahydrofurancarboxylat;
(41) Natrium-2-{(4S)-4-[2-(4-methyl-4H-1,2,4-triazol3-ylthio)acetamido]-3-oxo-2-isoxazolidinyl}-5-oxo-2-tetrahydrofurancarboxylat;
(42) Natrium-2-{(4S)-4-[(2R,3S)-2-(4-ethyl-2,3-dioxo-1-piperazincarboxamido)-3-formyloxybutylamido]-3-oxo-2-isoxazolidinyl)-5-oxo-2-tetrahydrofurancarboxylat;
(43) Dinatrium-2-[(4S)-4-(2-phenyl-2-sulfoacetamido)-3-oxo-2-isoxazolidinyl]-5-oxo-2-tetrahydrofurancarboxylat;
(44) Natrium-2-{(4S)-4-[2-(2-amino-4-thiazolyl)-(E)-2-methoxyiminoacetamido]-3-oxo-2-isoxazolidinyl}-5-oxo-2-tetrahydrofurancarboxylat;
(45) Natrium-2-[(4S)-4-(2-trimethylsilyl)-ethoxycarbonylamino-3-oxo-2-isoxazolidinyl]-5-oxo-2-tetrahydrofurancarboxylat;
(46) Natrium-2-[(4S)-4-(1-aminocyclohexylcarbonylamino)-3-oxo-2-isoxazolidinyl]-5-oxo-2-tetrahydrofurancarboxylat;
(47) Natrium-2-{(4S)-4-[3-(2,6-dichlorphenyl)-5-methyl-4-isoxazolylcarbonylamino]-3-oxo-2-isoxazolidinyl}-5-oxo-2-tetrahydrofurancarboxylat;
(48) Natrium-2-{(4S)-4-[2-(2-pyridylthio)acetamido]-3-oxo-2-isoxazolidinyl}-5-oxo-2-tetrahydrofurancarboxylat;
(49) Natrium-2-{(4S)-4-[2-(4-pyridylmethylthio)acetamido]-3-oxo-2-isoxazolidinyl)-5-oxo-2-tetrahydrofurancarboxylat;
(50) Natrium-2-{(4S)-4-[2-(2-pyrimidinylthio)acetamido]-3-oxo-2-isoxazolidinyl}-5-oxo-2-tetrahydrofurancarboxylat;
(51) Natrium-2-{(4S)-4-{2-[(E)-2-(acetamidovinyl)thio]acetamido}-3-oxo-2-isoxazolidinyl}-5-oxo-2-tetrahydrofurancarboxylat;
(52) Natrium-2-[(4S)-4-cyclopropylcarbonylamino-3-oxo-2-isoxazolidinyl]-5-oxo-2-tetrahydrofurancarboxylat;
(53) Natrium-2-[(4S)-4-methoxalylamino-3-oxo-2-isoxazolidinyl]-5-oxo-2-tetrahydrofurancarboxylat;
(54) Natrium-2-[(4S)-4-acrylamido-3-oxo-2-isoxazolidinyl]-5-oxo-2-tetrahydrofurancarboxylat;
(55) Natrium-2-{(4S)-4-[2-(2-chlor-4-pyridylthio)acetamido]-3-oxo-2-isoxazolidinyl}-5-oxo-2-tetrahydrofurancarboxylat;
(56) Natrium-2-[(4S)-4-(2-acetamido-5-oxo-2-tetrahydrofurancarbonylamino)-3-oxo-2-isoxazolidinyl]-5-oxo-2-tetrahydrofurancarboxylat;
(57) Natrium-2-{(4S)-4-[2-(1-pyrazolyl)acetamido]-3-oxo-2-isoxazolidinyl}-5-oxo-2-tetrahydrofurancarboxylat;
(58) Natrium-2-{(4S)-4-[2-(5-amino-1,2,4-thiadiazol-3-yl)-(Z)-2-ethoxyiminoacetamido]-3-oxo-2-isoxazolidinyl}-5-oxo-2-tetrahydrofurancarboxylat;
(59) Natrium-2-{(4S)-4-[2-(3-pyridazinylthiol)acetamido]-3-oxo-2-isoxazolidinyl}-5-oxo-2-tetrahydrofurancarboxylat;
(60) Natrium-2-[(4S)-4-dichloracetamido-3-oxo-2-isoxazolidinyl]-5-oxo-2-tetrahydrofurancarboxylat;
(61) Natrium-2-{(4S)-4-[(2R,3S)-2-(4-ethyl-2,3-dioxo-1-piperazincarboxamido)-3-hydroxybutyramido]-3-oxo-2-isoxazolidinyl}-5-oxo-2-tetrahydrofurancarboxylat;
(62) Natrium-2-{(4S)-4-[2-(5-trifluormethyl-1,3,4-thiadiazol-2-ylthio)acetamido]-3-oxo-2-isoxazolidinyl}-5-oxo-2-tetrahydrofurancarboxylat;
(63) Natrium-2-{(4S)-4-{2-[1-ethoxy(hydroxy)phosphinylmethyl-1H-5-tetrazolylthio]acetamido}-3-oxo-2-isoxazolidinyl}-5-oxo-2-tetrahydrofurancarboxylat;
(64) Natrium-2-{(4S)-4-{2-[5-(2-diethoxyphosphinylethylthio-1,3,4-thiadiazolylthio]acetamido}-3-oxo-2-isoxazolidinyl}-5-oxo-2-tetrahydrofurancarboxylat;
(65) Natrium-2-{(4S)-4-[2-(1-dimethylamino-1H-5-tetrazolylthio)acetamido]-3-oxo-2-isoxazolidinyl}-5-oxo-2-tetrahydrofurancarboxylat;
(66) Natrium-2-{(4S)-4-[2-(4,5-dimethyl-2-thiazolylthio)acetamido]-3-oxo-2-isoxazolidinyl}-5-oxo-2-tetrahydrofurancarboxylat;
(67) Natrium-2-{(4S)-4-[2-(4,5-dimethyl-2-oxazolylthio)acetamido]-3-oxo-2-isoxazolidinyl)-5-oxo-2-tetrahydrofurancarboxylat;
(68) Natrium-2-{(4S)-4-[2-(5-methoxymethyl-1,3,4-thiadiazol-2-ylthio)acetamido]-3-oxo-2-isoxazolidinyl}-5-oxo-2-tetrahydrofurancarboxylat;
(69) Natrium-2-{(4S)-4-[2-(5-methylsulfonylmethyl-1,3,4-thiadiazol-2-ylthio)acetamido]-3-oxo-2-isoxazolidinyl}-5-oxo-2-tetrahydrofurancarboxylat;
(70) Natrium-2-{(4S)-4-[2-(4-ethyl-4H-1,2,4-triazol-3-ylthio)acetamido]-3-oxo-2-isoxazolidinyl)-5-oxo-2-tetrahydrofurancarboxylat;
(71) Dinatrium-2-{(4S)-4-[2-(5-carboxymethyl-1,3,4-thiadiazol-2-ylthio)acetamido]-3-oxo-2-isoxazolidinyl}-5-oxo-2-tetrahydrofurancarboxylat;
(72) Natrium-2-{(4S)-4-{2-[1-(2-hydroxyethyl)-1H-5-tetrazolylthio]acetamido}-3-oxo-2-isoxazolidinyl}-5-oxo-2-tetrahydrofurancarboxylat;
(73) Natrium-2-{(4S)-4-{2-[1-(3-dimethylaminopropyl)-1H-5-tetrazolylthio]acetamido}-3-oxo-2-isoxazolidinyl}-5-oxo-2-tetrahydrofurancarboxylat;
(74) 2-[(4S)-4-(2-Amino-3-sulfamoylpropionamido)-3-oxo-2-isoxazolidinyl]-5-oxo-2-tetrahydrofurancarbonsäure;
(75) Natrium-2-{(4S)-4-[2-(4-cyan-3-hydroxy-5-isothiazolylthio)acetamido]-3-oxo-2-isoxazolidinyl}-5-oxo-2-tetrahydrofurancarboxylat;
(76) Natrium-2-{(4S)-4-[2-(2-amino-4-thiazolyl)-2-oxo-acetamido]-3-oxo-2-isoxazolidinyl}-5-oxo-2-tetrahydrofurancarboxylat;
(77) Natrium-2-{(4S)-4-[2-(2-amino-4-thiazolyl)-(Z)-2-isopropoxyiminoacetamido]-3-oxo-2-isoxazolidinyl}-5-oxo-2-tetrahydrofurancarboxylat;
(78) Natrium-2-[(4S)-4-trifluoracetamido-3-oxo-2-isoxazolidinyl]-5-oxo-2-tetrahydrofurancarboxylat;
(79) Dinatrium-2-{(4S)-4-[2-(2-amino-4-thiazolyl)-(Z)-2-(1-carboxy-1-methylethoxyimino)acetamido]-3-oxo-2-isoxazolidinyl}-5-oxo-2-tetrahydrofurancarboxylat;
(80) Natrium-2-{(4S)-4-[2-(2-amino-4-thiazolyl)-(Z)-2-(hydroxyimino)acetamido]-3-oxo-2-isoxazolidinyl}-5-oxo-2-tetrahydrofurancarboxylat;
(81) Natrium-2-[(4S)-4-isobutyloxycarbonylamino-3-oxo-2-isoxazolidinyl]-5-oxo-2-tetrahydrofurancarboxylat;
(82) 2-{(4S)-4-[N-(4-Nitrobenzyloxycarbonyl)glycylamino]-3-oxo-2-isoxazolidinyl}-5-oxo-2-tetrahydrofurancarbonsäure-diphenylmethylester;
(83) 2-{(4S)-4-[N-(2,2,2-Trichlorethoxycarbonyl)phenylglycylamino]-3-oxo-2-isoxazolidinyl}-5-oxo-2-tetrahydrofurancarbonsäure-diphenylmethylester;
(84) Natrium-2-{(4S)-4-[N-(benzyloxycarbonyl)-D-phenylglycylamino]-3-oxo-2-isoxazolidinyl}-5-oxo-2-tetrahydrofurancarboxylat;
(85) 2-[(4S)-4-L-Phenylglycylamino-3-oxo-2-isoxazolidinyl]-5-oxo-2-tetrahydrofurancarbonsäure;
(86) 2-[(4S)-4-D-Phenylglycylamino-3-oxo-2-isoxazolidinyl]-5-oxo-2-tetrahydrofurancarbonsäure;
(87) Natrium-2-{(4S)-4-[(2S)(2-hydroxy-2-phenyl)acetamido]-3-oxo-2-isoxazolidinyl}-5-oxo-2-tetrahydrofurancarboxylat;
(88) Natrium-2-{(4S)-4-[(2R)-(2-hydroxy-2-phenyl)acetamido]-3-oxo-2-isoxazolidinyl}-5-oxo-2-tetrahydrofurancarboxylat;
(89) Natrium-2-{(4S)-4-[2-(4-chlorphenyl)-2-hydroxyacetamido]-3-oxo-2-isoxazolidinyl}-5-oxo-2-tetrahydrofurancarboxylat;
(90) Natrium-2-{(4S)-4-[2-hydroxy-2-(4-hydroxyphenyl)acetamido]-3-oxo-2-isoxazolidinyl}-5-oxo-2-tetrahydrofurancarboxylat;
(91) Natrium-2-[(4S)-4-(2-hydroxypropionamido)-3-oxo-2-isoxazolidinyl]-5-oxo-2-tetrahydrofurancarboxylat;
(92) Dinatrium-2-[(4S)-4-(2-carboxy-2-phenylacetamido)-3-oxo-2-isoxazolidinyl]-5-oxo-2-tetrahydrofurancarboxylat;
(93) Natrium-2-[(4S)-4-benzyloxycarbonylamino-3-oxo-2-isoxazolidinyl]-5-oxo-2-tetrahydrofurancarboxylat;

(94) Natrium-2-[(4S)-4-pyruvamido-3-oxo-2-isoxazolidinyl]-5-oxo-2-tetrahydrofurancarboxylat;
(95) 2-{(4S)-4-[(2R)-(2-Hydroxy-2-phenyl)acetamido]-3-oxo-2-isoxazolidinyl}-5-oxo-2-tetrahydrofurancarbonsäure-pivaloyloxymethylester;
(96) 2-[(4S)-4-(4-Nitrobenzyliden)amino-3-oxo-2-isoxazolidinyl]-5-oxo-2-tetrahydrofurancarbonsäure-diphenylmethylester;
(97) Natrium-2-{(4S)-4-[2-(3-chlor-6-pyridazinylthio)acetamido]-3-oxo-2-isoxazolidinyl}-5-oxo-2-tetrahydrofurancarboxylat;
(98) Natrium-2-[(4S)-4-(2-phenylthioacetamido)-3-oxo-2-isoxazolidinyl]-5-oxo-2-tetrahydrofurancarboxylat;
(99) Natrium-2-[(4S)-4-(2-ethoxydithiocarbonylacetamido)-3-oxo-2-isoxazolidinyl]-5-oxo-2-tetrahydrofurancarboxylat;
(100) Natrium-2-{(4S)-4-[2-(2-amino-4-thiazolyl)-(Z)-2-(ethoxyimino)acetamido]-3-oxo-2-isoxazolidinyl}-5-oxo-2-tetrahydrofurancarboxylat;
(101) Natrium-2-{(4S)-4-[2-(2-amino-5-chlor-4-thiazolyl)-(Z)-2-(methoxyimino)acetamido]-3-oxo-2-isoxazolidinyl}-5-oxo-2-tetrahydrofurancarboxylat;
(102) Natrium-2-{(4S)-4-[2-(4-ethyl-2,3-dioxo-1-piperazinocarboxamido)-2-thienylacetamido]-3-oxo-2-isoxazolidinyl}-5-oxo-2-tetrahydrofurancarboxylat;
(103) Natrium-2-[(4S)-4-ureido-3-oxo-2-isoxazolidinyl]-5-oxo-2-tetrahydrofurancarboxylat;
(104) Natrium-2-[(4S)-4-phenylureylen-3-oxo-2-isoxazolidinyl]-5-oxo-2-tetrahydrofurancarboxylat;
(105) Natrium-2-{(4S)-4-[2-(pyridyl)acetamido]-3-oxo-2-isoxazolidinyl}-5-oxo-2-tetrahydrofurancarboxylat;
(106) Natrium-2-{(4S)-4-[2-(4-chlorphenyl)acetamido]-3-oxo-2-isoxazolidinyl}-5-oxo-2-tetrahydrofurancarboxylat;
(107) Natrium-2-{(4S)-4-[(2-chlor-2-phenyl)acetamido]-3-oxo-2-isoxazolidinyl}-5-oxo-2-tetrahydrofurancarboxylat;
(108) Natrium-2-{(4S)-4-[2-(3-pyridylthio)acetamido]-3-oxo-2-isoxazolidinyl}-5-oxo-2-tetrahydrofurancarboxylat;
(109) Natrium-2-{(4S)-4-[2-(4-chlorphenylthio)acetamido]-3-oxo-2-isoxazolidinyl}-5-oxo-2-tetrahydrofurancarboxylat;
(110) Natrium-2-[(4S)-4-(2-isopropylphenoxycarbonylamino)-3-oxo-2-isoxazolidinyl]-5-oxo-2-tetrahydrofurancarboxylat;
(111) Natrium-2-[(4S)-4-difluoracetamido-3-oxo-2-isoxazolidinyl]-5-oxo-2-tetrahydrofurancarboxylat;
(112) Natrium-2-[(4S)-4-(3-chlor)propionamido-3-oxo-2-isoxazolidinyl]-5-oxo-2-tetrahydrofurancarboxylat;
(113) 2-[(4S)-4-Chloracetamido-3-oxo-2-isoxazolidinyl]-5-oxo-2-tetrahydrofurancarbonsäure-pivaloyloxymethylester;
(114) 2-[(4S)-4-(2-Phenylthioacetamido)-3-oxo-2-isoxazolidinyl]-5-oxo-2-tetrahydrofurancarbonsäure-pivaloyloxymethylester;
(115) Natrium-2-[(4S)-4-propionamido-3-oxo-2-isoxazolidinyl]-5-oxo-2-tetrahydrofurancarboxylat;
(116) 2-[(4S)-4-Benzyloxycarbonylamino-3-oxo-2-isoxazolidinyl]-5-oxo-2-tetrahydrofurancarbonsäure-pivaloyloxymethylester;
(117) 2-[(4S)-4-Bromacetamido-3-oxo-2-isoxazolidinyl]-5-oxo-2-tetrahydrofurancarbonsäure-pivaloyloxymethylester;
(118) 2-[(4S)-4-(2,4-Hexadien)amido-3-oxo-2-isoxazolidinyl]-5-oxo-2-tetrahydrofurancarbonsäure-pivaloyloxymethylester;
(119) 2-[(4S)-4-(2-Thienyl)acetamido-3-oxo-2-isoxazolidinyl]-5-oxo-2-tetrahydrofurancarbonsäure-pivaloyloxymethylester;
(120) 2-[(4S)-4-(Hexahydro-1H-azepin-1-yl)methylenamino-3-oxo-2-isoxazolidinyl]-5-oxo-2-tetrahydrofurancarbonsäure-pivaloyloxymethylester;
(121) 2-[(4S)-4-(t-Butyldimethylsilyl)amino-3-oxo-2-isoxazolidinyl]-5-oxo-2-tetrahydrofurancarbonsäure-diphenylmethylester;
(122) 2-[(4S)-4-(t-Butyldiphenylsilyl)amino-3-oxo-2-isoxazolidinyl]-5-oxo-2-tetrahydrofurancarbonsäure-diphenylmethylester;
(123) Natrium-2-[(4S)-3-diphenylphosphorylamino-3-oxo-2-isoxazolidinyl]-5-oxo-2-tetrahydrofurancarboxylat;
(124) 2-[(4S)-4-Dimethylphosphorylamino-3-oxo-2-isoxazolidinyl]-5-oxo-2-tetrahydrofurancarbonsäure-diphenylmethylester;
(125) Natrium-2-[(4S)-4-ethylsulfonylamino-3-oxo-2-isoxazolidinyl]-5-oxo-2-tetrahydrofurancarboxylat;

(126) Natrium-2-[(4S)-4-vinylsulfonylamino-3-oxo-2-isoxazolidinyl]-5-oxo-2-tetrahydrofurancarboxylat;
(127) 2-[(4S)-4-Thienylacetamido-3-oxo-2-isoxazolidinyl]-5-oxo-2-tetrahydrofurancarbonylanilid;
(128) 2-[(4S)-4-Benzyloxycarbonylamino-3-oxo-2-isoxazolidinyl]-5-oxo-2-tetrahydrofurancarbonylanilid;
(129) 2-[(4S)-4-Benzyloxycarbonylamino-3-oxo-2-isoxazolidinyl]-5-oxo-2-tetrahydrofurancarbonylpyrrolidinamid;
(130) 2-[(4S)-4-Thienylacetamido-3-oxo-2-isoxazolidinyl]-5-oxo-2-tetrahydrofurancarbonylpyrrolidinamid;
(131) 2-[(4S)-4-Thienylacetamido-3-oxo-2-isoxazolidinyl]-5-oxo-2-tetrahydrofurancarbonylpropylamin;
(132) Natrium-2-[(4S)-4-γ-D-glutamoylamino-3-oxo-2-isoxazolidinyl]-5-oxo-2-tetrahydrofurancarboxylat; und
(133) Natrium-2-[(4S)-4-(2-hydroxy)isobutyrylamino-3-oxo-2-isoxazolidinyl]-5-oxo-2-tetrahydrofurancarboxylat;

zusammen mit einem pharmazeutisch annehmbaren Träger oder Verdünnungsmittel für diese.

2. Verwendung einer Verbindung (I') oder deren pharmazeutisch annehmbaren Salzes wie in Anspruch 1 definiert, zur Herstellung einer pharmazeutischen Zusammensetzung zur Hemmung des Wachstums von Mikroorganismen.

## Revendications

**Revendications pour les Etats contractants suivants : BE, CH, DE, FR, GB, IT, LI, LU, NL, SE**

1. Dérivé de l'acide 2-[(4S)-amino-3-oxo-2-isoxazolidinyl]-5-oxo-2-tétrahydrofurannecarboxylique de formule (I') ou sel pharmaceutiquement acceptable de celui-ci, choisi parmi:

(1) le 2-{(4S)-4-[2-(2-amino-4-thiazolyl)-(Z)-2-(méthoxyimino)acétamido]-3-oxo-2-isoxazolidiny|}-5-oxo-2-tétrahydrofurannecarboxylate de sodium;
(2) le 2-{(4S)-4-[D-2-(4-éthyl-2,3-dioxo-1-pipérazinocarboxamido)-2-phénylacétamido]-3-oxo-2-isoxazolidinyl}-5-oxo-2-tétrahydrofurannecarboxylate de sodium;
(3) le 2-{(4S)-4-[D-2-(3-méthylsulfonyl-2-oxoimidazolidine-1-carboxamido)-2-phénylacétamido]-3-oxo-2-isoxazolidinyl}-5-oxo-2-tétrahydrofurannecarboxylate de sodium;
(4) le 2-{(4S)-4-[D-2-(3-furfurylidène-amino-2-oxoimidazolidine-1-carboxamido-2-phénylacétamido]-3-oxo-2-isoxazolidinyl}-5-oxo-2-tétrahydrofurannecarboxylate de sodium;
(5) le 2-[(4S)-4-(2-thiénylacétamido)-3-oxo-2-isoxazolidinyl]-5-oxo-2-tétrahydrofurannecarboxylate de sodium;
(6) le 2-[(4S)-4-(2-cyanoacétamido)-3-oxo-2-isoxazolidinyl]-5-oxo-2-tétrahydrofurannecarboxylate de sodium;
(7) le 2-{(4S)-4-[(1H)-tétrazolylacétamido]-3-oxo-2-isoxazolidinyl}-5-oxo-2-tétrahydrofurannecarboxylate de sodium;
(8) le 2-[(4S)-4-(2,6-diméthoxybenzamido)-3-oxo-2-isoxazolidinyl]-5-oxo-2-tétrahydrofurannecarboxylate de sodium;
(9) le 2-[(4S)-4-(5-méthyl-3-phényl-2-isoxazoline-4-carboxamido)-3-oxo-2-isoxazolidinyl]-5-oxo-2-tétrahydrofurannecarboxylate de sodium;
(10) le 2-[(4S)-4-(2-éthoxynaphtalényl-1-carboxamido)-3-oxo-2-isoxazolidinyl]-5-oxo-2-tétrahydrofurannecarboxylate de sodium;
(11) le 2-[(4S)-4-(2-cyanométhylthioacétamido)-3-oxo-2-isoxazolidinyl]-5-oxo-2-tétrahydrofurannecarboxylate de sodium;
(12) le 2-[(4S)-4-(2-difluorométhylthioacétamido)-3-oxo-2-isoxazolidinyl]-5-oxo-2-tétrahydrofurannecarboxylate de sodium;
(13) le 2-{(4S)-4-[(R)-2-(4-cyclohexyl-2,3-dioxo-1-pipérazinocarboxamido-2-phénylacétamido]-3-oxo-2-isoxazolidinyl}-5-oxo-2-tétrahydrofurannecarboxylate de sodium;
(14) le 2-((4S)-4-chloracétamido-3-oxo-2-isoxazolidinyl)-5-oxo-2-tétrahydrofurannecarboxylate de sodium;
(15) le 2-{(4S)-4-[2-(4-pyridylthio)acétamido]-3-oxo-2-isoxazolidinyl}-5-oxo-2-tétrahydrofurannecarboxylate de sodium;
(16) le 2-[(4S)-4-acétamido-3-oxo-2-isoxazolidinyl]-5-oxo-2-tétrahydrofurannecarboxylate de méthyle;
(17) le 2-[(4S)-4-acétamido-3-oxo-2-isoxazolidinyl]-5-oxo-2-tétrahydrofurannecarboxylate de pivaloyloxyméthyle;
(18) le 2-[(4S)-4-acétamido-3-oxo-2-isoxazolidinyl]-5-oxotétrahydrofurannecarboxylate de 3,5-dinitro-

benzyle;
(19) le 2-[(4S)-4-acétamido-3-oxo-2-isoxazolidinyl]-5-oxo-2-tétrahydrofurannecarboxylate de p-bromo-phénacyle;
(20) le 2-[(4S)-4-acétamido-3-oxo-2-isoxazolidinyl]-5-oxo-2-tétrahydrofurannecarboxylate de p-bromo-benzyle;
(21) le 2-[(4S)-4-formylamino-3-oxo-2-isoxazolidinyl]-5-oxo-2-tétrahydrofurannecarboxylate de sodium;
(22) le 2-[(4S)-4-n-butyrylamino-3-oxo-2-isoxazolidinyl]-5-oxo-2-tétrahydrofurannecarboxylate de sodium;
(23) le 2-[(4S)-4-méthoxycarbonylamino-3-oxo-2-isoxazolidinyl]-5-oxo-2-tétrahydrofurannecarboxylate de sodium;
(24) le 2-[(4S)-4-(N-benzyloxycarbonyl)-glycylamino-3-oxo-2-isoxazolidinyl]-5-oxo-2-tétrahydrofurannecarboxylate de sodium;
(25) le 2-[(4S)-4-benzoylamino-3-oxo-2-isoxazolidinyl]-5-oxo-2-tétrahydrofurannecarboxylate de sodium;
(26) le 2-[(4S)-4-phénylacétylamino-3-oxo-2-isoxazolidinyl]-5-oxo-2-tétrahydrofurannecarboxylate de sodium;
(27) le 2-[(4S)-4-nicotinylamino-3-oxo-2-isoxazolidinyl]-5-oxo-2-tétrahydrofurannecarboxylate de sodium;
(28) le 2-[(4S)-4-phénoxyacétylamino-3-oxo-2-isoxazolidinyl]-5-oxo-2-tétrahydrofurannecarboxylate de pivaloyloxyméthyle;
(29) le 2-[(4S)-4-(p-toluènesulfonyl)amino-3-oxo-2-isoxazolidinyl]-5-oxo-2-tétrahydrofurannecarboxylate de sodium;
(30) le 2-[(4S)-4-(p-bromobenzoyl)-amino-3-oxo-2-isoxazolidinyl]-5-oxo-2-tétrahydrofurannecarboxylate de diphénylméthyle;
(31) le 2-[(4S)-4-(N-benzyloxy-carbonyl-D-alanyl)amino-3-oxo-2-isoxazolidinyl]-5-oxo-2-tétrahydrofurannecarboxylate de diphénylméthyle;
(32) l'acide 2-[(4S)-4-diméthylamino-3-oxo-2-isoxazolidinyl]-5-oxo-2-tétrahydrofurannecarboxylique;
(33) le 2-{(4S)-4-[2-(2-amino-4-thiazolyl)-acétamido]-3-oxo-2-isoxazolidinyl}-5-oxo-2-tétrahydrofurannecarboxylate de sodium;
(34) le 2-[4-méthoxy-4-(2-thiénylacétamido)-3-oxo-2-isoxazolidinyl]-5-oxo-2-tétrahydrofurannecarboxylate de sodium;
(35) le 2-{(4S)-4-[2-(2-amino-4-thiazolyl)-(Z)-2-(carboxyméthyloxyimino)acétamido]-3-oxo-2-isoxazolidinyl}-5-oxo-2-tétrahydrofurannecarboxylate disodique;
(38) l'acide 2-{(4S)-4-[2-(2-aminométhyl)phénylacétamido]-3-oxo-2-isoxazolidinyl}-5-oxo-2-tétrahydrofurannecarboxylique;
(39) le 2-[(4S)-4-(4-fluorobenzènesulfonyl-amino-3-oxo-2-isoxazolidinyl]-5-oxo-2-tétrahydrofurannecarboxylate de sodium;
(40) le 2-{(4S)-4-{2-[1-(2-diméthylaminoéthyl)-1H-5-tétrazolylthio]acétamido}-3-oxo-2-isoxazolidinyl}-5-oxo-2-tétrahydrofurannecarboxylate de sodium;
(41) le 2-{(4S)-4-[2-(4-méthyl-4H-1,2,4-triazolyl-3-ylthio)acétamido]-3-oxo-2-isoxazolidinyl}-5-oxo-2-tétrahydrofurannecarboxylate de sodium;
(42) le 2-{(4S)-4-[(2R,3S)-2-(4-éthyl-2,3-dioxo-1-pipérazinecarboxamido)-3-formyloxybutylamido]-3-oxo-2-isoxazo lidinyl}-5-oxo-2-tétrahydrofurannecarboxylate de sodium;
(43) le 2-[(4S)-4-(2-phényl-2-sulfoacétamido)-3-oxo-2-isoxazolidinyl]-5-oxo-2-tétrahydrofurannecarboxylate disodique;
(44) le 2-{(4S)-4-[2-(2-amino-4-thiazolyl)-(E)-2-méthoxyiminoacétamido]-3-oxo-2-isoxazolidinyl}-5-oxo-2-tétrahydrofurannecarboxylate de sodium;
(45) le 2-[(4S)-4-(2-triméthylsilyl)-éthoxy-carbonylamino-3-oxo-2-isoxazolidinyl]-5-oxo-2-tétrahydrofurannecarboxylate de sodium;
(46) l'acide 2-[(4S)-4-(1-aminocyclohexylcarbonylamino)-3-oxo-2-isoxazolidinyl]-5-oxo-2-tétrahydrofurannecarboxylique de sodium;
(47) le 2-{(4S)-4-[3-(2,6-dichlorophényl)-5-méthyl-4-isoxazolylcarbonylamino]-3-oxo-2-isoxazolidinyl}-5-oxo-2-tétrahydrofurannecarboxylate de sodium;
(48) le 2-{(4S)-4-[2-(2-pyridylthio)acétamido]-3-oxo-2-isoxazolidinyl}-5-oxo-2-tétrahydrofurannecarboxylate de sodium;
(49) le 2-{(4S)-4-[2-(4-pyridylméthylthio)-acétamido]-3-oxo-2-isoxazolidinyl}-5-oxo-2-tétrahydrofurannecarboxylate de sodium;

(50) le 2-{(4S)-4-[2-(2-pyrimidinylthio)-acétamido]-3-oxo-2-isoxazolidinyl}-5-oxo-2-tétrahydrofurannecarboxylate de sodium;
(51) le 2-{(4S)-4-{2-[(E)-2-(acétamidovinyl)-thio]acétamido}-3-oxo-2-isoxazolidinyl}-5-oxo-2-tétrahydrofurannecarboxylate de sodium;
(52) le 2-[(4S)-4-cyclopropylcarbonylamino-3-oxo-2-isoxazolidinyl]-5-oxo-2-tétrahydrofurannecarboxylate de sodium;
(53) le 2-[(4S)-4-méthoxalylamino-3-oxo-2-isoxazolidinyl]-5-oxo-2-tétrahydrofurannecarboxylate de sodium;
(54) le 2-[(4S)-4-acrylamido-3-oxo-2-isoxazolidinyl]-5-oxo-2-tétrahydrofurannecarboxylate de sodium;
(55) le 2-{(4S)-4-[2-(2-chloro-4-pyridylthio)acétamido]-3-oxo-2-isoxazolidinyl}-5-oxo-2-tétrahydrofurannecarboxylate de sodium;
(56) le 2-[(4S)-4-(2-acétamido-5-oxo-2-tétrahydrofurannecarbonylamino)-3-oxo-2-isoxazolidinyl]-5-oxo-2-tétrahydrofurannecarboxylate de sodium;
(57) le 2-{(4S)-4-[2-(1-pyrazolyl)acétamido]-3-oxo-2-isoxazolidinyl}-5-oxo-2-tétrahydrofurannecarboxylate de sodium;
(58) le 2-{(4S)-4-[2-(5-amino-1,2,4-thiadiazole-3-yl)-(Z)-2-éthoxyiminoacétamido]-3-oxo-2-isoxazolidinyl}-5-oxo-tétrahydrofurannecarboxylate de sodium;
(59) le 2-{(4S)-4-[2-(3-pyridazinylthio)-acétamido]-3-oxo-2-isoxazolidinyl}-5-oxo-2-tétrahydrofurannecarboxylate de sodium;
(60) le 2-[(4S)-4-dichloroacétamido-3-oxo-2-isoxazolidinyl]-5-oxo-2-tétrahydrofurannecarboxylate de sodium;
(61) le 2-{(4S)-4-[(2R,3S)-2-(4-éthyl-2,3-dioxo-1-pipérazinecarboxamido)-3-hydroxybutyramido]-3-oxo-2-isoxazolidinyl}-5-oxo-2-tétrahydrofurannecarboxylate de sodium;
(62) le 2-{(4S)-4-[2-(5-trifluorométhyl-1,3,4-thiadiazol-2-ylthio)acétamido]-3-oxo-2-isoxazolidinyl}-5-oxo-2-tétrahydrofurannecarboxylate de sodium;
(63) le 2-{(4S)-4-{2-[1-éthoxy(hydroxy)-phosphinylméthyl1H-5-tétrazolylthio]acétamido}-3-oxo-2-isoxazolidinyl}-5-oxo-2-tétrahydrofurannecarboxylate de sodium;
(64) le 2-{(4S)-4-{2-[5-(2-diéthoxyphosphinyléthylthio)-1,3,4-thiadiazolylthio]acétamido}-3-oxo-2-isoxazolidinyl}-5-oxo-2-tétrahydrofurannecarboxylate de sodium;
(65) le 2-{(4S)-4-[2-(1-diméthylamino-1H-5-tétrazolylthio)acétamido]-3-oxo-2-isoxazolidinyl}-5-oxo-2-tétrahydrofurannecarboxylate de sodium;
(66) le 2-{(4S)-4-[2-(4,5-diméthyl-2-thiazolylthio)acétamido]-3-oxo-2-isoxazolidinyl}-5-oxo-2-tétrahydrofurannecarboxylate de sodium;
(67) le 2-{(4S)-4-[2-(4,5-diméthyl-2-oxazolylthio)acétamido]-3-oxo-2-isoxazolidinyl}-5-oxo-2-tétrahydrofurannecarboxylate de sodium;
(68) le 2-{(4S)-4-[2-(5-méthoxyméthyl-1,3,4-thiadiazol-2-ylthio)acétamido]-3-oxo-2-isoxazolidinyl}-5-oxo-2-tétrahydrofurannecarboxylate de sodium;
(69) le 2-{(4S)-4-[2-(5-méthylsulfonyl-méthyl-1,3,4-thiadiazol-2-ylthio)acétamido]-3-oxo-2-isoxazolidinyl}-5-oxo-2-tétrahydrofurannecarboxylate de sodium;
(70) le 2-{(4S)-4-[2-(4-éthyl-4H-1,2,4-triazol-3-ylthio)acétamido]-3-oxo-2-isoxazolidinyl}-5-oxo-2-tétrahydrofurannecarboxylate de sodium;
(71) le 2-{(4S)-4-[2-(5-carboxyméthyl-1,3,4-thiadiazol-2-ylthio)acétamido]-3-oxo-2-isoxazolidinyl}-5-oxo-2-tétrahydrofurannecarboxylate disodique;
(72) le 2-{(4S)-4-{2-[1-(2-hydroxyéthyl)-1H-5-tétrazolylthio]acétamido}-3-oxo-2-isoxazolidinyl}-5-oxo-2-tétrahydrofurannecarboxylate de sodium;
(73) le 2-{(4S)-4-{2-[1-(3-diméthylamino-propyl)-1H-5-tétrazolylthio]acétamido}-3-oxo-2-isoxazolidinyl}-5-oxo-2-tétrahydrofurannecarboxylate de sodium;
(74) l'acide 2-[(4S)-4-(2-amino-3-sulfamoylpropionamido)-3-oxo-2-isoxazolidinyl]-5-oxo-2-tétrahydrofurannecarboxylique;
(75) le 2-{(4S)-4-[2-(4-cyano-3-hydroxy-5-isothiazolylthio)acétamido]-3-oxo-2-isoxazolidinyl}-5-oxo-2-tétrahydrofurannecarboxylate de sodium;
(76) le 2-{(4S)-4-[2-(2-amino-4-thiazolyl)-2-oxoacétamido]-3-oxo-2-isoxazolidinyl}-5-oxo-2-tétrahydrofurannecarboxylate de sodium;
(77) le 2-{(4S)-4-[2-(2-amino-4-thiazolyl)-(Z)-2-isopropoxyiminoacétamido]-3-oxo-2-isoxazolidinyl}-5-oxo-2-tétrahydrofurannecarboxylate de sodium;
(78) le 2-[(4S)-4-trifluoracétamido-3-oxo-2-isoxazolidinyl]-5-oxo-2-tétrahydrofurannecarboxylate de sodium;
(79) le 2-{(4S)-4-[2-(2-amino-4-thiazolyl)-(Z)-2-(1-carboxy-1-méthyléthoxyimino)acétamido]-3-oxo-2-isoxazolidinyl}-5-oxo-2-tétrahydrofurannecarboxylate disodique;
(80) le 2-{(4S)-4-[2-(2-amino-4-thiazolyl)-(Z)-2-(hydroxyimino)acétamido]-3-oxo-2-isoxazolidinyl}-5-oxo-2-té trahydrofurannecarboxylate de sodium;
(81) le 2-[(4S)-4-isobutyloxycarbonylamino-3-oxo-2-isoxazolidinyl]-5-oxo-2-tétrahydrofurannecarboxylate de sodium;
(82) le 2-{(4S)-4-[N-(4-nitrobenzyloxycarbonyl)glycylamino]-3-oxo-2-isoxazolidinyl}-5-oxo-2-tétrahydrofurannecarboxylate de diphénylméthyle;
(83) le 2-{(4S)-4-[N-(2,2,2-trichloroéthoxycarbonyl)phénylglycylamino]-3-oxo-2-isoxazolidinyl}-5-oxo-2-tétrahydrofurannecarboxylate de diphénylméthyle;
(84) le 2-{(4S)-4-[N-(benzyloxycarbonyl)-D-phénylglycylamino]-3-oxo-2-isoxazolidinyl}-5-oxo-2-tétrahydrofurannecarboxylate de sodium;
(85) l'acide 2-[(4S)-4-L-phénylglycylamino-3-oxo-2-isoxazolidinyl]-5-oxo-2-tétrahydrofuranne-carboxylique;
(86) l'acide 2-{(4S)-4-D-phénylglycylamino-3-oxo-2-isoxazolidinyl}-5-oxo-2-tétrahydrofuranne-carboxylique;
(87) le 2-{(4S)-4-[(2S)-(2-hydroxy-2-phényl)acétamido]-3-oxo-2-isoxazolidinyl}-5-oxo-2-tétrahydrofurannecarboxylate de sodium;
(88) le 2-{(4S)-4-[(2R)-(2-hydroxy-2-phényl)acétamido-3-oxo-2-isoxazolidinyl}-5-oxo-2-tétrahydrofurannecarboxylate de sodium;
(89) le 2-{(4S)-4-[2-(4-chlorophényl)-2-hydroxyacétamido]-3-oxo-2-isoxazolidinyl}-5-oxo-2-tétrahydrofurannecarboxylate de sodium;
(90) le 2-{(4S)-4-[2-hydroxy-2-(4-hydroxyphényl)-acétamido]-3-oxo-2-isoxazolidinyl}-5-oxo-2-tétrahydrofurannecarboxylate de sodium;
(91) le 2-[(4S)-4-(2-hydroxypropionamido)-3-oxo-2-isoxazolidinyl]-5-oxo-2-tétrahydrofurannecarboxylate de sodium;
(92) le 2-[(4S)-4-(2-carboxy-2-phénylacétamido)-3-oxo-2-isoxazolidinyl]-5-oxo-2-tétrahydrofurannecarboxylate disodique;
(93) le 2-[(4S)-4-benzyloxycarbonylamino-3-oxo-2-isoxazolidinyl]-5-oxo-2-tétrahydrofurannecarboxylate de sodium;
(94) le 2-((4S)-4-pyruvamido-3-oxo-2-isoxazolidinyl)-5-oxo-2-tétrahydrofurannecarboxylate de sodium;
(95) le 2-{(4S)-4-[(2R)-(2-hydroxy-2-phényl)acétamido]-3-oxo-2-isoxazolidinyl}-5-oxo-2-tétrahydrofurannecarboxylate de pivaloyloxyméthyle;
(96) le 2-[(4S)-4-(4-nitrobenzylidène)-amino-3-oxo-2-isoxazolidinyl]-5-oxo-2-tétrahydrofurannecarboxylate de diphénylméthyle;
(97) le 2-{(4S)-4-[2-(3-choro-6-pyridazinylthio)acétamido]-3-oxo-2-isoxazolidinyl}-5-oxo-2-tétrahydrofurannecarboxylate de sodium;
(98) le 2-[(4S)-4-(2-phénylthioacétamido)-3-oxo-2-isoxazolidinyl]-5-oxo-2-tétrahydrofurannecarboxylate de sodium;
(99) le 2-[(4S)-4-(2-éthoxydithiocarbonylacétamido)-3-oxo-2-isoxazolidinyl]-5-oxo-2-tétrahydrofurannecarboxylate de sodium;
(100) le 2-{(4S)-4-[2-(2-amino-4-thiazolyl)-(Z)-2-(éthoxyimino)acétamido]-3-oxo-2-isoxazolidinyl}-5-oxo-2-tétrahydrofurannecarboxylate de sodium;
(101) le 2-{(4S)-4-[2-(2-amino-5-chloro-4-thiazolyl)-(Z)-2-(méthoxyimino)acétamido]-3-oxo-2-isoxazolidinyl}-5-oxo-2-tétrahydrofurannecarboxylate de sodium;
(102) le 2-{(4S)-4-[2-(4-éthyl-2,3-dioxo-1-pipérazinocarboxamido)-2-thiénylacétamido]-3-oxo-2-isoxazolidinyl}-5-oxo-2-tétrahydrofurannecarboxylate de sodium;
(103) le 2-((4S)-4-uréido-3-oxo-2-isoxazolidinyl)-5-oxo-2-tétrahydofurannecarboxylate de sodium;
(104) le 2-((4S)-4-phényluréylène-3-oxo-2-isoxazolidinyl)-5-oxo-2-tétrahydrofurannecarboxylate de sodium;
(105) le 2-{(4S)-4-[2-(pyridyl)acétamido]-3-oxo-2-isoxazolidinyl}-5-oxo-2-tétrahydrofurannecarboxylate de sodium;
(106) le 2-{(4S)-4-[2-(4-chlorophényl)acétamido]-3-oxo-2-isoxazolidinyl}-5-oxo-2-tétrahydrofurannecarboxylate de sodium;
(107) le 2-{(4S)-4-[(2-chloro-2-phényl)acétamido]-3-oxo-2-isoxazolidinyl}-5-oxo-2-tétrahydrofurannecarboxylate de sodium;
(108) le 2-{(4S)-4-[2-(3-pyridylthio)acétamido]-3-oxo-2-isoxazolidinyl}-5-oxo-2-tétrahydrofurannecarboxylate de sodium;
(109) le 2-{(4S)-4-[2-(4-chlorophénylthio)acétamido]-3-oxo-2-isoxazolidinyl}-5-oxo-2-tétrahydrofuran-

necarboxylate de sodium;
(110) le 2-[(4S)-4-(2-isopropylphénoxy-carbonylamino)-3-oxo-2-isoxazolidinyl]-5-oxo-2-tétrahydrofurannecarboxylate de sodium;
(111) le 2-((4S)-4-difluoracétamido-3-oxo-2-isoxazolidinyl)-5-oxo-2-tétrahydrofurannecarboxylate de sodium;
(112) le 2-[(4S)-4-(3-chloro)propionamido-3-oxo-2-isoxazolidinyl]-5-oxo-2-tétrahydrofurannecarboxylate de sodium;
(113) le 2-((4S)-4-chloracétamido-3-oxo-2-isoxazolidinyl)-5-oxo-2-tétrahydrofurannecarboxylate de pivaloyloxyméthyle;
(114) le 2-[(4S)-4-(2-phénylthioacétamido)-3-oxo-2-isoxazolidinyl]-5-oxo-2-tétrahydrofurannecarboxylate de pivaloyloxyméthyle;
(115) le 2-((4S)-4-propionamido-3-oxo-2-isoxazolidinyl)-5-oxo-2-tétrahydrofurannecarboxylate de sodium;
(116) le 2-[(4S)-4-benzyloxy-carbonylamino-3-oxo-2-isoxazolidinyl]-5-oxo-2-tétrahydrofurannecarboxylate de pivaloyloxyméhtyle;
(117) le 2-[(4S)-4-bromacétamido-3-oxo-2-isoxazolidinyl]-5-oxo-2-tétrahydrofurannecarboxylate de pivaloyloxyméthyle;
(118) le 2-[(4S)-4-(2,4-hexadiène)-amido-3-oxo-2-isoxazolidinyl]-5-oxo-2-tétrahydrofurannecarboxylate de pivaloyloxyméthyle;
(119) le 2-[(4S)-4-(2-thiényl)-acétamido-3-oxo-2-isoxazolidinyl]-5-oxo-2-tétrahydrofurannecarboxylate de pivaloyloxyméthyle;
(120) le 2-[(4S)-4-(hexahydro-1H-azépine-1-yl)méthylèneamino-3-oxo-2-isoxazolidinyl]-5-oxo-2-tétrahydrofurannecarbo xylate de pivaloyloxyméthyle;
(121) le 2-[(4S)-4-(tert-butyl-diméthylsilyl)amino-3-oxo-2-isoxazolidinyl]-5-oxo-2-tétrahydrofurannecarboxylate de diphénylméthyle;
(122) le 2-[(4S)-4-tert-butyl-diphénylsilyl)amino-3-oxo-2-isoxazolidinyl]-5-oxo-2-tétrahydrofurannecarboxylate de diphénylméthyle;
(123) le 2-[(4S)-3-diphénylphosphorylamino-3-oxo-2-isoxazolidinyl]-5-oxo-2-tétrahydrofurannecarboxylate de sodium;
(124) le 2-[(4S)-4-diméthyl-phosphorylamino-3-oxo-2-isoxazolidinyl]-5-oxo-2-tétrahydrofurannecarboxylate de diphénylméthyle;
(125) le 2-[(4S)-4-éthylsulfonylamino-3-oxo-2-isoxazolidinyl]-5-oxo-2-tétrahydrofurannecarboxylate de sodium;
(126) le 2-[(4S)-4-vinylsulfonylamino-3-oxo-2-isoxazolidinyl]-5-oxo-2-tétrahydrofurannecarboxylate de sodium;
(127) le 2-[(4S)-4-thiénylacétamido-3-oxo-2-isoxazolidinyl]-5-oxo-2-tétrahydrofurannecarbonylanilide;
(128) le 2-[(4S)-4-benzyloxycarbonylamino-3-oxo-2-isoxazolidinyl]-5-oxo-2-tétrahydrofurannecarbonylanilide;
(129) le 2-[(4S)-4-benzyloxycarbonylamino-3-oxo-2-isoxazolidinyl]-5-oxo-2-tétrahydrofurannecarbonylpyrrolidinamide;
(130) le 2-[(4S)-4-thiénylacétamido-3-oxo-2-isoxazolidinyl]-5-oxo-2-tétrahydrofurannecarbonylpyrrolidinamide;
(131) la 2-[(4S)-4-thiénylacétamido-3-oxo-2-isoxazolidinyl]-5-oxo-2-tétrahydrofurannecarbonylpropylamine;
(132) le 2-[(4S)-4-$\gamma$-D-glutamylamino-3-oxo-2-isoxazolidinyl]-5-oxo-2-tétrahydrofurannecarboxylate de sodium et
(133) le 2-[(4S)-4-(2-hydroxy)isobutyryl-amino-3-oxo-2-isoxazolidinyl]-5-oxo-2-tétrahydrofurannecarboxylate de sodium.

**2.** Composition pharmaceutique pour l'inhibition de la croissance de microorganismes, qui contient une quantité efficace d'un composé (I') ou d'un sel pharmaceutiquement acceptable de celui-ci, tel que défini à la revendication 1, ensemble avec une matière de support ou un diluant pharmaceutiquement acceptable.

**3.** Composé (I') ou sel pharmaceutiquement acceptable de celui-ci, tel que défini à la revendication 1, pour son utilisation dans la préparation d'une composition pharmaceutique pour l'inhibition de la croissance de microorganismes.

**Revendications pour l'Etat contractant suivant : AT**

1. Composition pharmaceutique pour l'inhibition de la croissance de microorganismes, qui contient une quantité efficace d'un dérivé de l'acide 2-[(4S)-amino-3-oxo-2-isoxazolidinyl]-5-oxo-2-tétrahydrofuranne carboxylique, composé (I'), ou d'un sel pharmaceutiquement acceptable de celui-ci, choisi parmi:
(1) le 2-{(4S)-4-[2-(2-amino-4-thiazolyl)-(Z)-2-(méthoxyimino)acétamido]-3-oxo-2-isoxazolidinyl}-5-oxo-2-tétrahydrofurannecarboxylate de sodium;
(2) le 2-{(4S)-4-[D-2-(4-éthyl-2,3-dioxo-1-pipérazinocarboxamido)-2-phénylacétamido]-3-oxo-2-isoxazolidinyl}-5-oxo-2-tétrahydrofurannecarboxylate de sodium;
(3) le 2-{(4S)-4-[D-2-(3-méthylsulfonyl-2-oxoimidazolidine-1-carboxamido)-2-phénylacétamido]-3-oxo-2-isoxazolidinyl}-5-oxo-2-tétrahydrofurannecarboxylate de sodium;
(4) le 2-{(4S)-4-[D-2-(3-furfurylidène-amino-2-oxoimidazolidine-1-carboxamido-2-phénylacétamido]-3-oxo-2-isoxazolidinyl}-5-oxo-2-tétrahydrofurannecarboxylate de sodium;
(5) le 2-[(4S)-4-(2-thiénylacétamido)-3-oxo-2-isoxazolidinyl]-5-oxo-2-tétrahydrofurannecarboxylate de sodium;
(6) le 2-[(4S)-4-(2-cyanoacétamido)-3-oxo-2-isoxazolidinyl]-5-oxo-2-tétrahydrofurannecarboxylate de sodium;
(7) le 2-{(4S)-4-[(1H)-tétrazolylacétamido]-3-oxo-2-isoxazolidinyl}-5-oxo-2-tétrahydrofurannecarboxylate de sodium;
(8) le 2-[(4S)-4-(2-6-diméthoxybenzamido)-3-oxo-2-isoxazolidinyl]-5-oxo-2-tétrahydrofurannecarboxylate de sodium;
(9) le 2-[(4S)-4-(5-méthyl-3-phényl-2-isoxazoline-4-carboxamido)-3-oxo-2-isoxazolidinyl]-5-oxo-2-tétrahydrofurannecarboxylate de sodium;
(10) le 2-[(4S)-4-(2-éthoxynaphtalényl-1-carboxamido)-3-oxo-2-isoxazolidinyl]-5-oxo-2-tétrahydrofurannecarboxylate de sodium;
(11) le 2-[(4S)-4-(2-cyanométhylthioacétamido)-3-oxo-2-isoxazolidinyl]-5-oxo-2-tétrahydrofurannecarboxylate de sodium;
(12) le 2-[(4S)-4-(2-difluorométhylthioacétamido)-3-oxo-2-isoxazolidinyl]-5-oxo-2-tétrahydrofurannecarboxylate de sodium;
(13) le 2-{(4S)-4-[(R)-2-(4-cyclohexyl-2,3-dioxo-1-pipérazinocarboxamido-2-phénylacétamido]-3-oxo-2-isoxazolidinyl}-5-oxo-2-tétrahydrofurannecarboxylate de sodium;
(14) le 2-((4S)-4-chloracétamido-3-oxo-2-isoxazolidinyl)-5-oxo-2-tétrahydrofurannecarboxylate de sodium;
(15) le 2-{(4S)-4-[2-(4-pyridylthio)acétamido]-3-oxo-2-isoxazolidinyl}-5-oxo-2-tétrahydrofurannecarboxylate de sodium;
(16) le 2-[(4S)-4-acétamido-3-oxo-2-isoxazolidinyl]-5-oxo-2-tétrahydrofurannecarboxylate de méthyle;
(17) le 2-[(4S)-4-acétamido-3-oxo-2-isoxazolidinyl]-5-oxo-2-tétrahydrofurannecarboxylate de pivaloyloxyméthyle;
(18) le 2-[(4S)-4-acétamido-3-oxo-2-isoxazolidinyl]-5-oxo-tétrahydrofurannecarboxylate de 3,5-dinitrobenzyle;
(19) le 2-[(4S)-4-acétamido-3-oxo-2-isoxazolidinyl]-5-oxo-2-tétrahydrofurannecarboxylate de p-bromophénacyle;
(20) le 2-[(4S)-4-acétamido-3-oxo-2-isoxazolidinyl]-5-oxo-2-tétrahydrofurannecarboxylate de p-bromobenzyle;
(21) le 2-[(4S)-4-formylamino-3-oxo-2-isoxazolidinyl]-5-oxo-2-tétrahydrofurannecarboxylate de sodium;
(22) le 2-[(4S)-4-n-butyrylamino-3-oxo-2-isoxazolidinyl]-5-oxo-2-tétrahydrofurannecarboxylate de sodium;
(23) le 2-[(4S)-4-méthoxycarbonylamino-3-oxo-2-isoxazolidinyl]-5-oxo-2-tétrahydrofurannecarboxylate de sodium;
(24) le 2-[(4S)-4-(N-benzyloxycarbonyl)-glycylamino-3-oxo-2-isoxazolidinyl]-5-oxo-2-tétrahydrofurannecarboxylate de sodium;
(25) le 2-[(4S)-4-benzoylamino-3-oxo-2-isoxazolidinyl]-5-oxo-2-tétrahydrofurannecarboxylate de sodium;
(26) le 2-[(4S)-4-phénylacétylamino-3-oxo-2-isoxazolidinyl]-5-oxo-2-tétrahydrofurannecarboxylate de sodium;
(27) le 2-[(4S)-4-nicotinylamino-3-oxo-2-isoxazolidinyl]-5-oxo-2-tétrahydrofurannecarboxylate de sodium;

(28) le 2-[(4S)-4-phénoxyacétylamino-3-oxo-2-isoxazolidinyl]-5-oxo-2-tétrahydrofurannecarboxylate de pivaloyloxyméthyle;
(29) le 2-[(4S)-4-(p-toluènesulfonyl)amino-3-oxo-2-isoxazolidinyl]-5-oxo-2-tétrahydrofurannecarboxylate de sodium;
(30) le 2-[(4S)-4-(p-bromobenzoyl)-amino-3-oxo-2-isoxazolidinyl]-5-oxo-2-tétrahydrofurannecarboxylate de diphénylméthyle;
(31) le 2-[(4S)-4-(N-benzyloxy-carbonyl-D-alanyl)amino-3-oxo-2-isoxazolidinyl]-5-oxo-2-tétrahydrofurannecarboxylate de diphénylméthyle;
(32) l'acide 2-[(4S)-4-diméthylamino-3-oxo-2-isoxazolidinyl]-5-oxo-2-tétrahydrofurannecarboxylique;
(33) le 2-{(4S)-4-[2-(2-amino-4-thiazolyl)-acétamido-3-oxo-2-isoxazolidinyl}-5-oxo-2-tétrahydrofurannecarboxylate de sodium;
(34) le 2-[4-méthoxy-4-(2-thiénylacétamido)-3-oxo-2-isoxazolidinyl]-5-oxo-2-tétrahydrofurannecarboxylate de sodium;
(35) le 2-{(4S)-4-[2-(2-amino-4-thiazolyl)-(Z)-2-(carboxyméthyloxyimino)acétamido]-3-oxo-2-isoxazolidinyl}-5-oxo-2-tétrahydrofurannecarboxylate disodique;
(38) l'acide 2-{(4S)-4-[2-(2-aminométhyl)phénylacétamido]-3-oxo-2-isoxazolidinyl}-5-oxo-2-tétrahydrofurannecar boxylique;
(39) le 2-[(4S)-4-(4-fluorobenzènesulfonyl-amino-3-oxo-2-isoxazolidinyl]-5-oxo-2-tétrahydrofurannecarboxylate de sodium;
(40) le 2-{(4S)-4-{2-[1-(2-diméthylaminoéthyl)-1H-5-tétrazolylthio]acétamido}-3-oxo-2-isoxazolidinyl}-5-oxo-2-tétrahydrofurannecarboxylate de sodium;
(41) le 2-{(4S)-4-[2-(4-méthyl-4H-1,2,4-triazolyl-3-ylthio)acétamido]-3-oxo-2-isoxazolidinyl}-5-oxo-2-tétrahydrofurannecarboxylate de sodium;
(42) le 2-{(4S)-4-[(2R,3S)-2-(4-éthyl-2,3-dioxo-1-pipérazinecarboxamido)-3-formyloxybutylamido]-3-oxo-2-isoxazolidinyl}-5-oxo-2-tétrahydrofurannecarboxylate de sodium;
(43) le 2-[(4S)-4-(2-phényl-2-sulfoacétamido)-3-oxo-2-isoxazolidinyl]-5-oxo-2-tétrahydrofurannecarboxylate disodique;
(44) le 2-{(4S)-4-[2-(2-amino-4-thiazolyl)-(E)-2-méthoxyiminoacétamido]-3-oxo-2-isoxazolidinyl}-5-oxo-2-tétrahydrofurannecarboxylate de sodium;
(45) le 2-[(4S)-4-(2-triméthylsilyl)-éthoxy-carbonylamino3-oxo-2-isoxazolidinyl]-5-oxo-2-tétrahydrofurannecarboxylate de sodium;
(46) l'acide 2-[(4S)-4-(1-aminocyclohexylcarbonylamino)-3-oxo-2-isoxazolidinyl]-5-oxo-2-tétrahydrofurannecarboxylique de sodium;
(47) le 2-{(4S)-4-[3-(2,6-dichlorophényl)-5-méthyl-4-isoxazolylcarbonylamino]-3-oxo-2-isoxazolidinyl}-5-oxo-2-tétrahydrofurannecarboxylate de sodium;
(48) le 2-{(4S)-4-[2-(2-pyridylthio)acétamido]-3-oxo-2-isoxazolidinyl}-5-oxo-2-tétrahydrofurannecarboxylate de sodium;
(49) le 2-{(4S)-4-[2-(4-pyridylméthylthio)-acétamido]-3-oxo-2-isoxazolidinyl}-5-oxo-2-tétrahydrofurannecarboxylate de sodium;
(50) le 2-{(4S)-4-[2-(2-pyrimidinylthio)-acétamido]-3-oxo-2-isoxazolidinyl}-5-oxo-2-tétrahydrofurannecarboxylate de sodium;
(51) le 2-{(4S)-4-{2-[(E)-2-(acétamidovinyl)-thio]acétamido}-3-oxo-2-isoxazolidinyl}-5-oxo-2-tétrahydrofurannecarboxylate de sodium;
(52) le 2-[(4S)-4-cyclopropylcarbonylamino-3-oxo-2-isoxazolidinyl]-5-oxo-2-tétrahydrofurannecarboxylate de sodium;
(53) le 2-[(4S)-4-méthoxalylamino-3-oxo-2-isoxazolidinyl]-5-oxo-2-tétrahydrofurannecarboxylate de sodium;
(54) le 2-[(4S)-4-acrylamido-3-oxo-2-isoxazolidinyl]-5-oxo-2-tétrahydrofurannecarboxylate de sodium;
(55) le 2-{(4S)-4-[2-(2-chloro-4-pyridylthio)acétamido]-3-oxo-2-isoxazolidinyl}-5-oxo-2-tétrahydrofurannecarboxylate de sodium;
(56) le 2-[(4S)-4-(2-acétamido-5-oxo-2-tétrahydrofurannecarbonylamino)-3-oxo-2-isoxazolidinyl]-5-oxo-2-tétrahydrofurannecarboxylate de sodium;
(57) le 2-{(4S)-4-[2-(1-pyrazolyl)acétamido]-3-oxo-2-iso-xazolidinyl}-5-oxo-2-tétrahydrofurannecarboxylate de sodium;
(58) le 2-{(4S)-4-[2-(5-amino-1,2,4-thiadiazole-3-yl)-(Z)-2-éthoxyiminoacétamido]-3-oxo-2-isoxazolidinyl}-5-oxo-tétrahydrofurannecarboxylate de sodium;
(59) le 2-{(4S)-4-[2-(3-pyridazinylthio)-acétamido]-3-oxo-2-isoxazolidinyl}-5-oxo-2-tétrahydrofurannecarboxylate de sodium;

(60) le 2-[(4S)-4-dichloroacétamido-3-oxo-2-isoxazolidinyl]-5-oxo-2-tétrahydrofurannecarboxylate de sodium;

(61) le 2-{(4S)-4-[(2R,3S)-2-(4-éthyl-2,3-dioxo-1-pipérazinecarboxamido)-3-hydroxybutyramido]-3-oxo-2-isoxazolidinyl}-5-oxo-2-tétrahydrofurannecarboxylate de sodium;

(62) le 2-{(4S)-4-[2-(5-trifluorométhyl-1,3,4-thiadiazol-2-ylthio)acétamido]-3-oxo-2-isoxazolidinyl}-5-oxo-2-tétrahydrofurannecarboxylate de sodium;

(63) le 2-{(4S)-4-{2-[1-éthoxy(hydroxy)-phosphinylméthyl-1H-5-tétrazolylthio]acétamido}-3-oxo-2-isoxazolidinyl}-5-oxo-2-tétrahydrofurannecarboxylate de sodium;

(64) le 2-{(4S)-4-{2-[5-(2-diéthoxyphosphinyléthylthio)-1,3,4-thiadiazolylthio]acétamido}-3-oxo-2-isoxazolidinyl}-5-oxo-2-tétrahydrofurannecarboxylate de sodium;

(65) le 2-{(4S)-4-[2-(1-diméthylamino-1H-5-tétrazolylthio)acétamido]-3-oxo-2-isoxazolidinyl}-5-oxo-2-tétrahydrofurannecarboxylate de sodium;

(66) le 2-{(4S)-4-[2-(4,5-diméthyl-2-thiazolylthio)acétamido]-3-oxo-2-isoxazolidinyl}-5-oxo-2-tétrahydrofurannecarboxylate de sodium;

(67) le 2-{(4S)-4-[2-(4,5-diméthyl-2-oxazolylthio)acétamido]-3-oxo-2-isoxazolidinyl}-5-oxo-2-tétrahydrofurannecarboxylate de sodium;

(68) le 2-{(4S)-4-[2-(5-méthoxyméthyl-1,3,4-thiadiazol-2-ylthio)acétamido]-3-oxo-2-isoxazolidinyl}-5-oxo-2-tétrahydrofurannecarboxylate de sodium;

(69) le 2-{(4S)-4-[2-(5-méthylsulfonyl-méthyl-1,3,4-thiadiazol-2-ylthio)acétamido]-3-oxo-2-isoxazolidinyl}-5-oxo-2-tétrahydrofurannecarboxylate de sodium;

(70) le 2-{(4S)-4-[2-(4-éthyl-4H-1,2,4-triazol-3-ylthio)acétamido]-3-oxo-2-isoxazolidinyl}-5-oxo-2-tétrahydrofurannecarboxylate de sodium;

(71) le 2-{(4S)-4-[2-(5-carboxyméthyl-1,3,4-thiadiazol-2-ylthio)acétamido]-3-oxo-2-isoxazolidinyl}-5-oxo-2-tétrahydrofurannecarboxylate disodique;

(72) le 2-{(4S)-4-{2-[1-(2-hydroxyéthyl)-1H-5-tétrazolylthio]acétamido}-3-oxo-2-isoxazolidinyl}-5-oxo-2-tétrahydrofurannecarboxylate de sodium;

(73) le 2-{(4S)-4-{2-[1-(3-diméthylamino-propyl)-1H-5-tétrazolylthio]acétamido}-3-oxo-2-isoxazolidinyl}-5-oxo-2-tétrahydrofurannecarboxylate de sodium;

(74) l'acide 2-[(4S)-4-(2-amino-3-sulfamoylpropionamido)-3-oxo-2-isoxazolidinyl]-5-oxo-2-tétrahydrofurannecarboxylique;

(75) le 2-{(4S)-4-[2-(4-cyano-3-hydroxy-5-isothiazolylthio)acétamido]-3-oxo-2-isoxazolidinyl}-5-oxo-2-tétrahydrofurannecarboxylate de sodium;

(76) le 2-{(4S)-4-[2-(2-amino-4-thiazolyl)-2-oxoacétamido]-3-oxo-2-isoxazolidinyl}-5-oxo-2-tétrahydrofurannecarboxylate de sodium;

(77) le 2-{(4S)-4-[2-(2-amino-4-thiazolyl)-(Z)-2-isopropoxyiminoacétamido]-3-oxo-2-isoxazolidinyl}-5-oxo-2-tétrahydrofurannecarboxylate de sodium;

(78) le 2-[(4S)-4-trifluoracétamido-3-oxo-2-isoxazolidinyl]-5-oxo-2-tétrahydrofurannecarboxylate de sodium;

(79) le 2-{(4S)-4-[2-(2-amino-4-thiazolyl)-(Z)-2-(1-carboxy-1-méthyléthoxyimino)acétamido]-3-oxo-2-isoxazolidinyl}-5-oxo-2-tétrahydrofurannecarboxylate disodique;

(80) le 2-{(4S)-4-[2-(2-amino-4-thiazolyl)-(Z)-2-(hydroxyimino)acétamido]-3-oxo-2-isoxazolidinyl}-5-oxo-2-tétrahydrofurannecarboxylate de sodium;

(81) le 2-[(4S)-4-isobutyloxycarbonylamino-3-oxo-2-isoxazolidinyl]-5-oxo-2-tétrahydrofurannecarboxylate de sodium;

(82) le 2-{(4S)-4-[N-(4-nitrobenzyloxycarbonyl)glycylamino]-3-oxo-2-isoxazolidinyl}-5-oxo-2-tétrahydrofurannecarboxylate de diphénylméthyle;

(83) le 2-{(4S)-4-[N-(2,2,2-trichloroéthoxycarbonyl)phénylglycylamino]-3-oxo-2-isoxazolidinyl}-5-oxo-2-tétrahydrofurannecarboxylate de diphénylméthyle;

(84) le 2-{(4S)-4-[N-(benzyloxycarbonyl)-D-phénylglycylamino]-3-oxo-2-isoxazolidinyl}-5-oxo-2-tétrahydrofurannecarboxylate de sodium;

(85) l'acide 2-[(4S)-4-L-phénylglycylamino-3-oxo-2-isoxazolidinyl]-5-oxo-2-tétrahydrofuranne carboxylique;

(86) l'acide 2-{(4S)-4-D-phénylglycylamino-3-oxo-2-isoxazolidinyl}-5-oxo-2-tétrahydrofuranne carboxylique;

(87) le 2-{(4S)-4-[(2S)-(2-hydroxy-2-phényl)acétamido]-3-oxo-2-isoxazolidinyl}-5-oxo-2-tétrahydrofurannecarboxylate de sodium;

(88) le 2-{(4S)-4-[(2R)-(2-hydroxy-2-phényl)acétamido-3-oxo-2-isoxazolidinyl}-5-oxo-2-tétrahydrofurannecarboxylate de sodium;

(89) le 2-{(4S)-4-[2-(4-chlorophényl)-2-hydroxyacétamido]-3-oxo-2-isoxazolidinyl}-5-oxo-2-tétrahydrofurannecarboxylate de sodium;
(90) le 2-{(4S)-4-[2-hydroxy-2-(4-hydroxyphényl)-acétamido]-3-oxo-2-isoxazolidinyl}-5-oxo-2-tétrahydrofurannecarboxylate de sodium;
(91) le 2-[(4S)-4-(2-hydroxypropionamido)-3-oxo-2-isoxazolidinyl]-5-oxo-2-tétrahydrofurannecarboxylate de sodium;
(92) le 2-[(4S)-4-(2-carboxy-2-phénylacétamido)-3-oxo-2-isoxazolidinyl]-5-oxo-2-tétrahydrofurannecarboxylate disodique;
(93) le 2-[(4S)-4-benzyloxycarbonylamino-3-oxo-2-isoxazolidinyl]-5-oxo-2-tétrahydrofurannecarboxylate de sodium;
(94) le 2-((4S)-4-pyruvamido-3-oxo-2-isoxazolidinyl)-5-oxo-2-tétrahydrofurannecarboxylate de sodium;
(95) le 2-{(4S)-4-[(2R)-(2-hydroxy-2-phényl)acétamido]-3-oxo-2-isoxazolidinyl}-5-oxo-2-tétrahydrofurannecarboxylate de pivaloyloxyméthyle;
(96) le 2-[(4S)-4-(4-nitrobenzylidène)-amino-3-oxo-2-isoxazolidinyl]-5-oxo-2-tétrahydrofurannecarboxylate de diphénylméthyle;
(97) le 2-{(4S)-4-[2-(3-choro-6-pyridazinylthio)acétamido]-3-oxo-2-isoxazolidinyl}-5-oxo-2-tétrahydrofurannecarboxylate de sodium;
(98) le 2-[(4S)-4-(2-phénylthioacétamido)-3-oxo-2-isoxazolidinyl]-5-oxo-2-tétrahydrofurannecarboxylate de sodium;
(99) le 2-[(4S)-4-(2-éthoxydithiocarbonylacétamido)-3-oxo-2-isoxazolidinyl]-5-oxo-2-tétrahydrofurannecarboxylate de sodium;
(100) le 2-{(4S)-4-[2-(2-amino-4-thiazolyl)-(Z)-2-(éthoxyimino)acétamido]-3-oxo-2-isoxazolidinyl}-5-oxo-2-tétrahydrofurannecarboxylate de sodium;
(101) le 2-{(4S)-4-[2-(2-amino-5-chloro-4-thiazolyl)-(Z)-2-(méthoxyimino)acétamido]-3-oxo-2-isoxazolidinyl}-5-oxo-2-tétrahydrofurannecarboxylate de sodium;
(102) le 2-{(4S)-4-[2-4-éthyl-2,3-dioxo-1-pipérazinocarboxamido)-2-thiénylacétamido]-3-oxo-2-isoxazolidinyl}-5-oxo-2-tétrahydrofurannecarboxylate de sodium;
(103) le 2-((4S)-4-uréido-3-oxo-2-isoxazolidinyl)-5-oxo-2-tétrahydofurannecarboxylate de sodium;
(104) le 2-((4S)-4-phényluréylène-3-oxo-2-isoxazolidinyl)-5-oxo-2-tétrahydrofurannecarboxylate de sodium;
(105) le 2-{(4S)-4-[2-(pyridyl)acétamido]-3-oxo-2-isoxazolidinyl}-5-oxo-2-tétrahydrofurannecarboxylate de sodium;
(106) le 2-{(4S)-4-[2-(4-chlorophényl)acétamido]-3-oxo-2-isoxazolidinyl}-5-oxo-2-tétrahydrofurannecarboxylate de sodium;
(107) le 2-{4S)-4-[(2-chloro-2-phényl)acétamido]-3-oxo-2-isoxazolidinyl}-5-oxo-2-tétrahydrofurannecarboxylate de sodium;
(108) le 2-{(4S)-4-[2-(3-pyridylthio)acétamido]-3-oxo-2-iso-xazolidinyl}-5-oxo-2-tétrahydrofurannecarboxylate de sodium;
(109) le 2-{(4S)-4-[2-(4-chlorophénylthio)acétamido]-3-oxo-2-isoxazolidinyl}-5-oxo-2-tétrahydrofurannecarboxylate de sodium;
(110) le 2-[(4S)-4-(2-isopropylphénoxy-carbonylamino)-3-oxo-2-isoxazolidinyl]-5-oxo-2-tétrahydrofurannecarboxylate de sodium;
(111) le 2-((4S)-4-difluoracétamido-3-oxo-2-isoxazolidinyl)-5-oxo-2-tétrahydrofurannecarboxylate de sodium;
(112) le 2-[(4S)-4-(3-chloro)propionamido-3-oxo-2-isoxazolidinyl]-5-oxo-2-tétrahydrofurannecarboxylate de sodium;
(113) le 2-((4S)-4-chloracétamido-3-oxo-2-isoxazolidinyl)-5-oxo-2-tétrahydrofurannecarboxylate de pivaloyloxyméthyle;
(114) le 2-[(4S)-4-(2-phénylthioacétamido)-3-oxo-2-isoxazolidinyl]-5-oxo-2-tétrahydrofurannecarboxylate de pivaloyloxyméthyle;
(115) le 2-((4S)-4-propionamido-3-oxo-2-isoxazolidinyl)-5-oxo-2-tétrahydrofurannecarboxylate de sodium;
(116) le 2-((4S)-4-benzyloxy-carbonylamino-3-oxo-2-isoxazolidinyl)-5-oxo-2-tétrahydrofurannecarboxylate de pivaloyloxyméhtyle;
(117) le 2-((4S)-4-bromacétamido-3-oxo-2-isoxazolidinyl)-5-oxo-2-tétrahydrofurannecarboxylate de pivaloyloxyméthyle;
(118) le 2-[(4S)-4-(2,4-hexadiène)-amido-3-oxo-2-isoxazolidinyl]-5-oxo-2-tétrahydrofurannecarboxylate de pivaloyloxyméthyle;

(119) le 2-[(4S)-4-(2-thiényl)-acétamido-3-oxo-2-isoxazolidinyl]-5-oxo-2-tétrahydrofurannecarboxylate de pivaloyloxyméthyle;
(120) le 2-[(4S)-4-(hexahydro-1H-azépine-1-yl)méthylèneamino-3-oxo-2-isoxazolidinyl]-5-oxo-2-tétrahydrofurannecarboxylate de pivaloyloxyméthyle;
(121) le 2-[(4S)-4-(tert-butyl-diméthylsilyl)amino-3-oxo-2-isoxazolidinyl]-5-oxo-2-tétrahydrofurannecarboxylate de diphénylméthyle;
(122) le 2-[(4S)-4-tert-butyl-diphénylsilyl)amino-3-oxo-2-isoxazolidinyl]-5-oxo-2-tétrahydrofurannecarboxylate de diphénylméthyle;
(123) le 2-[(4S)-3-diphénylphosphorylamino-3-oxo-2-isoxazolidinyl]-5-oxo-2-tétrahydrofurannecarboxylate de sodium;
(124) le 2-[(4S)-4-diméthyl-phosphorylamino-3-oxo-2-isoxazolidinyl]-5-oxo-2-tétrahydrofurannecarboxylate de diphénylméthyle;
(125) le 2-[(4S)-4-éthylsulfonylamino-3-oxo-2-isoxazolidinyl]-5-oxo-2-tétrahydrofurannecarboxylate de sodium;
(126) le 2-[(4S)-4-vinylsulfonylamino-3-oxo-2-isoxazolidinyl]-5-oxo-2-tétrahydrofurannecarboxylate de sodium;
(127) le 2-[(4S)-4-thiénylacétamido-3-oxo-2-isoxazolidinyl]-5-oxo-2-tétrahydrofurannecarbonylanilide;
(128) le 2-[(4S)-4-benzyloxycarbonylamino-3-oxo-2-isoxazolidinyl]-5-oxo-2-tétrahydrofurannecarbonylanilide;
(129) le 2-[(4S)-4-benzyloxycarbonylamino-3-oxo-2-isoxazolidinyl]-5-oxo-2-tétrahydrofurannecarbonylpyrrolidinamide;
(130) le 2-[(4S)-4-thiénylacétamido-3-oxo-2-isoxazolidinyl]-5-oxo-2-tétrahydrofurannecarbonylpyrrolidinamide;
(131) la 2-[(4S)-4-thiénylacétamido-3-oxo-2-isoxazolidinyl]-5-oxo-2-tétrahydrofurannecarbonylpropylamine;
(132) le 2-[(4S)-4-$\gamma$-D-glutamylamino-3-oxo-2-isoxazolidinyl]-5-oxo-2-tétrahydrofurannecarboxylate de sodium et
(133) le 2-[(4S)-4-(2-hydroxy)isobutyryl-amino-3-oxo-2-isoxazolidinyl]-5-oxo-2-tétrahydrofurannecarboxylate de sodium;

ensemble avec une matière de support ou un diluant pharmaceutiquement acceptable.

**2.** Utilisation d'un composé (I') ou d'un sel pharmaceutiquement acceptable de celui-ci, tel que défini à la revendication 1, dans la préparation d'une composition pharmaceutique pour l'inhibition de la croissance de microorganismes.